# EUROPEAN PATENT SPECIFICATION

(11) **EP 0 888 278 B1**
(45) Date of publication and mention of the grant of the patent: **23.07.2003**
(21) Application number: 97906471.4
(22) Date of filing: 31.01.1997
(51) Int. Cl.: C07C 63/06, C07C 65/30, C07C 65/32, C07C 65/38, C07C 65/40, C07C 65/42, C07C 69/12, C07C 69/14, C07C 69/612, C07C 233/65, C07C 323/62

(54) **ANTIDIABETIC AGENTS**
ANTIDIABETISCHE MITTEL
AGENTS ANTI-DIABETE

(30) Priority: 02.02.1996 US 11093 P; 28.02.1996 GB 9604231; 23.12.1996 US 34435 P
(43) Date of publication of application: 07.01.1999
(73) Proprietor: MERCK & CO., INC., Rahway, New Jersey 07065 (US)
(72) Inventor: ADAMS, Alan, D., Rahway, NJ 07065 (US); DOEBBER, Thomas, W., Rahway, NJ 07065 (US); BERGER, Joel, P., Rahway, NJ 07065 (US); BERGER, Gregory, D., Rahway, NJ 07065 (US); JONES, Anthony, B., Rahway, NJ 07065 (US); VON LANGEN, Derek, Rahway, NJ 07065 (US); LEIBOWITZ, Mark, D., Rahway, NJ 07065 (US); MOLLER, David, E., Rahway, NJ 07065 (US); OLSON, John, T., Rahway, NJ 07065 (US); PATCHETT, Arthur, A., Rahway, NJ 07065 (US); SAHOO, Soumya, P., Rahway, NJ 07065 (US); TOLMAN, Richard, L., Rahway, NJ 07065 (US); TOUPENCE, Richard, B., Rahway, NJ 07065 (US); WALSH, Thomas, F., Rahway, NJ 07065 (US)
(74) Representative: Horgan, James Michael Frederic
(86) International application number: US9701689
(87) International publication number: WO97028115

(56) References cited:
- EP-A- 0 123 541
- EP-A- 0 180 416
- EP-A- 0 611 003
- CA-A- 1 328 662
- US-A- 4 672 066

## Description

### BACKGROUND OF THE INVENTION

Diabetes refers to a disease process derived from multiple causative factors and characterized by elevated levels of plasma glucose or hyperglycemia. Uncontrolled hyperglycemia is associated with increased and premature mortality due to an increased risk for microvascular and macrovascular diseases, including nephropathy, neuropathy, retinopathy, hypertension, stroke, and heart disease. Therefore, control of glucose homeostasis is a critically important approach for the treatment of diabetes.

Type I diabetes (IDDM) is the result of an absolute deficiency of insulin, the hormone which regulates glucose utilization. Type II, noninsulin dependent diabetes mellitus (NIDDM) is due to a profound resistance to insulin stimulating or regulatory effect on glucose and lipid metabolism in the main insulin-sensitive tissues, muscle, liver and adipose tissue. This resistance to insulin responsiveness results in insufficient insulin activation of glucose uptake, oxidation and storage in muscle and inadequate insulin repression of lipolysis in adipose tissue and of glucose production and secretion in liver.

The several treatments for NIDDM, which has not changed substantially in many years, are all with limitations. While physical exercise and reductions in dietary intake of calories will dramatically improve the diabetic condition, compliance with this treatment is very poor because of well-entrenched sedentary lifestyles and excess food consumption, especially high fat-containing food. Increasing the plasma level of insulin by administration of sulfonylureas (e.g. tolbutamide, glipizide) which stimulate the pancreatic β-cells to secrete more insulin or by injection of insulin after the response to sulfonylureas fails, will result in high enough insulin concentrations to stimulate the very insulin-resistant tissues. However, dangerously low levels of plasma glucose can result from these last two treatments and increasing insulin resistance due to the even higher plasma insulin levels could theoretically occur. The biguanides increase insulin sensitivity resulting in some correction of hyperglycemia. However, the two biguanides, phenformin and metformin, can induce lactic acidosis and nausea/diarrhea, respectively.

Thiazolidinediones (glitazones) are a recently disclosed class of compounds that are suggested to ameliorate many symptoms of NIDDM. These agents increase insulin sensitivity in muscle, liver and adipose tissue in several animal models of NIDDM resulting in complete correction of the elevated plasma levels of glucose, triglycerides and nonesterified free fatty acids without any occurrence of hypoglycemia. However, serious undesirable effects have occurred in animal and/or human studies including cardiac hypertrophy, hemadilution and liver toxicity resulting in few glitazones progressing to advanced human trials.

Hyperlipidemia is a condition which is characterized by an abnormal increase in serum lipids, such as cholesterol, triglycerides and phospholipids. These lipids do not circulate freely in solution in plasma, but are bound to proteins and transported as macromolecular complexes called lipoproteins. See the *Merck Manual*, 16th Ed. 1992 (see for example pp. 1039-1040) and "Structure and Metabolism of Plasma Lipoproteins" in *Metabolic Basis of Inherited Disease*, 6th Ed. 1989, pp. 1129-1138. One form of hyperlipidemia is hypercholesterolemia, characterized by the existence of elevated LDL cholesterol levels. The initial treatment for hypercholesterolemia is often to modify the diet to one low in fat and cholesterol, coupled with appropriate physical exercise, followed by drug therapy when LDL-lowering goals are not met by diet and exercise alone. LDL is commonly known as the "bad" cholesterol, while HDL is the "good" cholesterol. Although it is desirable to lower elevated levels of LDL cholesterol, it is also desirable to increase levels of HDL cholesterol. Generally, it has been found that increased levels of HDL are associated with lower risk for coronary heart disease (CHD). See, for example, Gordon, et al., Am. J. Med., *62*, 707-714 (1977); Stampfer, et al., N. England J. Med., *325*, 373-381 (1991); and Kannel, et al., Ann. Internal Med., *90*, 85-91 (1979). An example of an HDL raising agent is nicotinic acid, but the quantities needed to achieve HDL raising are associated with undesirable effects, such as flushing.

It is suggested that thiazolidinedione compounds exert their effects by binding to the peroxisome proliferator activated receptor (PPAR) family of receptors, controlling certain transcription elements having to do with the biological entities listed above. See Hulin et al., Current Pharm. Design (1996) 2, 85-102. Three sub-types of PPARs have been discovered and described; they are PPARα, PPARγ and PPARδ. PPARα is activated by a number of medium and long-chain fatty acids, and it is involved in stimulating β-oxidation of fatty acids. PPARα is also involved with the activity of fibrates in rodents and humans. Fibric acid derivatives such as clofibrate, fenofibrate, bezafibrate, ciprofibrate, beclofibrate and etofibrate, as well as gemfibrozil, produce a substantial reduction in plasma triglycerides along with moderate reduction in LDL cholesterol, and they are used particularly for the treatment of hypertriglyceridemia.

The PPARγ receptor subtypes are involved in activating the program of adipocyte differentiation and are not involved in stimulating peroxisome proliferation in the liver. The DNA sequences for the PPARγ receptors are described in Elbrecht, et al., BBRC *224*;431-437 (1996). Although peroxisome proliferators, including the fibrates and fatty acids, activate the transcriptional activity of PPAR's, only prostaglandin J₂ derivatives have been identified as natural ligands of the PPARγ subtype, which also binds thiazolidinedione antidiabetic agents with high affinity. The glitazones have been shown to bind exclusively to the PPARγ subtype.

The human nuclear receptor gene PPARδ (hPPARδ) has been cloned from a human osteosarcoma cell cDNA library and is fully described in A. Schmidt et al., *Molecular Endocrinology, 6* :1634-1641 (1992), herein incorporated by reference. It should be noted that PPARδ is also referred to in the literature as PPARβ and as NUC1, and each of these names refers to the same receptor; in Schmidt et al, the receptor is referred to as NUC1.

### SUMMARY OF THE INVENTION

This invention is concerned with the compounds of formula I below and its analogs, pharmaceutically acceptable salts thereof, and bioprecursors thereof, which differ from the thiazolidinediones in that they lack the thiazolidinedione moiety and they do not lead to the array of toxicity's associated with the thiazolidinediones. The instant compounds are effective in treating diabetes, atherosclerosis, hyperglycemia, hyperlipidemia and/or obesity because they lower one or more of the following biological entities in mammals; glucose, insulin, triglycerides, fatty acids, cholesterol and the like. Thus, it is an object of this invention to describe such compounds. It is a further object to describe the specific preferred stereoisomers of the substituted compounds. A still further object is to describe processes for the preparation of such compounds. Another object is to describe methods and compositions which use the compounds as the active ingredient thereof. Further objects will become apparent from reading the following description.

### DESCRIPTION OF THE INVENTION

The present invention is directed to a compound represented by formula I: 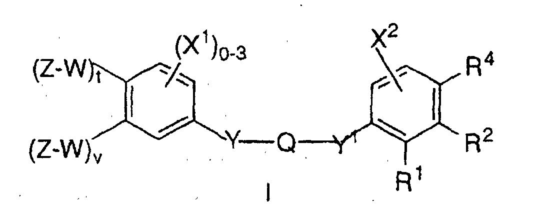 or a pharmaceutically acceptable salt thereof, wherein:
R is selected from the group consisting of H, C₁₋₆ alkyl, C₅₋₁₀ aryl, and C₅₋₁₀ heteroaryl, said alkyl, aryl and heteroaryl optionally substituted with 1 to 3 groups of R^{a};
R¹ is selected from a group consisting of: H, C₁₋₁₅ alkyl, C₂₋₁₅ alkenyl, C₂₋₁₅ alkynyl and C₃₋₁₀ cycloalkyl, said alkyl, alkenyl, alkynyl, and cycloalkyl optionally substituted with 1 to 3 groups of R^{a};
R² is selected from a group consisting of: H, C₁₋₁₅ alkyl, acyl, C₂₋₁₅ alkenyl, OR³, CO₂alkyl, C(O)R³, OH, -OC(O)R³, C₂₋₁₅ alkynyl, C₅₋₁₀ aryl, C₅₋₁₀ heteroaryl, said alkyl, alkenyl, alkynyl, aryl and heteroaryl optionally substituted with 1 to 3 groups of R^{a};
R³ is selected from a group consisting of: H, NHR¹, NHacyl, C₁₋₁₅ alkyl, C₂₋₁₅ alkenyl, C₁₋₁₅ alkoxy, CO₂alkyl, OH, C₂₋₁₅ alkynyl, C₅₋₁₀aryl, C₅₋₁₀ heteroaryl said alkyl, alkenyl, alkynyl, aryl and heteroaryl optionally substituted with 1 to 3 groups of R^{a;}
R⁴ is selected from the group consisting of: R², -B-R⁵ or 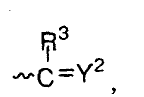 with the proviso that when (Z-W-) is Z-CR⁶R⁷, Y is O and R⁴ is R², then R² is not acetyl, H, alkyl, alkoxy or aryl; or when (Z-W-) is Z-CR⁶R⁷, Y is O and R⁴ is 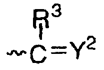 then Y² is not O, or when
(Z-W-) is Z-CR⁶R⁷-, or 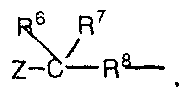 where R⁸ is O, Y is S or O and R⁴ is -B-R⁵, then B is not O or S:
R⁵ is selected from the group consisting of: C₅₋₁₀ aryl and C₅₋₁₀ heteroaryl, said aryl and heteroaryl optionally substituted with 1 to 3 groups of R^{a};
B is selected from the group consisting of: O, S(O)p and NR¹;
(Z-W-) is Z-CR⁶R⁷-, Z-CH=CH-, or 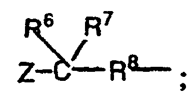
R⁸ is selected from the group consisting of CR⁶R⁷, O, NR⁶, and S(O)ₚ;
R⁶ and R⁷ are independently selected from the group consisting of H, C₁₋₆ alkyl;
X¹ and X² are independently selected from a group consisting of: H, OH, C₁₋₁₅ alkyl, C₂₋₁₅ alkenyl, C₂₋₁₅ alkynyl, halo, OR³, C₅₋₁₀ aryl, C₅₋₁₀ aralkyl, C₅₋₁₀ heteroaryl and C₁₋₁₀ acyl, said alkyl, alkenyl, alkynyl, aryl and heteroaryl optionally substituted with 1 to 3 groups of R^{a};
R^{a} represents a member selected from the group consisting of: halo, aryl, acyl, heteroaryl, CF₃, OCF₃, -O-, CN, NO₂, R³, OR³; SR³, S(O)R³, SO₂R³, NR³R³, NR³COR³, NR³CO₂R³, NR³CON(R³)₂, NR³SO₂R³, COR³, CO₂R³, CON(R³)₂, SO₂N(R³)₂, OCON(R³)₂ said aryl and heteroaryl optionally substituted with 1 to 3 groups of halo or C1-6 alkyl;
Y is selected from the group consisting of: S(O)ₚ, -CH₂-, -C(O)-, -C(O)NH-, -NR-, -O-, -SO₂NH, -NHSO₂;
Y¹ is selected from the group consisting of: O, NR and CH₂;
Y² is selected from the group consisting of: O, N(C₁₋₁₅) alkyl, N(CO₂)alkyl, N-Oalkyl, N-Oacyl and N-OH, with the proviso that if Y² is O and R³ is CH₃ then n is 2;
Z is selected from the group consisting of: CO₂R³ CONHSO₂R, CONH₂ and 5-(1H-tetrazole);
t and v are independently 0 or 1 such that t + v = 1
Q is a saturated or unsaturated straight chain hydrocarbon containing 2-4carbon atoms and
p is 0-2.

Included in the invention is a pharmaceutical composition which is comprised of a compound of formula I in combination with a pharmaceutically acceptable carrier.

Also included in the invention is a pharmaceutical composition which is comprised of a compound of formula I in combination with one or more known sulfonylureas, biguanides, α-glucosidase inhibitors, other insulin secretogogues as well as insulin.

Also included in the invention is a method for raising high densisty lipoprotein (HDL) plasma levels in a mammal in need of such treatment comprising administering an effective amount of a compound of formula 1.

Also included in the invention is a method for preventing, halting or slowing the progression of atherosclerotic cardiovascular diseases and related conditions and disease events in a mammal in need of such treatment comprising administering an effective amount of a compound of formula I.

Also included in the invention is a method for preventing, halting or slowing the progression of atherosclerotic cardiovascular diseases and related conditions and disease events in a mammal in need of such treatment comprising administering an effective amount of a compound of formula I in combination with one or more active agents such as antihyperlipidemic agents, HMG-CoA synthase inhibitors, squalene epoxidase inhibitors and the like..

Also included in the invention is a method of treating or controlling diabetes, which comprises administering to a diabetic patient an effective amount of a compound of formula I.

Also included in the invention is a method of treating or controlling diabetes, which comprises administering a compound of formula I in combination with one or more known sulfonylureas, biguanides, α-glucosidase inhibitors, other insulin secretogogues as well as insulin.

The invention is described herein in detail using the terms defined below unless otherwise specified.

The term "alkyl" refers to a monovalent alkane (hydrocarbon) derived radical containing from 1 to 15 carbon atoms unless otherwise defined. It may be straight, branched or cyclic. Preferred straight or branched alkyl groups include methyl, ethyl, propyl, isopropyl, butyl and t-butyl. Preferred cycloalkyl groups include cyclopentyl and cyclohexyl.

Alkyl also includes a straight or branched alkyl group which contains or is interrupted by a cycloalkylene portion.

Examples include the following: 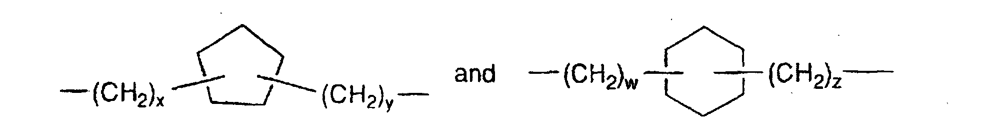 wherein: x and y = from 0-10; and w and z = from 0-9.

The alkylene and monovalent alkyl portion(s) of the alkyl group can be attached at any available point of attachment to the cycloalkylene portion.

When substituted alkyl is present, this refers to a straight, branched or cyclic alkyl group as defined above, substituted with 1-3 groups as defined with respect to each variable.

The term "alkenyl" refers to a hydrocarbon radical straight, branched or cyclic containing from 2 to 15 carbon atoms and at least one carbon to carbon double bond. Preferably one carbon to carbon double bond is present, and up to four nonaromatic (non-resonating) carbon-carbon double bonds may be present. Preferred alkenyl groups include ethenyl, propenyl, butenyl and cyclohexenyl. As described above with respect to alkyl, the straight, branched or cyclic portion of the alkenyl group may contain double bonds and may be substituted when a substituted alkenyl group is provided.

The term "alkynyl" refers to a hydrocarbon radical straight, branched or cyclic, containing from 2 to 15 carbon atoms and at least one carbon to carbon triple bond. Up to three carbon-carbon triple bonds may be present. Preferred alkynyl groups include ethynyl, propynyl and butynyl. As described above with respect to alkyl, the straight, branched or cyclic portion of the alkynyl group may contain triple bonds and may be substituted when a substituted alkynyl group is provided.

The term "alkoxy" refers to those groups of the designated carbon length in either a straight or branched configuration attached through an oxygen linkage and if two or more carbon atoms in length, they may include a double or a triple bond. Exemplary of such alkoxy groups are methoxy, ethoxy, propoxy, isopropoxy, butoxy, isobutoxy, tertiary butoxy, pentoxy, isopentoxy, hexoxy, isohexoxy allyloxy and propargyloxy.

The term halo as used herein, represents fluoro, chloro, bromo or iodo.

Aryl refers to aromatic rings e.g., phenyl, substituted phenyl and like groups as well as rings which are fused, e.g., naphthyl Aryl thus contains at least one ring having at least 5 atoms, with up to two such rings being present, containing up to 10 atoms therein, with alternating (resonating) double bonds between adjacent carbon atoms. The preferred aryl groups are phenyl and naphthyl. Aryl groups may likewise be substituted with 0-3 groups selected from R^{a}. The preferred aryl groups are phenyl and naphthyl. Aryl groups may likewise be substituted as defined below. Preferred substituted aryls include phenyl and naphthyl substituted with zero or three groups of R^{a}.

Heteroaryl is a group containing from 5 to 10 atoms, 1-4 of which are heteroatoms, 0-4 of which heteroatoms are N and 0-1 of which are O or S, said heteroaryl group being unsubstituted or substituted with 0-3 R^{a} groups; examples of heteroaryls are pyridyl, quinolyl, purinyl, imidazolyl, imidazopyridyl and pyrimidinyl

One embodiment of the novel compounds of the instant invention is realized when:
- Y is: O and all other variables are described as above.

Another embodiment of the novel compounds of the instant invention is realized when:
- Y is: S(O)ₚ, p is 0-2 and all other variables are described as above.

Still another embodiment of the novel compounds of the instant invention is realized when:
- Y is: -CH₂- and all other variables are described as above.

Yet another embodiment of the novel compounds of the instant invention is realized when:
- Y is: CO and all other variables are described as above.

A further embodiment of the novel compounds of the instant invention is realized when:
- Y is: NR and all other variables are described as above.

Another embodiment of the novel compounds of the instant invention is realized when:
- Y is: NHSO₂ or SO₂NH and all other variables are described as above.

Another embodiment of the novel compounds of the instant invention is realized when:
- Y is: -C(O)NH- and all other variables are described as above.

And another embodiment of the novel compounds of the instant invention is realized when:
R⁴ is B-R⁵, with the proviso that when (Z-W-) is Z-CR⁶R⁷-, or 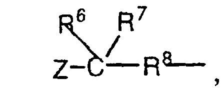 where R⁸ is O, and Y is S or O, then B is not O or S; and all other variables are described as above.

Still another embodiment of the novel compounds of the instant invention is realized when:
R⁴ is 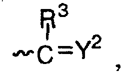 with the proviso that when (Z-W-) is Z-CR⁶R⁷, and Y is O then Y² is not O and all other variables are described as above.

Still another embodiment of the novel compounds of the instant invention is realized when:
R⁴ is R², with the proviso that when (Z-W-) is Z-CR⁶R⁷, and Y is O then R² is not acetyl, H, alkyl, alkoxy or aryl and all other variables are described as above.

Another embodiment of the novel compounds of the instant invention is realized when:
(Z-W-) is Z-CR⁶R⁷-, Z-CH=CH-, or 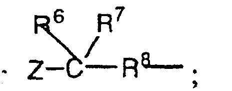 and all other variables are described as above and all other variables are
   described as above.

Still another embodiment of the novel compounds of the instant invention is realized when:
(Z-W-) is Z-CR⁶R⁷- or 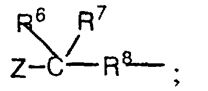 and all other variables are described as above and all other variables are
   described as above.

Another embodiment of the novel compounds of the instant invention is realized when:Ra is selected from the group consisting of C1-6 alkyl, CF₃, aryl, halo, acyl, OCF₃, -NO₂, OR³; COR³, CO₂R³, CON(R³)₂, and SO₂N(R³)₂; and Xl is selected from the group consisting of H, OH, C₁₋₆ alkyl, C₂₋₁₅ alkenyl, halo and OR³ and all other variables are described as above.

A preferred embodiment of the novel compounds of the instant invention is realized when:
R is C₁₋₆ alkyl or C₅₋₁₀ aryl, said alkylor aryl optionally substituted with 1 to 3 groups of R^{a};
R¹ is is C₁₋₁₅ alkyl;
X¹ & X² are independently H, C₁₋₆ alkyl, or halo;
Y is O, NH or S;
Y¹ is O;
(Z-W-) is Z-CR⁶R⁷- or 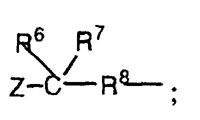
R⁴ is -B-R⁵ or 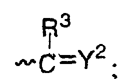 with the proviso that when (Z-W-) is Z-CR⁶R⁷,
Y is O and R4 is 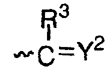 then Y² is not O, or when (Z-W-) is Z-CR⁶R⁷-, or 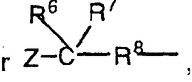 where R⁸ is O, Y is S or O and R⁴ is -B-R⁵, then B is not O or S;
R^{a} is a member selected from the group consisting of: halo, aryl, acyl heteroaryl, CF₃, OCF₃, -O-, CN, NO₂, R³, OR³; SR³, S(O)R³, SO₂R³, NR³COR³, COR³, CON(R³)₂, SO₂N(R³)₂, said aryl and heteroaryl optionally substituted with 1 to 3 groups of halo or C1-6 alkyl; and
Z is CO₂R³, CONHSO₂R, CONH₂ or 5-(1H-tetrazole).

Examples of the instant compounds are as follows:
Methyl 3-chloro-4-(3-(2-propyl-3-hydroxy-4-propionylphenoxy)-propylthio)-phenylacetate;
3-Chloro-4-(3-(2-propyl-3-hydroxy-4-propionylphenoxy)propylthio)phenyl-acetic acid;
Methyl 3-chloro-4-(3-(2-propyl-3-hydroxy-4-(1-oxobutyl)phenoxy)-propylthio)phenyl acetate;
3-Chloro-4-(3-(2-propyl-3-hydroxy-4-(1-oxobutyl)phenoxy)-propylthio)-phenylacetic acid;
Methyl 3-chloro-4-(3-(2-propyl-3-hydroxy-4-(1-oxo-2-methylpropyl)-phenoxy)propylthio)phenylacetate;
Methyl 3-chloro-4-(3-(2-propyl-3-hydroxy-4-(1-oxopropyl)phenoxy)-propyloxy)phenylacetate;
3-Chloro-4-(3-(2-propyl-3-methoxy-4-propionylphenoxy)propylthio)-phenylacetic acid;
methyl-3-chloro-4-(3-(2-propyl-3-methoxy-4-propionylphenoxy) propylthio)phenylacetate:
Methyl 3-chloro-4-(3-(2-propyl-3-acetoxy-4-propionylphenoxy)propylthio)phenylacetate;
Methyl 3- chloro-4-(3-(2-propyl-3-hydroxy-4-(1-hydroxyliminopropyl)-phenoxy)propylthio)phenylacetate;
3-Chloro-4-(3-(2-propyl-3-hydroxy-4-(1-hydroxyliminopropyl)phenoxy)-propylthio)phenylacetic acid;
Methyl 3- chloro-4-(3-(2-propyl-3-methoxy-4-(1-hydroxyliminopropyl)-phenoxy)propylthio)phenyl acetate;
3-Chloro-4-(3-(2-propyl-3-methoxy-4-(1-hydroxyliminopropyl)phenoxy)-propylthio)phenyl acetic acid;
Methyl 3-chloro-4-(3-(2-propyl-3-hydroxy-4-(1-hydroxyliminopropyl)-phenoxy)propyloxy)phenyl acetate;
Methyl 3-chloro-4-(3-(2-propyl-3-hydroxy-4-(1-methoxyliminopropyl)-phenoxy)propylthio)phenylacetate;
Methyl 3-chloro-4-(3-(2-propyl-3-hydroxy-4-( 1-hydroxyliminobutyl)-phenoxy)propylthio)pheny lacetate;
3-Chloro-4-(3-(2-propyl-3-hydroxy-4-(1-hydroxyliminobutyl)phenoxy)-propylthio)phenylacetic acid;
Methyl 3-chloro-4-(3-(2-propyl-3-hydroxy-4-(1-acetoxyiminobutyl)-phenoxy)propylthio)phenylacetate;
Methyl 3-chloro-4-(3-(2-propyl-3-hydroxy-4-(1-hydroxylimino-2-methyl-propyl)phenoxy)propylthio)phenylacetate;
3-Chloro-4-(3-(2-propyl-3-hydroxy-4-(1-hydroxylimino-2-methylpropyl)-phenoxy)propylthio)phenylacetic acid;
Methyl 3-chloro-4-(3-(2-propyl-3-acetoxy-4-(1-hydroxyiminopropyl) phenoxy)propylthio)phenyl acetate;
3-chloro-4-(3-(2-propyl-3-hydroxy-4-(1-oxopropyl)phenoxy)propylthio)-phenylacetamide
Methyl 3-chloro-4-(3-(2-cyclopropylmethyl-3-hydroxy-4-propionylphenoxy)propylthio)phenylacetate;
3-Chloro-4-(3-(2-cyclopropylmethyl-3-hydroxy-4-propionylphenoxy)-propylthio)phenylacetic acid;
Methyl 3-chloro-4-(3-(2-cyclopropylmethyl-3-hydroxy-4-(1-hydroxyimino-propyl)phenoxy)propylthio)phenylacetate;
3-Chloro-4-(3-(2-cyclopropylmethyl-3-hydroxy-4-(1-hydroxyiminopropyl)-phenoxy)propylthio)phenylacetic acid;
Methyl 3-chloro-4-(4-(2-propyl-3-hydroxy-4-propionylphenyl)butylthio)-phenylacetate;
3-Chloro-4-(4-(2-propyl-3-hydroxy-4-propionylphenyl)-butylthio)phenyl-acetic acid;
Methyl 3-chloro-4-(4-(2-propyl-3-hydroxy-4-(1-hydroxyiminopropyl)-phenyl)butylthio)phenylacetate;
3-chloro-4-(4-(2-propyl-3-hydroxy-4-(1-hydroxyiminopropyl)-phenyl)butylthio)phenylacetic acid;
Methyl 3-chloro-4-(3-(2-(2-propenyl)-3-hydroxy-4-propionylphenoxy)-propylthio)phenylacetate;
Methyl 3-chloro-4-(3-(2-(2-propenyl)-3-hydroxy-4-(1-hydroxyiminopropyl)-phenoxy)propylthio)phenylacetate;
3-Chloro-4-(3-(2-(2-propenyl)-3-hydroxy-4-(1-hydroxyiminopropyl)-phenoxy)propylthio)phenylacetic acid;
Methyl 3-chloro-4-(3-(2-propyl-4-propionylphenoxy)propylthio)phenyl-acetate;
3-Chloro-4-(3-(2-propyl-4-propionylphenoxy)propylthio)phenylacetic acid;
Methyl 3-chloro-4-(3-(2-propyl-4-(1-hydroxyiminopropyl)phenoxy)propyl-thio)phenylacetate;
3-Chloro-4-(3-(2-propyl-4-(1-hydroxyiminopropyl)phenoxy)propylthio)-phenylacetic acid;
Methyl 3-chloro-4-(4-(2-propyl-3-hydroxy-4-acetylphenoxy)butylthio)-phenylacetate;
3-Chloro-4-(4-(2-propyl-3-hydroxy-4-acetylphenoxy)butylthio)phenylacetic acid;
3-Chloro-4-(3-(2-propyl-3-hydroxy-4-acetyl)phenoxy)propylthiophenyl- 5-methyl-(1H)-tetrazole;
3-Chloro-4-(4-(2-propyl-3-hydroxy-4-acetylphenoxy)butylthio)phenyl-5-methyl-(1H)-tetrazole;
3-Chloro-4-(3-(2-propyl-3-hydroxy-4-propionylphenoxy)propylthio)-phenyl-5-methyl-( 1H)-tetrazole;
3-Chloro-4-(3-(2-propyl-3-hydroxy-4-( 1-hydroxyiminopropyl)-phenoxy)-propylthio)-phenyl-5-methyl-(1H)-tetrazole;
3-Chloro-4-(4-(2-propyl-3-hydroxy-4-propionylphenoxy)butylthio)-phenyl-5-methyl-(1H)-tetrazole;
3-Chloro-4-(4-(2-propyl-3-hydroxy-4-(1-hydroxyiminopropyl)-phenoxy)-butylthio)phenyl-5-methyl-(1H)-tetrazole;
4-(3-(2-Propyl-3-hydroxy-4-acetylphenoxy)propyloxy)-phenyl-5-methyl-( 1 H)-tetrazole;
4-(4-(2-Propyl-3-hydroxy-4-propionylphenoxy)butyloxy)-phenyl-5-methyl-(1H)-tetrazole;
4-(3-(2-Propyl-3-hydroxy-4-acetylphenoxy)propylthio)phenyl-5-methyl-(1H)-tetrazole;
3-chloro-4-(3-(2-propyl-3-hydroxy-4-(1-oxopentyl))phenoxy)propylthio phenyl acetic acid methyl ester;
3-chloro-4-(3-(2-propyl-3-hydroxy-4-(1-hydroxyliminopentyl))phenoxy) propylthio phenyl acetic acid methyl ester;
3-chloro-4-(3-(2-propyl-3-hydroxy-4-(1-hydroxylimino propyl)phenoxy)-propylthio)phenylacetamide;
3-chloro-4-(3-(2-propyl-3-hydroxy-4-(1-(hydroxylimino-3-(phenyl) propyl)phenoxy)-propylthio)phenylacetic acid;
Methyl 3-chloro-4-(4-phenoxy-2-propylphenoxy)-propylamino)phenylacetate;
3-Chloro-4-(4-phenoxy-2-propylphenoxy)-propylamino)phenylacetic acid;
3-chloro-4-(3-(2-propyl-3-hydroxy-4-(1-hydroxyliminopropyl)) phenoxy)propylthio phenyl acetic acid;
4-(2-(4-benzyl-2-propylphenoxy)propylthio)-3-chlorophenylacetic acid;
Methyl 3-chloro-4-(3-(2-propyl-3-hydroxy-4-(1-oxopentyl)phenoxy)-propyl)-phenylacetate;
4-(3-(4-(N-hydroxyiminobenzoyl)-2-propylphenoxy)propylthio)-3-chlorophenylacetic acid;
4-(3-(4-benzoyl-3-hydroxy-2-propylphenoxy)propylthio)-3-chlorophenylacetic acid;
4-(3-(4-Phenoxy-2-propylphenoxy)propylamino)-phenyl(2,2-dimethyl)acetic acid;
4-(3-(4-Phenoxy-2-propylphenoxy)propylamino)-phenylpropan-3-oic acid;
N-[4-(3-(4-Phenoxy-2-propylphenoxy)propylamino)-phenyl]glycine;
4-(3-(4-Phenoxy-2-propylphenoxy)propylamino)-phenylacetic acid;
4-(3-(4-Phenoxy-2-propylphenoxy)propylamino)-3-chlorophenylacetic acid;
4-(3-(4-Phenoxy-2-propylphenoxy)propylamino)-phenoxyacetic acid;
4-(3-(4-Phenoxy-2-propylphenoxy)propylthio)-phenylpropan-3-oic acid;
4-(3-(4-Phenoxy-2-propylphenoxy)propyloxy)-2-phenyl-2,2-dimethylacetic acid;
4-(4-(4-Phenoxy-2-propylphenoxy)butylamino)-phenoxy-2,2-dimethylacetic acid;
4-(3-(4-Phenoxy-2-propylphenoxy)propylthio)-3-methylphenylacetic acid;
4-(3-(4-Phenoxy-2-propylphenoxy)propyloxy)-3-butylphenylacetic acid;
4-(3-(4-Phenoxy-2-propylphenoxy)propylamino)-2-propylphenylacetic acid;
4-(4-(4-Phenoxy-2-propylphenoxy)butylthio)-2-fluorophenylacetic acid;
4-(3-(4-Phenoxy-2-propylphenoxy)propyloxy)-phenoxy-2-*spiro*-cyclopropylacetic acid;
3-(3-(4-Phenoxy-2-propylphenoxy)propyloxy)-phenoxyacetic acid;
3-(3-(4-Phenoxy-2-propylphenoxy)propylamino)-phenylpropan-3-oic acid;
3-(3-(4-Phenoxy-2-propylphenoxy)propylthio)-2-phenyl-2,2-dimethylacetic acid;
3-(4-(4-Phenoxy-2-propylphenoxy)butyloxy)-phenoxy-2,2-dimethylacetic acid;
3-(3-(4-Phenoxy-2-propylphenoxy)propylthio)-3-methylphenylacetic acid;
3-(3-(4-Phenoxy-2-propylphenoxy)propylthio)-3-chlorophenylacetic acid;
4-(3-(4-Phenoxy-2-propylphenoxy)propylthio)-3-chlorophenylacetic acid;
3-(4-(4-Phenoxy-2-propylphenoxy)butylamino)-3-butylphenylacetic acid;
3-(3-(4-Phenoxy-2-propylphenoxy)propyloxy)-2-propylphenylacetic acid;
3-(3-(4-Phenoxy-2-propylphenoxy)propylthio)-2-fluorophenylacetic acid;
3-(4-(4-Phenoxy-2-propylphenoxy)butylamino)-phenoxy-2-*spiro*-cyclopropylacetic acid;
4-(3-(4-Phenoxy-2-(cyclopropylmethyl)phenoxy)propylamino)-phenylacetic acid;
4-(3-(4-Phenoxy-2-(prop-2-enyl)phenoxy)propylamino)-phenylacetic acid;
4-(3-(4-Phenoxy-2-butylphenoxy)propylamino)-phenylacetic acid;
4-(3-(4-Phenoxy-2-propoxyphenoxy)propylamino)-phenylacetic acid;
3-(4-(4-Phenoxy-2-(cyclopropylmethyl)phenoxy)butyloxy)-phenylacetic acid;
3-(4-(4-Phenoxy-2-(prop-2-enyl)phenoxy)butylamino)-phenylacetic acid;
3-(4-(4-Phenoxy-2-butylphenoxy)butyloxy)-phenylacetic acid;
3-(4-(4-Phenoxy-2-propoxyphenoxy)butyloxy)-phenylacetic acid;
4-(3-(4-Phenoxy-3-hydroxy-2-propylphenoxy)propylthio)-phenylacetic acid;
3-(3-(4-Phenoxy-3-methoxy-2-propylphenoxy)propylthio)-phenylacetic acid;
4-(4-(4-Phenoxy-3-butyloxy-2-propylphenoxy)butyloxy)-phenylacetic acid;
4-(4-(4-Phenoxy-3-chloro-2-propylphenoxy)butyloxy)-phenylacetic acid;
4-(3-(4-Benzoyl-2-propylphenoxy)propylthio)-phenylacetic acid;
4-(3-(4-(4-Chloro)benzoyl-2-propylphenoxy)propylthio)-phenylacetic acid;
4-(4-(4-(4-Tolyl)benzoyl-2-propylphenoxy)butyloxy)-phenylacetic acid;
3-(4-(4-(4-Butylcarbonyl)-2-propylphenoxy)butyloxy)-phenylacetic acid;
4-(3-(4-(N-Hydroxyiminoacetyl)-2-propylphenoxy)propylthio)-phenylacetic acid;
3-(3-(4-(2-Phenylethylcarbonyl)-2-propylphenoxy)propylthio)-phenylacetic acid;
4-(4-(4-(2-Phenylethoxycarbonyl-2-propylphenoxy)butyloxy)-phenylacetic acid;
4-(4-(4-(4-Phenylthiocarbonyl)-2-propylphenoxy)butyloxy)-phenylacetic acid;
3-chloro-4-(3-((1,2-benzisoxazol-3-yl)-2-propylphenoxy) propylthio)phenylacetic acid;
3-chloro-4-(3-((1,2-benzisoxazol-3-yl)-2-propylphenoxy) butyloxy)phenylacetic acid;
3-(4-(4-benzoyl-2-propylphenoxy)butyloxy)phenyl acetic acid;
3-chloro-4-(3-(4-fluorobenzoyl-2-propylphenoxy)propylthio)phenyl acetic acid;
3-chloro-4-(3-(4-phenyl-2-propylphenoxy)propylthio)phenyl acetic acid;
3-chloro-4-(3-(N-ethyl-N-(4-acetyl-3-hydroxy-2-propylphenyl)-amino)propylthio)phenyl acetic acid;
3-chloro-4-(3-(N-(4-hydroximinoacetyl-3-hydroxy-2-propylphenyl)-amino)propylthio)phenyl acetic acid;
3-chloro-4-(4--phenoxy (4-trifluoromethyl)-propylamino) phenyl acetic acid;
3-chloro-4-(4- phenoxy-2-propyl-(4-(4-fluoro)phenylsulfonyl)-propylthio)-phenyl acetic acid;
3-fluoro-4-(4-(4-phenoxy-2-propylphenoxy)-butyloxy)phenylacetic acid;
3-(4-(1-(2-propyl-4-phenethyl)hydroquinolyl)butyloxy)phenylacetic acid;
3-Chloro-4-(3-(2-propyl-3-hydroxy-4-(1-acetoxyiminopropyl))-phenoxy)propylthio)phenylacetic acid;
3-Chloro-4-(3-(2-propyl-3-methoxy-4-methylaminocarbonylphenoxy)propylthio)phenylacetic acid;
3-chloro-4-(3-(4-phenoxy-2-propylphenoxy)propyl-thio)phenylacetic acid;
3-chloro-4-((2-propyl-5-phenoxy) phenoxy-propylthio)phenylacetic acid;
3-Chloro-4-(3-(2-propyl-3-benzoylphenoxy)propylthio)phenylacetic acid; and
4-(3-(2-propyl-3-hydroxy-4-acetyl-phenoxy)propyloxy)-phenoxy acetic acid.

Preferred examples of structural formula I are:
Methyl 3-chloro-4-(3-(2-propyl-3-hydroxy-4-propionylphenoxy)-propylthio)-phenylacetate;
3-Chloro-4-(3-(2-propyl-3-hydroxy-4-propionylphenoxy)propylthio)phenyl-acetic acid;
Methyl 3-chloro-4-(3-(2-propyl-3-hydroxy-4-(1-oxobutyl)phenoxy)-propylthio)phenyl acetate;
3-Chloro-4-(3-(2-propyl-3-hydroxy-4-(1-oxobutyl)phenoxy)-propylthio)-phenylacetic acid;
Methyl 3-chloro-4-(3-(2-propyl-3-hydroxy-4-(1-oxopropyl)phenoxy)-propyloxy)phenylacetate;
3-Chloro-4-(3-(2-propyl-3-hydroxy-4-(1-hydroxyliminopropyl)phenoxy)-propylthio)phenylacetic acid;
Methyl 3-chloro-4-(3-(2-propyl-3-hydroxy-4-(1-hydroxyliminopropyl)-phenoxy)propyloxy)phenyl acetate;
Methyl 3-chloro-4-(3-(2-propyl-3-hydroxy-4-(1-hydroxyliminobutyl)-phenoxy)propylthio)phenylacetate;
3-Chloro-4-(3-(2-propyl-3-hydroxy-4-(1-hydroxylinimobutyl)phenoxy)-propylthio)phenylacetic acid;
Methyl 3- chloro-4-(3-(2-propyl-3-hydroxy-4-(1-hydroxyliminopropyl)-phenoxy)propylthio)phenylacetate
3-Chloro-4-(3-(2-propyl-3-hydroxy-4-(1-hydroxylimino-2-methylpropyl)-phenoxy)propylthio)phenylacetic acid;
Methyl 3-chloro-4-(3-(2-cyclopropylmethyl-3-hydroxy-4-propionylphenoxy)propylthio)phenylacetate;
3-Chloro-4-(3-(2-cyclopropylmethyl-3-hydroxy-4-propionylphenoxy)-propylthio)phenylacetic acid;
Methyl 3-chloro-4-(4-(2-propyl-3-hydroxy-4-( I -hydroxyiminopropyl)-phenyl)butylthio)phenylacetate;
3-chloro-4-(4-(2-propyl-3-hydroxy-4-(1-hydroxyiminopropyl)-phenyl)butylthio)phenylacetic acid;
3-Chloro-4-(3-(2-propyl-3-hydroxy-4-propionylphenoxy)propylthio)-phenyl-5-methyl-(1H)-tetrazole;
3-chloro-4-(3-(2-propyl-3-hydroxy-4-(1-oxopentyl))phenoxy)propylthio phenyl acetic acid methyl ester;
3-chloro-4-(3-(2-propyl-3-hydroxy-4-(1-hydroxyliminopentyl))phenoxy) propylthio phenyl acetic acid methyl ester,
3-chloro-4-(3-(2-propyl-3-hydroxy-4-(1-hydroxylimino propyl)phenoxy)-propylthio)phenylacetamide;
3-chloro-4-(3-(2-propyl-3-hydroxy-4-(1-(hydroxylimino-3-(phenyl) propyl)phenoxy)-propylthio)phenylacetic acid;
Methyl 3-chloro-4-(4-phenoxy-2-propylphenoxy)-propylamino)phenylacetate;
3-Chloro-4-(4-phenoxy-2-propylphenoxy)-propylamino)phenylacetic acid;
3-chloro-4-(3-(2-propyl-3-hydroxy-4-(1-hydroxyliminopropyl)) phenoxy)propylthio phenyl acetic acid;
4-(2-(4-benzyl-2-propylphenoxy)propylthio)-3-chlorophenylacetic acid;
Methyl 3-chloro-4-(3-(2-propyl-3-hydroxy-4-(1-oxopentyl)phenoxy)-propyl)-phenylacetate;
4-(3-(4-(N-hydroxyiminobenzoyl)-2-propylphenoxy)propylthio)-3-chlorophenylacetic acid;
4-(3-(4-benzoyl-3-hydroxy-2-propylphenoxy)propylthio)-3-chlorophenylacetic acid;
4-(3-(4-Phenoxy-2-propylphenoxy)propyloxy)-phenylacetic acid;
4-(3-(4-Benzoyl-2-propylphenoxy)propyloxy)-phenylacetic acid;
3-(4-(4-Phenoxy-2-propylphenoxy)butyloxy)-phenylacetic acid;
3-(4-(4-Benzoyl-2-propylphenoxy)butyloxy)-phenylacetic acid;
4-(3-(4-Phenoxy-2-propylphenoxy)propyloxy)-phenoxyacetic acid;
4-(3-(4-Benzoyl-2-propylphenoxy)propyloxy)-phenoxyacetic acid;
4-(3-(4-Phenoxy-2-propylphenoxy)propylthio)-3-propylphenylacetic acid;
4-(4-(4-Benzoyl-2-propylphenoxy)butylthio)-3-chlorophenylacetic acid;
4-(3-(4-Phenoxy-2-propylphenoxy)propylsulfono)-3-propylphenylacetic acid;
4-(3-(4-Benzoyl-2-propylphenoxy)propylsulfono)-3-chlorophenylacetic acid;
4-(4-(4-Phenoxy-2-propylphenoxy)butylthio)-3-propylbenzyl-tetrazole;
4-(4-(4-Benzoyl-2-propylphenoxy)butylthio)-3-chlorobenzyl-tetrazole;
4-(3-(4-Phenoxy-2-propylphenoxy)propylamino)-phenylacetic acid;
4-(3-(4-Benzoyl-2-propylphenoxy)propylamino)-phenylacetic acid;
3-(4-(4-(4-Fluorophenoxy)-2-propylphenoxy)butyloxy)-phenylacetic acid;
3-(4-(4-(4-Fluorobenzoyl)-2-propylphenoxy)butyloxy)-phenylacetic acid;
3-chloro-4-(3-((1,2-benzisoxazol-3-yl)-2-propylphenoxy) propylthio)phenylacetic acid;
3-chloro-4-(3-(4-fluorobenzoyl-2-propylphenoxy)propylthio)phenyl acetic acid; and
3-chloro-4-(3-(4-phenoxy-2-propylphenoxy)propyl-thio)phenylacetic acid.

The compounds of the present invention may have asymmetric centers and occur as racemates, racemic mixtures, and as individual diastereomers, with all possible isomers, including optical isomers, being included in the present invention.

Compounds of the general Formula I may be separated into diastereoisomeric pairs of enantiomers by, for example, fractional crystallization from a suitable solvent, for example methanol or ethyl acetate or a mixture thereof. The pair of enantiomers thus obtained may be separated into individual stereoisomers by conventional means, for example by the use of an optically active acid as a resolving agent.

Alternatively, any enantiomer of a compound of the general Formula I may be obtained by stereospecific synthesis using optically pure starting materials of known configuration.

The instant compounds can be isolated in the form of their pharmaceutically acceptable acid addition salts, such as the salts derived from using inorganic and organic acids. Examples of such acids are hydrochloric, nitric, sulfuric, phosphoric, formic, acetic, trifluoroacetic, propionic, maleic, succinic and malonic. In addition, certain compounds containing an acidic function such as a carboxy or tetrazole, can be isolated in the form of their inorganic salt in which the counterion can be selected from sodium, potassium, lithium, calcium and magnesium, as well as from organic bases.

As previously indicated, the compounds of the present invention have valuable pharmacological properties. They are useful in treating or preventing diabetes, treating obesity, lowering triglyceride levels and prevention of vascular restenosis. They are useful in treating other disorders where insulin resistance is a component including ovarian hyperandrogenism (polycyctic ovarian syndrome). They are also useful in raising high density lipoprotein levels, preventing, halting or slowing the progression of atherosclerotic cardiovascular diseases and related conditions and disease events.

The present invention also provides a compound of the general Formula I or a pharmaceutically acceptable salt thereof for use as an active therapeutic substance.

The present invention further provides a compound of the general Formula I, or a pharmaceutically acceptable ester thereof; or pharmaceutically acceptable salt thereof, for use in the treatment of hyperglycemia (diabetes) in human or non-human animals.

The present invention further provides a compound of the general Formula I, or a pharmaceutically acceptable ester thereof; or pharmaceutically acceptable salt thereof, in combination with known sulfonylureas, other insulin secretogogues as well as insulin for use in the treatment of diabetes treating obesity, lowering triglyceride levels, prevention of vascular restenosis, treating other disorders where insulin resistance is a component including ovarian hyperandrogenism (polycyctic ovarian syndrome), raising high density lipoprotein levels, and preventing, halting or slowing the progression of atherosclerotic cardiovascular diseases and related conditions and disease events.and hypertension in human or non-human animals.

In one aspect, the present invention provides a compound of the general Formula I for use in the treatment of obesity in human or non-human animals. Said compound can be effectively used in combination with other known or proposed strategies for the treatment of obesity or obesity-related disorders; for example, fenfluramine, dexfenfluramine, phentiramine and β₃ adrenergic receptor agonist agents.

The disease diabetes mellitus is characterized by metabolic defects in production and utilization of glucose which result in the failure to maintain appropriate blood sugar levels. The result of these defects is elevated blood glucose or hyperglycemia. Research on the treatment of diabetes has centered on attempts to normalize fasting and postprandial blood glucose levels. Treatments have included parenteral administration of exogenous insulin, oral administration of drugs and dietary therapies. The instant compounds can be effectively used in combination with known therapies for diabetes including insulin, sulfonylureas, biguanides (such as metformin), α-glucosidase inhibitors (such as acarbose) and others.

Two major forms of diabetes mellitus are now recognized. Type I diabetes, or insulin-dependent diabetes, is the result of an absolute deficiency of insulin. the hormone which regulates glucose utilization. Type II diabetes, or non-insulin-independent diabetes, often occurs in the face of normal, or even elevated levels of insulin and appears to be the result of the inability of tissues to respond appropriately to insulin. Most of the Type II diabetics are also obese. Accordingly, in another aspect the present invention provides a method of lowering triglyceride levels which comprises administering, to an animal in need thereof, a therapeutically effective amount of a compound of the formula I or pharmaceutically acceptable salt or ester thereof.

In addition the compounds of the present invention lower or modulate triglyceride levels and/or cholesterol levels and raise HDL plasma levels and are therefore of use in combating medical conditions wherein such lowering (and raising) is thought to be beneficial. Thus they may be used in the treatment of hypertension, obesity, atherosclerotic disease events, diabetes and related conditions by administering to an animal in need thereof, a therapeutically effective amount of a compound of the formula (I) or pharmaceutically acceptable salt thereof. The compositions are formulated and administered in the same general manner as detailed below. They may also contain other active ingredients known for use in the treatment of atherosclerotic disease events, diabetes, hypertension, obesity and related conditions, for example fibrates such as clofibrate, bezafibrate and gemfibrozil; inhibitors of cholesterol biosynthesis such as HMG-CoA reductase inhibitors for example lovastatin, simvastatin and pravastatin; inhibitors of cholesterol absorption for example beta-sitosterol and (acyl CoA:cholesterol acyltransferase) inhibitors for example melinamide; anion exchange resins for example cholestyramine, colestipol or a dialkylaminoalkyl derivatives of a cross-linked dextran; nicotinyl alcohol, nicotinic acid or a salt thereof; vitamin E; and thyromimetics.

In particular the invention provides methods for preventing or reducing the risk of developing atherosclerosis, comprising the administration of a prophylactically effective amount of a compound of formula I alone or in combination with one or more additional pharmaceutically active agents, to a mammal, particularly human, who is at risk of developing atherosclerosis.

Atherosclerosis encompasses vascular diseases and conditions that are recognized and understood by physicians practicing in the relevant fields of medicine. Atherosclerotic cardiovascular disease, coronary heart disease (also known as coronary artery disease or ischemic heart disease), cerebrovascular disease and peripheral vessel disease are all clinical manifestations of atherosclerosis and are therefore encompassed by the terms "atherosclerosis" and "atherosclerotic disease."

The instant invention further provides methods for preventing or reducing the risk of a first or subsequent (where the potential exists for recurrence) atherosclerotic disease event, comprising the administration of a prophylactically effective amount, or more particularly an effective amount of cholesterol biosynthesis inhibitor, of a compound of formula I alone or in combination with one or more additional pharmaceutically active agents, to a mammal, particularly human, who is at risk for having an atherosclerotic disease event. The term "atherosclerotic disease event" as used herein is intended to encompass coronary heart disease events, cerebrovascular events, and intermittent claudication. Coronary heart disease events are intended to include CHD death, myocardial infarction (i.e., a heart attack), and coronary revascularization procedures. Cerebrovascular events are intended to include ischemic or hemorrhagic stroke (also known as cerebrovascular accidents) and transient ischemic attacks. Intermittent claudication is a clinical manifestation of peripheral vessel disease. It is intended that persons who have previously experienced one or more non-fatal atherosclerotic disease event are those for whom the potential for recurrence of such an event exists.

Persons to be treated with the instant therapy include those at risk of developing atherosclerotic disease and of having an atherosclerotic disease event. Standard atherosclerotic disease risk factors are known to the average physician practicing in the relevant fields of medicine. Such known risk factors include but are not limited to hypertension, smoking, diabetes, low levels of high density lipoprotein cholesterol, high levels of low density lipoprotein cholesterol, and a family history of atherosclerotic cardiovascular disease. Published guidelines for determining those who are at risk of developing atherosclerotic disease can be found in: National Cholesterol Education Program, Second report of the Expert Panel on *Detection, Evaluation, and Treatment of High Blood Cholesterol in Adults (Adult Treatment Panel II*), National Institute of Health, National Heart Lung and Blood Institute, NIH Publication No. 93-3095, September 1993; *abbreviated version:* Expert Panel on Detection, Evaluation, and Treatment of High Blood Cholesterol in Adults, *Summary of the second report of the national cholesterol education program (NCEP) Expert Panel on Detection, Evaluation, and Treatment of High Blood Cholesterol in Adults (Adult Treatment Panel II)*, *JAMA,* 1993, *269*, pp. 3015-23. People identified as having one or more of the above-noted risk factors, as well as people who already have atherosclerosis, are intended to be included within the group of people considered to be at risk for having an atherosclerotic disease event.

The active compounds of the present invention may be orally administered as a pharmaceutical composition, for example, with an inert diluent, or with an assimilable edible carrier, or they may be enclosed in hard or soft shell capsules, or they may be compressed into tablets, or they may be incorporated directly with the food of the diet. For oral therapeutic administration, which includes sublingual administration, these active compounds may be incorporated with excipients and used in the form of tablets, pills, capsules, ampules, sachets, elixirs, suspensions and syrups. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally as, for example, liquid drops or spray.

The effective dosage of active ingredient employed may vary depending on the particular compound employed, the mode of administration, the condition being treated and the severity of the condition being treated.

When treating or preventing diabetes mellitus and/or hyperglycemia or hypertriglyceridemia, or obesity, or when treating, preventing or slowing the progression of atherosclerosis generally satisfactory results are obtained when the compounds of the present invention are administered at a daily dosage of from about 0.1 milligram to about 100 milligram per kilogram of animal body weight, preferably given as a single daily dose or in divided doses two to six times a day, or in sustained release form. For most large mammals, the total daily dosage is from about 1.0 milligrams to about 1000 milligrams, preferably from about 1 milligrams to about 50 milligrams. In the case of a 70 kg adult human, the total daily dose will generally be from about 7 milligrams to about 350 milligrams. This dosage regimen may be adjusted to provide the optimal therapeutic response.

The compositions are formulated and administered in the same general manner as detailed below. The compounds of the instant invention may be used effectively alone or in combination with one or more additional active agents depending on the desired target therapy. Combination therapy includes administration of a single pharmaceutical dosage formulation which contains a compound of formula I and one or more additional active agents, as well as administration of a compound of formula I and each active agent in its own separate pharmaceutical dosage formulation. For example, a compound of formula I and an HMG-CoA reductase inhibitor can be administered to the patient together in a single oral dosage composition such as a tablet or capsule, or each agent administered in separate oral dosage formulations. Where separate dosage formulations are used, a compound of formual I and one or more additional active agents can be administered at essentially the same time, i.e., concurrently, or at separately staggered times, i.e, sequentially; combination therapy is understood to include all these regimens.

An example of combination treatment or prevention of atherosclerosis may be wherein a compound of formula I is administered in combination with one or more of the following active agents: *an antihyperlipidemic agent; a* plasma *HDL-raising agent; an antihypercholesterolemic agent such as* a cholesterol biosynthesis inhibitor, for example an HMG-CoA reductase inhibitor, *an HMG-CoA synthase inhibitor, a squalene epoxidase inhibitor, or a squalene synthetase inhibitor (also known as squalene synthase inhibitor); an* acyl-coenzyme A: cholesterol acyltransferase (ACAT) inhibitor such as melinamide; probucol; nicotinic acid and the salts thereof and niacinamide; a cholesterol absorption inhibitor such as beta-sitosterol; a bile acid sequestrant anion exchange resin such as cholestyramine, colestipol or dialkylaminoalkyl derivatives of a cross-linked dextran; an LDL (low density lipoprotein) receptor inducer; fibrates such as clofibrate, bezafibrate, fenofibrate, and gemfibrizol; vitamin B₆ (also known as pyridoxine) and the pharmaceutically acceptable salts thereof such as the HCl salt; vitamin B₁₂ (also known as cyanocobalamin); anti-oxidant vitamins such as vitamin C and E and beta carotene; a beta-blocker; an angiotensin II antagonist; an angiotensin converting enzyme inhibitor; and a platelet aggregation inhibitor such as fibrinogen receptor antagonists (i.e., glycoprotein IIb/IIIa fibrinogen receptor antagonists) and aspirin. As noted above, the compounds of formula I can be administered in combination with more than one additional active agent, for example, a combination of a compound of formula I with an HMG-CoA reductase inhibitor (e.g. lovastatin, simvastatin and pravastatin) and aspirin, or a compound of formula I with an HMG-CoA reductase inhibitor and a beta blocker.

Another example of combination therapy can be seen in treating obesity or obesity-related disorders, wherein the compounds of formula I may be effectively used in combination with for example, fenfluramine, dexfenfluramine, phentiramine and β₃ adrenergic receptor agonist agents.

Still another example of combination therapy can be seen in treating diabetes and related disorders wherein the compounds of formula I can be effectively used in combination with for example sulfonylureas, biguanides, α-glucosidase inhibitors, other insulin secretogogues, insulin as well as the active agents discussed above for treating atherosclerosis.

In accordance with this invention, a pharmaceutically effective amount of a compound of formula I can be used for the preparation of a medicament useful for treating diabetes, treating obesity, lowering tryglyeride levels, raising the plasma level of high density lipoprotein, and for treating, preventing or reducing the risk of developing atherosclerosis, and for preventing or reducing the risk of having a first or subsequent atherosclerotic disease event in mammals, particularly in humans.

Additionally, an effective amount of a compound of formula I and a therapeutically effective amount of one or more active agents selected from the group consisting of: *an antihyperlipidemic agent; a* plasma *HDL-raising agent; an antihypercholesterolemic agent such as* a cholesterol biosynthesis inhibitor, for example an HMG-CoA reductase inhibitor, *an HMG-CoA synthase inhibitor, a squalene epoxidase inhibitor, or a squalene synthetase inhibitor (also known as squalene synthase inhibitor);* ; an acyl-coenzyme A: cholesterol acyltransferase inhibitor, probucol; nicotinic acid and the salts thereof; niacinamide; a cholesterol absorption inhibitor; a bile acid sequestrant anion exchange resin; a low density lipoprotein receptor inducer; clofibrate, fenofibrate, and gemfbrozol; vitamin B₆ and the pharmaceutically acceptable salts thereof; vitamin B₁₂; an anti-oxidant vitamin; a beta-blocker; an angiotensin II antagonist; an angiotensin converting enzyme inhibitor; a platelet aggregation inhibitor; a fibrinogen receptor antagonist; aspirin; fenfluramines, dexfenfluramines, phentiramines, β₃ adrenergic receptor agonists; sulfonylureas, biguanides, α-glucosidase inhibitors, other insulin secretogogues, and insulin can be used together for the preparation of a medicament useful for the above-described treatments.

The tablets, pills and capsules, may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

These active compounds may also be administered parenterally. Solutions or suspensions of these active compounds can be prepared in water suitably mixed with a surfactant such as hydroxypropylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example. water, ethanol, polyol (e.g. glycerol, propylene glycol and liquid polyethylene glycol), suitable mixtures thereof, and vegetable oils.

Specific examples of formula I may require the use of protecting groups to enable their successful elaboration into the desired structure. Protecting groups may be chosen with reference to Greene, T.W., et al., Protective Groups in Organic Synthesis, John Wiley & Sons, Inc., 1991. The blocking groups are readily removable, i.e., they can be removed, if desired, by procedures which will not cause cleavage or other disruption of the remaining portions of the molecule. Such procedures include chemical and enzymatic hydrolysis, treatment with chemical reducing or oxidizing agents under mild conditions, treatment with fluoride ion, treatment with a transition metal catalyst and a nucleophile, and catalytic hydrogenation.

Examples of suitable hydroxyl protecting groups are: trimethylsilyl, triethylsilyl, o-nitrobenzyloxycarbonyl, p-nitrobenzyloxycarbonyl, t-butyldiphenylsilyl, t-butyldimethylsilyl, benzyloxycarbonyl, t-butyloxycarbonyl, 2,2,2-trichloroethyloxycarbonyl, and allyloxycarbonyl. Examples of suitable carboxyl protecting groups are benzhydryl, o-nitrobenzyl, p-nitrobenzyl, 2-naphthylmethyl, allyl, 2-chloroallyl, benzyl, 2,2,2-trichloroethyl, trimethylsilyl, t-butyldimethylsilyl, t-butldiphenylsilyl, 2-(trimethylsilyl)ethyl, phenacyl, p-methoxybenzyl, acetonyl, p-methoxyphenyl, 4-pyridylmethyl and t-butyl.

The process for making the compounds of the instant invention is generally depicted in Scheme 1 below: 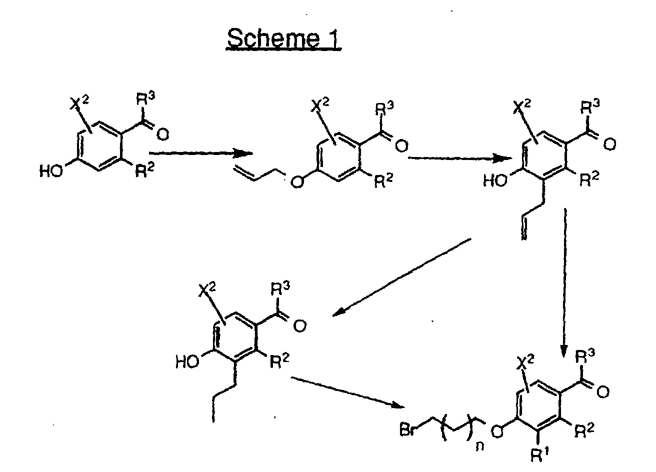 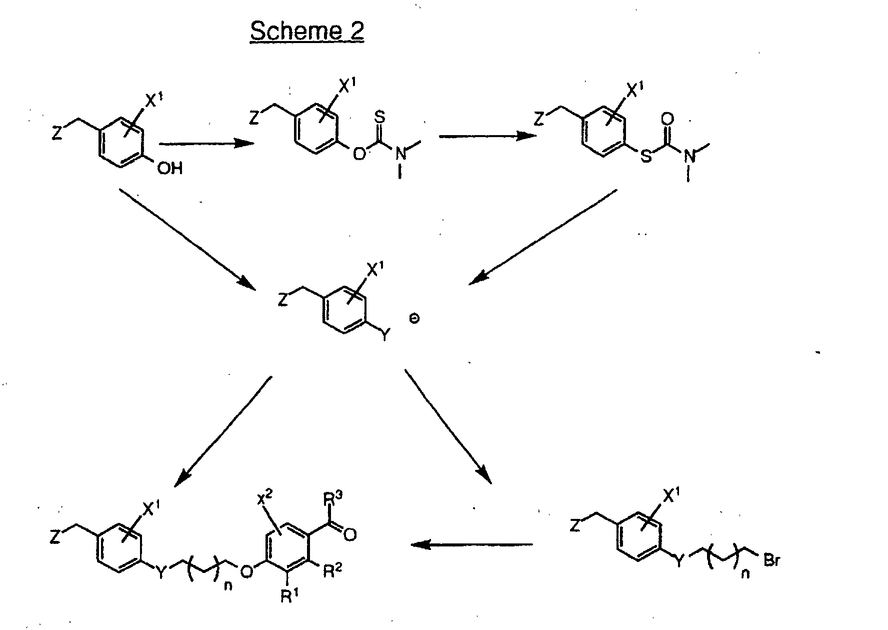 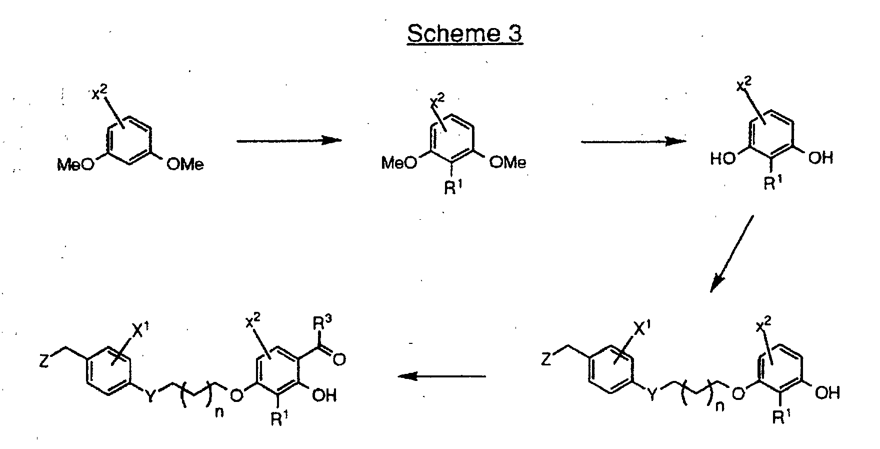 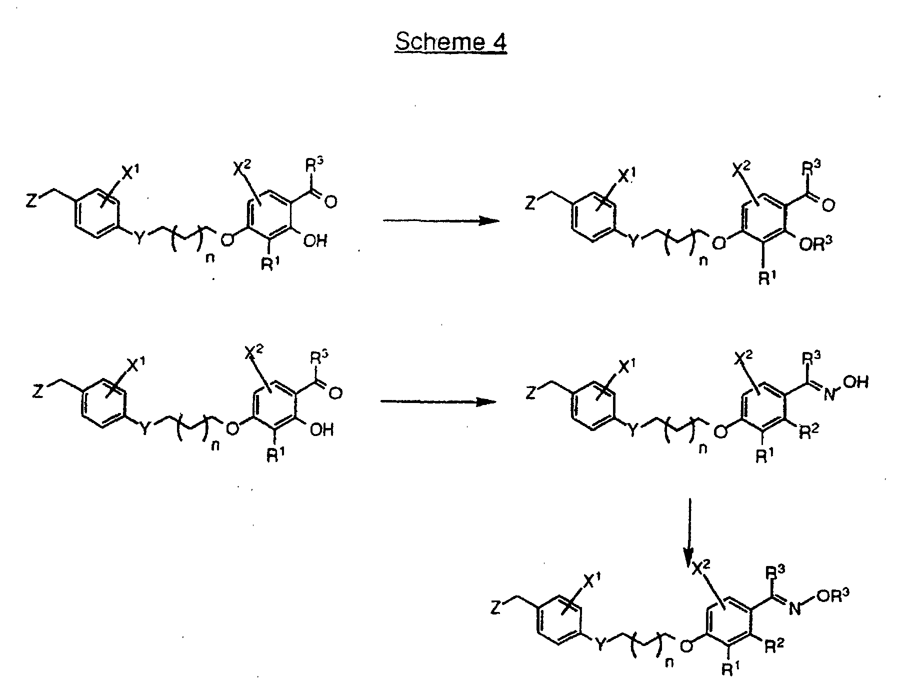 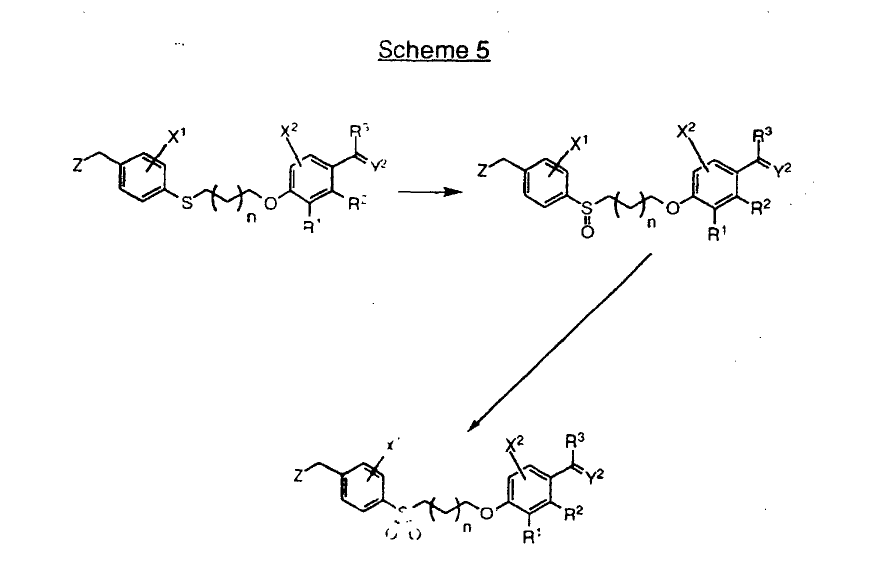 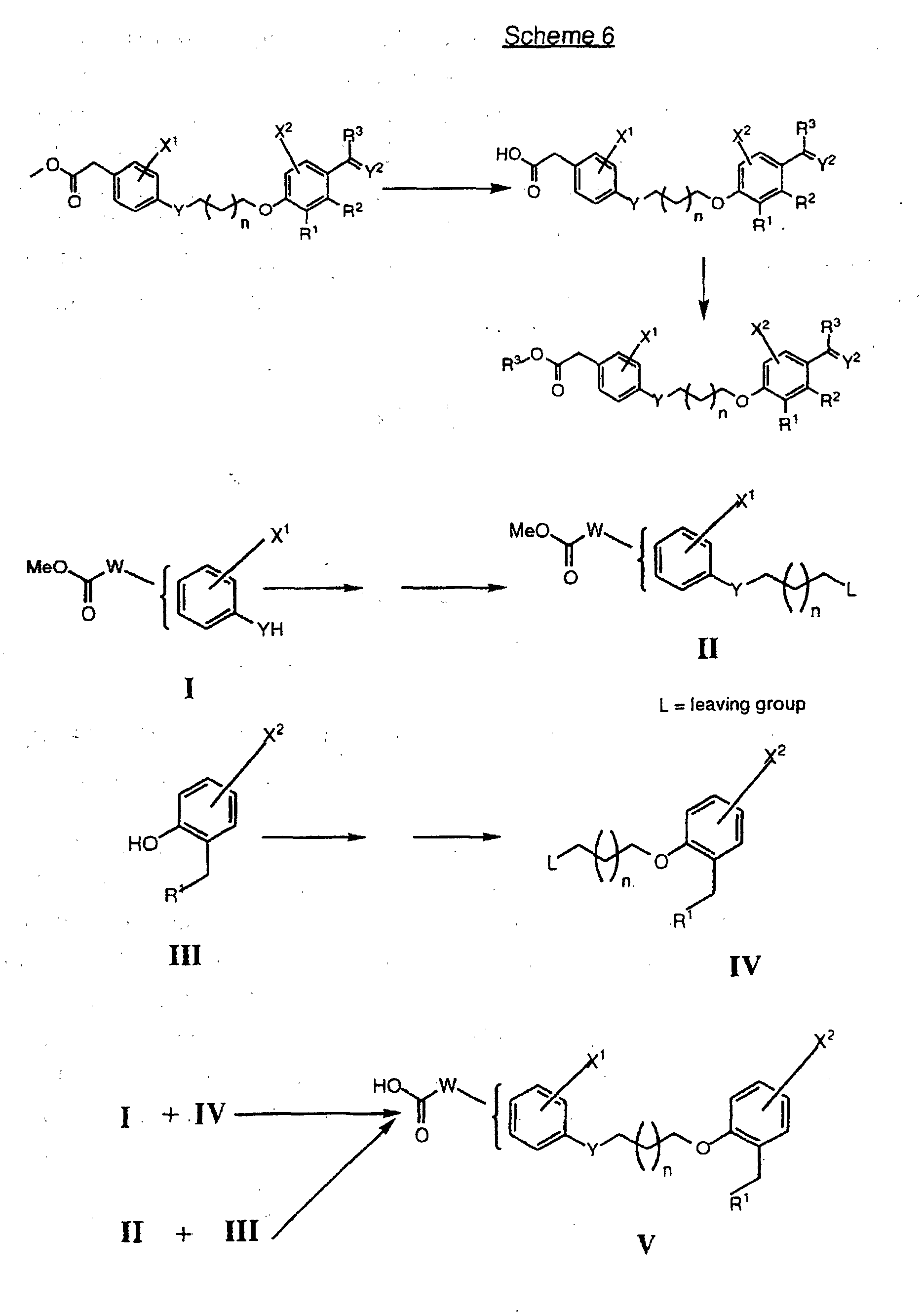 L is a leaving group such as halo, such as bromide, or sulfonyloxy, such as mesyloxy or tosyloxy.

The following examples are provided so that the invention might be more fully understood.

### EXAMPLE 1

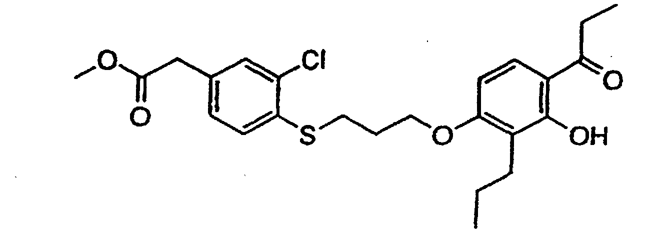

### Methyl 3-chloro-4-(3-(2-propyl-3-hydroxy-4-propionylphenoxy)-propylthio)-phenylacetate

### Step A: Preparation of 1-bromo-3-(2-propyl-3-hydroxy-4-propionyl-phenoxy)propane

A solution of 3-propyl-2,4-dihydroxypropiophenone (25.545 grams) in 2-butanone (300 mL) was treated with 1,3-dibromopropane (48.79 mL) and potassium carbonate (50.859 grams). The mixture was refluxed for 4 hours. The reaction mixture was partitioned between isopropyl acetate and pH 4 buffer. The organic was washed once with water, then dried over magnesium sulfate. The organic was filtered and evaporated to an oil which was chromatographed over silica gel with hexane/methylene chloride (2:1) to afford the title compound.
NMR (CDCl₃) δ 7.62 (d, 1H, J = 8.8 Hz), 6.43 (d, 1H, J = 8.8 Hz), 4.16 (t, 2H, J = 5.8 Hz), 3.60 (t, 2H, J = 6.4 Hz), 2.94 (quart, 2H, J = 7.3 Hz), 2.61 (bt, 2H, J = 7.5 Hz).

### Step B: Preparation of methyl 3-chloro-4-(3-(2-propyl-3-hydroxy-4-propionylphenoxy)propylthio)phenylacetate

A solution of 3-chloro-4-dimethylcarbamoylthiophenylacetic acid methyl ester (33.038 grams) in dry methanol (350 mL) was treated with a solution of sodium methoxide in methanol (25wt%; 34.15 mL). The solution was refluxed for 2 hours. HPLC analysis showed the disappearance of the carbamate. The solution was allowed to cool to 50°C. 1-bromo-3-(2-propyl-3-hydroxy-4-propionylphenoxy)propane (Example 1, Step A; 31.5 grams) was added and the solution stirred for 1 hour. The reaction was partitioned between isopropyl acetate and pH 4 buffer. The organic was washed once more with pH 4 buffer, then water. The organic was dried over magnesium sulfate, filtered and concentrated to an oil. The oil was applied to a silica gel column packed with hexane/methylene chloride (2:1). The column was eluted with this mobile phase until the product began to appear in the eluant. The mobile phase was switched to 100% methylene chloride and elution continued until all the product was recovered.
NMR (Acetone) δ 7.81 (d, 1H, J = 9.1 Hz), 7.25 (dd, 1H, J = 8.1, 1.8 Hz), 6.62 (d, 1H, J = 9.1 Hz), 4.27 (t, 2H, J = 5.9 Hz), 3.64 (s, 3H), 3.25 (t, 2H, J = 7.5 Hz), 3.04 (quart, 2H, J = 7.3 Hz), 2.65 (bt, 2H, J = 7.6 Hz).

### EXAMPLE 2

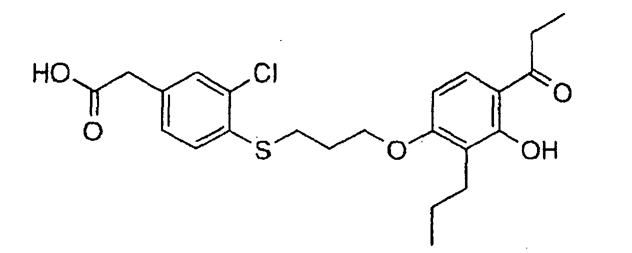

### 3-Chloro-4-(3-(2-propyl-3-hydroxy-4-propionylphenoxy)propylthio)-phenyl-acetic acid

A solution of methyl 3-chloro-4-(3-(2-propyl-3-hydroxy-4-propionylphenoxy)propylthio)phenylacetate (Example 1, 0.113 grams) in methanol (1.5 mL) was treated with a solution of lithium hydroxide in water (1.01 M; 0.362 mL). The reaction was refluxed 1 hour. The reaction mixture was partitioned between isopropyl acetate and 0.1N HCl. The organic was dried over magnesium sulfate, filtered and concentrated to a solid. The solid was suspended in methylene chloride/cyclohexane (1:1; 2 mL). The mixture was refluxed briefly and cooled to 0°C. The title compound was isolated by filtration.
NMR (CDCl₃) d 7.59 (d, 1H, J = 9.0 Hz), 7.11 (dd, 1H, J = 8.1, 1.8 Hz), 6.40 (d, 1H, J = 9.0 Hz), 4.14 (t, 2H, J = 5.8 Hz), 3.57 (s, 2H), 3.13 (t, 2H, J = 7.1 Hz), 2.94 (quart, 2H, J = 7.3 Hz), 2.62 (bt, 2H, J = 7.7 Hz).

### EXAMPLE 3

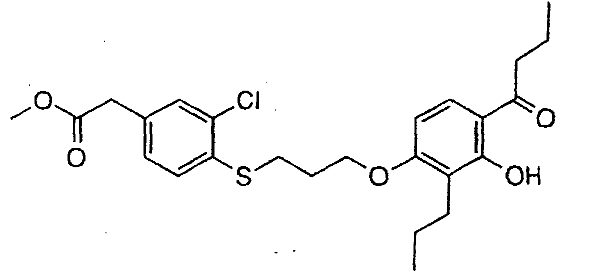

### Methyl 3-chloro-4-(3-(2-propyl-3-hydroxy-4-(1-oxobutyl)phenoxy)-propylthio)phenyl acetate

### Step A: Preparation of 1-(2,4-dihydroxy)phenyl-1-butanone

A 0°C suspension of resorcinol (7.523 grams) in dry 1,2-dichloroethane (100 mL) was treated with aluminum chloride (10.932 grams). The suspension was vigorusly stirred for 15 minutes. A solution of butyryl chloride (7.09 mL) in 1,2-dichloroethane (10 mL) was added dropwise. The mixture was allowed to stir and gradually warm to 25°C over 6 hours. The mixture was slowly added to a stirred mixture of water and methylene chloride. The organic was dried over magnesium sulfate, filtered and concentrated to a solid. Silica gel chromatography afforded the title compound.
NMR (CDCl₃) δ 12.90 (s, 1H), 7.63 (d, 1H, J = 9.0 Hz), 6.58 (vbs, 1H), 6.39 (d overlapping with dd, 2H), 2.86 (t, 2H, J = 7.3 Hz), 1.72 (hex, 2H, J = 7.3 Hz), 0.99 (t, 3H, J = 7.1 Hz).

### Step B: Preparation of 1-(2-hydroxy-4-(3-propenyloxy)phenyl-1-butanone

A solution of 1-(2,4-dihydroxy)phenyl-1-butanone (12.15 grams) in 2-butanone (125 mL) was treated with allyl bromide (5.56 mL) and potassium carbonate (9.318 grams). The mixture was refluxed for 4 hours. The reaction was partitioned between isopropyl acetate and pH 4 buffer. The organic was washed once with water and dried over magnesium sulfate. The organic was filtered and concentrated to an oil which was chromatographed over silica gel to afford the title compound.
NMR (CDCl₃) δ 7.62 (d, 1H, J = 9.0 Hz), 6.42 (dd, 1H, J = 9.0, 1.8 Hz), 6.40 (d, 1H, J = 1.8 Hz), 6.00 (mult, 1H), 5.40 (dd, 1H, J = 15.7, 1.3 Hz), 5.30 (dd, 1H, J = 11.9, 1.3 Hz), 4.52 (dd, 2H, J = 3.8, 1.3 Hz), 2.86 (t, 2H, J = 7.3 Hz), 1.72 (hex, 2H, J = 7.3 Hz), 0.99 (t, 3H, J = 7.1 Hz).

### Step C: Preparation of 1-(2,4-dihydroxy-3-(3-propenyl))phenyl-1-butanone

A solution of 1-(2-hydroxy-4-(3-propenyloxy)phenyl-1-butanone (14.22 grams) in ortho-dichlorobenzene (60 mL) was refluxed for 22 hours. The solvent was removed by distillation *in vacuo* and the semi-solid digested in refluxing cyclohexane (30 mL). The mixture was cooled to 25°C and filtered to afford the title compound.
NMR (CDCl₃) δ 13.22 (s, 1H), 7.58 (d, 1H, J = 8.9 Hz), 6.38 (d, 1H, J = 8.9 Hz), 5.98 (mult, 1H), 5.62 (bs, 1H), 5.18 (dd, 1H, J = 16.0, 1.3 Hz), 5.12 (dd, 1H, J = 11.5, 1.3 Hz), 3.49 (dd, 2H, J = 4.0, 1.3 Hz), 2.88 (t, 2H, J = 7.2 Hz), 1.72 (hex, 2H, J = 7.2 Hz), 0.99 (t, 3H, J = 7.1 Hz).

### Step D: Preparation of 1-(2,4-dihydroxy-3-propyl)phenyl-1-butanone

A solution of 1-(2,4-dihydroxy-3-(3-propenyl))phenyl-1-butanone (9.878 grams) in ethyl acetate (50 mL) was hydrogenated (21 psi) over 10% Pd/C catalyst (1.48 grams) for 3 hours. The reaction was filtered through Celite and all volatiles were removed to afford the title compound.
NMR (CDCl₃) δ 7.53 (d, 1H, J = 9.0 Hz), 6.35 (d, 1H, J = 9.0 Hz), 2.88 (t, 2H, J = 7.3 Hz), 2.60 (t, 2H, J = 7.5 Hz).

### Step E: 1-bromo-3-(2-propyl-3-hydroxy-4-(1-oxobutyl)phenoxy)-propane

The title compound was prepared according to the method described in Example 1, Step A.
NMR (CDCl₃) δ 7.61 (d, 1H, J = 8.8 Hz), 6.43 (d, 1H, J = 8.8 Hz), 4.16 (t, 2H, J = 5.7 Hz), 3.60 (t, 2H, J = 6.4 Hz), 2.87 (t, 2H, J = 7.4 Hz), 2.61 (bt, 2H, J = 7.5 Hz).

### Step F: Preparation of methyl 3-chloro-4-(3-(2-propyl-3-hydroxy-4-(1-oxobutyl)phenoxy)propylthio)phenyl acetate

The title compound was prepared according to the method described in Example 1, Step B.
NMR (CDCl₃) δ 7.61 (d, 1H, J = 9.0 Hz), 7.11 (dd, 1H, J = 8.1, 1.8 Hz), 6.41 (d, 1H, J = 8.9 Hz), 4.14 (t, 2H, J = 5.8 Hz), 3.68 (s,3H), 3.13 (t, 2H, J = 7.6 Hz), 2.87 (t, 2H, J = 7.1 Hz), 2.60 (bt, 2H, J = 7.4 Hz).

### EXAMPLE 4

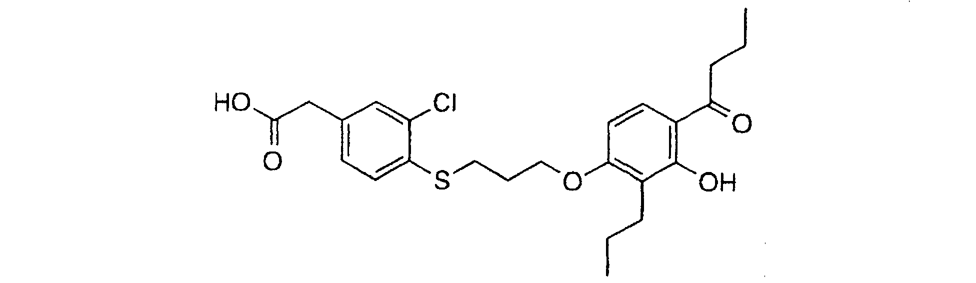

### 3-Chloro-4-(3-(2-propyl-3-hydroxy-4-(1-oxobutyl)phenoxy)-propylthio)-phenylacetic acid

Hydrolysis of Example 3 using the method described in Example 2 produced the title compound.
NMR (CDCl₃) δ 7.59 (d, 1H, J = 9.0 Hz), 7.11 (dd, 1H, J = 8.1, 1.8 Hz), 6.39 (d, 1H, J = 9.0 Hz), 4.14 (t, 2H, J = 5.8 Hz), 3.57 (s, 2H), 3.13 (t, 2H, J = 7.1 Hz), 2.87 (t, 2H, J = 7.4 Hz), 2.62 (bt, 2H, J = 7.5 Hz).

### EXAMPLE 5

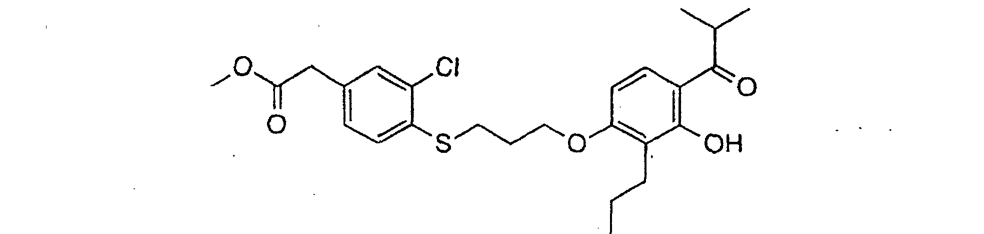

### Methyl 3-chloro-4-(3-(2-propyl-3-hydroxy-4-(1-oxo-2-methylpropyl)-phenoxy)propylthio)phenylacetate

### Step A: Preparation of 1-bromo-3-(2-propyl-3-hydroxy-4-(1-oxo-2-methylpropyl)phenoxy)propane

The title compound was prepared according to the method described in Example 3, Step A.
NMR (CDCl₃) δ 7.63 (d, 1H, J = 8.9 Hz), 6.43 (d, 1H, J = 8.9 Hz), 4.17 (t, 2H, J = 5.8 Hz), 3.60 (t, 2H, J = 6.4 Hz), 3.52 (hept, 1H, J = 6.8 Hz), 2.61 (bt, 2H, J = 7.5 Hz), 1.21 (d, 6H, J = 6.7 Hz).

### Step B: Preparation of methyl 3-chloro-4-(3-(2-propyl-3-hydroxy-4-(1 -oxo-2-methylpropyl)phenoxy)propylthio)-phenylacetate

The title compound was prepared according to the method described in Example 1, Step B.
NMR (CDCl₃) δ 7.63 (d, 1H, J = 9.1 Hz), 7.10 (dd, 1H, J = 8.1, 1.9 Hz), 6.42 (d, 1H, J = 9.0 Hz), 4.15 (t, 2H, J = 5.9 Hz), 3.68 (s, 3H), 3.52 (hept, 1H, J = 6.9 Hz), 3.13 (t, 2H, J = 7.6 Hz), 2.61 (bt, 2H, J = 7.3 Hz), 1.22 (d, 6H, J = 7.0 Hz).

### EXAMPLE 6

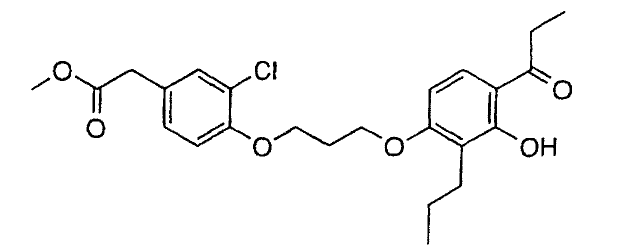

### Methyl 3-chloro-4-(3-(2-propyl-3-hydroxy-4-(1-oxopropyl)phenoxy)-propyloxy)phenylacetate

### Step A: Preparation of 3-chloro-4-(3-bromopropyloxy)phenylacetic acid methyl ester

The title compound was prepared from 3-chloro-4-hydroxyphenylacetic acid methyl ester according to the method described in Example 1, Step A.

### Step B: Preparation of methyl 3-chloro-4-(3-(2-propyl-3-hydroxy-4-(1-oxopropyl)phenoxy)propyloxy)phenylacetate

A solution of 3-chloro-4-(3-bromopropyloxy)phenylacetic acid methyl ester (Step A; 0.552 grams) in 2-butanone (6 mL) was treated with 3-propyl-2,4-dihydroxypropiophenone (0.299 grams). Potassium carbonate (0.217 grams) was added and the mixture refluxed for 4 hours. The reaction was partitioned between isopropyl acetate and pH 4 phosphate buffer. The organic was dried over magnesium sulfate, filtered and evaporated to a residue which was chromatographed over silica gel to give the product.
NMR (CD₃OD) δ 7.77 (d, 1H, J = 9.0 Hz), 7.29 (d, 1H, J = 1.8 Hz), 7.13 (dd, 1H, J = 8.1, 1.9 Hz), 7.02 (d, 1H, J = 8.0 Hz), 6.62 (d, 1H, J = 9.0 Hz), 4.31 (t, 2H, J = 5.9 Hz), 4.24 (t, 2H, J = 5.9 Hz), 3.68 (s, 3H), 3.00 (quart, 2H, J = 7.2 Hz), 2.60 (bt, 2H, J = 7.6 Hz).

### EXAMPLE 7

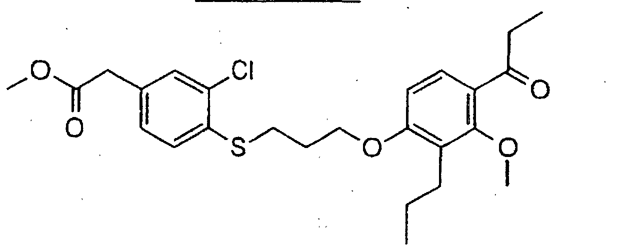

### Methyl 3-chloro-4-(3-(2-propyl-3-methoxy-4-propionylphenoxy) propyl-thio)phenylacetate

A solution of methyl 3-chloro-4-(3-(2-propyl-3-hydroxy-4-propionylphenoxy)propylthio)phenylacetate (Example 1; 65 mg) in dry DMF (1.0 mL) was treated with methyl iodide (0.017 mL) and cesium carbonate (55 mg). The reaction was stirred at 25°C for 14 hours. The reaction was partitioned between isopropyl acetate and pH 4 phosphate buffer. The organic was washed 3 times with water, then dried over magnesium sulfate, filtered and concentrated. The residue was chromatographed over silica gel to afford the titled methyl ether.
NMR (acetone) δ 7.49 (d, 1H, J = 9.0 Hz), 7.29 (dd, 1H, J = 8.0, 1.8 Hz), 6.82 (d, 1H, J = 8.9 Hz), 4.23 (t, 2H, J = 5.8 Hz), 3.72 (s, 3H), 3.68 (s, 3H), 3.24 (t, 2H, J = 7.3 Hz), 2.93 (quart, J = 7.4 Hz), 2.66 (t, 2H, J = 7.6 Hz).

### EXAMPLE 8

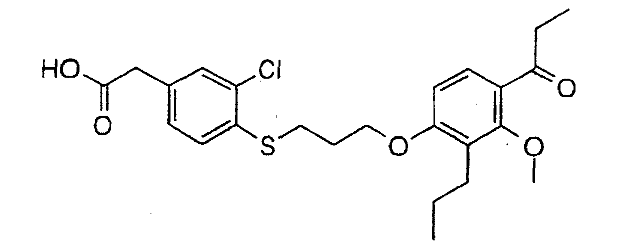

### 3-Chloro-4-(3-(2-propyl-3-methoxy-4-propionylphenoxy)propylthio)-phenylacetic acid

The product of Example 7 was coverted to its corresponding acid by lithium hydroxide hydrolysis as described in Example 2.
NMR (acetone) δ 7.48 (d, 1H, J = 9.0 Hz), 7.27 (dd, 1H, J = 8.0, 1.8 Hz), 6.82 (d, 1H, J = 8.9 Hz), 4.23 (t, 2H, J = 5.8 Hz), 3.72 (s, 3H), 3.25 (t, 2H, J = 7.2 Hz), 2.93 (quart, J = 7.3 Hz), 2.66 (t, 2H, J = 7.5 Hz).

### EXAMPLE 9

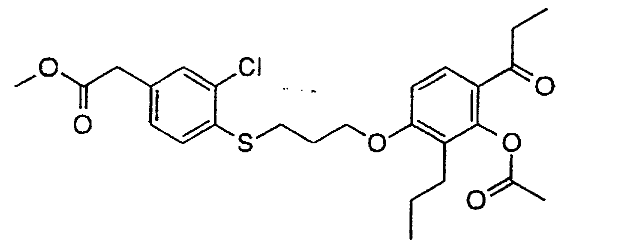

### Methyl 3-chloro-4-(3-(2-propyl-3-acetoxy-4-propionylphenoxy)propylthio)phenylacetate

A suspension of anhydrous sodium acetate (0.164 grams) in acetic anhydride (2 mL) was treated with trifluoroacetic anhydride (0.283 mL). The solution warmed and became homogenous. A solution of methyl 3-chloro-4-(3-(2-propyl-3-hydroxy-4-propionyl)phenoxy)propylthiophenyl-acetate (Example 1; 0.151 grams) in acetic anhydride (1.5 mL) was treated with the above solution of acetyl trifluoroacetate (0.768 mL) and potassioum carbonate (0.093 grams). The reaction was stirred at 70°C for 16 hours. The solvent was removed *in vacuo* and flushed once with toluene. The residue was partitioned between isopropyl acetate and pH 4 phosphate buffer. The organic was dried over magnesium sulfate, filtered and evaporated to afford the title compounds as a solid.
NMR (CDCl₃) δ 7.69 (d, 1H, J = 8.8 Hz), 7.11 (dd, 1H, J = 8.1, 1.8 Hz), 6.72 (d, 1H, J = 8.7 Hz), 4.14 (t, 2H, J = 5.7 Hz), 3.68 (s, 3H), 3.12 (t, 2H, J = 7.1 Hz), 2.86 (quart. 2H, J = 7.3 Hz), 2.51 (bt, 2H, J = 7.7 Hz), 2.36 (s, 3H).

### EXAMPLE 10

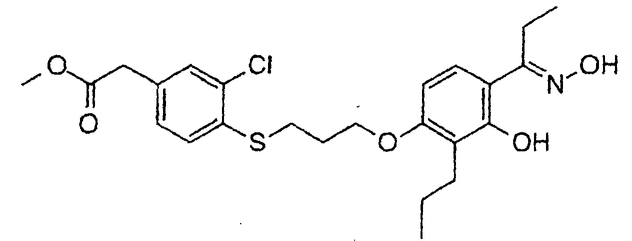

### Methyl 3- chloro-4-(3-(2-propyl-3-hydroxy-4-(1-hydroxyliminopropyl)-phenoxy)propylthio)phenylacetate

A solution of methyl 3-chloro-4-(3-(2-propyl-3-hydroxy-4-propionylphenoxy)propylthio)phenylacetate (Example 1; 25.655 grams) in dry methanol (260 mL) was treated with hydroxylamine hydrochloride (3.833 grams). Anhydrous sodium acetate (4.524 grams) was added and the mixture refluxed for 4 hours. The reaction mixture was partitioned between isopropyl acetate and pH 7 buffer. The organic phase was washed once with water and dried over magnesium sulfate, filtered and evaporated to give the title compound as an oil.
NMR (CDCl₃) δ 7.11 (dd, 1H, J = 8.1, 1.8 Hz), 6.42 (d, 1H, J = 8.9 Hz), 4.09 (t, 2H, J = 5.7 Hz), 3.68 (s, 3H), 3.14 (t, 2H, J = 7.2 Hz), 2.83 (quart, 2H, J = 7.7 Hz), 2.66 (bt, 2H, J = 7.7 Hz).

### EXAMPLE 11

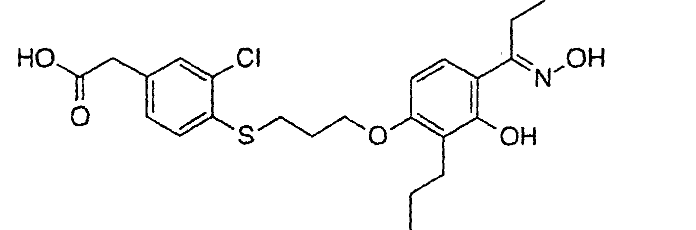

### 3-Chloro-4-(3-(2-propyl-3-hydroxy-4-(1-hydroxyliminopropyl)phenoxy)-propylthio)phenylacetic acid

A solution of methyl 3-chloro-4-(3-(2-propyl-3-hydroxy-4-(1-hydroxyiminopropyl)phenoxy)propylthio)phenylacetate (Example 10; 21.15 grams) in methanol (250 mL) was treated with a solution of lithium hydroxide in water (1.299 M; 67.84 mL). The reaction was refluxed 1 hour. The reaction mixture was partitioned between isopropyl acetate and 0.1N HCl. The organic was dried over magnesium sulfate, filtered and concentrated to a solid. The solid was dissolved in methylene chloride (80 mL) and heated to reflux. Cyclohexane (80 mL) was added dropwise. The solution was cooled to 0°C and the title compound was isolated by filtration.
NMR (CDCl₃) δ 7.11 (dd, 1H, J = 8.1, 1.8 Hz), 6.42 (d, 1H, J = 8.9 Hz), 4.09 (t, 2H, J = 5.7 Hz), 3.13 (t, 2H, J = 7.2 Hz), 2.82 (quart, 2H, J = 7.7 Hz), 2.64 (bt, 2H, J = 7.7 Hz).

### EXAMPLE 12

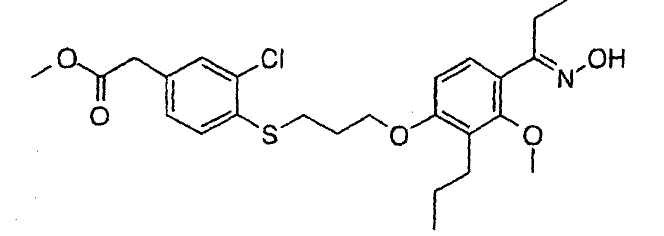

### Methyl 3- chloro-4-(3-(2-propyl-3-methoxy-(1-hydroxyliminopropyl)-phenoxy)propylthio)phenyl acetate

The title compound was prepared from methyl 3-chloro-4-(3-(2-propyl-3-methoxy-4-( 1-oxopropyl)phenoxy)propylthio)phenylacetate (Example 7) using the method described in Example 10. NMR (acetone) d 7.26 (dd, 1H, J = 8.1, 1.7 Hz), 7.03 (d, 1H, J = 9.0 Hz), 6.83 (d, 1H, J = 8.9 Hz), 4.17 (t, 2H, J = 5.8 Hz), 3.68 (s, 3H), 3.66 (s, 3H), 3.25 (t, 2H, J = 7.2 Hz), 2.73 (quart, 2H, J = 7.8 Hz), 2.64 (bt, 2H, J = 7.7 Hz).

### EXAMPLE 13

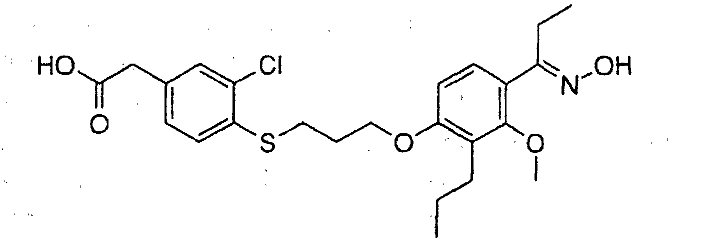

### 3-Chloro-4-(3-(2-propyl-3-methoxy-4-(1-hydroxyliminopropyl)-phenoxy)-propylthio)phenyl acetic acid

Hydrolysis of Example 12 using the method described in Example 11 produced the title compound.
NMR (acetone) δ 7.27 (dd, 1H, J = 8.1, 1.8 Hz), 7.02 (d, 1H, J = 8.9 Hz), 6.71 (d, 1H, J = 9.0 Hz), 4.18 (t, 2H, J = 5.7 Hz), 3.68 (s, 3H), 3.63 (s, 2H), 3.26 (t, 2H, J = 7.4 Hz), 2.75 (quart, J = 7.5 Hz), 2.67 (bt, 2H, J = 7.3 Hz).

### EXAMPLE 14

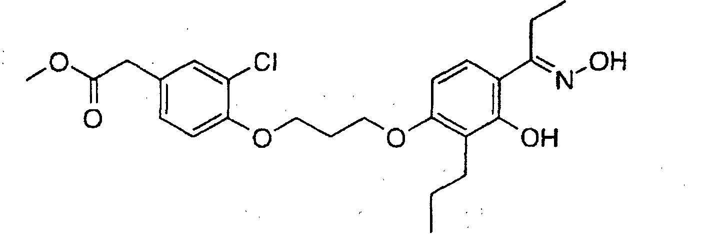

### Methyl 3-chloro-4-(3-(2-propyl-3-hydroxy-4-(1-hydroxyliminopropyl)-phenoxy)propyloxy)phenyl acetate

The titled compound was prepared from methyl 3-chloro-4-(3-(2-propyl-3-hydroxy-4-propionylphenoxy)propyloxy)phenylacetate (Example 6) using the method described in Example 10.
NMR (CD₃OD) δ 7.14 (dd, 1H, J = 8.1, 1.9 Hz), 7.02 (d, 1H, J = 8.1 Hz), 6.54 (d, 1H, J = 8.9 Hz), 4.23 (2 overlapping quarts, 4H), 3.67 (s, 3H), 2.82 (quart, 2H, J = 7.4 Hz), 2.61 (bt, 2H, J = 7.5 Hz).

### EXAMPLE 15

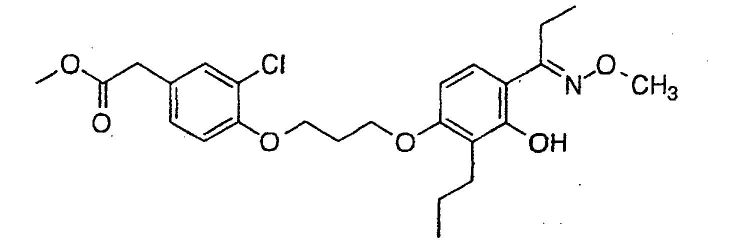

### Methyl 3-chloro-4-(3-(2-propyl-3-hydroxy-4-(1-methoxyliminopropyl)-phenoxy)propylthio)phenylacetate

A solution of methyl 3-chloro-4-(3-(2-propyl-3-hydroxy-4-propionylphenoxy)propyloxy)phenylacetate (Example 6; 87 mg) in methanol (1 mL) was treated with anhydrous sodium acetate (159 mg) and methoxylamine hydrochloride (162 mg). The mixture was refluxed for 24 hours. The reaction was partitioned between isopropyl acetate and pH 7 phosphate buffer. The organic was dried over magnesium sulfate, filtered and concentrated to a semi-solid which was chromatographed over silica gel to afford the titled compound.
NMR (CDCl₃) δ 7.10 (dd, 1H, J = 8.1, 1.9 Hz), 6.88 (d, 1H, J = 8.8 Hz), 6.47 (d, 1H, J = 8.9 Hz), 4.20 (2 overlapping quarts, 4H), 3.93 (s, 3H), 3.68 (s, 3H), 2.67 (quart, 2H, J = 7.6 Hz), 2.62 (bt, 2H, J = 7.5 Hz).

### EXAMPLE 16

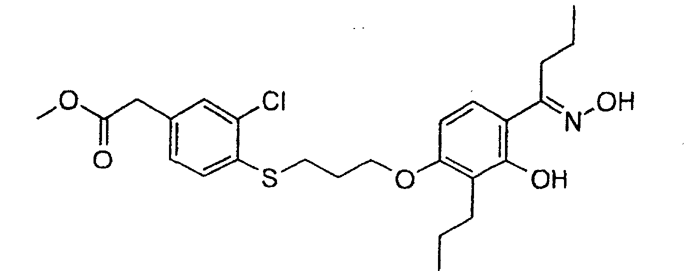

### Methyl 3-chloro-4-(3-(2-propyl-3-hydroxy-4-(1-hydroxyliminobutyl)-phenoxy)propylthio)phenylacetate

The titled compound was prepared from methyl 3-chloro-4-(3-(2-propyl-3-hydroxy-4-(1-oxobutyl)phenoxy)propylthio)-phenylacetate (Example 3) using the method described in Example 10. NMR (CDCl₃) δ 7.12 (dd, 1H, J = 8.0, 1.8 Hz), 6.43 (d, 1H, J = 8.9 Hz), 4.09 (t, 2H, J = 5.7 Hz), 3.69 (s, 3H), 3.13 (t, 2H, J = 7.3 Hz), 2.79 (bt, 2H, J = 7.9 Hz), 2.65 (bt, 2H, J = 7.6 Hz).

### EXAMPLE 17

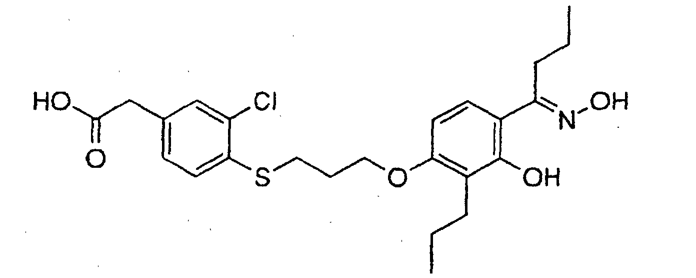

### 3-Chloro-4-(3-(2-propyl-3-hydroxy-4-(1-hydroxyliminobutyl)phenoxy)-propylthio)phenylacetic acid

Hydrolysis of Example 16 using the method described in Example 11 produced the title compound.
NMR (CDCl₃) δ 7.12 (dd, 1H, J = 8.0, 1.8 Hz), 6.41 (d, 1H, J = 8.9 Hz), 4.08 (t, 2H, J = 5.7 Hz), 3.57 (s, 2H), 3.14 (t, 2H, J = 7.3 Hz), 2.79 (bt, 2H, J = 7.9 Hz), 2.64 (bt, 2H, J = 7.6 Hz).

### EXAMPLE 18

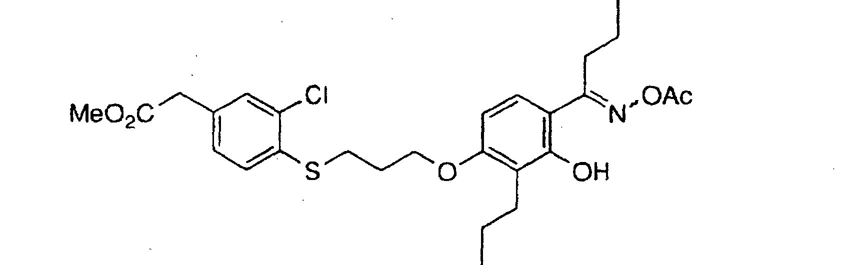

### Methyl 3-chloro-4-(3-(2-propyl-3-hydroxy-4-(1-acetoxyiminobutyl)-phenoxy)propylthio)phenylacetate

A solution of methyl 3-chloro-4-(3-(2-propyl-3-hydroxy-4-(1-hydroxyliminobutyl)phenoxy)propylthio)phenylacetate (Example 16; 253 mg) in acetic anhydride (3 mL) was stirred for 16 hours. The solvent removed *in vacuo*. The remaining residue was dissolved in isopropyl acetate and washed with pH 7 phosphate buffer. The organic phase was dried over magnesium sulfate, filtered and evaporated to give the titled compound.
NMR (CDCl₃) δ 7.11 (dd, 1H, J - 7.9, 1.8 Hz), 6.43 (d,1H, J = 8.8 Hz), 4.09 (t, 2H, J = 5.8 Hz), 3.69 (s, 3H), 3.13 (t, 2H, J = 7.2 Hz), 2.82 (bt, 2H, J = 7.8 Hz), 2.68 (bt, 2H, J = 7.7 Hz).

### EXAMPLE 19

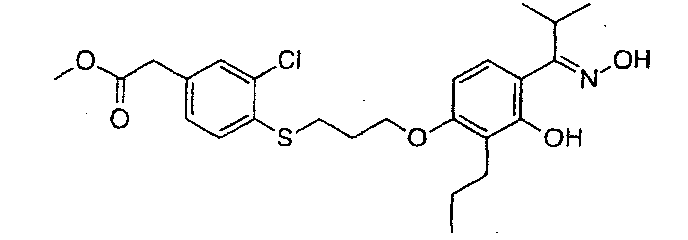

### Methyl 3-chloro-4-(3-(2-propyl-3-hydroxy-4-(1-hydroxylimino-2-methyl-propyl)phenoxy)propylthio)phenylacetate

The titled compound was prepared from methyl 3-chloro-4-(3-(2-propyl-3-hydroxy-4-(1-oxo-2-methylpropyl)phenoxy)-propylthio)phenyl-acetate (Example 5) using the method described in Example 10.
NMR (CDCl₃) δ 7.11 (dd, 1H, J = 8.0, 1.8 Hz), 6.39 (d, 1H, J = 9.0 Hz), 4.08 (t, 2H, J = 5.7 Hz), 3.68 (s,3H), 3.51 (hept, 1H, J = 7.2 Hz), 3.13 (t, 2H, J = 7.2 Hz), 2.65 (bt, 2H, J = 7.8 Hz), 1.40 (d, 6H, J = 7.1 Hz).

### EXAMPLE 20

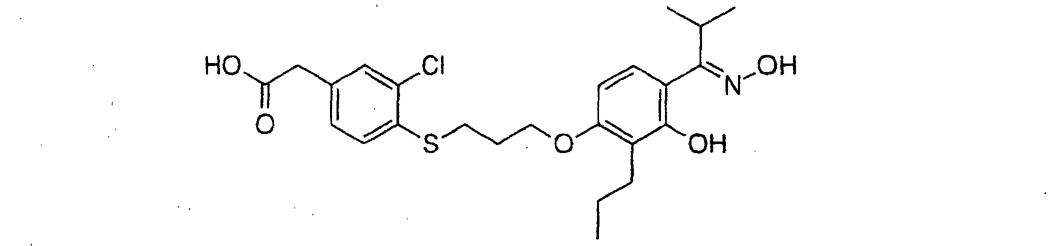

### 3-Chloro-4-(3-(2-propyl-3-hydroxy-4-(1-hydroxylimino-2-methylpropyl)-phenoxy)propylthio)phenylacetic acid

Hydrolysis of Example 19 using the method described in Example 11 produced the title compound.
NMR (CDCl₃) δ 7.11 (dd, 1H, J = 8.0, 1.8 Hz), 6.39 (d, 1H, J = 9.0 Hz), 4.09 (t, 2H, J = 5.7 Hz), 3.58 (s, 2H), 3.50 (hept, 1H, J = 7.2 Hz), 3.14 (t, 2H, J = 7.2 Hz), 2.63 (bt, 2H, J = 7.8 Hz), 1.39 (d, 6H, J = 7.2 Hz).

### EXAMPLE 21

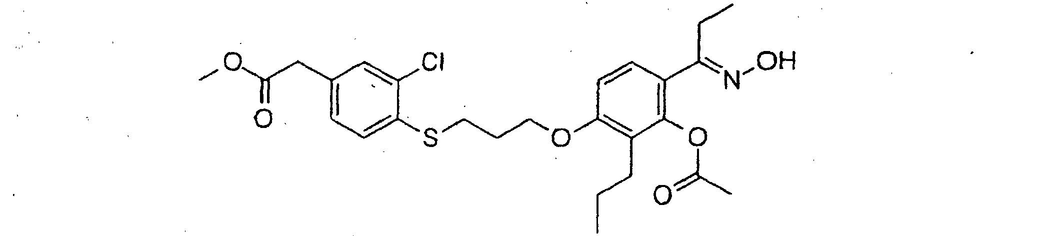

### Methyl 3-chloro-4-(3-(2-propyl-3-acetoxy-4-(1-hydroxyiminopropyl) phenoxy)propylthio)phenyl acetate

A solution of methyl 3-chloro-4-(3-(2-propyl-3-acetoxy-4-(1-oxopropyl)phenoxy)propylthio)phenylacetate (Example 9; 112 mg) in methanol (1.2 mL) was treated with anhydrous sodium acetate (19 mg) and hydroxylamine hydrochloride (16 mg). The solution was stirred at 40°C for 24 hours. The reaction mixture was partitioned between isopropyl acetate and pH 7 phosphate buffer. The organic was dried over magnesium sulfae, filtered and evaporated to a residue. Silica gel chromatography afforded the titled compound.
NMR (CDCl₃) δ 8.21 (vbs, 1H), 6.72 (d, 1H, J = 8.9 Hz), 4.10 (t, 2H, J = 5.8 Hz), 3.69 (s, 3H), 3.12 (t, 2H, J = 7.2 Hz), 2.67 (quart, 2H, J = 7.7 Hz), 2.48 (bt, 2H, J = 7.6 Hz), 2.25 (s, 3H).

### EXAMPLE 22

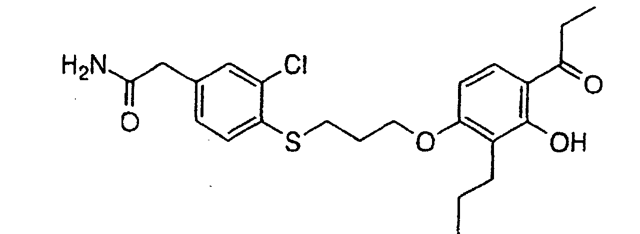

### 3-chloro-4-(3-(2-propyl-3-hydroxy-4-(1-oxopropyl)phenoxy) propylthio)-phenylacetamide

A solution of 3-chloro-4-(3-(2-propyl-3-hydroxy-4-propionyl-phenoxy)propylthio)phenylacetic acid (Example 2; 0.103 grams) in dry methylene chloride (1.5 mL) was treated with oxalyl chloride (0.030 mL). The reaction was stirred at 25°C for 6 hours. All volatiles were removed *in vacuo*. The derived residue was dissolved in dry THF (2 mL) and cooled to 0°C. The solution was treated with con. ammonia (0.5 mL) and allowed to warm slowly to 25° over a one hour period. The reaction mixture was partitioned between isopropyl acetate and saturated aqueous sodium bicarbonate. The organic was washed once with water, dried over magnesium sulfate, filtered and concentrated to a solid. The solid was suspended in methylene chloride/cyclohexane (1:1; 2 mL). The mixture was refluxed briefly and cooled to 0°C. The title compound was isolated by filtration.
NMR (CDCl₃) δ 7.60 (d, 1H, J = 9.0 Hz), 7.11 (dd, 1H, J = 8.1, 1.8 Hz), 6.39 (d, 1H, J = 9.0 Hz), 5.51 (vbs, 1H), 5.39 (vbs, 1H), 4.15 (t, 2H, J = 5.7 Hz), 3.49 (s, 2H), 3.14 (t, 2H, J = 7.1 Hz), 2.94 (quart, 2H, J = 7.3 Hz), 2.62 (bt, 2H, J = 7.7 Hz).

### EXAMPLE 23

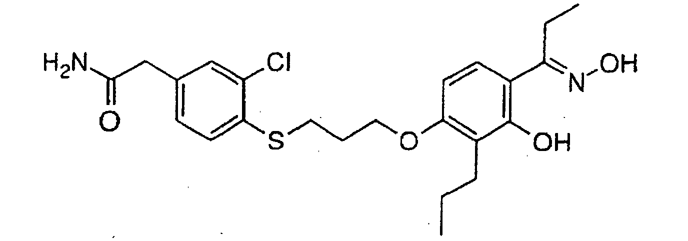

### 3-Chloro-4-(3-(2-propyl-3-hydroxy-4-(1-hydroxyliminopropyl)phenoxy)-propylthio)phenylacetamide

Starting from 3-chloro-4-(3-(2-propyl-3-hydroxy-4-propionyl-phenoxy)propylthio)phenylacetamide (Example 22), the title compound was prepared according to the method described in Example 9. The product is a 2:1 mixture of isomers.
NMR (acetone) δ 10.19 (vbs, 0.67H), 8.02 (vbs, 0.67H), 7.25 (dd, J = 8.1, 1.8 Hz), 6.91 (vbs, 0.33H), 6.55 (d, 1H, J = 8.9 Hz). 6.30 (vbs, 0.33H), 4.17 (t, 2H, J = 5.7 Hz), 3.48 (bs, 0.67H), 3.40 (bs, 1.34H), 3.22 (t, 2H, J = 7.2 Hz), 2.88 (quart overlapping H₂O, J = 7.2 Hz), 2.68 (bt, 2H, J = 7.4 Hz).

### EXAMPLE 24

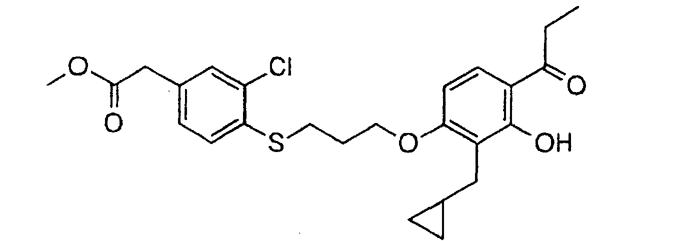

### ethyl-3-hydroxy-4-propionyl-phenoxy)propylthio)phenylacetate

### Step A: Preparation of 2,4-dihydroxy-3-cyclopropylmethylpropiophenone

To a solution of 2,4-dihydroxy-3-allyl-propiophenone (0.500 g, 2.42 mmol) in 2 mL of ethyl ether was added diazomethane (15 mL of a 0.70M soln. in Et₂O) under nitrogen followed by the addition of palladium acetate (cat., 2 mg). The reaction mixture was stirred at ambient temperature for 30 min until gas evolution had ceased. The ether was evaporated in vacuo, and the residue was purified on a silica gel flash chromatography column eluted with 10% EtOAc:hexane. Evaporation of the purified fractions and solvent removal in vacuo afforded the product.
¹H NMR (400 MHz, CDCl₃, ppm): δ 0.24 m, 2H), 0.40 (m, 2H), 0.97 (m, 1H), 1.18 (t, *J*=7.34 Hz, 3H), 2.61 (d, *J*=6.38 Hz, 2H), 2.91 (q, *J*=7.33 Hz, 2H), 6.32 (d, *J*=8.76 Hz, 1H), 7.52 (d, *J*=8.82 Hz, 1H).

### Step B: Preparation of 3-(2-cyclopropylmethyl-3-hydroxy-4-propionylphenoxy)propy] bromide

To a suspension of the product from Step A (2.30 g, 10.4 mmol), and potassium carbonate (1.58 g, 11.4 mmol) in methylethyl ketone was added 1,3-dibromopropane (6.30 g, 31.2 mmol). The reaction mixture was heated to reflux and stirred for 4 h under nitrogen. The reaction mixture was cooled to ambient temperature, filtered, and the solvent was evaporated in vacuo. The resulting residue was purified on a silica gel flash chromatography column eluted with 20% EtOAc:hexane. Evaporation of the purified fractions and solvent removal in vacuo afforded the product.
¹H NMR (400 MHz, CDCl₃, ppm): δ 0.20 (m, 2H), 0.34 (m, 2H), 0.98 (m, 1H), 1.21 (t, *J*=7.32 Hz, 3H), 2.34 (m, 2H), 2.58 (d, *J*=6.76 Hz, 2H), 2.95 (q, *J*=7.33 Hz, 2H), 3.60 (t, *J*=6.31 Hz, 2H), 4.17 (t, *J*=5.78 Hz, 2H), 6.45 (d, *J*=9.00 Hz, 1H), 7.63 (d, *J*=8.95 Hz, 1H).
CI-MS: *m*/*e* = 341 (M + 1).

### Step C: Preparation of methyl 3-chloro-4-(3-(2-cyclopropylmethyl-3-hydroxy-4-propionylphenoxy)propylthio)phenylacetate

To a solution of methyl 3-chloro-4-[(*N,N*-dimethylcarbamoyl)thio]phenylacetate (3.12 g, 9.68 mmol) in 5 mL of methanol was added sodium methoxide (19.5 mL of 0.5 M soln., 1.5 eq.) slowly at ambient temperature under nitrogen. The reaction mixture was stirred at ambient temperature for 40 minutes. The product of Step B (2.20 g, 6.45 mmol) was added to the reaction mixture, and the mixture was heated to reflux for 2 h. The reaction was cooled to ambient temperature, quenched with pH=4 phosphate buffer (20 mL), and partitioned with ethyl acetate. The aqueous portion was separated. The organic layer was washed with brine (2 x 20 mL), dried (MgSO₄), filtered, and evaporated in vacuo. The residue was purified on a silica gel flash chromatography column eluted with 20% EtOAc:hexane. Evaporation of the purified fractions and solvent removal in vacuo afforded the product.
¹H NMR (400 MHz, CDCl₃, ppm): δ 0.20 (m, 2H), 0.34 (m, 2H), 0.97 (m, 1H), 1.21 (t, *J*=7.36 Hz, 3H), 2.16 (m, 2H), 2.59 (d, *J*=6.68 Hz, 2H), 2.95 *(q, J*=7.32 Hz, 2H), 3.13 (t, *J*=7.10 Hz, 2H), 3.55 (s, 2H), 3.68 (s, 3H), 4.15 (t, *J*=5.77 Hz, 2H), 6.42 (d, *J*=9.00 Hz, 1H), 7.15 (d, 1H), 7.27 (m, 2H), 7.61 (d, *J*=8.99 Hz, 1H).
ESI-MS: *m*/*e* = 477 (M + 1).

### EXAMPLE 25

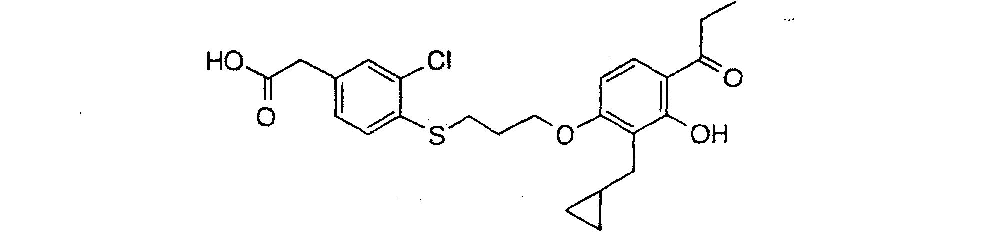

### 3-Chloro-4-(3-(2-cyclopropylmethyl-3-hydroxy-4-propionylphenoxy)-propy]thio)phenylacetic acid

### Preparation of 3-chloro-4-(3-(2-cyclopropylmethyl-3-hydroxy-4-propionylphenoxy)propylthio)phenylacetic acid

To a solution of the product of Example 24 (0.025 g, 0.0524 mmol) in 0.5 mL of methanol and 0.1 mL of THF was added NaOH soln. (0.05 mL of a 5 N aq. soln., 5.0 eq) at rt. The reaction mixture was initially heated with a heat gun to reflux in order to dissolve the starting material. After heating, the reaction mixture stirred at rt for 1.5 h. The reaction mixture was diluted with EtOAc and 0.1N HCl aq soln.(5 mL) was added. The organic layer was separated from the aqueous portion, and washed with 0.1N HCl (5 mL) followed by 10 mL brine. The organic layer was dried (MgSO₄), filtered, and evaporated in vacuo to afford the title compound. TLC: (80:20:1-CHCl₃:MeOH:NH₄OH).
¹H NMR (400 MHz, CD₃OD, ppm): δ 0.19 (m, 2H), 0.30 (m, 2H), 1.00 (m, 1H), 1.18 (t, *J*=7.33 Hz, 3H), 2.13 (m, 2H), 2.58 (d, *J*=6.74 Hz, 2H), 2.99 (q, *J*=7.33 Hz, 2H), 3.17 (t, *J*=7.24 Hz, 2H), 3.55 (s, 2H), 4.18 (t, *J*=5.81 Hz, 2H), 6.55 (d, *J*=9.09 Hz, 1H), 7.15-7.18 (m, 1H), 7.33-7.37 (m, 2H), 7.75 (d, *J*=9.04 Hz, 1H).
ESI-MS: *m*/*e* = 463 (M + 1).

### EXAMPLE 26

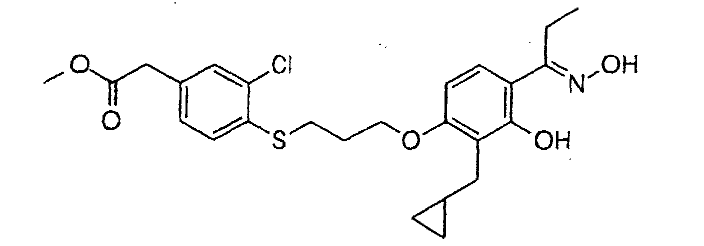

### Methyl 3-chloro-4-(3-(2-cyclopropylmethyl-3-hydroxy-4-(1-hydroxyimino-propyl)phenoxy)propylthio)phenylacetate

### Preparation of methyl 3-chloro-4-(3-(2-cyclopropylmethyl-3-hydroxy-4-(1-hydroxyiminopropyl)phenoxy)propylthio)-phenylacetate

To a suspension of the product of Example 24 (0.521 g, 1.09 mmol) in 5 mL of methanol was added hydroxylamine-HCl (0.379 g, 5.46 mmol) and sodium acetate (0.448 g, 5.46 mmol). The reaction mixture was heated to reflux for 4 h. The reaction mixture was allowed to cool to ambient temperature, and partitioned between EtOAc and pH=7 phosphate buffer. The organic layer was separated from the aqueous layer and washed with pH=7 buffer and brine. The organic portion was dried (MgSO₄), filtered, and evaporated in vacuo. The residue was purified on a silica gel flash chromatography column eluted with 1% MeOH/CHCl₃. Evaporation of the purified fractions and solvent removal in vacuo afforded the product.
¹H NMR (400 MHz, CDCl₃, ppm): δ 0.22 (m, 2H), 0.34 (m, 2H), 1.05 (m, 1H), 1.20 (t, *J*=7.64 Hz, 3H), 2.15 (m, 2H), 2.63 (d, *J*=6.59 Hz, 2H), 2.83 (q, *J*=7.65 Hz, 2H), 3.13 (t, *J*=7.32 Hz, 2H), 3.55 (s, 2H), 3.68 (s, 3H), 4.10 (t, *J*=5.78 Hz, 2H), 6.43 (d, *J*=8.86 Hz, 1H), 6.92 (s, 1H), 7.10-7.15 (m, 1H), 7.23-7.29 (m, 3H).
CI-MS: *m*/*e* = 494 (M + 1).

### EXAMPLE 27

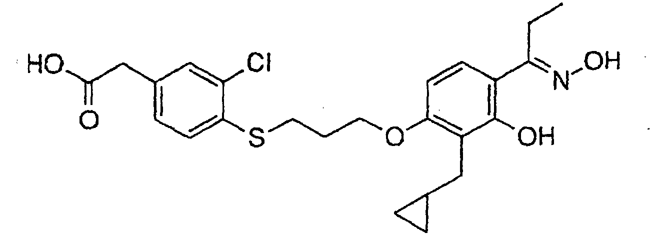

### 3-Chloro-4-(3-(2-cyclopropylmethyl-3-hydroxy-4-(1-hydroxyiminopropyl)-phenoxy)propylthio)phenylacetic acid

### Preparation of 3-chloro-4-(3-(2-cyclopropylmethyl-3-hydroxy-4-(1-hydroxyiminopropyl)phenoxy)-propylthio)phenylacetic acid

To a solution of the product of Example 26 (0.030 g, 0.061 mmol) in 0.5 mL of methanol and 0.1 mL of THF was added 5N NaOH (0.06 mL, 5 eq) at ambient temperature. The reaction mixture was heated initially with a heat gun to reflux in order to dissolve the starting material. After heating, the reaction mixture was stirred at rt for 1.5 h. The reaction mixture was diluted with EtOAc and 0.1N HCl aq soln.(5 mL). The organic layer was separated from the aqueous portion, and washed with 0.1N HCl (5 mL) followed by 10 mL brine. The organic layer was dried (MgSO₄), filtered, and evaporated in vacuo to afford the title compound. TLC (80:20:1-CHCl₃:MeOH:NH₄OH).
¹H NMR (400 MHz, CDCl₃, ppm): δ 0.21-0.22 (m, 2H), 0.31-0.34 (m, 2H), 1.04 (m, 1H), 1.18-1.25 (m, 3H), 2.15 (m, 2H), 2.62 (d, *J*=6.59 Hz, 2H), 2.83 (q, *J*=7.61 Hz, 2H), 3.14 (t, *J*=7.32 Hz, 2H), 3.58 (s, 2H), 4.08-4.11 (m, 2H), 6.43 (d, *J*=8.83 Hz, 1H), 7.11-7.14 (m, 1H), 7.22-7.31 (m, 3H).
ESI-MS: *m*/*e* = 478 (M + 1).

### EXAMPLE 28

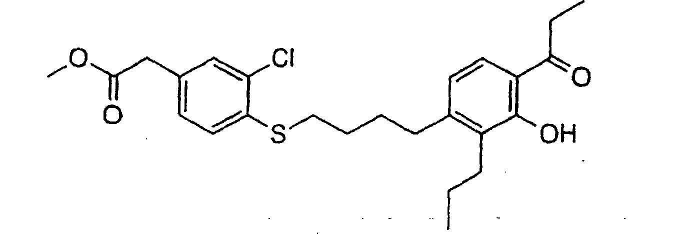

### Methyl 3-chloro-4-(4-(2-propyl-3-hydroxy-4-propionylphenyl)butylthio)-phenylacetate

### Step A: Preparation of 2-hydroxy-3-propyl-4-trifluoromethanesulfonyloxypropiophenone

To a solution of 2,4-dihydroxy-3-propylpropiophenone (2.0 g, 9.62 mmol) in 50 mL of dry DMF was added a 60% dispersion of sodium hydride (0.576 g, 1.5 eq) at 0°C. The reaction mixture was allowed to warm to ambient temperature and was stirred under nitrogen until hydrogen evolution had ceased. *N*-Phenyltrifluoromethanesulfonimide (3.78 g, 10.6 mmol) was added to the reaction mixture which was then stirred at ambient temperature under nitrogen for an additional 12 h. The reaction mixture was partitioned between 10% aq. NaHSO₄ and EtOAc. The organic layer was separated, washed with water and brine, dried (MgSO₄), filtered, and evaporated in vacuo. The residue was purified on a silica gel flash chromatography column eluted with 20% CH₂Cl₂:hexane. Evaporation of the purified fractions and solvent removal in vacuo afforded the product.
¹H NMR (400 MHz, CDCl₃, ppm): δ 0.95 (t, *J*=7.33 Hz, 3H), 1.23 (t, *J*=7.25 Hz, 3H), 1.53-1.61 (m, 2H), 2.66-2.70 (m, 2H), 3.01 (q, *J*=7.16 Hz, 2H), 6.81 (d, *J*=8.99 Hz, 1H), 7.69 (d, *J*=9.07 Hz, 1H).

### Step B: Preparation of 2-hydroxy-4-(4-hydroxy-1-butynyl)-3-propyl-propiophenone

A mixture of the product from Step A (0.117 g, 0.344 mmol), 3-butyn-1-ol (0.036 g, 0.516 mmol), triethylamine (0.18 mL), and bis(triphenylphosphine)palladium(II) chloride (3 mol%) in 1 mL of dry DMF was stirred at 90°C for 2 h under nitrogen. The reaction mixture was cooled to ambient temperature, partitioned between water and EtOAc. The organic portion was separated, washed with water and brine, dried (MgSO₄), filtered, and evaporated in vacuo. The residue was purified on a silica gel flash chromatography column eluted with 20% EtOAc:Hexane. Evaporation of the purified fractions and solvent removal in vacuo afforded the product.
¹H NMR (400 MHz, CDCl₃, ppm): δ 0.95 (t, *J*=7.45 Hz, 3H), 1.21 (t, *J*=7.29 Hz, 3H), 1.52-1.62 (m, 2H), 1.72 (t, 1H), 2.72-2.81 (m, 4H), 3.00 (q, *J*=7.29 Hz, 2H), 3.83 (q, *J*=6.35 Hz, 2H), 6.88 (d, *J*=8.38 Hz, 1H), 7.52 (d, *J*=8.38 Hz, 1H).
CI-MS: *m*/*e* = 261 (M + 1).

### Step C: Preparation of 2-hydroxy-4-(4-hydroxybutyl)-3-propylpropiophenone

A mixture of product from Step B (0.108 g, 0.415 mmol) and 10% palladium on carbon catalyst in 5 mL of ethanol was hydrogenated for 2 h at 45 psi H₂. The reaction mixture was filtered through celite filter cake, then filtered again through a pad of silica gel. The filtrate was evaporated in vacuo to afford a white solid which was used in the next step without further purification.
¹H NMR (400 MHz, CDCl₃, ppm): δ 0.98 (t, *J*=7.24 Hz, 3H), 1.19-1.22 (m, 3H), 1.50-1.56 (m, 4H), 1.62-1.65 (m, 4H), 2.60-2.66 (m, 4H), 2.96-3.01 (m, 2H), 3.66-3.67 (m, 2H), 6.68 (d, *J*=8.30 Hz, 1H), 7.52 (d, *J*=8.30 Hz).
CI-MS: *m*/*e* = 265 (M + 1).

### Step D: Preparation of 4-(2-propyl-3-hydroxy-4-propionylphenyl)butyl bromide

To a solution of the product from Step C (0.522 g, 1.98 mmol) and triphenylphosphine (0.622 g, 2.37 mmol) in 10 mL of CH₂Cl₂ at 0°C under nitrogen was slowly added NBS (0.422 g, 2.37 mmol). The reaction mixture was stirred at 0°C for 15 min. and then 2 h at rt. The solvent was evaporated in vacuo, and the residue was purified on a silica gel flash chromatography column eluted with 20% EtOAc:hexane. Evaporation of the purified fractions in vacuo afforded the product.
¹H NMR (400 MHz, CDCl₃, ppm): δ 0.98 (t, *J*=7.36 Hz, 3H), 1.19-1.23 (m, 3H), 1.50-1.56 (m, 2H), 1.69-1.74 (m, 2H), 1.90-1.94 (m, 2H), 2.60-2.65 (m, 4H), 2.96-3.01 (m, 2H), 3.40-3.43 (m, 2H), 6.67 (d, *J*=8.30 Hz, 1H), 7.53 (d, *J*=8.34 Hz, 1H).
CI-MS: *m*/*e* = 327 (M + 1).

### Step E: Preparation of methyl 3-chloro-4-(4-(2-propyl-3-hydroxy-4-propionylphenyl)butylthio)phenylacetate

To a solution of methyl 3-chloro-4-[(*N,N*-dimethylcarbamoyl)thio]phenylacetate (0.855 g, 2.65 mmol) in 3 mL of methanol was added sodium methoxide (5.3 mL of 0.5M soln., 1.5 eq.) slowly at ambient temperature under nitrogen. The reaction mixture was stirred at ambient temperature for 40 min. The product (0.577 g. 1.77 mmol) of Step D was added to the reaction mixture, and the mixture was heated to reflux for 2 h. The reaction was cooled to ambient temperature, queched with pH=4 phosphate buffer (20 mL), and partitioned with ethyl acetate. The aqueous portion was separated, and the organic portion was washed with brine (2 x 20 mL), dried (MgSO₄), filtered, and evaporated in vacuo. The residue was purified on a silica gel flash chromatography column eluted with 20% EtOAc:hexane. Evaporation of the purified fractions and solvent removal in vacuo afforded the product.
¹H NMR (400 MHz, CDCl₃, ppm): δ 0.97 (t, *J*=7.40 Hz, 3H), 1.21 (t, *J*=7.32 Hz, 3H), 1.53 (m, 3H), 1.72-1.74 (m, 4H), 2.59-2.63 (m, 4H), 2.93-2.99 (m, 4H), 3.55 (s, 2H), 3.68 (s, 3H), 6.66 (d, *J*=8.30 Hz, 1H), 7.12-7.28 (m, 3H), 7.52 (d, *J*=8.30 Hz, 1H).
CI-MS: *m*/*e* = 463 (M + 1).

### EXAMPLE 29

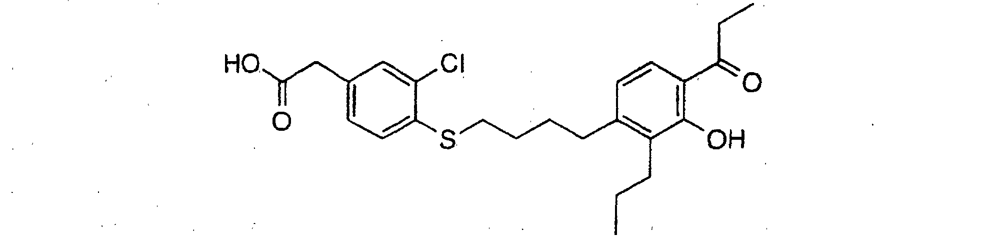

### 3-Chloro-4-(4-(2-propyl-3-hydroxy-4-propionylphenyl)-butylthio)phenyl-acetic acid

### Preparation of 3-chloro-4-(4-(2-propyl-3-hydroxy-4-propionylphenyl)butylthio)phenylacetic acid

To a solution of the product of Example 28 (0.024 g, 0.051 mmol) in 1.0 mL of methanol was added 5N NaOH soln (0.05 mL, 5 eq) at ambient temperature. The reaction mixture was initially heated with a heat gun to reflux in order to dissolve the starting material. After heating, the reaction mixture was then stirred at ambient temperature for 1 h. The reaction mixture was diluted with EtOAc and 0.1N HCl (5 mL). The organic layer was separated from the aqueous portion, and washed with 0.1N HCl (5 mL) followed by 10 mL brine. The organic layer was dried (MgSO₄), filtered, and evaporated in vacuo to afford the title compound. TLC (80:20:1-CHCl₃:MeOH:NH₄OH).
¹H NMR (400 MHz, CD₃OD, ppm): δ 0.96 (t, *J*=7.29 Hz, 3H), 1.18 (t, *J*=7.33 /hz, 3H), 1.49-1.54 (m, 2H), 1.71-1.73 (m, 4H), 2.61-2.67 (m, 4H), 2.96-3.06 (m, 4H), 3.55(s, 2H), 6.72 (d, *J*=8.35 Hz, 1H), 7.14-7.31 (m, 3H), 7.63 (d, *J*=8.35 Hz, 1H).
CI-MS: *m*/*e* = 449 (M + 1).

### EXAMPLE 30

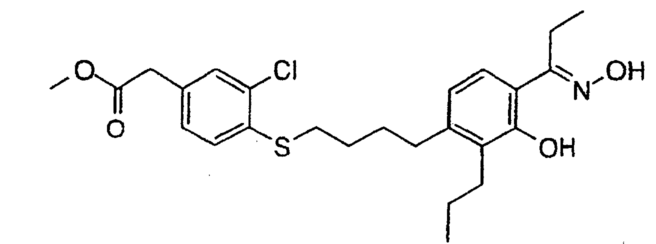

### Methyl 3-chloro-4-(4-(2-propyl-3-hydroxy-4-(1-hydroxyiminopropyl)-phenyl)butylthio)phenylacetate

### Preparation of methyl 3-chloro-4-(4-(2-propyl-3-hydroxy-4-(1-hydroxyiminopropyl)phenyl)butylthio)phenylacetate

To a suspension of the product of Example 28 (0.422 g, 0.913 mmol) in 5 mL of methanol was added hydroxyamine-HCl (0.317 g, 4.57 mmol) and sodium acetate (0.375 g, 4.57 mmol). The reaction mixture was heated to reflux for 4 h. The reaction mixture was allowed to cool to ambient temperature, and partitioned between EtOAc and pH=7 phosphate buffer. The organic layer was separated from the aqueous layer and washed with pH=7 buffer and brine. The organic portion was dried (MgSO₄), filtered, and evaporated in vacuo. The residue was purified on a silica gel flash chromatography column eluted with 1% MeOH/CHCl₃. Evaporation of the purified fractions and solvent removal in vacuo afforded the product.
¹H NMR (400 MHz, CDCl₃, ppm): δ 0.98 (t, *J*=7.40 Hz, 3H), 1.20 (t, *J*=7.69, Hz, 3H), 1.54 (m, 2H), 1.73-1.75 (m, 4H), 2.60-2.63 (m, 4H), 2.84 (q, *J*=7.65 Hz, 2H), 2.92 (m, 2H), 3.54 (s, 2H), 3.68 (s, 3H), 6.69 (d, *J*=8.30 Hz, 1H), 7.00-7.28 (m, 4H).
CI-MS: *m*/*e* = 478 (M + 1).

### EXAMPLE 31

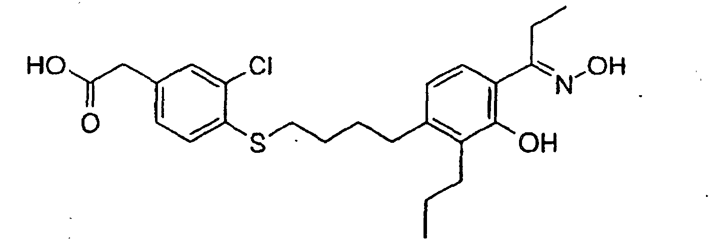

### 3-chloro-4-(4-(2-propyl-3-hydroxy-4-(1-hydroxyiminopropyl)-phenyl)butylthio)phenylacetic acid

### Preparation of 3-chloro-4-(4-(2-propyl-3-hydroxy-4-(1-hydroxyiminopropyl)phenyl)butylthio)phenylacetic acid

To a solution of the product of Example 30 (0.021 g, 0.044 mmol) in 0.5 mL of methanol was added 5N NaOH soln (0.04 mL, 5 eq) at rt. The reaction mixture was heated initially with a heat gun to reflux in order to dissolve the starting material. After heating, the reaction mixture stirred at ambient temperature for 1 h. The reaction mixture was diluted with EtOAc and 0.1N HCl (5 mL). The organic layer was separated from the aqueous portion, and washed with 0.1N HCl (5 mL) followed by 10 mL brine. The organic layer was dried (MgSO₄), filtered, and evaporated in vacuo to afford the title compound. TLC: (80:20:1-CHCl₃:MeOH:NH₄OH).
¹H NMR (400 MHz, CD₃OD, ppm): δ 0.96 (t, *J*=7.42 Hz, 3H), 1.16 (t, *J*=7.65 Hz, 3H), 1.49-1.55 (m, 2H), 1.70-1.72 (m, 4H), 2.59-2.64 (m, 4H), 2.84 (q, *J*=7.65 Hz, 2H), 2.94-2.98 (m, 2H), 3.55 (s, 2H), 6.67 (d, *J*=8.25 Hz, 1H), 7.13-7.31 (m, 4H).
ESI-MS: *m*/*e* = 464 (M + 1).

### EXAMPLE 32

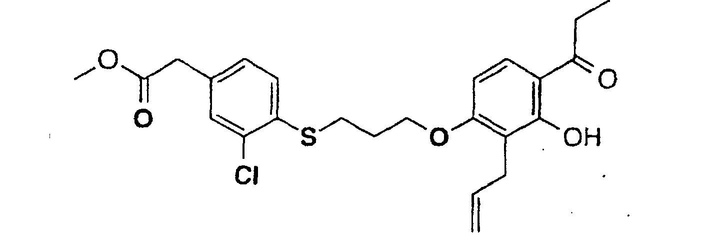

### Methyl 3-chloro-4-(3-(2-(2-propenyl)-3-hydroxy-4-propionylphenoxy)-propylthio)phenylacetate

### Step A: Preparation of 1-bromo-3-(2-(2-propenyl)-3-hydroxy-4-propionylphenoxy)propane

2,4-dihydroxy-3-(2-propenyl)phenyl ethyl ketone (1.05 g, 5.09 mmol) was dissolved in dry methyl ethyl ketone (15 mL), potassium carbonate (0.75 g, 15.3 mmol) and 1,3-dibromopropane (1.55 mL, 15.3 mmol) were added. The reaction mixture protected by nitrogen was stirred and refluxed overnight. pH 4 buffer solution was added, and the mixture was extracted with isopropyl acetate. The extracts were washed with water, and dried with sodium sulfate. The solvent was removed under reduced pressure, and the residue was purified by chromatography (silica gel, 1/1 methylene chloride / hexane) to afford the title compound.
¹H NMR( 400MHz, CDCl₃): δ 7.64(d,1H, J= 8.95 Hz), 6.45(d, 1H, J= 9.0 Hz), 5.95-6.86(ddt, 1H), 5.00-4.92(mult, 2H), 4.17(t, 2H, J= 5.70 Hz), 3.59(t, 2H, J= 6.35 Hz), 3.38(d, 2H, J= 1.51 Hz), 2.95(quart. 2H, J= 7.36 Hz), 2.31 (quint, 2H, J= 5.70, 6.35 Hz), 1.21 (t, 3H, J= 7.32 Hz)

### Step B: Preparation of methyl 3-chloro-4-(3-(2-(2-propenyl)-3-hydroxy-4-propionylphenoxy)propylthio)phenylacetate

To a solution of methyl 3-chloro-4-dimethylcarbamoylthiobenzeneacetate (0.84 g, 2.92 mmol) and methanol (7.34 mL), was added 4.37M sodium methoxide (0.87 mL, 3.80 mmol). The reaction mixture was refluxed for 2 hr, was then allowed to cool to 50°C. 1-bromo-3-(2-(2-propenyl)-3-hydroxy-4-propionylphenoxy)propane (Step A; 0.8 g, 2.43 mmol) was added , and the mixture was stirred at 50°C for 2.5 hr. Aqueous buffer solution (pH 4) was added, and the mixture was extracted with isopropyl acetate. The extracts were washed with water, and dried with sodium sulfate. The solvent was removed under reduced pressure, and the residue was purified by chromatography (silica gel, 1/1 methylene chloride / hexane) to afford the title compound.
¹H NMR( 400MHz, CDCl₃): δ 7.62(d, 1H, J= 8.95 Hz), 7.30-7.26(mult,2H), 7.1 1(dd,1H, J= 8.05 Hz), 6.41(d, 1H, J= 8.99 Hz), 5.90-5.20(mult,1H), 5.00-4.92(mult, 2H), 4.16(t, 2H, J= 5.77 Hz), 3.68(s, 3H), 3.54(s, 2H), 3.41(d, 2H, J= 4.60 Hz), 3.12(t, 2H, J= 7.12 Hz), 2.94(quant, 2H, J= 7.37 Hz), 2.14(quint, 2H, J= 5.77, 7.12 Hz), 1.11(t, 3H, J= 7.32 Hz).

### EXAMPLE 33

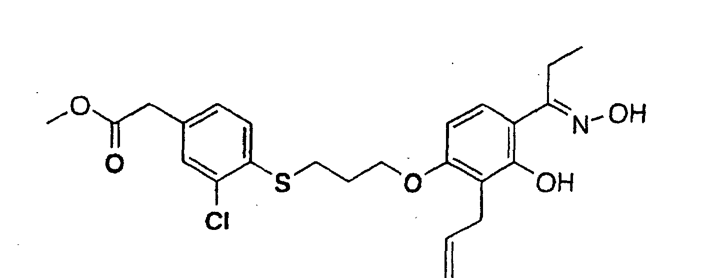

### Methyl 3-chloro-4-(3-(2-(2-propenyl)-3-hydroxy-4-(1-hydroxyimuiopropyl)-phenoxy)propylthio)phenylacetate

A solution of methyl 3-chloro-4-(3-(2-(2-propenyl)-3-hydroxy-4-propionylphenoxy)propylthio)phenylacetate (Example 32; 0.40 g, 0.865 mmol), hydroxylamine hydrochloride (0.30 g, 4.32 mmol) and sodium acetate (0.36 g, 4.32 mmol) in methanol (4 mL) was refluxed for 5 hr. The reaction mixture was then poured into pH 7 buffer solution and extracted with isopropyl acetate. The extracts were washed with water, and dried with sodium sulfate. The solvent was removed under reduced pressure, and the residue was purified by chromatography (silica gel, 1/1 methylene chloride / hexane) to afford the title compound.
¹H NMR( 400MHz, CDCl₃): δ 7.11-7.09(mult, 3H), 7.10(dd, 1H, J= 8.06 Hz), 6.43(d, 1H, J= 9.23 Hz), 6.00-5.87(mult, 1H), 5.15-4.90(mult, 2H), 4.09(t, 2H, J= 5.58 Hz), 3.68(s, 3H), 3.54(s, 2H), 3.44(d, 2H, J= 6.02 Hz), 3.11(t, 2H, J= 6.52 Hz), 2.82(quat, 2H, J= 7.29 Hz), 2.12(quint, 2H, J= 5.58, 6.52 Hz), 1.20(t, 3H, J= 7.27Hz).

### EXAMPLE 34

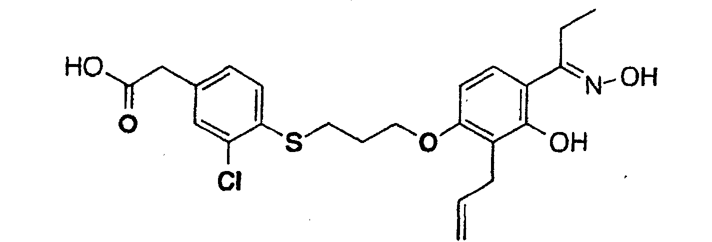

### 3-Chloro-4-(3-(2-(2-propenyl)-3-hydroxy-4-(1-hydroxyiminopropyl)-phenoxy)propylthio)phenylacetic acid

A solution of methyl 3-chloro-4-(3-(2-(2-propenyl)-3-hydroxy-4-(1-hydroxyiminopropyl)phenoxy)propylthio)phenylacetate (Example 33; 20 mg, 0.04 mmol) and 1.299 M lithium hydroxide (0.06 mL, 0.08 mmol) in methanol (1 mL) was refluxed for 1 hr. The reaction mixture was then taken up in pH 4 buffer solution and extracted with isopropyl acetate. The extracts were washed with water, dried with sodium sulfate and concentrated under reduced pressure to afford the title compound.
¹H NMR( 400MHz, CDCl₃): δ 7.11-7.09(mult, 3H), 7.10(dd, 1H, J= 8.02 Hz), 6.43(d, 1H, J= 9.25 Hz), 6.00-5.87(mult, 1H), 5.15-4.90(mult, 2H), 4.09(t, 2H, J= 5.56 Hz), 3.54(s, 2H), 3.44(d, 2H, J= 6.01 Hz), 3.11(t, 2H, J= 6.50 Hz), 2.82(quat, 2H, J= 7.31 Hz), 2.12(quint, 2H, J= 5.56, 6.50 Hz), 1.18(t, 3H, J= 7.30 Hz)

### EXAMPLE 35

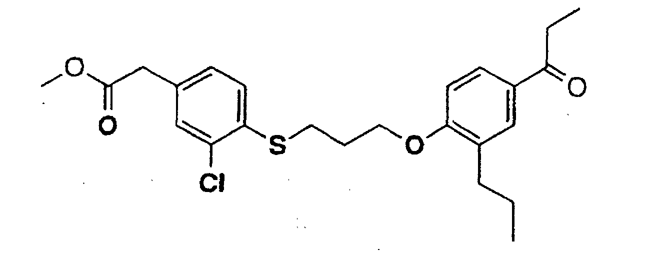

### Methyl 3-chloro-4-(3-(2-propyl-4-propionylphenoxy)propylthio)phenyl-acetate

### Step A: Preparation of 4-propionyl-2-propylphenol

Using the method for preparation of 3-propyl-2,4-dihydroxy-propiophenone but substituting 4-hydroxypropiophenone for 2,4-dihydroxypropiophenone, the title compound was obtained.
¹H NMR( 400MHz, CDCl₃): δ 7.79(S, 1H), 7.72(dd, 1H), 6.83(d, 1H), 6.12(S, 1H), 2.95(quart, 2H),2.61(t, 2H), 1.68(hex, 2H), 1.22(t, 3H), 0.98(t, 3H);

### Step B: Preparation of 1-bromo-3-(2-propyl-4-propionylphenoxy)-propane

Using the method for preparation of 1-bromo-3-(2-propyl-3-hydroxy-4-propionylphenoxy)propane (Example 1, Step A) but substituting 4-propionyl-2-propylphenol (Step A) for 2,4-dihydroxy-3-propyl-propiophenone, the title compound was obtained.
¹H NMR( 400MHz, CDCl₃): δ 7.80(dd, 1H), 7.77(S, 1H), 6.84(d, 1H), 4.14(t, 2H), 3.60(t, 2H), 2.92(quart, 2H), 2.58(t, 2H), 2.32(hex, 2H), 1.61(quint, 2H), 1.19(t, 3H), 0.92(t, 3H);

### Step C: Preparation of methyl 3-chloro-4-(3-(2-propyl-4-propionyl-phenoxy)propylthio)phenylacetate

Using the method described in Step B of Example 1 but substituting 1-bromo-3-(2-propyl-4-propionylphenoxy)propane for 1-bromo-3-(2-propyl-3-hydroxy-4-propionylphenoxy)propane, the title compound was obtained.
¹H NMR( 400MHz, CDCl₃): δ 7.80-7.75(mult, 2H), 7.29-7.24(mult, 2H), 7.11(dd, 1H, J= 8.11 Hz), 6.80(d, 1H, J= 8.4 Hz), 4.13(t, 2H, J= 5.70 Hz), 3.67(s, 3H), 3.54(s, 2H), 3.13(t, 2H, J= 7.08 Hz), 2.92(quart, 2H, J= 7.24 Hz), 2.61(t, 2H, 7.77 Hz), 2.18(quint, 2H, J= 5.70, 7.08 Hz), 1.61(hex, 2H, J= 7.40, 7.77 Hz), 1.19(t, 3H, J= 7.24 Hz), 0.92(t, 3H, J= 7.40 Hz).

### EXAMPLE 36

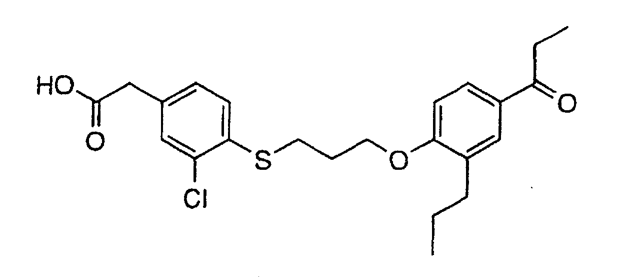

### 3-Chloro-4-(3-(2-propyl-4-propionylphenoxy)propylthio)phenylacetic acid

Using the method described in Example 34 but substituting methyl 3-chloro-4-(3-(2-propyl-4-propionylphenoxy)propylthio)phenylacetate (Example 35) for methyl 3-chloro-4-(3-(2-(2-propenyl)-3-hydroxy-4-(1-hydroxyiminopropyl)phenoxy)propylthio)phenylacetate (Example 33), the title compound was obtained.
¹H NMR( 400MHz, CDCl₃): δ 7.80-7.75(mult, 2H), 7.30-7.24(mult, 2H), 7.11(dd, 1H, J= 8.11 Hz), 6.80(d, 1H, J= 8.44 Hz), 4.13(t, 2H, J= 5.85 Hz), 3.57(s, 2H), 3.13(t, 2H, J= 7.03 Hz), 2.92(quart, 2H, J= 7.34 Hz), 2.61(t, 2H, 7.50 Hz), 2.17(quint, 2H, J= 5.85, 7.03 Hz), 1.61(hex, 2H, J= 7.32, 7.50 Hz), 1.20(t, 3H, J= 7.32 Hz), 0.92(t, 3H, J= 7.32 Hz). MS: 438.2, 435.1(M+1), 416.1, 391.2, 364.1, 279.1, 241.4, 233.1(base peak), 203.1, 177.1, 147.1, 133.1.

### EXAMPLE 37

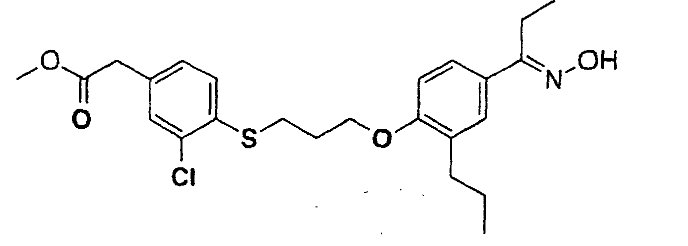

### Methyl 3-chloro-4-(3-(2-propyl-4-(1-hydroxyiminopropyl)phenoxy)propyl-thio)phenylacetate

Using the method described in Example 33 but substituting methyl 3-chloro-4-(3-(2-propyl-4-propionylphenoxy)propylthio)phenylacetate (Example 35) for methyl 3-chloro-4-(3-(2-(2-propenyl)-3-hydroxy-4-propionylphenoxy)propylthio)phenylacetate (Example 32), the title compound was obtained.
¹H NMR( 400MHz, CDCl₃): δ 7.30-7.22(mult, 4H), 7.01(dd, 1H, J= 8.10 Hz), 6.67(d, 1H, J= 9.15 Hz), 4.12(t, 2H, J= 5.84 Hz), 3.61(s, 3H), 3.55(s, 2H), 3.14(t, 2H, J= 7.1 Hz), 2.83(quart, 2H, J= 7.70 Hz), 2.55(t, 2H,J= 7.65 Hz), 2.13(quint, 2H, J= 5.86, 7.00 Hz), 1.68(hex, 2H, J= 6.30, 7.65 Hz), 1.26(t, 3H, J= 7.65 Hz), 0.91(t, 3H, J= 6.30 Hz).

### EXAMPLE 38

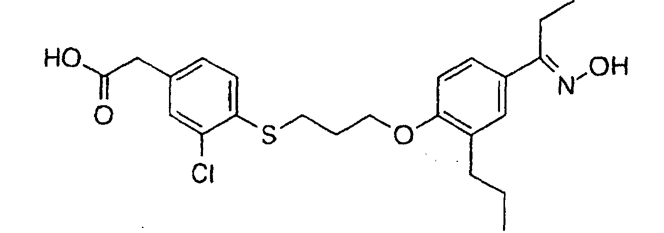

### 3-Chloro-4-(3-(2-propyl-4-(1-hydroxyiminopropyl)phenoxy)propylthio)-phenylacetic acid

Using the method described in Example 34 but substituting methyl 3-chloro-4-(3-(2-propyl-4-(1-hydroxyiminopropyl)phenoxy)propylthio)-phenylacetate (Example 37) for methyl 3-chloro-4-(3-(2-(2-propenyl)-3-hydroxy-4-(1-hydroxyiminopropyl)phenoxy)propylthio)phenylacetate (Example 33), the title compound was obtained.
¹H NMR( 400MHz, CDCl₃): δ 7.31-7.23(mult, 4H), 7.03(dd, 1H, J= 8.13 Hz), 6.69(d, 1H, J= 9.15 Hz), 4.11(t, 2H, J= 5.87 Hz), 3.55(s, 2H), 3.14(t, 2H, J= 7.0 Hz), 2.81(quart, 2H, J= 7.70 Hz), 2.55(t, 2H,J= 7.65 Hz), 2.13(quint, 2H, J= 5.86, 7.00 Hz), 1.68(hex, 2H, J= 6.30, 7.65 Hz), 1.26(t, 3H, J= 7.65 Hz), 0.91(t, 3H, J= 6.30 Hz).
MS: 434.2(M+1), 432.1, 405.1, 392.3, 391.3, 388.0, 369.2, 343.0, 329.3, 301.3, 287.0, 279.1, 183.1 (base peak).

### EXAMPLE 39

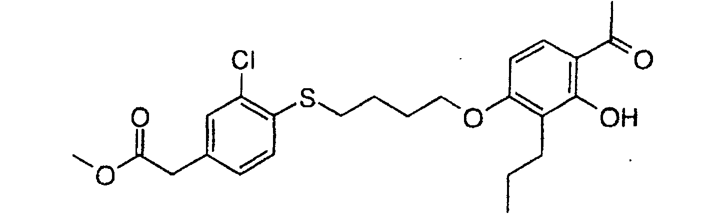

### Methyl 3-chloro-4-(4-(2-propyl-3-hydroxy-4-acetylphenoxy)butylthio)-phenylacetate

### Step A: Preparation of 1-bromo-4-(2-propyl-3-hydroxy-4-acetylphenoxy)butane

To a solution of 2,4-dihydroxy-3-propylacetophenone (5.0 g, 25.74 mmol) in 2-butanone (250 mL) was added K₂CO₃ (4.3 g. 30.9 mmol) and 1,4-dibromobutane (9.8 mL, 82.4 mmol). The reaction was heated to reflux until TLC analysis indicated that the reaction was complete. The reaction was cooled to room temperature and diluted with ethyl acetate washed with 2N HCl, brine, dried (MgSO₄), filtered and concentrated. Column chromatography (6:1 hexanes:ethyl acetate) of the residue gave the title compound.

### Step B: Preparation of methyl 3-chloro-4-(4-(2-propyl-3-hydroxy-4-acetylphenoxy)butylthio)phenylacetate

To a solution of the 3-chloro-4-(dimethylcarbamoylthio)-phenylacetic acid methyl ester (200 mg, 0.69 mmol) in anhydrous methanol (5.0 mL) was added sodium methoxide (0.21 mL of a 4.37 molar solution in CH₃OH). The reaction was heated to reflux until TLC analysis indicated the complete consumption of the starting material. To this recrion was then added 1-bromo-4-(2-propyl-3-hydroxy-4-acetylphenoxy)butane (329.2 mg, 1.04 mmol) in CH₃OH (2x1 mL). The reaction was stirred at reflux for 20 minutes, cooled to room temperature and diluted with ether. The mixture was washed with 2N HCl, dried (MgSO4), filtered and the filtrate concentrated. Column chromatography (4:1 hexanes:ethyl acetate) of the residue gave the title compound.
MS(CI) 465.1 (M+H)

### EXAMPLE 40

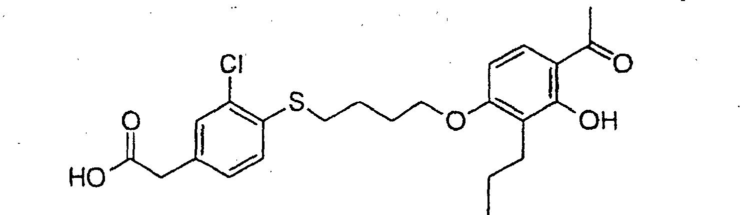

### 3-Chloro-4-(4-(2-propyl-3-hydroxy-4-acetylphenoxy)butylthio)phenylacetic acid

To a solution of methyl 3-chloro-4-(4-(2-propyl-3-hydroxy-4-acetylphenoxy)butylthio)phenylacetate (Example 39; 100 mg) in CH₃OH:H₂O:THF (6 mL:2 mL:2 mL) was added lithium hydroxide (176 mg). The reaction was stirred at room temperature for 2 hours. The reaction was then diluted with ethyl acetate and washed with 2N HCl, brine, dried (MgSO₄), filtered and the filtrate was concentrated to give the title compound.
MS(CI) 451.2 (M+H)

### EXAMPLE 41

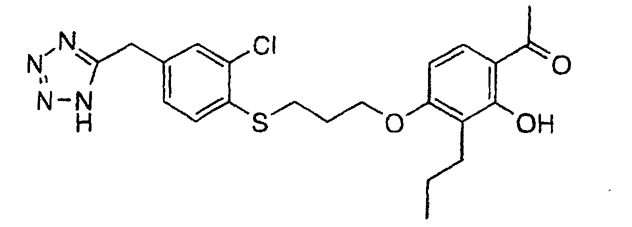

### 3-Chloro-4-(3-(2-propyl-3-hydroxy-4-acetyl)phenoxy)propylthiophenyl-5-methyl-(1H)-tetrazole

### Step A: Preparation of 3-chloro-4-(dimethylcarbamoylthio)phenylaceto-nitrile

Trichloroethane (30mL) was cooled to 0°C and saturated with ammonia (g) for approximately 30 minutes. To this solution was added Al(CH₃)₃ (26.06 mL, of a 2 molar solution in toluene, 52.16 mmol). The reaction was slowly heated to 80°C for 1 hour whereupon 3-chloro-4-(dimethylcarbamoylthio)phenylacetic acid methyl ester (6.0 g, 20.85 mmol) in trichloroethane (10 mL) was added dropwise. The reaction was then heated to 120°C for 1.5 hours, cooled to room temperature and diluted with ether. The mixture was then washed with 2N HCl, dried (MgSO₄), filterd and the filtrate was concentrated. Column chromatography (3:1 hexanes:ethyl acetate) of the residue gave the title compound.

### Step B: Preparation of 3-chloro-4-(3-(2-propyl-3-hydroxy-4-acetyl-phenoxy)propylthio)phenylacetonitrile

To a solution of 3-chloro-4-(dimethylcarbamoylthio)-phenyl-acetonitrile (Step A; 127.8 mg, 0.50 mmol) in anhydrous methanol (3.0 mL) was added sodium methoxide (0.15 mL of a 4.37 molar solution in CH₃OH, 0.65 mmol). The reaction was heated to reflux until TLC analysis indicated the complete consumption of the starting material. To this rection was then added 1-bromo-3-(2-propyl-3-hydroxy-4-acetylphenoxy)propane (189 mg, 0.6 mmol) in CH₃OH (1 mL). The reaction was stirred at reflux for 20 minutes, cooled to room temperature and diluted with ether. The mixture was washed with 2N HCl, dried (MgSO₄), filtered and the filtrate concentrated. Radial chromatography (2 mm plate; 2:1 hexanes:ethyl acetate) of the residue gave the title compound.
MS (CI) 418.1 (M+H)

### Step C: Preparation of 3-chloro-4-(3-(2-propyl-3-hydroxy-4-acetyl-phenoxy)propylthio)phenyl-5-methyl-(1H)-tetrazole

To solution of the nitrile prepared in Step B of this example (90 mg, 0.22 mmol) in DMF (2.0 mL)was added NH₄Cl (115.3 mg, 2.15 mmol) and NaN₃ (140.1 mg, 2.15 mmol). The reaction was heated to 130°C overnight. The following morning the reaction was cooled to room temperature and diluted with CH₂Cl₂, washed with 2N HCl, H₂O, brine, dried (MgSO₄), filtered and concentrated. Chromatography (10:1 CHCl₃:CH₃OH) of the residue gave the title compound.
MS (CI) 461.1 (M+H)

### EXAMPLE 42

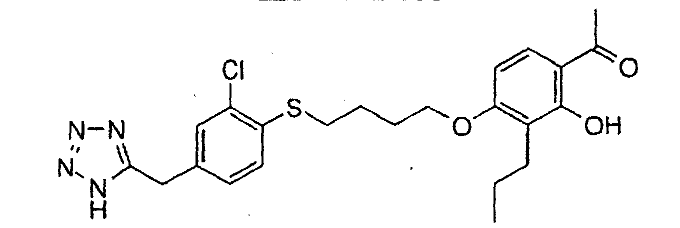

### 3-Chloro-4-(4-(2-propyl-3-hydroxy-4-acetylphenoxy)butylthio)phenyl-5-methyl-(1H)-tetrazole

### Step A: Preparation of 3-chloro-4-(4-(2-propyl-3-hydroxy-4-acetyl-phenoxy)butylthio)phenylacetonitrile

To a solution of 3-chloro-4-(dimethylcarbamoylthio)-phenylaceto-nitrile (Example 41, Step A; 123 mg, 0.48 mmol) in anhydrous methanol (5.0 mL) was added sodium methoxide (0.16 mL of a 4.37 molar solution in CH₃OH, 0.73 mmol). The reaction was heated to reflux until TLC analysis indicated the complete consumption of the starting material. To this rection was then added the 1-bromo-4-(2-propyl-3-hydroxy-4-acetylphenoxy)butane (Example 39, Step A; 200 mg, 0.61 mmol) in CH₃OH (1 mL). The reaction was stirred at reflux for 20 minutes, cooled to room temperature and diluted with ether. The mixture was washed with 2N HCl, dried (MgSO₄), filtered and the filtrate concentrated. Radial chromatography (2 mm plate; 2:1 hexanes:ethyl acetate) of the residue gave the title compound.
MS (CI) 432.2 (M+H)

### Step B: Preparation of 3-chloro-4-(4-(2-propyl-3-hydroxy-4-acetyl-phenoxy)butylthio)phenyl-5-methyl-(1H)-tetrazole

To solution of 3-chloro-4-(4-(2-propyl-3-hydroxy-4-acetylphenoxy)butylthio)phenylacetonitrile (Step A; 100 mg, 0.23 mmol) in DMF (2.0 mL) was added NH₄Cl (123.6 mg, 2.31 mmol) and NaN₃ (150.2 mg, 2.31 mmol). The reaction was heated to 130°C overnight. The following morning the reaction was diluted with CH₂Cl₂, washed with 2N HCl, H₂O, brine, dried (MgSO₄), filtered and concentrated. Chromatography (10:1 CHCl₃:CH₃OH) of the residue gave the title compound.
MS (CI) 475.1 (M+H)

### EXAMPLE 43

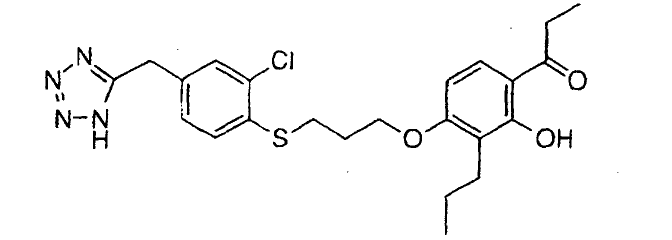

### 3-Chloro-4-(3-(2-propyl-3-hydroxy-4-propionylphenoxy)propylthio)-phenyl-5-methyl-(1H)-tetrazole

### Step A: Preparation of 3-chloro-4-(3-(2-propyl-3-hydroxy-4-propionylphenoxy)propylthio)phenylacetonitrile

To a solution of 3-chloro-4-(dimethylcarbamoylthio)-phenylaceto-nitrite (Example 41, Step A; 470 mg, 1.84 mmol) in anhydrous methanol (10.0 mL) was added sodium methoxide (0.54 mL of a 4.37 molar solution in CH₃OH, 2.35 mmol). The reaction was heated to reflux until TLC analysis indicated the complete consumption of the starting material. To this rection was then added 1-bromo-3-(2-propyl-3-hydroxy-4-propioyl-phenoxy)propane (Example 1, Step A; 700 mg, 2.05 mmol) in CH₃OH (5 mL). The reaction was stirred at reflux for 20 minutes, cooled to room temperature and diluted with ether. The mixture was washed with 2N HCl, dried (MgSO₄), filtered and the filtrate concentrated. Column chromatography (4:1 hexanes:ethyl acetate) of the residue gave the title compound.
MS (CI) 432.2 (M+H)

### Step B: Preparation of 3-chloro-4-(3-(2-propyl-3-hydroxy-4-propionyl-phenoxy)propylthio)phenyl-5-methyl-(1H)-tetrazole

To solution of 3-chloro-4-(3-(2-propyl-3-hydroxy-4-propionyl-phenoxy)propylthio)phenylacetonitrile (Step A; 245 mg, 0.59 mmol) in DMF (10.0 mL) was added NH₄Cl (313 mg, 5.86 mmol) and NaN3 (380 mg, 5.86 mmol). The reaction was heated to 130°C overnight. The following morning the reaction was diluted with CH₂Cl₂, washed with 2N HCl, H₂O, brine, dried (MgSO4), filtered and concentrated. Chromatography (10:1 CHCl₃:CH₃OH) of the residue gave the title compound.
MS (CI) 475.1 (M+H)

### EXAMPLE 44

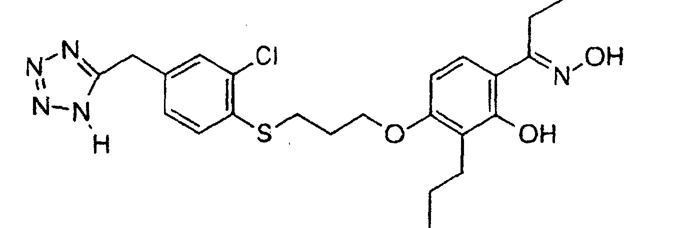

### 3-Chloro-4-(3-(2-propyl-3-hydroxy-4-( 1 -hydroxyiminopropyl)-phenoxy)-propylthio)-phenyl-5-methyl-(1H)-tetrazole

To a solution of 3-chloro-4-(3-(2-propyl-3-hydroxy-4-propionylphenoxy)propylthio)phenyl-5-methyl-(1H)-tetrazole (Example 43; 200 mg, 0.41 mmol) in CH₃OH (10 mL) was added NaOAc (315 mg, 4.1 mmol) and hydroxylamine hydrochloride (284.2 mg, 4.1 mmol). The reaction was stirred at room temperature until complete as determined by TLC analysis. The reaction was then diluted with ethyl acetate, washed with water, brine, dried (MgSO₄), filtered and concetrated to give the title compound.
MS (CI) 490.2 (M+H)

### EXAMPLE 45

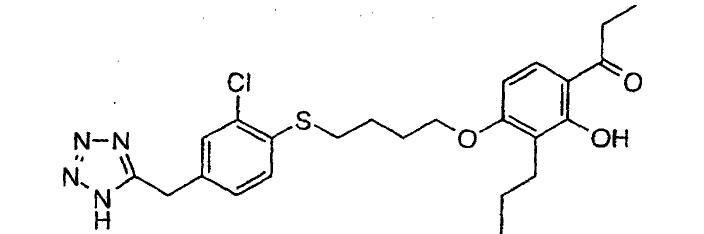

### 3-Chloro-4-(4-(2-propyl-3-hydroxy-4-propionylphenoxy)-butylthio)phenyl-5-methyl-(1H)-tetrazole

### Step A: Preparation of 3-chloro-4-(4-(2-propyl-3-hydroxy-4-propionylphenoxy)butylthio)phenylacetonitrile

To a solution of 3-chloro-4-(dimethylcarbamoylthio)-phenylacetonitrile (Example 41, Step A; 451 mg, 1.77 mmol) in anhydrous methanol (12.0 mL) was added sodium methoxide (0.52 mL of a 4.37 molar solution in CH₃OH, 2.26 mmol). The reaction was heated to reflux until TLC analysis indicated the complete consumption of the starting material. To this rection was then added 1-bromo-4-(2-propyl-3-hydroxy-4-propionyl-phenoxy)butane (prepared using the method described in Example 39, Step A; 700 mg, 1.97 mmol) in CH₃OH (5 mL). The reaction was stirred at reflux for 20 minutes, cooled to room temperature and diluted with ether. The mixture was washed with 2N HCl, dried (MgSO₄), filtered and the filtrate concentrated. Column chromatography (4:1 hexanes:ethyl acetate) of the residue gave the title compound.
MS (CI) 446.2 (M+H)

### Step B: Preparation of 3-chloro-4-(4-(2-propyl-3-hydroxy-4-propionyl-phenoxy)butylthio)phenyl-5-methyl-(1H)-tetrazole

To solution of the nitrile prepared in Step A of this example (450 mg, 1.04 mmol) in DMF (10.0 mL) was added NH₄Cl (556.3 mg, 10.4 mmol) and NaN₃ (676.1 mg, 10.4 mmol). The reaction was heated to 130°C overnight. The following morning the reaction was diluted with CH₂Cl₂, washed with 2N HCl, H₂O, brine, dried (MgSO₄), filtered and concentrated. Chromatography (10:1 CHCl₃:CH₃OH) of the residue gave the title compound.
MS (CI) 489.2 (M+H)

### EXAMPLE 46

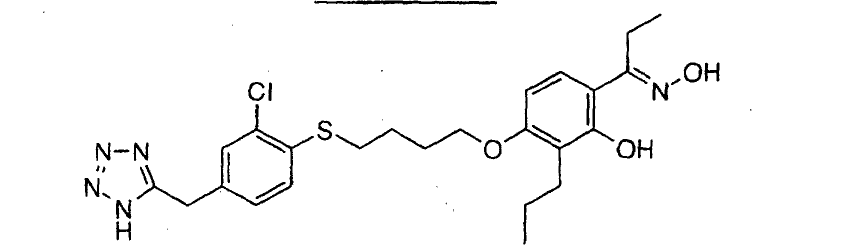

### 3-Chloro-4-(4-(2-propyl-3-hydroxy-4-(1-hydroxyiminopropyl)-phenoxy)-butylthio)phenyl-5-methyl-( 1H)-tetrazole

To a solution of 3-chloro-4-(4-(2-propyl-3-hydroxy-4-propionylphenoxy)butylthio)phenyl-5-methyl-(1H)-tetrazole (Example 45; 75 mg, 0.16 mmol) in CH₃OH (3 mL) was added NaOAc (62.7 mg, 0.81 mmol) and hydroxylamine hydrochloride (56.6 mg, 0.81 mmol). The reaction was stirred at room temperature until complete as determined by TLC analysis. The reaction was then diluted with ethyl acetate, washed with water, brine, dried (MgSO₄), filtered and concetrated to give the title compound.
MS (CI) 506 (M+H)

### EXAMPLE 47

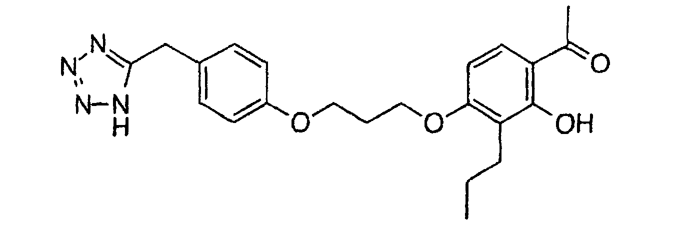

### 4-(3-(2-Propyl-3-hydroxy-4-acetylphenoxy)propyloxy)-phenyl-5-methyl-(1H)-tetrazole

### Step A: Preparation of 4-(3-(2-propyl-3-hydroxy-4-acetylphenoxy)-propyloxy)phenylacetonitrile

To a solution of the 1-bromo-3-(2-propyl-3-hydroxy-4-acetylphenoxy)propane (568 mg, 1.50 mmol) in dry 2-butanone (15.0 mL) was added the p-hydroxyphenylacetonitrile (200 mg, 1.80 mmol) and K₂CO₃ (250 mg, 1.80 mmol). The reaction was heated to reflux for 16 hours whereupon it was diluted with ether. The mixture was washed with 10% HCl, brine, dried (MgSO₄), filtered and concentrated. Column chromatography (4:1 hexane:ethyl acetate) of the residue gave the title compound.
MS (ESI) 368.2 (M+H). ¹HNMR: (CDCl₃; 300 MHz) 7.21 (d,2H), 6.88 (d,2H), 3.67 (s, 2H).

### Step B: Preparation of 4-(3-(2-propyl-3-hydroxy-4-acetylphenoxy)-propyloxy)-phenyl-5-methyl-(1H)-tetrazole

To solution of the nitrile (Step A; 100 mg, 0.27 mmol) in DMF (8.0 mL)was added NH₄Cl (73 mg, 1.36 mmol) and NaN₃ (90 mg, 1.36 mmol). The reaction was heated to 130°C overnight. The following morning the reaction was diluted with EtOAc, washed with 2N HCl, H₂O, brine, dried (MgSO₄), filtered and concentrated. Chromatography (10:1 CHCl₃:CH₃OH) of the residue gave the title compound.
MS (ESI) 411.2 (M+H). ¹HNMR: (CDCl₃; 400 MHz) 7.17 (d,2H), 6.90 (d,2H), 4.22 (s, 2H).

### EXAMPLE 48

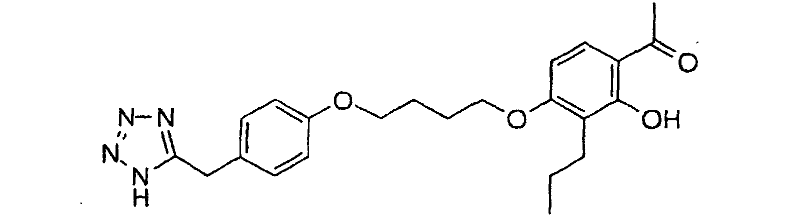

### 4-(4-(2-Propyl-3-hydroxy-4-propionylphenoxy)butyloxy)-phenyl-5-methyl-(lH)-tetrazole

### Step A: Preparation of 4-(4-(2-propyl-3-hydroxy-4-acetylphenoxy)-butyloxy)phenylacetonitrile

To a solution of 1-bromo-4-(2-propyl-3-hydroxy-4-acetylphenoxy)-butane (Example 39, Step A; 250 mg, 0.76 mmol) in dry 2-butanone (12.0 mL) was added the p-hydroxyphenylacetonitrile (84 mg, 0.63 mmol) and K₂CO₃ (105 mg, 0.94 mmol). The reaction was heated to reflux for 16 hours whereupon it was diluted with ether. The mixture was washed with 10% HCl, brine, dried (MgSO₄), filtered and concentrated. Column chromatography (4:1 hexanes:ethyl acetate) of the residue gave the title compound.
MS (ESI) 382.1 (M+H). ¹HNMR: (CDCl₃; 400 MHz) 7.23 (d,2H), 6.89 (d,2H), 3.69 (s, 2H).

### Step B: Preparation of 4-(4-(2-propyl-3-hydroxy-4-acetylphenoxy)-butyloxy)phenyl-5-methyl-(1H)-tetrazole

To solution of the nitrile (Step A; 125 mg, 0.33 mmol) in DMF (8.0 mL)was added NH₄Cl (88 mg, 1.64 mmol) and NaN₃ (107 mg, 1.64 mmol). The reaction was heated to 130°C overnight. The following morning the reaction was diluted with EtOAc, washed with 2N HCl, H₂O, brine, dried (MgSO₄), filtered and concentrated.

Chromatography (10:1 CHCl₃:CH₃OH) of the residue gave the title compound.

MS (CI) 425.2 (M+H). ¹HNMR: (CDCl₃; 400 MHz) 7.16 (d,2H), 6.88 (d,2H), 4.21 (s, 2H).

### EXAMPLE 49

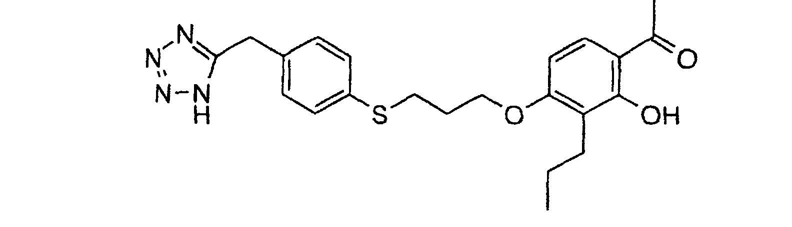

### 4-(3-(2-Propyl-3-hydroxy-4-acetylphenoxy)propylthio)phenyl-5-methyl-(1H)-tetrazole

### Step A: Preparation of 4-(dimethylthiocarbamoyloxy) phenylacetonitrile

A stirred solution of *p*-hydroxyphenylacetonitrile (2 g, 15 mmol) and thiocarbamoyl chloride (2.8 g, 22.5 mmol) in pyridine (35 mL) was heated to 60°C for 2.5 hr. The reaction was diluted with ether and extracted with 10% HCl. The aqueous portion was back extracted twice with ether and the combined ether extracts washed three times with 10% HCl, brine and dried over MgSO₄. The solution was concentrated and purified by column chromatography (SiO₂, 4:1 hexane/EtOAc) to provide the title compound.
MS (CI) 221.1 (M+H). ¹HNMR: (CDCl₃; 400 MHz) 7.34 (d,2H), 7.06 (d,2H), 3.75 (s,2H), 3.44 (s, 3H), 3.34 (s, 3H).

### Step B: Preparation of 4-(dimethylcarbamoylthio) phenylacetonitrile

Under an inert atmosphere of N₂, 4-(dimethylthiocarbamoyloxy)phenylacetonitrile (Step A; 120 mg, 0.54 mmol) was heated to 240C° for 3 hr. The rearranged material was purified by column chromatography (SiO₂, 4:1 hexane/EtOAc) to provide the title compound.
MS (CI) 221.1 (M+H). ¹HNMR: (CDCl₃; 400 MHz) 7.49 (d,2H), 7.33 (d,2H), 3.75 (s,2H), 3.08 (s, 3H), 3.01 (s, 3H).

### Step C: Preparation of 4-(3-(2-propyl-3-hydroxy-4-acetylphenoxy)-propylthio)phenylacetonitrile

To a solution of 4-(dimethylcarbamoylthio)-phenylacetonitrile (Step C; 132 mg, 0.60 mmol) in anhydrous methanol (3.0 mL) was added sodium methoxide (0.18 mL of a 4.37 molar solution in CH₃OH, 0.78 mmol). The reaction was heated to reflux until TLC analysis indicated the complete consumption of the starting material. To this reaction was then added 1-bromo-3-(2-propyl-3-hydroxy-4-acetylphenoxy)propane (283 mg, 0.9 mmol) in CH₃OH (1 mL). The reaction was stirred at reflux for 20 minutes, cooled to room temperature and diluted with ether. The mixture was washed with 2N HCl, dried (MgSO₄), filtered and the filtrate concentrated. Radial chromatography (2 mm plate; 2:1 hexanes:ethyl acetate) of the residue gave the title compound.
MS (CI) 384.2 (M+H). ¹HNMR: (CDCl₃; 400 MHz) 7.33 (d,2H), 7.21 (d,2H), 3.69.

### Step D: Preparation of 4-(3-(2-propyl-3-hydroxy-4-acetylphenoxy)-propylthio)phenyl-5-methyl-(1H)-tetrazole

To solution of the nitrile (Step C; 100 mg, 0.27 mmol) in DMF (8.0 mL)was added NH₄Cl (73 mg, 1.36 mmol) and NaN₃ (90 mg, 1.36 mmol). The reaction was heated to 130°C overnight. The following morning the reaction was diluted with EtOAc, washed with 2N HCl, H₂O, brine, dried (MgSO₄), filtered and concentrated. Chromatography (10:1 CHCl₃:CH₃OH) of the residue gave the title compound.
MS (ESI) 427.2 (M+H). ¹HNMR: (CDCl₃; 400 MHz) 7,24 (d,2H), 7.10 (d,2H), 4.23.

### EXAMPLE 50

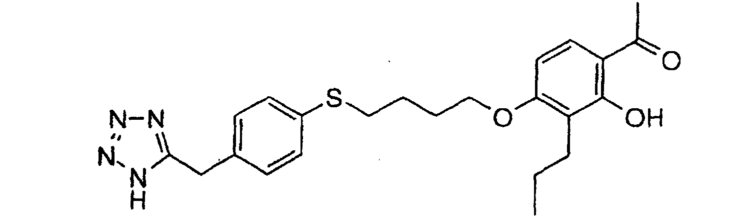

### 3-Chloro-4-(4-(2-propyl-3-hydroxy-4-propionylphenoxy)butylthio)-phenyl-5-methyl-(1H)-tetrazole

### Step A: Preparation of 4-(4-(2-propyl-3-hydroxy-4-acetylphenoxy)-butylthio)phenylacetonitrile

To a solution of 4-(dimethylcarbamoylthio)-phenylacetonitrile (Example 49, Step B; 132 mg, 0.60 mmol) in anhydrous methanol (3.0 mL) was added sodium methoxide (0.18 mL of a 4.37 molar solution in CH₃OH, 0.78 mmol). The reaction was heated to reflux until TLC analysis indicated the complete consumption of the starting material. To this reaction was then added 1-bromo-4-(2-propyl-3-hydroxy-4-acetylphenoxy)butane (Example 37, Step A; 296 mg, 0.9 mmol) in CH30H (1 mL). The reaction was stirred at reflux for 20 minutes, cooled to room temperature and diluted with ether. The mixture was washed with 2N HCl, dried (MgSO₄), filtered and the filtrate concentrated. Radial chromatography (2 mm plate; 2:1 hexanes:ethyl acetate) of the residue gave the title compound.
MS (CI) 398.2 (M+H). ¹HNMR: (CDCl₃; 400 MHz) 7.33 (d,2H), 7.21 (d,2H), 3.69.

### Step B: Preparation of 4-(4-(2-propyl-3-hydroxy-4-acetylphenoxy)-butylthio)phenyl-5-methyl-(1H)-tetrazole

To solution of the nitrile (Step A; 97 mg, 0.24 mmol) in DMF (8.0 mL)was added NH₄Cl (131 mg, 2.44 mmol) and NaN₃ (159 mg, 2.44 mmol). The reaction was heated to 130°C overnight. The following morning the reaction was diluted with EtOAc, washed with 2N HCl, H₂O, brine, dried (MgSO₄), filtered and concentrated. Chromatography (10:1 CHCl₃:CH₃OH) of the residue gave the title compound.
MS (ESI) 441.1 (M+H). ¹HNMR: (CDCl₃; 400 MHz) 7.23 (d,2H), 7.12 (d,2H), 4.27.

### EXAMPLE 51

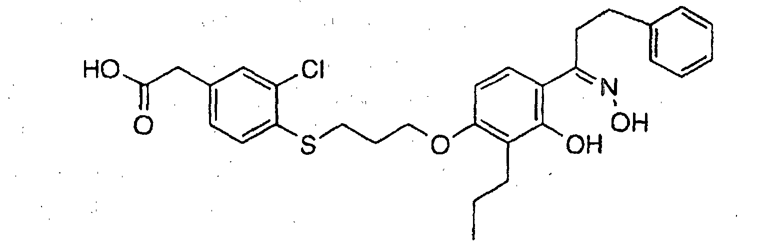

### 3-Chloro-4-(3-(2-propyl-3-hydroxy-4-(1-(hydroxylimino)-3-(phenyl)propyl)phenoxy)propylthio)phenylacetic acid

### Step A: Preparation of methyl 3-chloro-4-(3-bromopropylthio)-phenylacetate

To a solution of methyl 3-chloro-4-dimethylcarbamoylthiophenylacetate (85 g, 0.295 mol) in methanol (250 mL) was added 25% NaOMe in methanol (74 mL, 0.34 mol). The reaction was heated to reflux for 2 h. TLC analysis shows residual starting carbamate. Additional NaOMe/MeOH (10 mL) was added and the mixture stirred an additional 30 min at reflux. After cooling to ambient temperature, the thiolate solution was added dropwise to a solution of 1,3-dibromopropane (120 mL, 1.18 mol) in methanol (250 mL). The resulting solution was refluxed for 3 h then cooled to ambient temperature. After standing overnight, the reaction was quenched by pouring into ice water (2 L). After adjusting to pH 1 with conc. HCl (*ca*. 10 mL), the aqueous was extracted with EtOAc (2 L then 2 x 1 L). The combined organics were washed with water (2 x I L), brine (1 L), dried over anhyd. MgSO₄, filtered, and concentrated. The residue was dissolved in EtOAc/ hexane (1/9) and eluted through a plug of silica gel (70-230 mesh, *ca.* 2 L, packed in EtOAc/hexane, 1/9). The fractions containing product were combined and evaporated to give title compound (48 g, 48% yield) as an off-white solid.
NMR (CDCl₃) δ 7.25-7.32 (m, 2H), 7.15 (dd, 1H, J = 8.1, 1.8 Hz), 3.71 (s, 3H), 3.57 (s, 2H), 3.55 (t, 2H, J = 7.7 Hz), 3.10 (t, 2H, J = 7.7 Hz), 2.18 (m, 2H).

### Step B: Preparation of 4-(3-phenylpropionyl)-2-(n-propyl)resorcinol

To a mixture of 2-(n-propyl)resorcinol (7.71 g, 50 mmoles) and 3-phenylpropionic acid (9.0 g, 60 mmoles) was added trifluoro-methanesulfonic acid (20 ml). After the exotherm subsided and the solids dissolved, the yellow-brown solution was heated at 85° for 2.5 hr. The cooled mixture was poured into a mixture of ice (200 g) and ethyl acetate (100 ml) with vigorous stirring. The aqueous phase was extracted twice with ethyl acetate. The combined ethyl acetate phases were washed with water, 10% NaHCO₃, water, and saturated NaCI solution and dried (MgSO₄). Evaporation of the ethyl acetate *in vacuo* and purification by chromatography on silica gel with CH₂Cl₂ and recrystallization from hexane-ethyl acetate (15:1) gave the title compound as a pale yellow solid.
NMR (CDCl₃): δ 7.52 (d, 1H, J = 8.1 Hz), 7.19-7.33 (m, 5H), 6.33 (d, 1H, J = 8.1 Hz), 5.28 (s, 1H), 3.24 (t, 2H, J = 8.0), 3.04 (t, 2H, J = 8.0 Hz), 2.63 (t, 2H, J = 8.0 Hz), 1.6 (m, 2H), 0.99 (t, 3H, J = 8.0 Hz).

### Step C: Preparation of methyl 3-chloro-4-(3-(4-(3-phenylpropionyl)-2-(n-propyl)-3-hydroxyphenoxy)propyl)thio)phenylacetate

A mixture of methyl 3-chloro-4-(3-bromopropylthio)-phenylacetate (196 mg, 0.58 mmoles), 4-(3-phenylpropionyl)-2-(n-propyl)resorcinol (165 mg, 0.58 mmoles), cesium carbonate (189 mg, 0.58 mmoles), and DMF (2.3 ml) was heated at 80° in a nitrogen atmosphere for 5 hrs with magnetic stirring. The suspension was partitioned between ethyl acetate and dilute HCl solution. The aqueous phase was extracted twice with ethyl acetate. The combined organic phases were washed three times with water, once with saturated NaCl solution and dried (MgSO₄). Evaporation *in vacuo* gave the title compound as an orange oil. It was used in the next reaction without purification.
NMR (CDCl₃): δ 7.60 (d, 1H, J = 8.1 Hz), 7.20-7.34 (m, 7H), 7.13 (dd, 8.1, 1.8 Hz), 6.40 (d, 1H, J = 8.1 Hz), 4.15 (t, 2H, J = 5.7 Hz), 3.70 (s, 3H), 3.56 (s, 2H), 3.25 (t, 2H), 3.14 (t, 2H), 3.05 (t, 2H), 2.65 (t, 2H), 2.17 (m, 2H), 1.53 (m, 2H), 0.93 (t, 3H).

### Step D: Preparation of methyl 3-chloro-4-(3-(4-(3-(phenyl)-1-hydroxyliminopropyl)-2-(n-propyl)-3-hydroxyphenoxy)-propyl)thio)phenylacetate

A mixture of methyl 3-chloro-4-(3-(4-(3-phenylpropionyl)-2-(n-propyl)-3-hydroxyphenoxy)propyl)thio)phenylacetate (328 mg, 0.58 mmoles), hydroxylamine hydrochloride (202 mg, 2.9 mmoles), anhydrous sodium acetate (238 mg, 2.9 mmoles) and ethanol (4 ml) was heated under reflux in a nitrogen atmosphere with magnetic stirring for 2 hr. The mixture was partitioned between ethyl acetate and water. The aqueous phase was extracted twice with ethyl acetate. The combined ethyl acetate phases were washed with water, 10% NaHCO₃ solution, and saturated NaCl solution, and dried (MgSO₄). Evaporation *in vacuo* and purification by chromatography (silica gel, 4:1 hexane-ethyl acetate) gave the title compound as a colorless solid.
NMR (CDCl₃): δ 7.21-7.34 (m, 8H), 7.14 (dd, 1H, J = 8.1, 1.8 Hz), 6.44 (d, 1H, J = 8.9 Hz), 4.11 (t, 2H, J= 5.7 Hz), 3.69 (s, 3H), 3.55 (s, 2H), 3.14 (t, 2H, J = 7.2 Hz), 3.12 (m, 2H), 2.90 (m, 2H), 2.67 (dd, 2H, J = 7.7, 7.2 Hz), 2.15 (m, 2H), 1.56 (m, 2H), 0.94 (t, 3H, J = 7.7 Hz).

### Step E: Preparation of 3-chloro-4-(3-(4-(3-(phenyl)-1-hydroxyliminopropyl)-2-(n-propyl)-3-hydroxyphenoxy)propyl)-thio)phenylacetic acid

A solution of methyl 3-chloro-4-(3-(4-(3-(phenyl)-1-hydroxyliminopropyl)-2-(n-propyl)-3-hydroxyphenoxy)propyl)thio)-phenylacetate (205 mg, 0.37 mmoles), LiOH solution (1.0 M, 1.11 ml, 1.11 mmoles), and methanol (11 ml) was kept at room temperature for 16 hr. It was heated under reflux for 15 min, and most of the methanol was removed *in vacuo*. The residue was suspended in water and acidified with dilute HCl. The suspension was extracted three times with ethyl acetate. The combined extracts were washed with water and saturated brine and dried (MgSO₄). The solid residue after evaporation of the solvent *in vacuo* was triturated with CH₂Cl₂, filtered and dried to. give the title compound as an off-white solid, mp 153-154°.
NMR (CDCl₃ plus 1 drop of CD₃OD): δ 7.19-7.34 (m, 8H), 7.13 (dd, 1H, J = 8.1, 1.8 Hz), 6.43 (d, 1H, J = 8.9 Hz), 4.10 (t, 2H, J = 5.7 Hz), 3.54 (s, 2H), 3.15 (t, 2H, J = 7.2 Hz), 3.11 (m, 2H), 2.90 (m, 2H), 2.66 (dd, 2H, J = 7.7, 7.2 Hz), *2.15* (m, 2H), 1.55 (m, 2H), 0.95 (t, 3H, J = 7.7 Hz).

### EXAMPLE 52

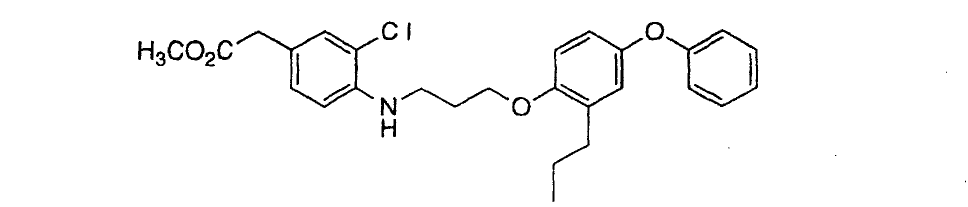

### Methyl 3-chloro-4-(4-phenoxy-2-propylphenoxy)-propylamino)-phenylacetate

### Step A: Preparation of 3-chloro-4-acetamidophenylacetic acid

Acetic anhydride (152 mL, 1.6 moles) was added dropwise to a rapidly stirring mixture of 4-aminophenylacetic acid (195 grams, 1.3 moles) in acetic acid (600 mL) and water (250 mL) at room temperature. After a slight exotherm, the dark brown solution was stirred for one hour at room temperature. The solution was diluted with ethanol (500 mL) and water (250 mL), and a suspension of Calcium hypochlorite (340 grams, 2.3 moles) in water (1 L plus 500mL rinse) was added portionwise. The temperature rose to 50°C and the mixture was stirred for 16 hours at room temperature. The mixture was poured into ice-water (8 L) and extracted with ethyl acetate (3x 2L). The combined extracts were washed with saturated brine, dried over magnesium sulfate and concentrated *in vacuo* to a small volume. Hexane was added and the resulting precipitate filtered, washed with hexane and dried to give the title compound(180 grams) as a brown solid
NMR (CDCl₃ + 10% CD₃OD): δ 2.12 (s, 3H); 3.45 (s, 2H); 7.10 (dd, 2H)); 8.02 (dd, 1H).

### Step B: Preparation of methyl 3-chloro-4-aminophenylacetate·HCl

A solution of 3-chloro-4-acetamidophenylacetic acid (180 grams, 0.79 moles) in methanol (2 L), was treated with concentrated HCl (200 mL) and the resulting solution refluxed for 6 hours and then stirred at room temperature for 16 hours. The mixture was concentrated *in vacuo* to about one-half its volume and ether (4 L) was added. The resulting precipitate was filtered, washed with ether and dried to give the title compound (173 grams) as a tan solid NMR, (CD₃OD): δ 3.70 (s, 2H); 3.73 (s, 3H); 7.35 (d, 1H); 7,43 (d, 1H); 7.56 (s, 1H).

### Step C: Preparation of methyl 3-chloro-4-(3 bromopropylamino)-phenylacetate

Magnesium oxide (10 grams, 250 mmoles), was added to a solution of 1,3-dibromopropane (139 grams, 70 mL, 700 mmoles) in dimethylacetamide (150 mL). A solution of methyl 3-chloro-4-aminophenylacetate·HCl (23.6 grams, 100 mmoles) in dimethylacetmide (200 mL) was added dropwise over 30 minutes and the mixture stirred at 80°C for 6 hours. The cooled mixture was partitioned with methylene chloride and water. The aqueous phase was extracted with methylene choride and the combined organic phases washed with brine, dried over magnesium sulfate and concentrated *in vacuo* to an oil. The crude product was chromatographed on a silica gel column eluting with hexane:ethyl acetate (9:1). The product was further purified by a second silica gel chromatography in methylene chloride:hexane (2:3) to give the title compound as an oil. NMR, (CDCl₃): δ 2.15 (qnt, 2H); 3.35 (q, 2H); 3.47 (s,2H); 3.49 (t, 2H); 3.67 (s, 3H); 6.63 (d, 1H); 7.03 (dd, 1H); 7.17 (d, 1H).

### Step D: Preparation of 1-phenoxy-(4-propenyloxy)benzene

A solution of 4-phenoxy phenol (2.0 grams) was treated with allyl bromide (1.2 mL) and potassium carbonate (1.5 grams). The mixture was stirred over night at 60°. The reaction was partitioned between methylene chloride and water. The organic was washed once with water and dried over sodium sulfate. The organic layer was filtered and concentrated to an oil which was chromatographed over silica gel to afford the title compound.
NMR (CDCl₃) δ 7.10 (m, 9H, ), 6.07 (m, 1H), 5.40 (dd, 1H, J = 17.4, 3.2 Hz), 4.52 (dd, 2H, J = 3.8, 1.3 Hz).

### Step E: Preparation of 2-propyl-4-phenoxy phenol

A solution of 1-phenoxy-(4-propenyloxy)benzene (0.9 grams) in ortho-dichlorobenzene 8 mL) was refluxed for 22 hours. Mixture was cooled to room temperature and was chromatographed over silica gel to afford the intermediate which was hydrogenated over 10% Pd/C catalyst (90 mg) in ethyl acetate for 18 hours. The reaction was filtered through Celite and all volatiles were removed to afford the title compound.
NMR (CDCl₃) δ 7.15 (m, 8H), 2.53 (t, 2H J=7.4 Hz), 1.60 (m, 2H), 0.94 (t, 3H, J = 7.3Hz).

### Step F: Preparation of 3-chloro-4-(4-phenoxy-2-propylphenoxy)-propylamino)phenylacetate

A solution of methyl 3-chloro-4-(3-bromopropylamino)phenylacetate (4.55grams, 14.19 mmoles) and 2-propyl-4-phenoxy phenol(0.95grams, 14.19 mmoles) in 2-butanone (50 mL) was treated with potassium carbonate (2.35 grams, 17.0 mmoles). The mixture was refluxed for 8 hours, stirred at room temperature for 16 hours and filtered. Evaporation *in vacuo* followed by flash chromatography on silica gel in hexane:ethyl acetate (9:1) afforded the title compound as a white solid. NMR, (CD₃OD): δ 7.27(m, 1H), 7.18 (s, 1H), 7.03 (m, 2H), 6.92 (d, 2H), 6.91 (s, 1H), 6.80 (q,2H), 6.65(d, 2H), 4.04 (t, 2H), 3.72(s, 3H), 3.50 (s, 2H), 3.42 (m, 2H), 2.59 (t, 2H), 2.14 (m, 2H), 1.60 (m, 2H), 0.91 (t, 3H).

### EXAMPLE 53

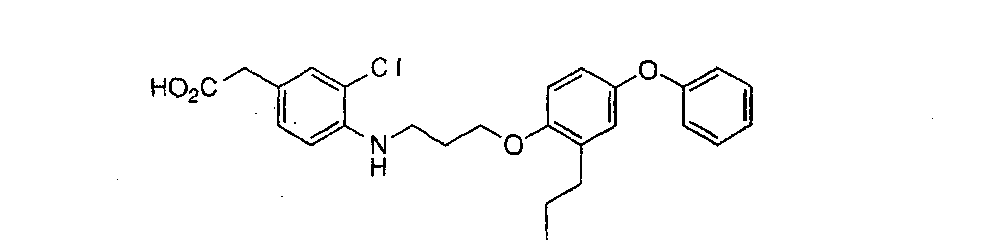

### 3-Chloro-4-(4-phenoxy-2-propylphenoxy)-propylamino)phenylacetic acid

To a solution of methyl 3-chloro-4-(4- phenoxy-2-propylphenoxy)-propylamino)phenylacetate (4.67 grams, 10 mmoles) in methanol (90 mL) was added a solution of lithium hydroxide (1M, 20mL, 20 mmoles), and the resulting mixture stirred at 60°C for 2.5 hours. The solution was evaporated *in vacuo* and the residue diluted with water and ethyl acetate. The pH was brought to 5.0 with 1M hydrochloric acid and organic phase separated. The aqueous phase was extracted 3 times with ethyl acetate and the combined organic extracts dried over magnesium sulfate and evaporated in vacuo to give the title compound as an oil.
NMR (CDCl₃) δ 7.27(m, 1H), 7.18 (s, 1H), 7.03 (m, 2H), 6.92 (d, 2H), 6.91 (s, 1H), 6.80 (q,2H). 6.65(d, 2H), 4.04 (t, 2H), 3.50 (s, 2H), 3.42 (m, 2H), 2.59 (t, 2H), 2.14 (m, 2H), 1.60 (m, 2H), 0.91 (t, 3H). ESI-MS: m/e= 454(M+1)

### EXAMPLE 54

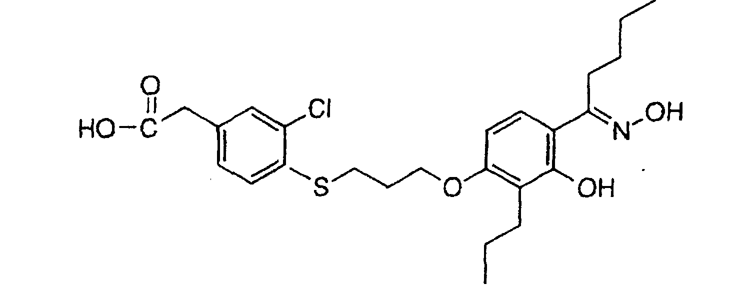

### 3-chloro-4-(3-(2-propyl-3-hydroxy-4-(1-hydroxyliminopropyl)) phenoxy)propylthio phenyl acetic acid

### Preparation of 1-(2,4-dihydroxy)phenyl-1-pentanone

A 0°C suspension of resorcinol (2.028 grams) in dry 1,2-dichloroethane (25 mL) was treated with aluminum chloride (2.701 grams). The suspension was vigorusly stirred for 15 minutes. A solution of valeryl chloride (2.41 mL) in 1,2-dichloroethane (4 mL) was added dropwise. The mixture was allowed to stir and gradually warm to 25°C over 6 hours. The mixture was slowly added to a stirred mixture of water and methylene chloride. The organic was dried over magnesium sulfate, filtered and concentrated to a solid. Silica gel chromatography afforded the title compound.
NMR (CDCl₃) δ 12.90 (s, 1H), 7.63 (d, 1H, J = 9.0 Hz), 6.58 (vbs, 1H), 6.39 (d overlapping with dd, 2H), 2.86 (t, 2H, J = 7.3 Hz), 1.72 (hex, 2H, J = 7.3 Hz), 0.99 (t, 3H, J = 7.1 Hz).

### Preparation of 1-(2-hydroxy-4-(3-propenyloxy)phenyl-1-pentanone

A solution of 1-(2,4-dihydroxy)phenyl-1-pentanone (3.059 grams) in 2-butanone (35 mL) was treated with allyl bromide (1.43 mL) and potassium carbonate (2.394 grams). The mixture was refluxed for 4 hours. The reaction was partitioned between isopropyl acetate and pH 4 buffer. The organic was washed once with water and dried over magnesium sulfate. The organic was filtered and concentrated to an oil which was chromatographed over silica gel to afford the title compound.
NMR (CDCI₃) δ 7.62 (d, 1H, J = 9.0 Hz), 6.42 (dd, 1H, J = 9.0, 1.8 Hz), 6.40 (d, 1H, J = 1.8 Hz), 6.00 (mult, 1H), 5.40 (dd, 1H, J = 15.7, 1.3 Hz), 5.30 (dd, 1H, J = 11.9, 1.3 Hz), 4.52 (dd, 2H, J = 3.8, 1.3 Hz), 2.86 (t, 2H, J = 7.3 Hz), 1.72 (hex, 2H, J = 7.3 Hz), 0.99 (t, 3H, J = 7.1 Hz).

### Preparation of 1-(2,4-dihydroxy-3-(3-propenyl))phenyl-1-pentanone

A solution of 1-(2-hydroxy-4-(3-propenyloxy)phenyl-1-pentanone (2.59 grams) in ortho-dichlorobenzene (25 mL) was refluxed for 23 hours. The solvent was removed by distillation *in vacuo* and the semi-solid digested in refluxing cyclohexane (30 mL). The mixture was cooled to 25°C and filtered to afford the title compound.
NMR (CDCl₃) δ 13.22 (s, 1H), 7.58 (d, 1H, J = 8.9 Hz), 6.38 (d, 1H, J = 8.9 Hz), 5.98 (mult, 1H), 5.62 (bs, 1H), 5.18 (dd, 1H, J = 16.0, 1.3 Hz), 5.12 (dd, 1H, J = 11.5, 1.3 Hz), 3.49 (dd, 2H, J = 4.0, 1.3 Hz), 2.88 (t, 2H, J = 7.2 Hz), 1.72 (hex, 2H, J = 7.2 Hz), 0.99 (t, 3H, J = 7.1 Hz).

### Preparation of 1-(2,4-dihydroxy-3-propyl)phenyl-1-pentanone

A solution of 1-(2.4-dihydroxy-3-(3-propenyl))phenyl-1-pentanone (1.855 grams) in ethyl acetate (20 mL) was hydrogenated (21 psi) over 10% Pd/C catalyst (0.27 grams) for 2 hours. The reaction was filtered through Celite and all volatiles were removed to afford the title compound.
NMR (CDCl₃) δ 13.20 (s, 1H), 7.60 (d, 1H, J = 9.0 Hz), 6.36 (d, 1H, J = 9.0 Hz), 2.95 (bt, 2H, J = 7.6 Hz). 2.84 (t, 2H, J = 7.2 Hz), 1.72 (hex, 2H, J = 7.3 Hz), 0.96 (t, 3H, J = 7.1 Hz).

### 1-(2-hydroxy-3-propyl-4-(3-bromo)propyloxy)phenyl-1-pentanone

A solution of 1-(2,4-dihydroxy-3-propyl)phenyl-1-pentanone (2.368 grams) in 2-butanone (25 mL) was treated with 1,3-dibromopropane (3.99 mL) and potassium carbonate (4.155 grams). The mixture was refluxed for 4 hours. The reaction mixture was partitioned between isopropyl acetate and pH 4 buffer. The organic was washed once with water, then dried over magnesium sulfate. The organic was filtered and evaporated to an oil which was chromatographed over silica gel with hexane/methylene chloride (2:1) to afford the title compound.
NMR (CDCl₃) d 7.60 (d, 1H, J = 8.9 Hz), 6.41 (d, 1H, J = 9.0 Hz), 4.15 (t, 2H, J = 5.8 Hz), 3.60 (t, 2H, J = 6.4 Hz), 2.83 (t, 2H, J = 7.6 Hz), 2.60 (bt, 2H, J = 7.5 Hz).

### 3-chloro-4-(3-(2-propyl-3-hydroxy-4-(1-oxopentyl))phenoxy)-propylthio phenyl acetic acid methyl ester

A solution of 3-chloro-4-dimethylcarbamoylthiophenyl acetic acid methyl ester (0.668 grams) in dry methanol (6 mL) was treated with a solution of sodium methoxide in methanol (25wt%; 0.691 mL). The solution was refluxed for 2 hours. LC analysis showed the disappearance of the carbamate. The solution was allowed to cool to 50°C. 1-(2-hydroxy-3-propyl-4-(3-bromo)propyloxy)phenyl-1-pentanone (0.691 grams) was added and the solution stirred for 1 hour. The reaction was partitioned between isopropyl acetate and pH 4 buffer. The organic was washed once more with pH 4 buffer, then water. The organic was dried over magnesium sulfate, filtered and concentrated to an oil. The oil was chromatograhphed over silica gel to afford the title compound.
NMR (CDCl₃) d 7.60 (d, 1H, J = 8.9 Hz), 7.12 (dd, 1H, J = 8.0, 1.9 Hz), 6.39 (d, 1H, J = 9.0 Hz), 4.12 (t, 2H, J = 5.7 Hz), 3.69 (s,3H), 3.13 (t, 2H, J = 7.7 Hz), 2.87 (t, 2H, J = 7.1 Hz), 2.60 (bt, 2H, J = 7.4 Hz).

### 3-chloro-4-(3-(2-propyl-3-hydroxy-4-(1-hydroxyliminopentyl))phenoxy)propylthio phenyl acetic acid methyl ester

A solution of L-786,353 (0.308 grams) in dry methanol (4 mL) was treated with hydroxylamine hydrochloride (0.218 grams). Anhydrous sodium acetate (0.257 grams) was added and the mixture refluxed for 8 hours. The reaction mixture was partitioned between isopropyl acetate and pH 7 buffer. The organic phase was washed once with water and dried over magnesium sulfate, filtered and evaporated to an oil. The title compound was recovered by chromatography over silica gel.
NMR (CDCl₃) d 7.11 (dd, 1H, J = 8.1, 1.9 Hz), 6.41 (d, 1H, J = 9.0 Hz), 4.11 (t, 2H, J = 5.8 Hz), 3.70 (s, 3H), 3.11 (t, 2H, J = 7.4 Hz), 2.80 (bt, 2H, J = 7.8 Hz), 2.65 (bt, 2H, J = 7.7 Hz).

### 3-chloro-4-(3-(2-propyl-3-hydroxy-4-(1-hydroxyliminopropyl)) phenoxy)propylthio phenyl acetic acid

A solution of L-786,355 (0.208 grams) in methanol (4 mL) was treated with a solution of lithium hydroxide in water (1.032 M; 0.500 mL). The reaction was refluxed 1 hour. The reaction mixture was partitioned between isopropyl acetate and 0.1N HCl. The organic was dried over magnesium sulfate, filtered and concentrated to a solid. The solid was dissolved in methylene chloride (0.5 mL) and heated to reflux. Cyclohexane (1.50 mL) was added dropwise. The solution was cooled to 0°C, and the resultant solid isolated by filtration (0.171 grams).
NMR (CDCl₃) d 7.14 (dd, 1H, J = 8.0, 1.9 Hz), 6.38 (d, 1H, J = 9.0 Hz), 4.10 (t, 2H, J = 5.8 Hz), 3.58 (s, 2H), 3.16 (t, 2H, J = 7.5 Hz), 2.81 (bt, 2H, J = 7.8 Hz), 2.65 (bt, 2H, J = 7.7 Hz).

### EXAMPLE 55

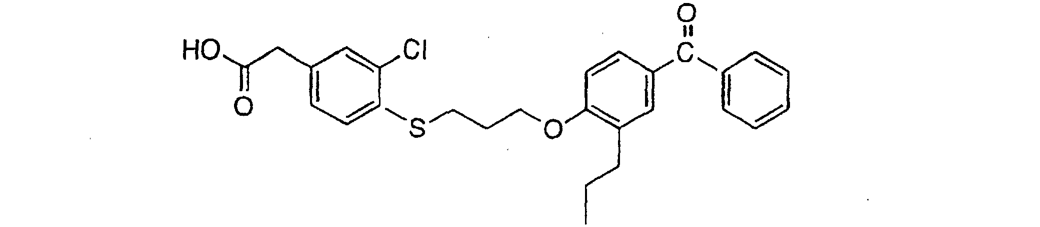

### 4-(2-(4-benzyl-2-propylphenoxy)propylthio)-3-chlorophenylacetic acid

### Step 1A

A solution of 3-chloro-4-dimethylcarbamoylthiophenyl acetic acid methyl ester (10.028 grams; 34.848 mmol) in dry MeOH (90 mL) was treated with a solution of sodium methoxide (4.37 M; 11.16 mL; 48.788 mmol). The reaction was refluxed for 2 hours. The reaction mixture was cooled to 20° C and transferred to a dropping funnel. The dropping funnel was placed atop a flask containing a solution of dibromopropane (14.15 mL; 139.392 mmol) in dry MeOH (50 mL). The contents of the dropping funnel were added to the flask dropwise, and the solution stirred for 2 hours. The reaction mixture was partitioned between isopropyl acetate and pH 4 buffer. The layers were separated and the organic washed once with water. The organic was dried over magnesium sulfate, filtered and concentrated. Silica gel chromatography afforded 3-chloro-4-(3-bromopropyl)thiophenyl acetic acid methyl ester.

### Step I

Commercially available 2-propylphenol ( 1.5 g, 1.0 Eq, 11 mmol ) and benzoic acid (1.62 g, 1.2 Eq, 13 mmol ) were dissolved in trifluoromethanesulfonic acid ( 5.5 ml ) at RT. The mixture was heated at 85 C for 2 Hrs. The reaction mixture was poured into ice and EtOAc. The aqueous phase was extracted with EtOAc and the EtOAc extracts washed with sat'd aq NaHCO₃, followed by sat'd aq NaCl. The EtOAc extracts were dried over MgSO₄ and reduced *i. vac.* The crude material was crystallized from Hexanes : EtOAc (6:1) with cooling. 2.4 g 90% Characteristic NMR Resonances; ¹H NMR 400MHz (CDCl₃); 7.78-7.8 ( 2 overlapping d, 2H), 7.72 (d, 1H, J=2Hz), 7.62 (m, 2H), 7.5 (m, 2H), 6.86 (d, 1H, J=8.4 Hz), 2.67 (t, 2H, J=7.6 Hz), 1.69 (sext, 2H, J=7.5 Hz), 1.02 (t, 3H, J=7.4 Hz).

### Step 2

The phenol ( 53 mg, 1.03 Eq, 0.221 mmol) was dissolved in DMF (0.4 ml) with the bromide ( 72.6 mg, 1.0Eq, 0.215 mmol ) and CsCO₃ ( 74 mg, 1.06 Eq, 0.227 mmol ). The suspension was stirred 16 Hrs at RT. The mixture was poured into 0.2 N HCl and EtOAc. The aqueous phase was extracted with EtOAc and the EtOAc extracts washed with sat'd aq NaCI. The extracts were dried over Na₂SO₄ and reduced *i*. *vac.* The product was purified by elution from a silica gel column (4 g E. Merck 40-63 µ ) with 97 : 3 Toluene : EtOAc. The ester is obtained as a glass.
Characteristic NMR Resonances; ¹H NMR 400MHz (CDCl₃); 7.74 (m, 2H), 7.65 (m, 2H), 7.53 (m, 1H), 7.46 (t, fine splitting, 2H, J= ∼6Hz), 7.29 (m, 2H), 7.12 (dd, 1H, J= 8.1, 1.8 Hz), 6.84 (d, 1H, J=8.4 Hz), 4.16 (t, 2H, J=5.7 Hz), 3.68 (s, 3H), 3.55 (s, 3H), 3.15 (t, 2H, J=7.2 Hz), 2.62 (dd, 2H, J unresolved), 2.18 (pent, 2H, J=6.2 Hz), 1.60 (sext, 2H, J=7.6 Hz), 0.93 (t, 3H, J=7.4 Hz).

### Step 3

The ester ( 34.5 mg, 1.0 Eq, 0.069 mmol ) was dissolved in approximately 0.5 ml 2 : 1 dioxane : H₂O. 1.5 M Aqueous LiOH ( 72 µl, 2 N, 2 Eq) was added dropwise at RT and the mixture stirred 3 Hrs. The reaction mixture was diluted into 0.2 N HCl and EtOAc. The aqueous phase was extracted with EtOAc and the EtOAc extracts washed with sat'd aq NaCI. The extracts were dried over Na₂SO₄ and reduced i. *vac.* The crude acid was purified by elution from an E. Merck 40-63 µ RP-8 column with 70 : 30 CH₃CN : H₂O containing 0.1% TFA. Material was lyophilized.
Characteristic NMR Resonances; ¹H NMR 400MHz (CDCl₃); 7.74 (m, 2H), 7.62 (m, 2H), 7.54 (m. 1H), 7.45 (t, 2H, J=7.5 Hz), 7.28 (m, 2H), 7.09 (dd, 1H), 6.79 (d, 1H, J=8.4 Hz), 4.15 (t, 2H, J=5.8 Hz), 3.55 (s, 2H), 3.16 (t, 2H, J=7.0 Hz), 2.59 (dd, 2H, J=7.5, 7.9 Hz), 2.18 (pent, 2H), 1.59 (sext, 2H, J=7.6), 0.92 (t, 3H, J=7.4).

### EXAMPLE 56

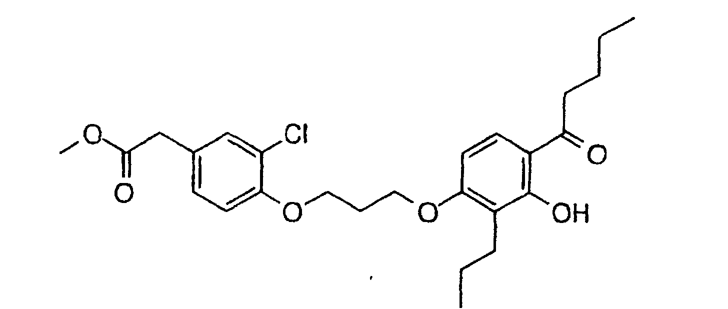

### Methyl 3-chloro-4-(3-(2-propyl-3-hydroxy-4-(1-oxopentyl)phenoxy)-propyl)-phenylacetate

### Step A: Preparation of 1-(2,4-dihydroxy-3-propyl)phenyl-1-pentanone

The title compound was prepared according to the method described in Example 3, Step A.
NMR (CDCl₃) δ 7.62 (d, 1H, J = 9.0 Hz), 6.55 (vbs, 1H), 6.39 (d overlapping with dd, 2H), 2.88 (t, 2H, J = 7.3 Hz), 1.72 (hex, 2H, J = 7.3 Hz), 0.98 (t, 3H, J = 7.1 Hz).

### Step B: Preparation of 1-(2-hydroxy-4-(3-propenyloxy)phenyl-1-pentanone

The title compound was prepared according to the method described in Example 3, Step B
NMR (CDCl₃) δ 7.63 (d. 1H, J = 9.0 Hz), 6.40 (dd, 1H, J = 9.0, 1.8 Hz), 6.40 (d, 1H, J = 1.8 Hz), 6.00 (m, 1H), 5.40 (dd, 1H, J = 15.8, 1.3 Hz), 5.30 (dd, 1H, J = 11.9, 1.3 Hz), 4.52 (dd, 2H, J = 3.8, 1.3 Hz), 2.89 (t, 2H, J = 7.3 Hz), 1.72 (hex, 2H, J = 7.3 Hz), 0.99 (t, 3H, J = 7.1 Hz).

### Step C: Preparation of 1-(2,4-dihydroxy-3-propyl)phenyl-1-pentanone

The title compound was prepared according to the method described in Example 3, Step C & D.
NMR (CDCl₃) δ 7.53 (d, 1H, J = 9.0 Hz), 6.36 (d, 1H, J = 9.0 Hz), 2.87 (t, 2H, J = 7.3 Hz), 2.61 (t, 2H, J = 7.5 Hz).

### Step D: Preparation of 1-bromo-3-(2-propyl-3-hydroxy-4-(1-oxopentyl) henoxy)propane

A solution of 3-propyl-2,4-dihydroxypentophenone (25.545 grams) in 2-butanone (300 mL) was treated with 1,3-dibromopropane (48.79 mL) and potassium carbonate (50.859 grams). The mixture was refluxed for 4 hours. The reaction mixture was partitioned between isopropyl acetate and pH 4 buffer. The organic was washed once with water, then dried over magnesium sulfate. The organic was filtered and evaporated to an oil which was chromatographed over silica gel with hexane/methylene chloride (2:1) to afford the title compound.
NMR (CDCl₃) δ 7.62 (d, 1H, J = 8.8 Hz), 6.43 (d, 1H, J = 8.8 Hz), 4.16 (t, 2H, J = 5.8 Hz), 3.60 (t, 2H, J = 6.4 Hz), 2.94 (quart, 2H, J = 7.3 Hz), 2.61 (t, 2H, J = 7.5 Hz).

### Step E: Preparation of methyl 3-chloro-4-(3-(2-propyl-3-hydroxy-4-(1-oxopentyl)-phenoxy)-propyl)-phenylacetate

A solution of methyl 3-chloro-4-hydroxyphenylacetate (105 mg) in dry DMF (4.0 mL) and potassium carbonate (78 mg) were stirred for 0.5 hr at 60°C. 1-Bromo-3-(2-propyl-3-hydroxy-4-(1-oxopentyl) phenoxy)propane (160 mg) was added and stirred at 60° overnight. The reaction was partitioned between ethyl acetate and water. The organic layer was dried over sodium sulfate, filtered and concentrated to an oil. The oil was applied to a silica gel plate and eluted with hexane/ethyl acetate (2:1). Isolated a band at rf=0.5 to afford the title compound.
NMR (CD₃OD) δ 7.60 (d, 1H, J = 9.0 Hz), 7.27 (d, 1H, J = 2.1, Hz), 7.10 (dd, 1H, J = 8.5, 2.2 Hz), 6.87 (d, 1H, J = 8.4 Hz), 6.46 (d, 1H, J = 9.0 Hz), 4.25 (t, 2H, J = 5.9 Hz), 4.20 (t, 2H, J = 5.9 Hz), 3.67 (s, 3H), 3.52 (s, 2H,), 2.88 (t, 2H, J = 7.6 Hz).
ESI-MS: m/e = 477 (M+1)

### 3-Chloro-4-(3-(2-propyl-3-hydroxy-4-(1-oxopentyl)-phenoxy) propyloxy)phenyl-acetic acid

A solution of methyl 3-chloro-4-(3-(2-propyl-3-hydroxy-4-(1-oxopentyl)phenoxy)-propyl)-phenylacetate (0.113 grams) in methanol (1.5 mL) was treated with a solution of lithium hydroxide in water (1.01 M; 0.362 mL). The reaction was stirred at room temperature overnight. The reaction mixture was partitioned between ethyl acetate and 0.1N HCl. The organic layer was dried over sodium sulfate, filtered and concentrated to an oil.
NMR (CDCl₃) d 7.59 (d, 1H, J = 8.8 Hz), 7.28 (d, 1H, J = 1.9 Hz), 7.10 (dd, 1H, J = 8.5, 1.8 Hz), 6.88 (d, 1H, J = 8.5 Hz), 6.46 (d, 1H, J = 9.1 Hz), 4.25 (t, 2H, J = 6.0 Hz), 4.20 (t, 2H, J = 6.0 Hz), 3.55 (s, 2H,), 2.88 (t, 2H, J = 7.6 Hz).
ESI-MS: m/e = 463 (M+1)

### EXAMPLE 57

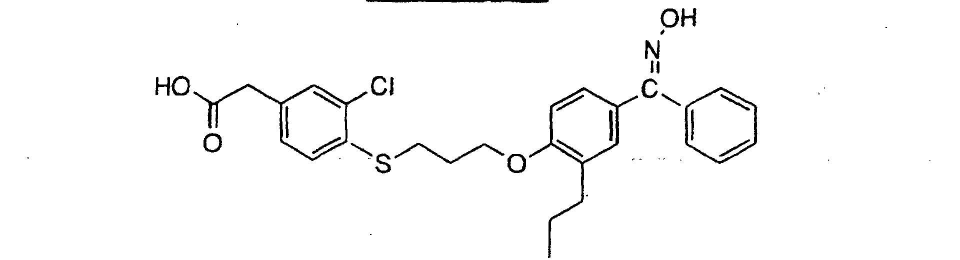

### 4-(3-(4-(N-hydroxyiminobenzoyl)-2-propylphenoxy)propylthio)-3-chlorophenylacetic acid

### Step 1

The ester prepared in Example 55, Step 2 above was converted to the oxime according to the general procedure of Example 10. Heating under reflux was continued for 16 Hrs. The mixture was cooled and reduced *i*. *vac.* The residue was taken up in EtOAc and H₂O. The aqueous phase was extracted with EtOAc and the EtOAc extracts washed with sat'd aq NaCl. The EtOAc extracts were dried over MgSO₄ and reduced *i*. *vac*. The product was purified by elution from a silica gel column (4 g E. Merck 40-63 µ ) with 60 : 37 : 3 toluene : hexanes : EtOAc. The ester is obtained as a glass.
Characteristic NMR Resonances; ¹H NMR 400MHz (CDCl₃); Product is a mixture of oxime isomers; approx. 55 : 45. Where 'minor' or 'major' isomer is not indicated, proton count is the indicated number for both minor and major isomer.
7.47-7.11 (overlapping aromatic multiplets, 9H), 6.86 (d, 1H minor isomer, J=8.5 Hz), 6.71 (d, 1H major isomer, J=8.6 Hz), 4.13 (t, 2H minor isomer, J=5.7 Hz), 4.07 (t, 2H major isomer, J=5.8 Hz), 3.685 (s, 3H minor isomer), 3.68 (s, 3H major isomer), 3.553 (s, 2H minor isomer), 3.546 (s, 3H major isomer), 3.14 ( 2 overlapping t, 2H), 2.59 (dd, 2H minor isomer), 2.55 (dd, 2H major isomer), 2.15 (overlapping m, 2H), 1.57 (overlapping m, 2H), 0.91 (t, 3H unassignable to major or minor, J=7.3 Hz), 0.89 (t, 3H unassignable to major or minor, J=7.4 Hz).

### Step 2

The ester ( 11.4 mg, 1.0 Eq, 0.022 mmol ) was cleavedwith LiOH aq ( 25 µl, 2 N, 2 Eq ) as for Example 55, Step 3. The product is a mixture of oxime isomers.
Characteristic NMR Resonances; ¹H NMR 400MHz (CDCl₃); Product is a mixture of oxime isomers; approx. 55 : 45. Where 'minor' or 'major' isomer is not indicated, proton count is the indicated number for both minor and major isomer.
7.42-7.08 (overlapping aromatic multiplets, 9H), 6.81 (d, 1H major isomer, J=8.5 Hz), 6.68 (d, 1H minor isomer, J=8.6 Hz), 4.05-4.12 (Overlapping m, 2H), 3.69 (s, 3H), 3.56 (s, 2H), 3.14/3.12 ( 2 overlapping t, 2H, J=7.2 Hz for both), 2.57/2.52 (2 overlapping dd, 2H, J=∼7.4, ∼7.7 Hz for each), 2.14 (overlapping m, 2H), 1.55 (overlapping m, 2H), 0.888 (t, 3H unassignable to major or minor, J=7.4 Hz), 0.875 (t, 3H unassignable to major or minor, J=7.4 Hz).
MS ESI CH₃CN / NH₄CO₂ aq. M+1 498.3.

### EXAMPLE 58

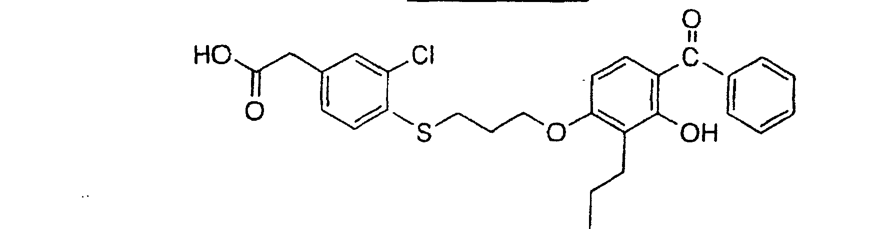

### 4-(3-(4-benzoyl-3-hydroxy-2-propylphenoxy)propylthio)-3-chlorophenylacetic acid

### Step 1

Commercially available 4-allyloxy-2-hydroxybenzophenone (5 g) was rearranged as in Example 3 Step C. The product was isolated by dilution of the reaction mixture with 5 volumes hexanes to give a crystalline product as fine needles.
Characteristic NMR Resonances; ¹H NMR 400MHz (CDCl₃); 7.62-7.59 (m, 2H), 7.56-7.52 (m, 2H), 7.49-7.44 (m, 2H), 7.40 (d, 1H, J=8.9 Hz), 6.34 (d, 1H, J=8.8 Hz), 6.02 (ddt, 1H, J=17.21, 10.1, 6.2 Hz), 5.72 (s, 1H, phenol OH), 5.14-5.24 (m, 2H), 3.53 (d with fine splitting, 2H, J=6.2 Hz).

### Step 2

2,4-dihydroxy-3-(2-propenyl)benzophenone (3 g) was hydrogenated as in Example 3 Step D. The product was purified by crystallization from methanol/water. The product is obtained as small yellow plates. Characteristic NMR Resonances; ¹H NMR 400MHz (CDCl₃); 7.61-7.59 (m, 2H), 7.55-7.51 (m, 1H). 7.48-7.44 (m, 2H), 7.33 (d, 1H, J=8.8 Hz), 6.29 (d, 1H, J=8.8 Hz), 5.51 (s, 1H, phenol OH), 2.66 (dd, 2H, J=7.6, 9.3 Hz), 1.61 (sext, 2H, J=7.7 Hz), 0.99 (t, 3H, J=7.3 Hz).

### Step 3

The phenol (102.6 mg, 1.0 Eq, 0.4 mmol) and the indicated bromide (169 mg, 1.2 Eq. 0.5 mmol) were coupled with CsCO3 (135 mg, 1.05 Eq, 0.42 mmol) as for the procedure reported for Example 55, Step 2. The product was purified by elution from a silica gel column (10 g E. Merck 40-63 µ ) with toluene : hexanes : EtOAc 40 : 57 : 3.
Characteristic NMR Resonances; ¹H NMR 400MHz (CDCl₃); 7.62-7.59 (m, 2H), 7.54 (m, 1H), 7.47 (m, 2H), 7.40 (d, 1H, J=9.0 Hz), 7.30 (d, 1H, J=1.9 Hz), 7.26 (d, 1H, J=8.1 Hz), 7.12 (dd, 1H), 6.36 (d, 1H, J=9 Hz), 4.15 (t, 2H, J=5.7 Hz), 3.68 (s, 3H), 3.55 (s, 2H), 3.14 (t, 2H, J=7.1 Hz), 2.68 (dd, 2H), 2.16 (pent, 2H), 1.56 (sext, 2H), 0.95 (t, 3H, J=7.4 Hz).

### Step 3

The ester ( 36.2 mg, 1.0 Eq, 0.071 mmol ) was cleavedwith LiOH aq (100 µl, 1.5 N, 2 Eq ) as for Example 55, Step 3.
Characteristic NMR Resonances; ¹H NMR 400MHz (CDCl₃); 7.62-7.59 (m, 2H), 7.54-7.52 (m, 1H), 7.48-7.44 (m, 2H), 7.40 (d, 1H, J=9 Hz), 7.30 (d, 1H, J=1.8 Hz), 7.26 (d, 1H, J=8.1 Hz), 7.12 (dd, 1H, J=8.1, 1.8 Hz), 6.37 (d, 1H, J=9 Hz), 4.15 (t, 2H, J=5.8 Hz), 3.58 (s, 2H), 3.14 (t, 2H, J=7.1 Hz), 2.67 (dd, 2H, J=8.9, 7.5 Hz), 2.16 (sext, 2H, J=7.5 Hz), 0.95 (t, 3H, J=7.3 Hz).
MS ESI CH₃CN / NH₄CO₂ aq. M+1 499.1.

### EXAMPLE 59

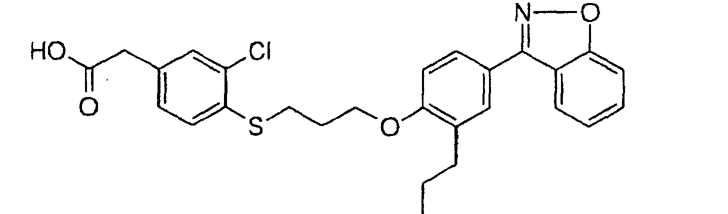

### 3-chloro-4-(3-((1,2-benzisoxazol-3-yl)-2-propylphenoxy) propylthio)phenylacetic acid

### Step A: Preparation of 4-hydroxy-5-propyl-2'-fluorobenzophenone

A 0°C suspension of 2-fluorobenzoyl chloride (1.3 mL, 11.03 mmol) in 15 mL of 1,2-dichloroethane was treated with aluminum chloride (1.0 g, 7.35 mmol). The suspension was vigorously stirred for 15 minutes. A solution of 2-propylphenol (1.0 g, 7.35 mmol) in 5 mL of 1,2-dichloroethane was added dropwise. The mixture was allowed to stir and gradually warm to 25°C over 6 hours. The mixture was slowly added to a stirred mixture of water and methylene chloride. The organic phase was dried over magnesium sulfate, filtered and concentrated to a solid. The crude residue was purified via flash chromatography on silica gel (15% ethyl acetate/hexane eluent) to afforded the title compound.
¹NMR (CDCl₃, ppm) δ 0.93 (t, 3H), 1.65 (m, 2H), 2.14 (t, 2H), 5.29 (s, 1H), 7.08-7.36 (m, 2H), 7.46-7.78 (m, 3H), 7.84 (s, 1H), 8.12 (td, 1H)

### Step B: Preparation of 4-hydroxy-5-propyl-2'-fluorobenzophenone oxime

A solution of 4-hydroxy-5-propyl-2'-fluorobenzophenone (Step A; 1.35 g, 5.23 mmol) in 15 mL of pyridine was treated with hydroxylamine hydrochloride (1.82 g, 26.15 mmol). The mixture was refluxed for 24 hours, cooled and the pyridine removed in vacuo. The residue was taken up in ethyl acetate and washed with 1N hydrochloric acid, water, brine, dried over MgSO₄, filtered and concentrated in vacuo.The crude residue was purified via flash chromatography on silica gel (20 % ethyl acetate/hexane eluent) to yield the title compound.
¹NMR (CDCI₃, ppm) δ 0.96 (t, 3H), 1.22 (m, 2H), 1.56 (t, 2H), 6.82 (d, 1H), 7.13-7.57 (m, 7H)
ESI: MS m/e = 259 (M+1)

### Step C:Preparation of 4-(1,2-benzisoxazol-3-yl)-2-propylphenol

Sodium hydride (60%; 160 mg, 4.0 mmol) was taken up in 7 mL of dimethylformamide (DMF) and 7 mL of benzene. 4-hydroxy-5-propyl-2'-fluorobenzophenone oxime (Step B; 517 mg, 2.0 mmol) was added in 3 mL of DMF and 3 mL of benzene and the reaction stirred at 50 °C for 2 hours. After cooling, the reaction was quenched with saturated aqueous ammonium chloride and extracted with ethyl acetate. The organic phase was washed with water, brine, dried (MgSO₄), filtered and concentrated in vacuo. The crude residue was purified by flash chromatography on silica gel (10% ethyl acetate/hexane eluent) to afford the title compound.
¹NMR (CDCl₃, ppm) δ 1.02 (t, 3H), 1.71 (m, 2H), 2.69 (t, 2H), 5.54 (broad s, 1H), 6.95 (d, 1H, J = 8 Hz), 7.48 (td, 1H), 7.91 (dd, 1H, J = 8 Hz, 1 Hz)
ESI: MS m/e = 239 (M+1)

### Step D: Preparation of methyl-3-chloro-4-(3-((1,2-benzisoxazol-3-yl)-2-propylphenoxy)propylthio)phenylacetate

A solution of potassium carbonate (31 mg, 0.222 mmol) and 4-(1,2-benzisoxazol-3-yl)-2-propylphenol (Step C; 39 mg, 0.163 mmol) in 1 mL dimethylformamide (DMF) at 60 °C was stirred for 30 minutes. Methyl-3-chloro-4-(3-bromopropylthio)phenylacetate (example 51, step A; 50 mg, 0.148 mmol) was added in 1mL DMF and the reaction stirred 18 hours at 60 °C. The reaction was cooled, diluted with ethyl acetate and washed with water, brine, dried over MgSO₄, filtered and concentrated in vacuo. The crude residue was purified via flash chromatography on silica gel (15% ethyl acetate/hexane) to afford the title compound.
¹NMR (CDCI₃, ppm) δ 1.02 (t, 3H), 1.72 (m, 2H), 2.24 (m, 2H), 2.75 (t, 2H), 3.21 (t, 2H), 3.58 (s, 2H), 3.70 (s, 3H), 4.21 (t, 2H), 6.95 (d, 1H, J = 8 Hz), 7.49 (td, 1H), 7.91 (dd, 1H, J = 8 Hz, 1 Hz)
ESI: MS m/e = 511 (M+1)

### Step E: Preparation of 3-chloro-4-(3-((1,2-benzisoxazol-3-yl)-2-propylphenoxy)propylthio)phenvlacetic acid

Using the method of Example 2, and substituting methyl-3-chloro-4-(3-((1,2-benzisoxazol-3-yl)-2-propylphenoxy)propylthio)phenylacetate (Step D) as the starting material, the title compound was obtained.
¹NMR (CDCl₃, ppm) δ 0.99 (t, 3H), 1.68 (m, 2H), 2.21 (m, 2H), 2.68 (t, 2H), 3.19 (t, 2H), 3.58 (s, 2H), 4.17 (t, 2H), 6.95 (d, 2H, J = 8 Hz), 7.13 (dd, 1H), 7.27-7.42 (m, 3H), 7.55-7.65 (m, 2H), 7.71-7.79 (m, 2H), 7.92 (dd, 1H, J = 8 Hz, 1 Hz)
ESI: MS m/e = 497 (M+1)

### EXAMPLE 60

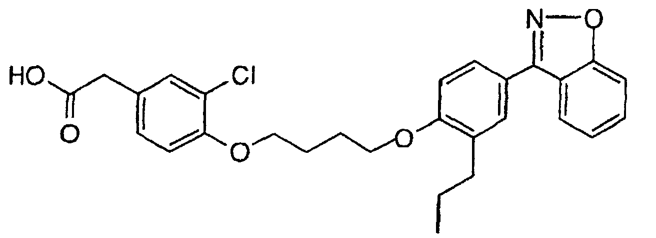

### 3-chloro-4-(3-(( 1,2-benzisoxazol-3-yl)-2-propylphenoxy) butyloxy)phenylacetic acid

### Step A: Preparation of methyl-3-chloro-4-(3-((1,2-benzisoxazol-3-yl)-2-propylphenoxy)butyloxy)phenylacetate

The title compound was prepared using the method described in example 59, step D, but substituting methyl-3-chloro-4-(4-bromobutyloxy)phenylacetate for methyl-3-chloro-4-(3-bromopropylthio)phenyl acetate (methyl-3-chloro-4-(4-bromobutyloxy)phenylacetate was obtained by the following procedure: a solution of methyl 3-chloro-4-hydroxyphenylacetate and 1,4-dibromobutane (0.021 g, 0.044 mmol) in 0.5 mL of methanol was added 5N NaOH soln (0.04 mL, 5 eq) at rt. The reaction mixture was heated initially with a heat gun to reflux in order to dissolve the starting material. After heating, the reaction mixture stirred at ambient temperature for 1 h. The reaction mixture was diluted with EtOAc and 0.1N HCl (5 mL). The organic layer was separated from the aqueous portion, and washed with 0.1N HCl (5 mL) followed by 10 mL brine. The organic layer was dried (MgSO₄), filtered, and evaporated in vacuo to afford the title compound. This compound was taken forward without further purification. the title compound was obtained.
¹NMR (CDCl₃, ppm) δ 0.99 (t, 3H), 1.68 (m, 2H), 2.69 (t, 2H), 2.02-2.15 (broad t, 4H), 3.54 (s, 2H), 3.69 (s, 3H), 4.04-4.19 (broad q, 4H), 6.88 (d, 1H, J = 8 Hz), 7.0 (d. 1H, J = 8Hz), 7.11 (dd, 1H, J = 8 Hz, 1 Hz), 7.30-7.41 (m, 2H). 7.54-7.65 (m, 3H), 7.75 (s, 1H), 7.93 (dd, 1H, J = 8 Hz, 1 Hz)

### Step B: Preparation of 3-chloro-4-(3-((1,2-benzisoxazol-3-yl)-2-propylphenoxy)butyloxy)phenylacetic acid

Using the method of Example 2 and substituting methyl-3-chloro-4-(3-((1,2-benzisoxazol-3-yl)-2-propylphenoxy)butyloxy)-phenylacetate (Step A) as the starting material, the title compound was obtained.
¹NMR (CDCl₃, ppm) δ 0.98 (t, 3H), 1.68 (m, 2H), 2.0-2.15 (broad t, 4H), 2.68 (t, 2H), 3.57 (s, 2H), 4.08-4.20 (broad q, 4H), 6.89 (d, 1H, J = 8 Hz), 7.00 (d, 1H, J = 8 Hz), 7.12 (dd, 1H, J = 8 Hz, 1 Hz), 7.27-7.42 (m, 2H), 7.53-7.67 (m, 2H), 7.75 (s, 1H), 7.94 (dd, 1H, J = 8 Hz, 1 Hz) ESI: MS m/e = 495 (M+1)

### Example 61

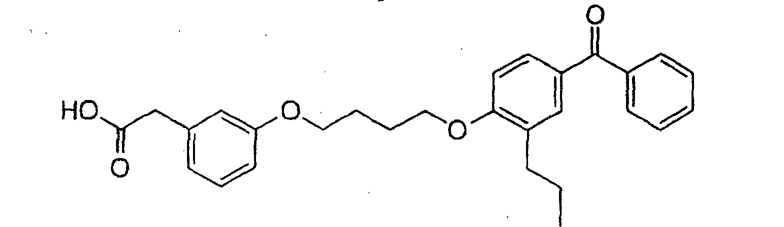

### 3-(4-(4-benzoyl-2-propylphenoxy)butyloxy)phenyl acetic acid

### Step 1

Methyl 3-hydroxyphenylacetate was obtained from the Fischer esterification of the commercially available 3-hydroxyphenylacetic acid in methanol. The 3-hydroxyphenylacetic acid (25g) was dissolved in methanol (100 ml) with approximately 0.4 ml H₂SO₄ conc. The mixture was heated 16 Hrs under reflux. The mixture was cooled and reduced i. *vac*. The residue was taken up in ethyl acetate and washed with sat'd aq NaHCO., followed by sat'd aq NaCl. The EtOAc extracts were dried over MgSO₄ and reduced *i. vac.* The ester was used without further purification.
Characteristic NMR Resonances; ¹H NMR 400MHz (CDCl₃); 7.15 (t, 1H, J=7.7 Hz), 6.80 (t, 1H, J=8.1 Hz), 6.75 (brd s, 1H), 6.72 (dd, 1H, J=2.6, 8.1 Hz), 3.68 (s, 3H), 3.56 (s, 2H).

### Step 2

Methyl 3-hydroxyphenylacetate prepared in Example 61 step 1 ( 4.0 g, 1 Eq, 0.024 mol ) was dissolved in DMF ( 30 ml ) with 1,4-dibromobutane ( 14.4 ml, 5 Eq, 0.121 mol ) and CsCO₃ (8.3 g, 1.05 Eq, 0.025 mol). The suspension was stirred 1.5 Hrs at RT. The mixture was poured into 0.2 N HCl and EtOAc. The aqueous phase was extracted with EtOAc and the EtOAc extracts washed three times with water, followed by sat'd aq NaCl. The extracts were dried over MgSO₄ and reduced *i. vac.* The product was purified by elution from a silica gel column (150 g E. Merck 40-63 µ ) with 9 : 1 Hexanes : EtOAc. The bromide is obtained as an oil.
Characteristic NMR Resonances; ¹H NMR 400MHz (CDCl₃); 7.21 (t, 1H, J=7.9 Hz), 6.86-6.76 (m, 3H), 3.97 (t, 2H, J=6.0 Hz), 3.67 (s, 3H), 3.58 (s, 2H), 3.47 (t, 2H, J=6.6 Hz), 2.02-2.09 (complex m, 2H), 1.89-1.96 (complex m, 2H).
MS CI NH₃ M+NH₄⁺ =318 / 320. MW = 300 / 302 ( Bromine Isotopes).

### Step 3

The 4-hydroxy-3-propylbenzophenone described in Example 55 Step 1 (38 mg, 1.0Eq, 0.16 mmol ) and the 3-(4-bromobut-1-oxy)phenylacetate from Example 61 Step ( 54 mg, 1.0 Eq, 0.17 mmol) were coupled with CsCO₃ (50 mg, 1.0 Eq, 0.15 mmol) as for the procedure reported for Example 55, Step 2. The product was purified by elution from a silica gel column (3 g E. Merck 40-63 µ ) with toluene : hexanes : EtOAc 60 : 35 : 5.
Characteristic NMR Resonances; ¹H NMR 400MHz (CDCl₃); 7.74 (m, 2H), 7.66 (m, 2H), 7.54 (m, 1H), 7.45 (t, 2H, J = 7.5 Hz), 7.21 (t, 1H, J = 7.8 Hz), 6.83 (m, 4H), 4.09 (t. 2H, J = 5.9 Hz), 4.05 (t, 2H, J = 5.7 Hz), 3.67 (s, 3H), 3.58 (s, 2H), 2.61 (dd, 2H, J unresolved), 2.00 (m, 4H), 1.60 (sext, 2H, J = 7.5 Hz), 0.92 (t, 3H, J = 7.5 Hz).
MS ESI M+1 = 461.4. MW = 460.2.

### Step 4

The ester of Example 61 step 3 ( 54.3 mg, 1.0 Eq, 0.118 mmol) was cleavedwith LiOH aq ( 150 µl, 1.5 N, 2 Eq ) as for Example 55, Step 3. Characteristic NMR Resonances; ¹H NMR 400MHz (CDCl₃); 7.75 (m, 2H), 7.67 (m, 2H), 7.54 (m, 1H), 7.47 (t, 2H, J = 7.6 Hz & fine splitting.), 7.25 (m, 1H), 6.8-6.9 (m, 4H), 4.13 (t, 2H, J = 5.2 Hz), 4.05 (t, 2H, J = 5.8 Hz), 3.62 (2, 2H), 2.62 (dd, 2H, J unresolved), 2.02 (m, 4H), 1.61 (sext, 2H, J = 7.5 Hz), 0.94 (t, 3H, J = 7.3 Hz).
MS ESI M+1 = 447.3. MW = 446.0.

### Example 62

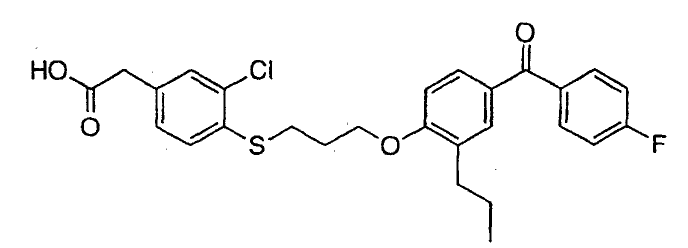

### 3-chloro-4-(3-(4-fluorobenzoyl-2-propylphenoxy)propylthio)phenyl acetic acid

### Step 1

Commercially available 4-fluoro-4'-hydroxybenzophenone ( 2 g, 1.0 Eq, 9.3 mmol ) was combined with allyl bromide ( 1.6 ml, 2.0 Eq, 18.5 mmol ) in methanol ( 20 ml ) with K₂CO₃ ( 2.6 g, 2.0 Eq, 18.5 mmol ). The mixture was heated under reflux for 24 Hrs. The mixture was cooled to RT, reduced *i*. *vac.* and dissolved in EtOAc and 0.25 M aq K₂CO₃. The mixture was extracted with EtOAc, the extracts washed with NH₄Cl aq sat'd, and dried over Na₂SO₄. The EtOAc solution was reduced to an oil *i*. *vac.* The product was purified by recrystallization from methanol ( 50 ml ).
Characteristic NMR Resonances; ¹H NMR 400MHz (CDCl₃); 7.78 (collapsed ABq, 4H), 7.13 (t, 2H, J = 8.8 Hz plus fine splitting), 6.96 (d, 2H, J = 9 Hz plus fine splitting), 6.05 (m, 1H), 5.41 (dq, 1H, J = 17.3, 1.6 Hz), 5.31 (dq, 1H, J = 16.5, 1.6 Hz), 4.60 (dt, 2H, J = 5.3, 1.5 Hz).

### Step 2

The 4-fluoro-4'-allyloxybenzophenone of Example 62 step 1 (1.4 g, 5.5 mmol ) was rearranged as in Example 3 Step C. The mixture was heated under reflux for 40 Hrs. The product was purified by elution from a silica gel column (E. Merck 40-63 µ ) with hexanes : EtOAc 80 : 20.
Characteristic NMR Resonances; ¹H NMR 400MHz (CDCl₃); 7.77 (dd, 2H, J = 8.8, 5.5 Hz plus fine splitting.), 7.64 (d, 1H, 2.2 Hz), 7.58 (dd, 1H, J = 8.3, 2.2 Hz), 7.13 (t, 2H, 8.8 Hz plus fine splitting), 6.86 (d, 1H, J = 8.3 Hz), 5.99 (m, 1H), 5.65 (s, 1H), 5.17 (m, 2H), 3.45 (brd d, 2H, J = 6.4 Hz).

### Step 3

The 3-allyl-4-fluoro-4'-hydroxybenzophenone of Example 62 Step 2 ( 33.4 mg, 0.13 mmol ) was reduced in EtOAc ( 6 ml ) as for Example 3 Step D. The product was purified by elution from a silica gel column (E. Merck 40-63 µ ) with hexanes : EtOAc 80 : 20.
Characteristic NMR Resonances; ¹H NMR 400MHz (CDCl₃); ); 7.79 (dd, 2H, J = 8.8, 5.5 Hz plus fine splitting.), 7.64 (d, 1H, 2.1 Hz), 7.56 (dd, 1H, J = 8.3, 2.2 Hz), 7.15 (t, 2H, 8.7 Hz plus fine splitting), 6.82 (d, 1H, J = 8.3 Hz), 5.52 (s, 1H), 2.63 (t, 2H, J = 7.4 Hz), 1.66 (sext, 2H, J = 7.5 Hz), 0.98 (t, 3H, J = 7.4 Hz).
MS ESI M+1 = 259.1. MW =258.1.

### Step 4

The 4-fluoro-4'-hydroxy-3-propylbenzophenone of Example 62 Step 3 (25.5 mg, 1.0 Eq, 0.099 mmol ) and the bromide from Example 51 step A ( 35 mg, 1.05 Eq, 0.105 mmol) were coupled with CsCO₃ (33.7 mg, 1.05 Eq, 0.105 mmol) as for the procedure reported for Example 55, Step 2. The product was purified by elution from a silica gel column (2 g E. Merck 40-63 µ ) with toluene : hexanes : EtOAc 60 : 35 : 5.
Characteristic NMR Resonances; ¹H NMR 400MHz (CDCl₃); 7.78 (dd, 2H, J = 8.8, 5.5 Hz plus fine splitting), 7.62 (m, 2H), 7.26 (m, 2H), 7.13 (m, 3H), 6.84 (d, 1H, J = 8.4 Hz), 4.16 (t, 2H, J = 5.8 Hz), 3.68 (s, 3H), 3.55 (s, 2H), 3.15 (t, 2H, J = 7.3 Hz), 2.62 (dd, 2H, J unresolved), 2.18 (pent, 2H, J = 7.1 Hz), 1.60 (sext, 2H, J = 7.5 Hz), 0.93 (t, 3H, J = 7.3 Hz).
MS ESI M+1 = 515.3. MW = 514.1.

### Step 5

The ester of Example 62 Step 4 ( 31 mg, 1.0 Eq, 0.06 mmol ) was cleavedwith LiOH aq ( 90 µl, 1.5 N, 2 Eq ) as for Example 55, Step 3. Characteristic NMR Resonances; ¹H NMR 400MHz (CDCl₃); 7.79 (m, 2H), 7.58 (m, 2H), 7.28 (m, 2H), 7.13 (t, 2H, J = 8.8 Hz), 7.08 (dd, 1H, J = 8.0, 2.0 Hz), 6.79 (d, 1H, J = 8.4 Hz), 4.15 (t, 2H, J = 5.7 Hz), 3.55 (s, 2H), 3.16 (t, 2H, J = 7.0 Hz), 2.60 (dd, 2H, J unresolved), 2.18 (pent, 2H, J = 6.0 Hz), 1.60 (sext, 2H, J = 7.5 Hz), 0.93 (t, 3H, J = 7.4 Hz).
MS ESI M+1 = 501.2. MW = 500.1.

### Example 63

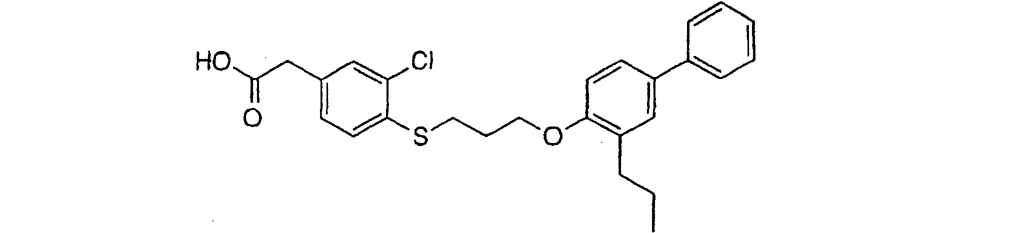

### 3-chloro-4-(3-(4-phenyl-2-propylphenoxy)propylthio)phenyl acetic acid

### Step 1

Commercially available 4-hydroxybiphenyl ( 2.0 g, 1.0 Eq, 0.012 mol ) and allyl bromide ( 1 ml, 1.0 Eq, 0.012 mol ) were combined in DMF ( 5 ml ) with K₂CO₃ ( 3.27 g, 2.0 Eq, 0.024 mol ). The mixture was stirred 2 1/2 Hrs at RT. The mixture was diluted with EtOAc and H₂O, extracted with EtOAc, the extracts washed with NH₄Cl aq sat'd, and dried over MgSO₄. The EtOAc solution was reduced to an oil *i*. *vac* The product was purified by elution from a silica gel column (97 g E. Merck 40-63 µ ) with hexanes : EtOAc 95 : 5.
Characteristic NMR Resonances; ¹H NMR 400MHz (CDCl₃); 7.52 (m, 4H, 2 doublets with fine splitting), 7.39 (t, 2H, J = 7.4 Hz), 7.28 (m, 1H), 6.97 (d, 2H, J = 8.9 Hz plus fine splitting), 6.05 (m, 1H), 5.42 (dq, 1H, J = 17.3, 1.7 Hz), 5.29 (dq, 1H, J = 10.5, 1.4 Hz), 4.57 (dt, 2H, J = 5.3, 1.6 Hz).
MS EI M+ 210.1 MW = 210.1.

### Step 2

The 4-allyloxybiphenyl of Example 63 Step 1 ( 2.2 g, 0.01 mol ) was rearranged as in example 3 Step C. The mixture was heated under reflux for 40 Hrs. The product was purified by elution from a silica gel column ( 80 g E. Merck 40-63 µ ) with hexanes : EtOAc 80 : 20. Characteristic NMR Resonances; ¹H NMR 400MHz (CDCl₃); 7.52 (m, 2H), 7.39 (t, 2H, J = 7.4 Hz), 7.35 (m, 2H), 7.29 (m, 1H), 6.86 (d, 1H, J = 8.1 Hz), 6.04 (m, 1H), 5.18 (m, 2H), 4.94 (s, 1H), 3.46 (d, 2H, J = 5.7 Hz).
MS EI M+ = 210.1. MW = 210.1.

### Step 3

The 3-allyl-4-hydroxybiphenyl of Example 63 Step 2 ( 869 mg, 4.1 mmol ) was reduced in EtOAc ( 40 ml ) as for Example 3 Step D.
The product was used in step 4 without purification.
Characteristic NMR Resonances: ¹H NMR 400MHz (CDCl₃);); 7.52 (m, 2H), 7.39 (t, 2H, J = 7.4 Hz), 7.34 (d, 2H, J = 2.3 Hz), 7.29 (m, 2H), 6.81 (d, 1H, J = 8.2 Hz), 4.68 (s, 1H), 2.63 (dd, 2H, J unresolved), 1.67 (sext, 2H, J = 7.6 Hz), 0.99 (t, 3H, J = 7.3 Hz).
MS CI NH₃ M+ = 212.1. MW = 212.1.

### Step 4

The 4-hydroxy-3-propylbiphenyl described in Example 63 Step 3 ( 232 mg, 1.0 Eq, 1.1 mmol ) and the bromide from Example 51 step A ( 380 mg, 1.0 Eq, 1.1 mmol) were coupled with CsCO₃ ( 377 mg, 1.05 Eq, 1.15 mmol) as for the procedure reported for Example 55, Step 2. The product was purified by elution from a silica gel column (21 g E.
Merck 40-63 µ ) with toluene : hexanes : EtOAc 60 : 35 : 5.
Characteristic NMR Resonances; ¹H NMR 400MHz (CDCl₃); 7.53 (m, 2H), 7.39 (t, 2H, J = 7.5 Hz), 7.36 (m, 2H), 7.29 (m, 3H), 7.11 (dd, 1H), 6.86 (d, 1H, J = 9.1 Hz), 4.12 (t, 2H, J = 5.7 Hz), 3.68 (s, 3H), 3.55 (s, 2H), 3.16 (t, 2H, J = 7.1 Hz), 2.64 (dd, 2H, J unresolved), 2.17 (m, 2H), 1.64 (sext, 2H, J = 7.5 Hz), 0.95 (t, 3H, J = 7.3 Hz).

### Step 5

The ester of Example 63 Step 4 ( 272 mg, 1.0 Eq, 0.58 mmol ) was cleavedwith LiOH aq ( 800 µl, 1.5 N, 2 Eq ) as for Example 55, Step 3. Characteristic NMR Resonances; ¹H NMR 400MHz (CDCl₃); ); 7.55 (m, 2H), 7.41 (t, 2H, J = 7.3 Hz), 7.38 (m, 2H), 7.29 (m, 3H), 7.14 (dd, 1H, J = 7.2, 2.0 Hz), 6.88 (d, 1H, J = 9.1 Hz), 4.13 (t, 2H, J = 5.8 Hz), 3.60 (s, 2H), 3.18 (t, 2H, J = 7.4 Hz), 2.66 (dd, 2H, J = 8.9, 7.4 Hz), 2.19 (pent, 2H, J = 5.8 Hz), 1.65 (sext, 2H, J = 7.6 Hz), 0.97 (t, 3H, J = 7.4 Hz).
MS EI M+ = 454.1. MW = 454.1.

### Example 64

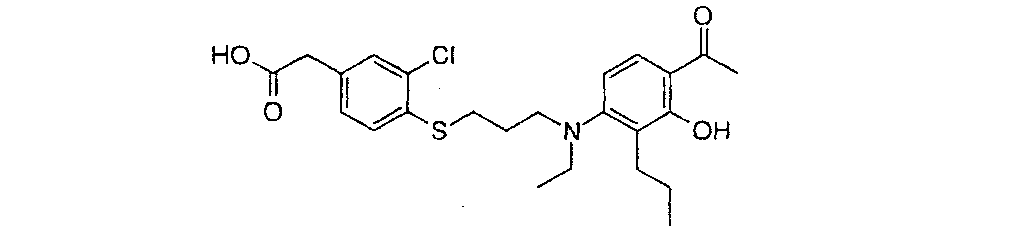

### 3-chloro-4-(3-(N-ethyl-N-(4-acetyl-3-hydroxy-2-propylphenyl)-amino)propylthio)phenyl acetic acid

### Step 1

Commercially available N-ethyl-4-amino-2-hydroxy-3-propylacetophenone ( 120 mg, 1.0 Eq, 0.54 mmol ) was alkylated with the bromide prepared in Example 51 Step A ( 189 mg, 1.05 Eq, 0.56 mmol ) in N-methylpyrrolidin-2-one ( 0.6 ml ) with MgO (22 mg, 1.0 Eq, 0.5 mmol) as for Example 66 Step 1 below. The mixture was heated at 80 C for 72 Hrs. The product was purified by elution from a reversed phese RP-8 col (10 g E. Merck 40-63 µ ) with 65 : 35 CH₃CN : H₂O 0.1 % TFA. The TFA salt of the ester is obtained as a glass. Characteristic NMR Resonances; ¹H NMR 400MHz (CD30D); 7.77 (brd, 1H), 7.29 (d, 1H, J = 1.8 Hz), 7.16 (d, 1H, J = 8.2 Hz), 7.09 (m, 1H), 6.88 (brd, 1H), 3.68 (s, 3H), 3.59 (s, 2H), 3.43 (brd. 1H), 3.30 (brd, 1H), 2.96 (t, 2H, J = 7.0 Hz), 2.64 (m, 2H), 2.62 (s, 3H), 1.75 (brd, 2H), 1.55 (m, 2H), 1.06 (t, 3H, J = 7.1 Hz), 0,97 (t, 3H, J = 7.4 Hz).
MS ESI M+1 = 478.3. MW = 477.2.

### Step 2

The ester of Example 64 Step 1 ( 23 mg, 1.0 Eq, 0.048 mmol ) was cleavedwith LiOH aq ( 100 µl, 1.5 N, 3 Eq ) as for Example 55, Step 3. Characteristic NMR Resonances; ¹H NMR 400MHz (CD₃OD); 7.78 (brd, 1H), 7.31 (d, 1H, J = 1.9 Hz), 7.18 (d, 1H, J = 8.0 Hz), 7.10 (m, 1H), 6.90 (brd, 1H), 3.55 (s, 2H), 3.47 (brd, 1H), 3.33 (brd. 1H), 2.96 (t, 2H, J = 6.8 Hz), 2.65 (m, 2H), 2.61 (s, 3H), 1.75 (brd, 2H), 1.55 (m, 2H), 1.06 (t, 3H, J = 7.1 Hz), 0,97 (t, 3H, J = 7.3 Hz).
MS ESI M+1 = 464.1. MW = 463.1.

### Example 65 (Reference)

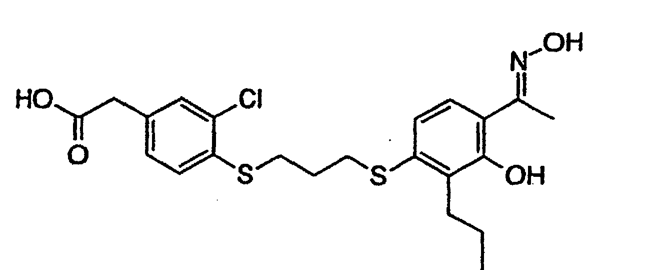

### 3-chloro-4-(3-((4-hydroximinoacetyl-3-hydroxy-2-propylphenyl)-thio)propylthio)phenyl acetic acid

### Step 1

Commercially available 2-hydroxy-3-propyl-4-(N,N-dimethylthiocarbamoyl)acetophenone ( 102 mg, 1.0 Eq, 0.36 mmol ) was treated with a solution of sodium methoxide ( 165 µL 4.63 M MeOH, 2.1 Eq, 0.76 mmol ) in methanol ( .5 ml ) with heating to 80 C under reflux. Stirring was continued until the starting thiocarbamate was consumed, 2 1/2 Hrs. The methanol solvent was removed *i*. *vac.* The residue was dissolved in DMF (0.3 ml ) with the bromide described in Example 51 Step A 124 mg, 1.0 Eq, 0.36 mmol ). The reaction mixture was stirred 16 Hrs at RT. The mixture was diluted with EtOAc and H₂O, acidified with dilute HCl and extracted with EtOAc. The extracts were washed with NaCl aq sat'd, and dried over MgSO₄. The EtOAc solution was reduced to an oil *i. vac*
The product was purified by elution from a silica gel column (7 g E. Merck 40-63 µ ) with toluene : EtOAc 97 : 3.
Characteristic NMR Resonances; ¹H NMR 400MHz (CDCl₃); 7.50 (d, 1H, J = 8.6 Hz), 7.31 (d, 1H, J = 1.8 Hz), 7.22 (m, 2H), 7.08 (dd, 1H), 6.72 (d, 1H, J = 8.7 Hz), 3.70 (s, 3H), 3.56 (s, 2H), 3.14 (t, 2H, J = 7.1 Hz), 3.09 (t, 2H, J = 6.9 Hz), 2.74 (m, 2H), 2.59 (s, 3H), 2.05 (pent, 2H, J = 7.0 Hz), 1.55 (m, 2H), 0.99 (t, 3H, J = 7.4 Hz).
MS ESI M+1 = 467.3. MW = 466.1

### Step 2

The ester of Example 65 Step 1 ( 76 mg, 1.0 Eq, 0.16 mmol ) was converted to the oxime according to the general procedure of Example 10. Heating under reflux was continued for 16 Hrs.
The product was purified by elution from a silica gel column (2.5 g E. Merck 40-63 µ ) with 97 : 3 toluene : EtOAc. The ester is obtained as a glass.
Characteristic NMR Resonances; ¹H NMR 400MHz (CDCl₃); 7.50 (d, 1H, J = 8.6 Hz), 7.31 (d, 1H, J = 1.8 Hz), 7.22 (m, 1H), 7.10 (dd, 1H, J = 8.1, 1.8 Hz), 6.72 (d, 1H, J = 8.6 Hz), 3.70 (s, 3H), 3.56 (s, 2H), 3.14 (t, 2H, J = 7.1 Hz), 3.09 (t, 2H, J = 7.0 Hz), 2.74 (m, 2H), 2.59 (s, 3H), 2.05 (pent, 2H, J = 7.0 Hz), 1.56 (m, 2H), 0.99 (t, 3H, J = 7.4 Hz). Spectrum is essentially identical to that of the ketone at 400 MHz.
MS ESI M+1 482.2 MW 481.1

### Step 3

The ester of Example 65 step 2 ( 14 mg, 1.0 Eq, 0.03 mmol ) was cleavedwith LiOH aq ( 30 µl, 2.0 N, 2 Eq ) as for Example 55, Step 3. Characteristic NMR Resonances; ¹H NMR 400MHz (CDCl₃); ); 7.27 (d, 1H, J = 1.8 Hz), 7.18 (d, 1H, J = 8.5 Hz), 7.14 (d, 1H, J = 8.2 Hz), 7.04 (dd, 1H, J = 8.1, 1.9 Hz), 6.77 (d, 1H, J = 8.7 Hz), 3.56 (s, 2H), 3.07 (t, 2H, J = 6.9 Hz), 3.05 (t, 2H, J = 7.3 Hz), 2.77 (m, 2H), 2.30 (s, 3H), 1.99 (pent, 2H, J = 7.0 Hz), 1.55 (sext, 2H, J = 7.6 Hz), 0.97 (t; 3H, J = 7.4 Hz).
MS ESI M+1 = 468.3. MW = 467.1.

### Example 66

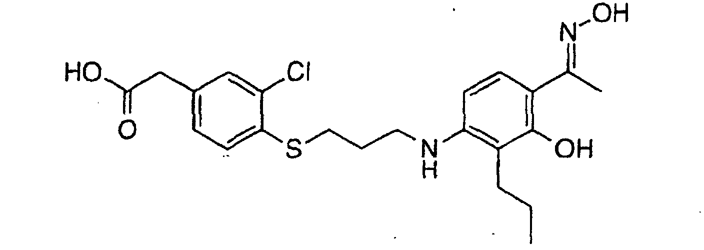

### 3-chloro-4-(3-(N-(4-hydroximinoacetyl-3-hydroxy-2-propylphenyl)-amino)propylthio)phenyl acetic acid

### Step 1

Commercially available 4-amino-3-propyl-2-hydroxyacetophenone ( 93 mg, 1.0 Eq, 0.48 mmol ) and the bromide described in Example 51 Step A ( 178 mg, 1.1 Eq, 0.53 mmol ) were dissolved in N-methylpyrrolidin-2-one ( 0.6 ml ) with MgO (20 mg, 1.05 Eq, 0.5 mmol). The mixture was heated at 80 C for 22 Hrs. The reaction mixture was poured into dilute NaHCO₃ aq. and EtOAc. The aqueous phase was extracted with EtOAc and the EtOAc extracts washed with sat'd aq NaCl. The EtOAc extracts were dried over MgSO₄ and reduced *i. vac* The product was purified by elution from a silica gel column (10 g E. Merck 40-63 µ ) with 97 : 3 Toluene : EtOAc. The ester is obtained as a glass.
Characteristic NMR Resonances; ¹H NMR 400MHz (CD₃OD); 7.52 (d, 1H, J = 9.0 Hz), 7.31 (d, 1H, J = 1.7 Hz), 7.26 (d, 1H, J = 8.2 Hz), 7.10 (dd, 1H), 6.24 (d, 1H, J = 8.5 Hz), 3.67 (s, 3H), 3.60 (s, 2H), 3.43 (q, 2H, J = 6.3 Hz), 3.04 (t, 2H, J = 7.1 Hz), 2.51 (t, 2H, J = 7.0 Hz), 2.46 (s, 3H), 1.95 (pent, 2H, J =7.1 Hz), 1.47 (sext, 2H, J = 7.7 Hz), 0.94 (t, 3H, J = 7.4 Hz).
MS ESI M+1 = 450.6. MW = 449.1.

### Step 2

The ester of Example 66 Step 1 ( 87 mg, 1 Eq, 0.2 mmol ) was converted to the oxime according to the general procedure of Example 10. Heating under reflux was continued for 16 Hrs.
The product was purified by elution from a silica gel column (7 g E. Merck 40-63 µ ) with 97 :3 toluene : EtOAc. The ester is obtained as a glass.
Characteristic NMR Resonances; ¹H NMR 400MHz 400MHz (CD₃OD); 7.53 (d, 1H, J = 9.0 Hz), 7.31 (d, 1H, J = 1.8 Hz), 7.26 (d, 1H, J = 8.1 Hz), 7.09 (dd, 1H, J = 8.3, 1.9 Hz), 6.23 (d, 1H, J = 9.1 Hz), 3.67 (s, 3H), 3.59 (s, 2H), 3.43 (t, 2H, J = 6.8 Hz), 3.03 (t, 2H, J = 7.1 Hz), 2.53 (dd, J = 9.1, 7.8 Hz), 2.46 (s, 3H), 1.95 (pent, 2H, J =7.5 Hz), 1.47 (sext, 2H, J = 7.6 Hz), 0.94 (t, 3H, J = 7.4 Hz).
MS ESI M+1 = 465.3. MW = 464.2

### Step 3

The ester of ( 17.6 mg, 1.0 Eq, 0.04 mmol ) was cleavedwith LiOH aq (40 µl, 2.0 N, 2 Eq ) as for Example 55, Step 3.
Characteristic NMR Resonances; ¹H NMR 400MHz 400MHz (CD₃OD); 7.31 (d, 1H, J = 1.8 Hz), 7.26 (d, 1H, J = 8.1 Hz), 7.14 (d, 1H, J = 8.8 Hz), 7.10 (dd, 1H), 6.21 (d, 1H, J = 8.8 Hz), 3.55 (s, 2H), 3.34 (t, 2H, J = 6.6 Hz), 3.03 (t, 2H, J = 7.1 Hz), 2.53 (dd, J = 9.4, 7.7 Hz)), 2.22 (s, 3H), 1.95 (pent, 2H, J =7.0 Hz), 1.49 (sext, 2H, J = 7.6 Hz), 0.95 (t, 3H, J = 7.4 Hz).
MS ESI M+1 = 451.2. MW = 450.

### Example 67

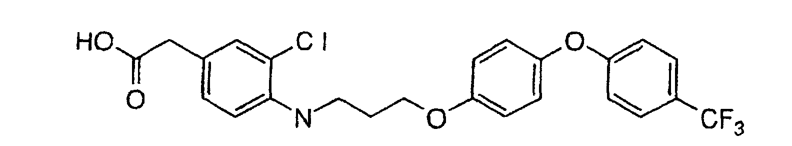

### 3-chloro-4-(4--phenoxy (4-trifluoromethyl)-propylamino) phenyl acetic acid.

### Step A: Preparation of methyl 3-chloro-4-(3 bromopropylamino)-phenylacetate

The titled compound was pepared according to the method described in Example 52, Step C.

### Step B: Preparation of methyl-3-chloro-4-(4--phenoxy (4-trifluoromethyl)-propylamino) phenylacetate

The titled compound was pepared according to the method described in Example 52, Step F, using 4-(4-(trifluoromethyl)-phenoxy)-phenol as starting material.
¹H NMR (400 MHz, CDCl₃, ppm ): δ 7.52-6.61(m, 11H,),), 7.03 (m, 4.09 (t, 2H, J=4.7 Hz), 3.66(s, 3H), 3.47 (s, 2H), 3.67-3.41 (m, 2H), 2.14 (t, 2H, J=5.9Hz).

### Step C: Preparation of 3-chloro-4-(4- phenoxy-(4-trifluoromethyl)-propylamino)-phenyl acetatic acid.

The titled compound was prepared according to the method described in Example 53, Step A. using methyl-3-chloro-4-(4-phenoxy (4-trifluoromethyl-phenoxy)-propylamino) phenylacetate.
¹H NMR (400 MHz, CDCl₃, ppm ): δ 7.53-6.62(m, 11H), 4.08 (t, 2H, J=5.8), 3.50 (s, 2H), 3.39 (m, 2H, J=6.3), 2.14 (m, 2H, J=6.0), 1.60 (m, 2H),
ESI: MS: m/e= 479(M+1)

### Example 68

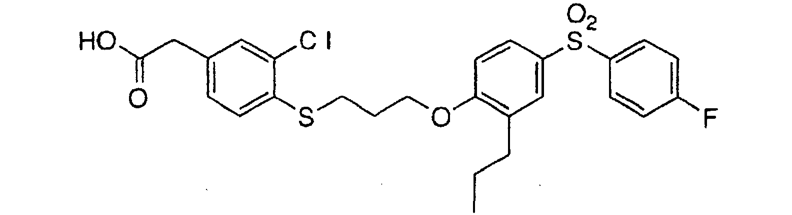

### 3-chloro-4-(4- phenoxy-2-propyl-(4-(4-fluoro)phenylsulfonyl)-propylthio)-phenyl acetic acid.

### Step A: Preparation of methyl 3-chloro-4-(3-bromopropvlthio)-phenylacetate

The titled compound was prepared according to the method described in Example 51, Step A,.

### Scheme B: Preparation of 1-propenyloxy-phenyl-4-(4-fluorophenyl)sulfone.

A solution of allyl alcohol (0.0197 mol) in DMF (30 ml)was reacted with sodium hydride (0.0197 mol) for 1/2 hr. Then 4-fluoro phenyl sulfone (0.0197 mol) was added and the mixture was stirred over night at 24°. The reaction was quenched with water. Extracted with ethyl acetate. The organic layer was washed with water, brine, dried over sodium sulfate, filtered, concentrated in vacuo, and the crude residue was purified by flash chromatography on silica gel (10% ethyl acetate/hexane) to provide the title compound.
¹H NMR (400 MHz, CDCl_{3,} ppm ): δ 7.93-6.95(m, 5H), 6.02-5.95(m, 1H), 5.40-5.27 (m, 1H), 4.54 (d, 2H, J = 3.8 Hz).

### Scheme C: Preparation of 2-propyl-(4-(4-fluorophenyl)-sulfonyl)-1-phenol

This compound was prepared according to the method described in Example 52, StepE, using 1-propenyloxy-phenyl-4-(4-fluoro-phenyl)sulfone.
¹H NMR (400 MHz, CDCl_{3,} ppm ): δ 7.93-6.81(m, 5H), 2.56(t, 2H, J=7.6 Hz), 1.64-1.40 (m, 2H), 0.92 (t, 3H, J = 7.4 Hz).

### Scheme D: Preparation of methyl-3-chloro-4- ((4-(4- fluorophenyl)-2-propylphenoxy sulfonyl) -propylthio)-phenyl acetate.

The titled compound was prepared according to the method described in Example 51, Step C, using 2-propyl-(4-(4-fluorophenyl)-sulfonyl)-1-phenol
¹H NMR (400 MHz, CDCl_{3,} ppm ): δ 7.91-6.83(m, 10H), 4.11(t, 2H, J=5.6 Hz), 3.67(s, 3H), 3.53 (s, 2H), 3.10(t, 2H, J=7.2 Hz), 2.57(t, 2H, J=7.4 Hz), 2.14(t, 2H, J=6.4 Hz), 1.59-1.51 (m, 2H), 0.89 (t, 3H, J = 7.3 Hz)

### Step E: Preparation of 3-chloro-4-(4- phenoxy-2-propyl-(4-(4-fluoro)phenylsulfonyl) -propylthio)-phenyl acetatic acid.

The titled compound was prepared according to the method described in Example 51, Step E, using methyl-3-chloro-4- ((4-(4-fluorophenyl)- 2-propylphenoxy sulfonyl) -propylthio)- phenyl acetate. ¹H NMR (400 MHz, CDCl_{3,} ppm ): δ 7.91-6.78(m, 10H), 4.10(t, 2H, J=5.8 Hz), 3.55 (s, 2H), 3.11(t, 2H, J=6.9 Hz), 2.55(t, 2H, J=7.4 Hz), 2.14(t, 2H, J=6.3 Hz), 1.59-1.51 (m, 2H), 0.89 (t, 3H, J = 7.2 Hz)
ESI: MS: m/e= 554(M+17).

### Example 69

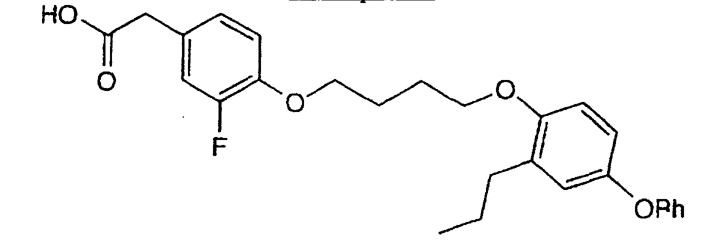

### 3-fluoro-4-(4-(4-phenoxv-2-propylphenoxy)-butyloxy)phenylacetic acid

### STEP A: Preparation of 2-propyl-4-phenoxy phenol

A solution of 1-phenoxy-(4-propenyloxy)benzene (0.9 grams) in ortho-dichlorobenzene 8 mL) was refluxed for 22 hours. Mixture was cooled to room temperature and was chromatographed over silica gel to afford the intermediate which was hydrogenated over 10% Pd/C catalyst (90 mg) in ethyl acetate for 18 hours. The reaction was filtered through Celite and all volatiles were removed to afford the title compound.
NMR (CDCl₃) δ 7.15 (m, 8H), 2.53 (t, 2H J=7.4 Hz), 1.60 (m, 2H), 0.94 (t, 3H, J = 7.3Hz).

### STEP B: Preparation of Methyl 3-fluoro-4-(4-(4-bromobutvloxy)-phenylacetate

Using the method in example 6 step A, substituting methyl 3-fluoro-4-hydroxyphenylacetate and 1,4-dibromobutane as the starting materials, the titled compound was obtained. This compound was taken forward without further purification.

### STEP C: Preparation of Methyl 3-fluoro-4-(4-(4-phenoxy-2-propylphenoxy)-butyloxy)phenylacetate

A solution of methyl 3-fluoro-4-(4-bromobutyloxy)-phenylacetate and 2-propyl-4-phenoxy phenol(0.21 grams) and potassium carbonate (0.07 grams) in 2-butanone (4 mL). The mixture was refluxed overnight. The reaction mixture was cooled to room temperature and partitioned between isopropyl acetate and pH 4 buffer. The organic layer was separated, washed with water, dried over MgSO4, and concentrated. Column Chromatography (silica gel 60, 50% methylene chloride in hexane) gave the tittle compound.
NMR (CDCl₃) δ 7.27 (m, 3H), 7.00 (m, 2H), 6.91 (m, 3H), 6.73 (m, 2H); 4.09 (t, 2H); 4.03 (t, 2H); 3.67 (s, 3H); 3.46 (s, 2H); 2.54 (t, 2H, J = 7.36 Hz); 2.01 (m, 4H); 1.56 (m, 2H); 0.90 (t, 3H, J = 7.4 Hz).

### STEP D: Preparation of 3-fluoro-4-(4-(4-phenoxy-2-propylphenoxy)-butyloxy)phenylacetic acid

Using the method in example 2 step A, substituting Methyl 3-fluoro-4-(4-(4-phenoxy-2-propylphenoxy)-butyloxy)phenylacetate as the starting material, the titled compound was obtained.
NMR (CDCl₃) δ 7.27 (m, 3H), 7.00 (m, 2H), 6.91 (m, 3H), 6.73 (m, 2H); 4.09 (t, 2H); 4.03 (t, 2H); 3.56 (s, 2H); 2.54 (t, 2H, J = 7.36 Hz); 2.01 (m, 4H); 1.56 (m, 2H); 0.90 (t, 3H, J = 7.4 Hz).
ESI: Mass spec: m/e = 453 (M+1).

### EXAMPLE 70

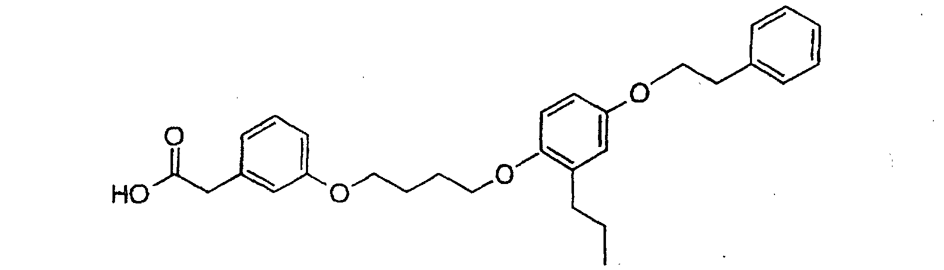

### 3-(4-(1-(2-propyl-4-phenethyl)hydroquinolyl)butyloxy)phenylacetic acid

### Step A: Preparation of 1-Allylhydroquinone

To a solution of 200 mg (1.82 mmol) hydroquinone in 1.5 ml methylethyl ketone was added 252 mg (1.82 mmol) potassium carbonate, and 158 µL (1.82 mmol) allyl bromide. The reaction mixture was stirred at 100°C for 2 hours. The reaction was quenched with water at room temperature and extracted with isopropyl acetate. The combined organic layers were washed with brine, and dried over sodium sulfate. After filtration and concentration of the filtrate *in vacuo*, the crude product was flash chromatographed (eluted with methylene chloride) to yield the titled compound, homogeneous by TLC.
¹HNMR (500 MHz, CDCl₃, ppm): δ 6.8 (m, 4H), 6.06 (m, 1H), 5.41 (m, 1H), 5.29 (m, 1H), 4.59 (d, 1H, J = 1.8 Hz), 4.50 (m, 2H). Probe EI-
MS: (M+1) = 150.

### Step B: Preparation of 4-Phenethyl-1-allylhydroquinone

The product obtained in Step A of Example 70 (200 mg; 1.34 mmol) was dissolved in 1.5 ml DMF and treated with 437 mg (1.34 mmol) cesium carbonate and 183 µL (1.34 mmol) phenethyl bromide. The reaction mixture stirred at room temperature for 16 hours. The reaction was quenched with water and acidified using 10% citric acid solution, then extracted with ethyl acetate. The combined organics were washed with water, brine, and dried over sodium sulfate. After filtration and concentration of the filtrate *in vacuo*, the crude product was flash chromatographed (eluted with 1.0% ethyl acetate/hexane) to yield 61 mg (18%) of the titled compound as a pale orange liquid, homogeneous by TLC.
¹HNMR (500MHz, CDCl₃, ppm): δ 7.30 (m, 4H), 6.86 (m, 5H), 6.07 (m, 1H), 5.42 (m, 1H), 5.29 (m, 1H), 4.50 (m, 2H), 4.15 (t, 2H, J = 7.2 Hz), 3.10 (t, 2H, J = 7.1 Hz). CI-MS: (M+NH₄) = 272.

### Step C: Preparation of 2-Allyl-4-phenethylhydroquinone

The product obtained in Step B of Example 70 (61 mg; 0.240 mmol) was dissolved in 1.5 ml dichlorobenzene. The reaction mixture stirred at 205°C for 16 hours. The crude reaction mixture was loaded directly onto a flash column and purified (gradient elution with 5% - 20% ethyl acetate/hexane) to yield 46 mg (75%) of the titled compound as a pale orange gum, homogeneous by TLC.
¹HNMR (500MHz, CDCl₃, ppm): δ 7.29 (m, 5H), 6.72 (m, 3H), 6.00 (m, 1H), 5.16 (m, 2H), 4.59 (s, 1H), 4.13 (m, 2H), 3.38 (d, 2H, J = 6.2 Hz), 3.09 (t, 2H, J = 7.2 Hz).

### Step D: Preparation of 2-Propyl-4-phenethylhydroquinone

The product obtained in Step C of Example 70 (188 mg; 0.740 mmol) was dissolved in 2.0 ml methanol, and 30 mg 10% palladium on carbon added. The reaction mixture stirred under hydrogen for 16 hours. The contents of the reaction flask were centrifuged, and the residual catalyst washed with methanol three times. Solvents were evaporated *in vacuo*. The crude product was purified by flash chromatography (gradient elution with 1.0% - 6.0% ethyl acetate/hexane) to afford 163 mg (86%) of the titled compound as a white solid, homogeneous by TLC.
¹HNMR (500MHz, CDCl₃, ppm): δ 7.30 (m, 5H), 6.67 (m, 3H), 4.42 (s, 1H), 4.13 (t, 2H, J = 7.3 Hz), 3.09 (t, 2H, J = 7.3 Hz), 2.56 (t, 2H, J = 7.6 Hz), 1.65 (m, 2H), 0.99 (t, 3H, J = 7.3 Hz). CI-MS: (M+1) = 257; (M+ NH₄) = 274.

### Step E: Preparation of 3-(4-(1-(2-Propyl-4-phenethyl)hydroquinolyl)-butyloxy)phenylacetate

The product obtained in Step D of Example 70 (90 mg; 0.352 mmol) was dissolved in 1.5 ml DMF, and 54 mg (0.387 mmol) potassium carbonate and 116 mg (0.387 mmol) 3-(4-bromobutoxy)phenylacetate (Example 61 Step 1) were added. The reaction mixture stirred at 60°C for 16 hours. The reaction was quenched with water and extracted with ethyl acetate. The combined organic layers were washed with brine, and dried over sodium sulfate. The crude product was flash chromatographed to afford the titled compound as a mixture with residual starting material, homogeneous by TLC. The material was used without further purification.
¹HNMR (500MHz, CDCl₃, ppm): δ 7.29 (m, 6H), 6.85 (m, 2H), 6.71 (m, 2H), 6.63 (m, 2H), 4.13 (m, 2H), 4.05 (t, 2H, J = 5.8 Hz), 3.98 (t, 2H, J = 5.7 Hz), 3.71 (s, 3H), 3.61 (s, 2H), 3.09 (m, 2H), 2.56 (m, 2H), 1.99 (m, 4H), 1.58 (m, 2H), 1.57 (s, 2H), 0.97 (m, 3H). ESI-MS: (M+NH₄) = 494.

### Step F: Preparation of 3-(4-(1-(2-propyl-4-phenethyl)hydroquinolyl)butyloxy)phenylacetic acid

The product obtained in step E of Example 70 (110 mg; 0.231 mmol) was dissolved in 2.0 ml tetrahydrofuran and 308 mL (0.462 mmol) of a 1.5N lithium hydroxide solution was added. The reaction mixture stirred at room temperature for 3 hours. The reaction was quenched with 2N HCl and extracted with ethyl acetate. The combined organic layers were washed with water, brine, and dried over sodium sulfate. After filtration and concentration of the filtrate *in vacuo*, the crude material was purified by flash chromatography (gradient elution with 1.0% - 5.0% methanol/methylene chloride) to afford 91 mg (85%) of the titled compound as a cream-colored solid, homogeneous by TLC.
¹HNMR (500MHz, CDCl₃, ppm): δ 7.29 (m, 6H), 6.85 (m, 3H), 6.75 (m, 2H), 6.68 (m, 1H), 4.14 (t, 2H, J = 7.2 Hz), 4.04 (t, 2H, J = 5.8 Hz), 3.98 (t, 2H, J = 5.7 Hz), 3.63 (s, 2H), 3.09 (t, 2H, J = 7.3 Hz), 2.57 (t, 2H, J = 7.5 Hz), 1.98 (m, 4H), 1.61 (m, 2H), 0.95 (t, 3H, J = 7.3 Hz).
ESI-MS: (M+NH₄) = 480.

### Example 71

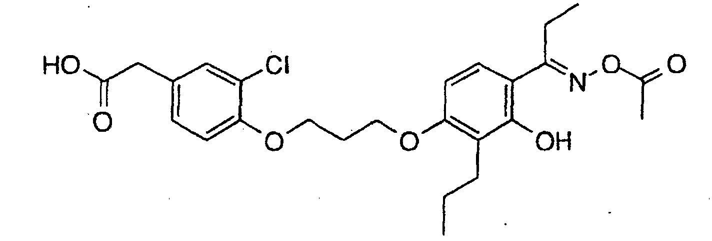

### 3-Chloro-4-(3-(2-propyl-3-hydroxy-4-(1-acetoxyiminopropyl))-phenoxy)propylthio)phenylacetic acid

### 1. 3-Chloro-4-(3-(2-propyl-3-hydroxy-4-(1-oxopropyl))phenoxy) propyloxy phenyl acetic acid methyl ester

A solution of 3-chloro-4-(3-bromo)propyloxyphenyl acetic acid methyl ester (0.552 grams) in 2-butanone (6 mL) was treated with 3-propyl-2,4-dihydroxy propiophenone (0.299 grams). Potassium carbonate (0.217 grams) was added and the mixture refluxed for 4 hours. The reaction was partitioned between isopropyl acetate and pH 4 buffer. The organic was dried over magnesium sulfate, filtered and evaporated to a residue which was chromatographed over silica gel to give the product.
NMR (CD₃OD) δ 7.77 (d, 1H, J = 9.0 Hz), 7.29 (d, 1H, J = 1.8 Hz), 7.13 (dd, 1H, J = 8.1, 1.9 Hz), 7.02 (d, 1H, J = 8.0 Hz), 6.62 (d, 1H, J = 9.0 Hz), 4.31 (t, 2H, J = 5.9 Hz), 4.24 (t, 2H, J = 5.9 Hz), 3.68 (s, 3H), 3.00 (quart, 2H, J = 7.2 Hz), 2.60 (bt, 2H, J = 7.6 Hz).

### 2. 3-Chloro-4-(3-(2-propyl-3-hydroxy-4-(1-hydroxyliminopropyl)) phenoxy)propyloxy phenyl acetic acid methyl ester

A solution of L-164,807 (0.05 grams; 0.111 mmol) in dry methanol (2 mL) was treated with hydroxylamine hydrochloride (0.008 grams; 0.117 mmol). Anhydrous sodium acetate (0.010 grams); 0.122 mmol) was added and the mixture refluxed for 4 hours. The reaction mixture was partitioned between isopropyl acetate and pH 7 buffer. The organic phase was washed once with water and dried over magnesium sulfate, filtered and evaporated to an oil. The product was used without further purification.
NMR (CD₃OD): 7.14 (dd, 1H, J = 8.1, 1.9 Hz), 7.02 (d, 1H, J = 8.1 Hz), 6.54 (d, 1H, J = 8.9 Hz), 4.23 (2 overlapping quarts, 4H), 3.67 (s, 3H), 2.82 (quart, 2H, J = 7.4 Hz), 2.61 (bt, 2H, J = 7.5 Hz).

### 3. 3-chloro-4-(3-(2-propyl-3-hydroxy-4-(1-hydroxyminopropyl)) phenoxy)propyloxy phenyl acetic acid

A solution of L-164,887 (0.113 grams; 0.252 mmol) in methanol (3 mL) was treated with a solution of lithium hydroxide in water (1.032 M; 0.305 mL). The reaction was refluxed 1 hour. The reaction mixture was partitioned between isopropyl acetate and 0.1N HCl. The organic was dried over magnesium sulfate, filtered and concentrated to a solid. The solid was covered with cyclohexane/methylene chloride (2:1; 2 mL), briefly refluxed, cooled and filtered. The title compound was used without further purification.
NMR (CDCl₃): 7.27 (d, 1H, J = 2.1 Hz); 7.22 (d, 1H, J = 9.1 Hz); 7.10 (dd, 1H, J = 8.6, 2.2 Hz); 6.88 (d, 1H, J = 8.5 Hz); 6.47 (d, 1H, J = 9.0 Hz); 4.20 (quart, 4H, J = 6.2 Hz); 3.55 (s, 2H); 2.82 (quart, 2H, J = 7.7 Hz); 2.63 (bt, 2H, J = 7.6 Hz).

### 4. 3-chloro-4-(3-(2-propyl-3-hydroxy-4-(1-acetoxyiminopropyl)) phenoxy)propyloxy phenyl acetic acid

A solution of 3-chloro-4-(3-(2-propyl-3-hydroxy-4-(1-hydroxyiminopropyl))phenoxy)propyloxy phenyl acetic acid (0.030 grams; 0.064 mmol) in freshly distilled acetic anhydride (1 mL) was stirred for 1 hour. The acetic anhydride was removed *in vacuo* and the residue flushed twice with toluene. The residue was dissolved in THF (1 mL). Water (0.25 mL) and saturated aqueous sodium bicarbonate (0.25 mL) were added. The mixture was stirred 30 minutes, then partitioned between isopropyl acetate and 0.01 N HCl. The organic was dried over magnesium sulfate, filtered and evaporated. The residue was digested in cyclohexane/methylene chloride (2:1; 1 mL). After stirring for 1 hour, the title compound was recovered by filtration.
NMR (CDCl₃): 7.25 (two overlapping d overlapping CHCl₃, 2H); 7.10 (dd, 1H, J = 8.4, 1.9 Hz); 6.88 (d, 1H, J = 8.4 Hz); 6.49 (d, 1H, J = 9.1 Hz); 4.21 (t , 4H, J = 5.9 Hz); 3.54 (s, 2H); 2.85 (quart, 2H, J = 7.7 Hz); 2.65 (bt, 2H, J = 7.7 Hz); 2.22 (s, 3H).

### Example 72

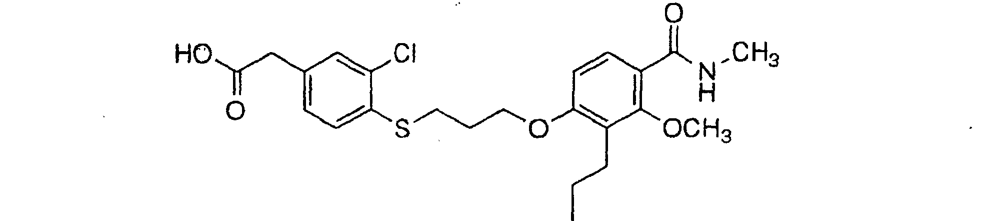

### 3-Chloro-4-(3-(2-propyl-3-methoxy-4-methylaminocarbonylphenoxy)propylthio)phenylacetic acid

### 1. Methyl 2-methoxy-3-propyl-4-benzyloxy benzoate

A solution of methyl 3-propyl-4-benzyloxy salicylate (0.719 grams; 2.394 mmol) in dry DMF (6 mL) was treated with methyl iodide (0.298 mL; 4.788 mmol) and cesium carbonate (0.936 grams; 2.873 mmol). The reaction was stirred at 25°C for 14 hours. The reaction was partitioned between isopropyl acetate and pH 4 buffer. The organic was washed 2 times with water and dried over magnesium sulfate, filtered and concentrated. The residue was chromatographed over silica gel to afford the product methyl ether.
NMR (CDCl₃): 7.67 (d, 1H, J = 8.9 Hz); 7.42-7.29 (m, 5H); 6.48 (d, 1H, J = 8.9 Hz); 5.12 (bs, 2H); 3.89 (s, 3H); 3.85 (s, 3H); 2.69 (bt, 2H, J = 7.4 Hz).

### 2. 2-Methoxy-3-propyl-4-benzyloxy benzoic acid

A solution of methyl 3-propyl-4-benzyloxy salicylate (0.350 grams; 1.113 mmol) in methanol (3.6 mL) was treated with a solution of lithium hydroxide in water (1.032 M; 1.08 mL; 1.115 mmol). The reaction was refluxed 1 hour. The reaction mixture was partitioned between isopropyl acetate and 0.1 N HCl. The organic was dried over magnesium sulfate, filtered and concentrated to the solid title compound, which was used without further purification (0.322 grams).
NMR (CDCl₃): 7.97 (d, 1H, J = 8.6 Hz); 7.62-7.41 (m, 5H); 6.83 (d, 1H, J = 8.7 Hz); 5.13 (bs, 2H); 3.90 (s, 3H); 2.67 (bt, 2H, J = 7.5 Hz).

### 3. N-methyl 2-Methoxy-3-propyl-4-benzyloxy benzamide

A solution of 2-methoxy-3-propyl-4-benzyloxy benzoic acid (0.307 grams; 1.022 mmol) in dry methylene chloride (4 mL) was treated with dry DMF (10µL). Oxalyl chloride (0.107 mL; 1.227 mmol) was added dropwise. Stirring was continued until gas evolution ceased (4 hours). The reaction was cooled to 0° C and treated with a solution of methylamine in THF (2.0 M; 3.0 mL; 6.0 mmol). The reaction was allowed to warm to 20° C and stir one hour. The reaction was partitioned between isopropyl acetate and water. The organic phase was dried over magnesium sulfate, filtered and evaporated *in vacuo* to afford the title compound as a white solid, which was used without further purification (0.333 grams).
NMR (CDCl₃): 7.90 (d, 1H, J = 8.4 Hz); 7.66 (vbs, 1H); 7.62-7.40 (m, 5H); 6.78 (d, 1H, J = 8.5 Hz); 5.11 (bs, 2H); 3.73 (s, 3H); 3.00 (d, 3H, J = 6.0 Hz); 2.67 (bt, 2H, J = 7.5 Hz).

### 4. N-methyl 2-Methoxy-3-propyl-4-hydroxy benzamide

A solution of N-methyl 2-methoxy-3-propyl-4-benzyloxy benzamide (0.333 grams) in ethyl acetate (10 mL) was hydrogenated (50 psi) using 10% Pd/C as catalyst (0.065 grams). After shaking for 9 hours, the mixture was filtered through Celite and evaporated to afford the title compound (0.217 grams).
NMR (CDCl₃): 8.38 (vbs, 1H); 7.89 (vbquart, 1H, J = 6.0 Hz); 7.76 (d, 1H, J = 8.3 Hz); 6.78 (d, 1H, J = 8.4 Hz); 3.72 (s, 3H); 3.01 (d, 3H, J = 5.9 Hz); 2.60 (bt, 2H, J = 7.8 Hz);

### 5. Methyl 3-chloro-4-(3-(2-propyl-3-methoxy-4-methylaminocarbonyl) phenoxy)propylthio phenyl acetate

A solution of N-methyl 2-methoxy-3-propyl-4-hydroxy benzamide (0.217 grams; 0.972 mmol) in dry DMF (3 mL) was treated with methyl 3-chloro-4-(3-bromopropyl)thiophenyl acetate (0.345 grams; 1.022 mmol). Cesium carbonate was added (0.348 grams; 1.068 mmol) and the mixture was stirred at 40° C for 16 hours. The reaction mixture was partitioned between isopropyl acetate and pH 4 buffer. The organic was washed twice more with water, then dried over magnesium sulfate. Filtration and evaporation afforded a residue which was chromatographed over silica gel to afford the title compound (0.328 grams).
NMR (CDCl₃): 7.89 (d, 1H, J = 8.7 Hz); 7.64 (vbquart, 1H, J = 4.4 Hz); 7.30 (d, 1H, J = 1.8 Hz); 7.25 (d, 1H, J = 9.2 Hz); 7.12 (dd, 1H, J = 8.1, 1.8 Hz); 6.69 (d, 1H, J = 8.8 Hz); 4.10 (t, 2H, J = 5.7 Hz); 3.72 (s, 3H); 3.68 (s, 3H); 3.55 (s, 2H); 3.12 (t, 2H, J = 7.2 Hz); 2.99 (d, 3H, J = 4.8 Hz); 2.60 (bt, 2H, J = 7.7 Hz).

### 6. 3-chloro-4-(3-(2-propyl-3-methoxy-4-methylaminocarbonyl) phenoxy)propylthio phenyl acetic acid

A solution of methyl 3-chloro-4-(3-(2-propyl-3-methoxy-4-methylaminocarbonyl)phenoxy)propylthio phenyl acetate (0.322 grams; 0.671 mmol) in methanol (4 mL) was treated with a solution of lithium hydroxide in water (1.032 M; 0.683 mL). The reaction was refluxed 1 hour. The reaction mixture was partitioned between isopropyl acetate and 0.1N HCl. The organic was dried over magnesium sulfate, filtered and concentrated to a stiff foam. The foam was stirred in methylene chloride/cyclohexane (3:1; 2 mL) for 2 hours. Stirring was stopped and the resultant oil allowed to settle. The supernatant was drawn off and replaced with methylene chloride/cyclohexane (3:1). After brief agitation the suspended viscous oil was allowed to settle. The supernatant was drawn off and the oil allowed to stand under high vacuum for 1 hour, affording the title compound as a white foam (0.302 grams).
NMR (CDCl₃): 7.59 (vbquart, 1H, J = 5.1 Hz); 7.56 (d, 1H, J = 8.8 Hz); 7.25 (d, 1H, J = 1.9 Hz); 7.22 (d, 1H, J = 8.1 Hz); 6.89 (dd, 1H, J = 8.1, 2.0 Hz); 6.31 (d, 1H, J = 8.8 Hz); 4.02 (t, 2H, J = 5.8 Hz); 3.74 (s, 3H); 3.42 (s, 2H); 3.15 (t, 2H, J = 6.6 Hz); 3.02 (d, 3H, J = 5.0 Hz); 2.58 (bt, 2H, J = 7.7 Hz).

### Example 73

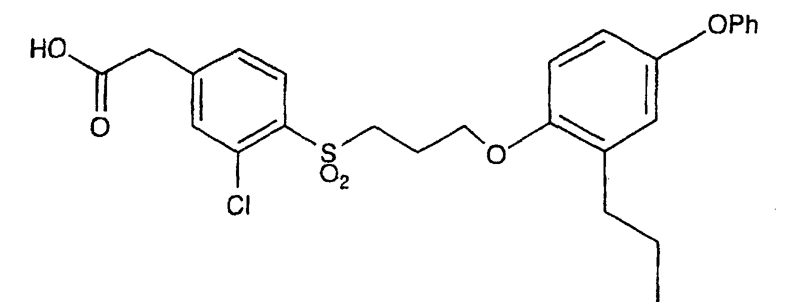

### 3-chloro-4-(3-(4-phenoxy-2-propylphenoxy)propyl-thio)phenylacetic acid

### STEP A: Preparation of 4-phenoxy-2-propylpheno]

Using the method in example 69 step A the titled compound was obtained.

### STEP B: Preparation of methyl 3-chloro-4-(3-bromopropylthio)-phenyl acetate

Using the method in example 51 step A the titled compound was obtained.

### STEP C: Preparation of Methyl 3-chloro-4-(3-(4-phenoxy-2-propylphenoxy)propyl-thio)phenylacetate

The title compound was obtained using the method of example 69 step C, substituting ethyl 3-chloro-4-(3-bromopropylthio)phenyl acetate for methyl 3-fluoro-4-(4-bromobuyloxy)-phenyl acetate.
NMR (CDCl₃) δ 7.27 (m, 3H), 7.14 (d, 1H); 7.02 (m, 1H); 6.85 (m, 5H); 4.18 (t, 2H); 3.67 (s, 3H); 3.52 (s, 2H); 3.19 (t, 2H); 2.58 (t, 2H); 2.18 (m, 2H); 1.56 (m, 2H); 0.90 (t, 3H).

### STEP D: Preparation of Methyl 3-chloro-4-(3-(4-phenoxy-2-propylphenoxy)propyl-thio)phenylacetate S, S-dioxide

Using the method in example 13, substituting Methyl 3-chloro-4-(3-(4-phenoxy-2-propylphenoxy)propyl-thio)phenylacetate as the starting material and using 2 equivalents of the oxidating agent, the titled compound was obtained. This compound was filtered through a pad of silica gel using ethyl ether and hexane (1:1) as the mobile phase, and taken forward without further purification.

### STEP E: Preparation of 3-chloro-4-(3-(4-phenoxy-2-propylphenoxy)-propyl-thio)phenylacetic acid S, S-dioxide

The title compound was prepared using the method of Example 2 and methyl 3-chloro-4-(3-(4-phenoxy-2-propylphenoxy)propylthio)phenyl acetate S,S-dioxide as the starting material.
NMR (CDCl₃) δ 7.27 (m, 3H), 7.14 (d, 1H); 7.02 (m, 1H); 6.85 (m, 5H); 4.18 (t, 2H); 3.67 (s, 2H); 3.64 (t, 2H); 2.58 (t, 2H); 2.29 (m, 2H); 1.56 (m, 2H); 0.90 (t, 3H).
ESI: Mass spec: m/e = 516 (M+1).

### Example 74

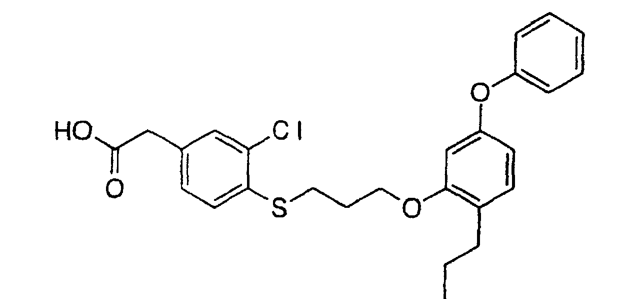

### 3-chloro-4-((2-propyl-5-phenoxy) phenoxy-propylthio)phenylacetic acid.

### Scheme A: Preparation of methyl 3-chloro-4-(3-bromopropylthio)-phenylacetate

The titled compound was pepared according to the methoddescribed in Example 51, Step A.

### Scheme B: Preparation of 3-phenoxy-(1-propenyloxy)benzene

The titled compound was pepared according to the methoddescribed in Example 52, Step D, using 5-phenoxy phenol. ¹H NMR (400 MHz, CDCl_{3,} ppm ): δ 7.34-6.56(m, 9H), 6.05-5.98(m, 1H), 5.40-5.24 (m, 1H), 4.48 (d, 2H, J = 5.3 Hz).

### Scheme C: Preparation of 2-propyl-5-phenoxy phenol

The titled compound was pepared according to the methoddescribed in Example 52. Step E, using 5-phenoxy-(1-propenyloxy)benzene
¹H NMR (400 MHz, CDCl_{3,} ppm ): δ 7.32-6.43(m, 8H), 2.52(t, 2H, J=7.4 Hz), 1.64-1.56 (m. 2H), 0.96 (t, 3H, J = 7.8 Hz).

### Scheme D: Preparation of methyl-3-chloro-4-((2-propyl-5-phenoxy) phenoxy-propylthio)phenylacetate.

The titled compound was prepared according to the method described in Example 51, Step C, using 2-propyl-5-phenoxy phenol
¹H NMR (400 MHz, CDCl_{3,} ppm ): δ 7.32-6.47(m, 10H), 3.99(t, 2H, J=5.8 Hz), 3.68(s, 3H), 3.54 (s, 2H), 3.12(t, 2H, J=7.2 Hz), 2.55(t, 2H, J=7.5 Hz), 2.19-2.09(m, 2H,), 1.61-1.56 (m, 2H), 0.92 (t, 3H, J = 7.3 Hz)

### Scheme D: Preparation of 3-chloro-4-((2-propyl-5-phenoxy) phenoxy-propylthio)phenylacetic acid.

The titled compound was prepared according to the method described in Example 51, Step E, using methyl-3-chloro-4-((2-propyl-5-phenoxy) phenoxy-propylthio)phenylacetate.
¹H NMR (400 MHz, CDCl_{3,} ppm ): δ 7.32-6.47(m, 10H), 3.99(t, 2H, J=5.8 Hz), 3.57 (s, 2H), 3.12(t, 2H, J=7.2 Hz), 2.54(t, 2H, J=7.4 Hz), 2.16-2.03(m, 2H,), 1.61-1.56 (m, 2H), 0.92 (t, 3H, J = 7.4 Hz)
ESI: MS: m/e= 471(M+).

### EXAMPLE 75

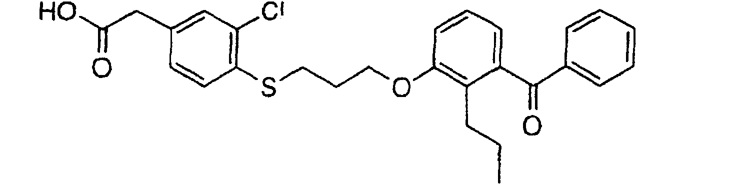

### 3-Chloro-4-(3-(2-propyl-3-benzoylphenoxy)propylthio)phenylacetic acid

### Step A : Preparation of 3-propenyloxybenzophenone

3-Hydroxybenzophenone (2.0g, 10mMol) was dissolved in DMF (50mL, 0.2M) at room temperature and potassium carbonate (1.67g, 12mMol) added followed by allyl bromide (870µL, 10mMol). The mixture was stirred overnight then diluted with water and extracted with ethyl acetate. The organic extract was washed with water (3x), dried over magnesium sulphate, filtered and concentrated. Chromatography (silica gel, 10% ethyl acetate/hexanes) gave 3-propenyloxybenzophenone (1.86g).
Nmr: 400MHz, CDCl₃; δ 4.56 (2H, d), 5.30 (1H, dd), 5.41 (1H, dd), 6.04 (1H, m), 7.12 (1H, m), 7.35 (3H, m), 7.46 (2H, dd), 7.57 (1H, dd), 7.79 (2H,d) ppm.

### Step B : Preparation of 1-((3-hydroxy-2-propyl)phenyl)-benzyl alcohol

The product from Example 75 Step A (1.86g, 7.8mMol) was dissolved in o-dicholorobenzene (35mL, 0.2M) and heated to 200°C. After stirring at this temperature overnight the mixture was cooled, loaded onto a silica chromatography column and eluted with 15% ethyl acetate/hexanes to collect a mixture of isomeric C-allyl benzophenones (4-hydroxy-3-propenyl-benzophenone and 2-hydroxy-3-propenyl-benzophenone in a ratio of 3:1 ; 1.17g).This mixture (1.17g, 4.9mMol) was dissolved in ethyl acetate (30mL, 0.16M) and 10% palladium on carbon (521mg, 0.49mMol) added. The mixture was hydrogenated at 1 atm hydrogen and room temperature. After 2h it was filtered through celite and concentrated. The residual oil was chromatographed (silica gel; 10→15% ethyl acetate/hexanes) to collect a mixture of isomeric C-propyl benzophenones (4-hydroxy-3-propylbenzophenone and 2-hydroxy-3-propyl-benzophenone in a ratio of 5:1 ; 780mg), the required product 1-((3-hydroxy-2-propyl)phenyl)-benzyl alcohol (120mg) and the isomeric 1-((3-hydroxy-4-propyl)phenyl)-benzyl alcohol (25mg).
Nmr: 300MHz, CD₃OD; δ 0.92 (3H, t), 1.29 (1H, m), 1.45 (1H, m), 2.55 (2H, t), 6.0 (1H, s), 6.69 (1H, dd), 6.92 (1H, dd), 6.99 (1H, dd), 7.22 (1H, m), 7.29 (4H,m) ppm.

### Step C: Preparation of Methyl 3-chloro-4-(3-(2-propyl-3-(hydroxybenzyl)phenoxy)-propylthio)phenylacetate

To a solution of the product of Step B of Example 75 (120 mg, 0.496 mmol) in 2.0 ml DMF was added 75 mg (0.546 mmol) potassium carbonate, and 184 mg (0.546 mmol) methyl 3-chloro-4-(3-bromopropylthio)phenylacetate (Example 51 Step A). The reaction mixture was stirred at 60°C for 16 hours. The reaction was quenched with pH 4 buffer at room temperature and extracted with ethyl acetate. The combined organic layers were washed with water, brine, and dried over sodium sulfate. After filtration and concentration of the filtrate *in vacuo*, the crude product was flash chromatographed (gradient elution with 5% - 15% ethyl acetate/hexane) to yield 175 mg (71%) of the titled compound as a yellow oil, homogeneous by TLC (20% ethyl acetate/hexane).
¹HNMR (500 MHz, CDCl₃, ppm): δ 7.35 (m, 4H), 7.28 (m, 2H), 7.19 (t, 1H, J = 8.0 Hz), 7.13 (dd, 1H, J = 8.0, 1.8 Hz), 7.09 (dd, 1H, J = 7.7, 1.0 Hz), 6.81 (dd, 1H, J = 8.0, 0.8 Hz), 6.11 (d, 1H, J = 4.1 Hz), 4.10 (m, 2H), 3.72 (s, 3H), 3.58 (s, 2H), 3.17 (t, 2H, J = 7.2 Hz), 2.66 (m, 2H), 2.18 (m, 2H), 2.12 (d, 1H, J = 4.1), 1.49 (m, 1H), 1.35 (m, 1H), 0.94 (t, 3H, J = 7.3 Hz) ppm.

### Step D: Preparation of Methyl 3-chloro-4-(3-(2-propyl-3-benzoylphenoxy)propylthio)-phenylacetate

The product obtained in Step C of Example 75 (170 mg; 0.341 mmol) was dissolved in 1.5 ml methylene chloride and treated with 52 mg (0.443 mmol) N-methylmorpholine N-oxide and 14 mg (0.040 mmol; 12 mole %) tetrapropyl ammonium perruthenate. The reaction mixture stirred at room temperature for 2 hours. The crude reaction mixture was loaded directly onto a flash column and purified (gradient elution with 5% - 12% ethyl acetate/hexane) to yield 137 mg (81%) of the titled compound as a colorless oil, homogeneous by TLC (20% ethyl acetate/hexane).
¹HNMR (500MHz, CDCl₃, ppm): δ 7.83 (m, 2H), 7.59 (m, 1H), 7.46 (m, 2H), 7.33 (d, 1H, J = 1.9 Hz), 7.30 (d, 1H, J = 8.0 Hz), 7.21 (t, 1H, J = 8.0 Hz), 7.15 (dd, 1H, J = 8.0, 2.0 Hz), 6.96 (d, 1H, J = 8.3 Hz), 6.86 (dd, 1H, J = 7.6, 1.0 Hz), 4.17 (t, 2H, J = 5.8 Hz), 3.72 (s, 3H), 3.58 (s, 2H), 3.19 (t, 2H, J = 7.2 Hz), 2.60 (m, 2H), 2.21 (m, 2H), 1.52 (m, 2H), 0.841 (t, 3H, J = 7.3 Hz) ppm. CI-MS: (M+1) = 497.

### Step E: Preparation of 3-Chloro-4-(3-(2-propyl-3-benzoylphenoxy)propylthio)phenylacetic acid.

The product obtained in Step D of Example 75 (130 mg; 0.262 mmol) was dissolved in 1.5 ml tetrahydrofuran and 0.5 ml water, and 350 µL (0.524 mmol) of a 1.5N lithium hydroxide solution was added. The reaction mixture stirred at room temperature for 3 hours. The reaction was quenched with 2N HCl and extracted with ethyl acetate. The combined organic layers were washed with water, brine, and dried over sodium sulfate. After filtration and concentration of the filtrate *in vacuo*, the crude material was flash chromatographed (gradient elution with 1.0% - 4.0% methanol/methylene chloride) to give 89 mg (71%) of the titled compound as a colorless glass, homogeneous by TLC (10% methanol/methylene chloride).
¹HNMR (500MHz, CDCl₃, ppm): δ 7.82 (m, 2H), 7.59 (m, 1H), 7.46 (t, 2H, J = 7.8 Hz), 7.33 (d, 1H, J = 8.1 Hz), 7.31 (d, 1H, J = 8.1 Hz), 7.21 (t, 1H, J = 8.0 Hz), 7.14 (dd, 1H, J = 8.2, 1.8 Hz), 6.96 (d, 1H, J = 7.8 Hz), 6.86 (dd, 1H, J = 7.5, 0.9 Hz), 4.16 (t, 2H, J = 5.8 Hz), 3.59 (s, 2H), 3.19 (t, 2H, J = 7.1 Hz), 258 (m, 2H), 2.21 (m, 2H), 1.50 (m, 2H), 0.83 (t, 3H, J = 7.4 Hz) ppm. CI-MS: (M+1) = 483.

### EXAMPLE 76

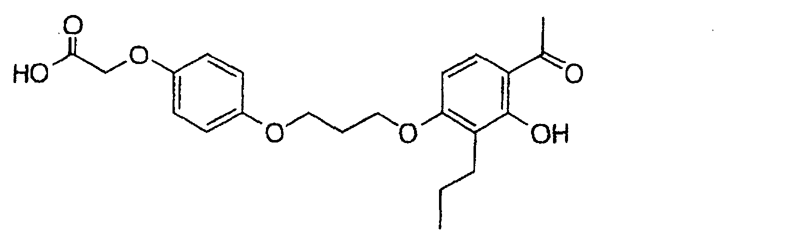

### Preparation of 4-(3-(2-propyl-3-hydroxy-4-acetyl-phenoxy)propyloxy)-phenoxy acetic acid

### Step 1: Preparation of methyl 4-hydroxyphenoxyacetate

Commercially available 4-hydroxyphenoxyacetic acid ( 2 g) was dissolved in methanol (10 ml) with approximately 0.04 ml H₂SO₄ conc. The mixture was heated 16 hrs under reflux. The mixture was cooled and reduced *in vacuo.* The residue was taken up in ethyl acetate and washed with saturated aq NaHCO₃, followed by saturated aq NaCl. The EtOAc extracts were dried over MgSO₄ and reduced *in vacuo*. The ester was used without further purification.
Characteristic NMR Resonances: ¹H NMR 400MHz (CDCl₃); 6.76 (Aromatic ABq, 4H), 4.56 (s, 2H), 3.78 (s, 3H). MS CI NH₃ M+NH₄⁺ = 200. MW = 182.1

### Step 2: Preparation of 4-(3-(2-propyl-3-hydroxy-4-acetylphenoxy)propyloxy)phenoxy acetic acid

To a solution of 2,4-dihydroxy-3-propylacetophenone (4.0 g, 20.6 mmol) in 2-butanone (50 mL) was added K₂CO₃ (6.0 g) and 1,3-dibromopropane (10 mL). The reaction was heated to reflux until TLC analysis indicated that the reaction was complete. The reaction was filtered and concentrated to a small volume. Column chromatography (0→10% ethyl acetate/hexanes) gave 1-bromo-3-(2-propyl-3-hydroxy-4-acetyl-phenoxy)propane. To a solution of methyl 4-hydroxyphenoxyacetate Step 1 (156 mg) and 1-bromo-3-(2-propyl-3-hydroxy-4-acetyl-phenoxy)propane (above; 300mg) in 2-butanone (5mL) was added potassium carbonate (196 mg). The mixture was refluxed for 48h. The reaction mixture was cooled to room temperature and partitioned between diethyl ether and 2N HCI. The organic layer was separated, washed with brine, dried over MgSO₄, and concentrated. Column chromatography (30% ethyl acetate/hexanes) gave methyl 4-(3-(2-propyl-3-hydroxy-4-acetyl-phenoxy)propyloxy) phenoxy acetate. A solution of methyl 4-(3-(2-propyl-3-hydroxy-4-acetylphenoxy)propyloxy) phenoxy acetate (above; 100 mg) in 3:1:1 methanol / water / tetrahydrofuran (4 mL) was treated with LiOH.H₂O (100 mg). The solution was stirred for 2 hour. The solution was partitioned between ethyl acetate and 2N HCl. The organic layer was washed with brine, dried over magnesium sulfate, filtered and evaporated to give the title compound as a solid.

Characteristic NMR Resonances; ¹H NMR (acetone): 7.77 (d, 1H, J = 8.8 Hz); 6.89 (bs, 4H); 6.67 (d, 1H, J = 8.9 Hz); 4.62 (bs, 2H); 4.30 (t, 2H, J = 6.1 Hz); 4.18 (t, 2H, J = 6.0 Hz); 2.62 (bt, 2H, J = 7.4 Hz); 2.58 (s, 3H).

### BIOLOGICAL ASSAYS

### I. White Adipose Tissue in vitro Assay

The ability of compounds of the present invention to enhance the insulin activation of ¹⁴C-glucose incorporation into glycogen in white adipose tissue (WAT) was determined by the following assay.

This assay measures the efficacy of the instant compounds to enhance the insulin activation of ¹⁴C-glucose incorporation into glycogen in white adipose tissue (WAT) in a 5 hour completely *in vitro* system. All procedures are performed in medium 199 containing 1% bovine serum albumen, 5 mM HEPES, and antibiotic (100 units/ml penicillin, 100 µg/ml streptomycin sulfate, 0.25 µg/ml amphotericin B), hereafter called culture medium. Epididymol fat pads are minced with scissors into small fragments , approximately 1 mm in diameter. Minced WAT fragments (100 mg) are incubated in a total volume of 0.9 ml culture medium containing 1 mU/ml insulin and test compound in tissue culture incubator at 37°C with 5% CO₂ with orbital shaking for 3 hours. ¹⁴C-labeled glucose is added and incubation continued for 2 hours. Tubes are centrifuged at low speed, infranatant is removed and 1 M NaOH is added. Incubation of alkali-treated WAT for 10 minutes at 60°C solubilizes tissue. Resulting tissue hydrolyzate is applied to Whatman filter paper strips which are then rinsed in 66% ethanol followed by 100% acetone which removes unincorporated ¹⁴C-glucose from bound ¹⁴C-glycogen. The dried paper is then incubated in solution of amyloglucosidase to cleave glycogen into glucose. Scintillation fluid is added and samples are counted for ¹⁴C activity. Test compounds that resulted in ¹⁴C activity substantially above incubations with insulin alone are considered active insulin-enhancing agents. Active compounds were titrated to determine the compound concentration which resulted in 50% of maximum enhancement of insulin activation and were termed EC₅₀ values. EC₅₀ values for the instant compounds were found to be 50 µM or less, preferably 5.0 to .0001 µM or less.

### II. PPAR Receptor Binding and/or Transactivation Assays

Compounds of the instant invention which are useful for the above discussed treatments can be identified and/or characterized by employing the PPAR δ, and γ binding assays and/or PPAR δ, PPAR α and PPARγ transactivation assays. The assays are useful in predicting or quantitating in vivo effects having to do with the control or modulation of glucose, free fatty acid, triglyceride, insulin or cholesterol. To evaluate IC₅₀ or EC_{50,} values the compounds were titrated in the appropriate assay using different concentrations of the compound to be tested. To obtain the appropriate values (%Inhibition-IC₅₀, or %Activation-EC₅₀), the data resulting from the assays were then analyzed by determining the best fit of a 4 parameter function to the data using the Levenberg-Marquardt non-linear fitting algorithm in Kaleidagraph (Synergy Software, Reading, PA). The human nuclear receptor cDNA for PPARδ (hPPARδ) has been cloned from a human osteosarcoma cell cDNA library and is fully described in A. Schmidt et al., Molecular Endocrinology, 6:1634-1641 (1992), herein incorporated by reference in its entirety. See A. Elbrecht et al., Biochem. and Biophy. Res. Comm. 224:431-437 (1996) and T. Sher et al., Biochem. 32:5598-5604 (1993) for a description of the human nuclear receptor gene PPARγ and α.

The hPPARδ binding assay comprises the steps of:
(a) preparing multiple test samples by incubating separate aliquots of the receptor hPPARδ with a test compound in TEGM containing 5-10% COS-1 cell cytoplasmic lysate and 2.5 nM labeled ([³H₂]Compound D, 17 Ci/mmole) for a minimum of 12 hours, and preferably for about 16 hours, at 4 °C, wherein the concentration of the test compound in each test sample is different, and preparing a control sample by incubating a further separate aliquot of the receptor hPPARδ under the same conditions but without the test compound; then
(b) removing unbound ligand by adding dextran/gelatin-coated charcoal to each sample while maintaining the samples at 4 °C and allowing at least 10 minutes to pass, then
(c) subjecting each of the test samples and the control sample from step (b) to centrifugation at 4 °C until the charcoal is pelleted; then
(d) counting a portion of the supernatant fraction of each of the test samples and the control sample from step (c) in a liquid scintillation counter and analyzing the results to determine the IC₅₀ of the. test compound.

In the hPPARδ binding assay, preferably at least four test samples of varying concentrations of a single test compound are prepared in order to determine the IC₅₀.

The hPPARδ transactivation assay comprises the steps of:
(a) seeding an hPPARδ/GR stable CHO-K1 cell line into alpha MEM containing 10% FCS, 10 mM HEPES, and 500 mg/ml G418 at 37°C in an atmosphere of 10% CO₂ in air,
(b) incubating the cells from step (a) for 16 to 48 hours, preferably about 20 hours, at 37°C in an atmosphere of 10% CO₂ in air;
(c) washing the cells from step (b) with alpha MEM;
(d) preparing multiple test cell groups by incubating separate groups of the cells from step (c) with the test compound in alpha MEM containing 5% charcoal stripped FCS, 10 mM HEPES, and 500 mg/ml G418, for 24 to 48 hours, preferably about 24 hours, at 37°C in an atmosphere of 10% CO₂ in air, wherein the concentration of the test compound in each test cell group is different, and preparing a control cell group by incubating a further separate group of the cells from step (c) under the same conditions but without the test compound; then
(e) preparing cell lysates from each of the test cell groups and the control cell group of step (d) using an aqueous detergent lysis buffer, and
(f) measuring the luciferase activity of the test cell groups and the control cell group of step (e) and analyzing the results to determine the EC50 of the test compound.

In the hPPARδ transactivation assay, preferably at least four test cell groups of varying concentrations of a single test compound are prepared in order to determine the EC₅₀.

Particular terms and abbreviations used herein are defined as follows: gst is glutathione-S-transferase; EDTA is ethylenediaminetetraacetic acid; HEPES is N-[2-hydroxyethyl]-piperazine-N'-[2-ethanesulfonic acid]; FCS is fetal calf serum; Lipofectamine is a 3:1 (w/w) liposome formulation of the polycationic lipid 2,3-dioleyloxy-N-[2(spermine-carboxamido)ethyl]-N,N-dimethyl- 1-propanaminiumtrifluoroacetate and the neutral lipid dioleoyl phosphatidylethanolamine in water; G418 is geneticin; MEM is Minimum Essential Medium; Opti MEM 1 Reduced-Serum Medium is an aqueous composition containing HEPES buffer, 2400 mg/L sodium bicarbonate, hypoxanthine, thymidine, sodium pyruvate, L-glutamine, trace elements, growth factors, and phenol red reduced to 1.1 mg/L; Luciferase Assay Reagent (in re-constituted form) is an aqueous composition containing 20 mM tricine, 1.07 mM (MgCO₃)₄Mg(OH)₂•5H₂O, 2.67 mM MgSO₄, 0.1 mM EDTA, 33.3 mM DTT, 270 µM coenzyme A, 470 µM luciferin, 530 µM ATP, having a final pH of 7.8.

AD-5075 has the following structure:

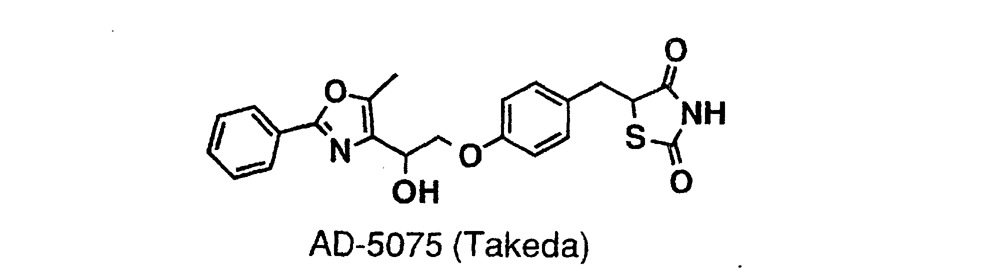

Opti MEM 1 Reduced-Serum Medium, alpha MEM, G418, and Lipofectamine are commercially available from GibcoBRL Life Technologies, Gaithersburg. Maryland. Alpha MEM is an aqueous composition having the following components:

| **Component: Inorganic Salts** | **mg/L** |
|---|---|
| CaCl₂ (anhyd.) | 200.00 |
| CaCl₂•2H₂O | -- |
| KCl | 400.00 |
| MgSO₄ (anhyd.) | 97.67 |
| MgSO₄•7H₂O | -- |
| NaCl | 6800.00 |
| NaHCO₃ | 2200.00 |
| NaH₂PO₄•H₂O | 140.00 |
| NaH₂PO₄•2H₂O | -- |

| **Other Components:** | **mg/L** |
|---|---|
| D-Glucose | 1000.00 |
| Lipoic Acid | 0.20 |
| Phenol Red | 10.00 |
| Sodium Pyruvate | 110.00 |

| **Amino Acids:** | **mg/L** |
|---|---|
| L-Alanine | 25.00 |
| L-Arginine•HCl | 126.00 |
| L-Asparagine•H₂O | 50.00 |
| L-Aspartic Acid | 30.00 |
| L-Cystine | -- |
| L-Cystine•2HCl | 31.00 |
| L-Cysteine HCl | -- |
| L-Cysteine HCl•H₂O | 100.00 |
| L-Glutamic Acid | 75.00 |
| L-Glutamine | 292.00 |
| L-Alanyl-L-Glutamine | -- |
| Glycine | 50.00 |
| L-Histidine HCl•H₂O | 42.00 |
| L-Isoleucine | 52.00 |
| L-Leucine | 52.00 |
| L-Lysine•HCl | 73.00 |
| L-Methionine | 15.00 |
| L-Phenylalanine | 32.00 |
| L-Proline | 40.00 |
| L-Serine | 25.00 |
| L-Threonine | 48.00 |
| L-Tryptophan | 10.00 |
| L-Tyrosine | -- |
| L-Tyrosine (disodium salt) | 52.00 |
| L-Valine | 46.00 |

| **Vitamins:** | **mg/L** |
|---|---|
| L-Ascorbic acid | 50.00 |
| Biotin | 0.10 |
| D-Ca Pantothenate | 1.00 |
| Choline Chloride | 1.00 |
| Folic acid | 1.00 |
| i-Inositol | 2.00 |
| Niacinamide | 1.00 |
| Pyridoxal HCl | 1.00 |
| Riboflavin | 0.10 |
| Thiamine HCl | 1.00 |
| Vitamin B₁₂ | 1.40 |

| **Ribonucleosides** | **mg/L** |
|---|---|
| Adenosine | 10.00 |
| Cytidine | 10.00 |
| Guanosine | 10.00 |
| Uridine | 10.00 |

| **Deoxyribonucleosides** | **mg/L** |
|---|---|
| 2' Deoxyadenosine | 10.00 |
| 2' Deoxycytidine HCl | 11.00 |
| 2' Deoxyguanosine | 10.00 |
| Thymidine | 10.00 |

The instant compounds, which are useful for treating the above discussed disease states, will preferably have IC₅₀ values at one, two or all of the PPAR (PPARγ, PPARδ or PPARα) receptor cites of equal to or less than 10 µM binding assay, and an EC₅₀ equal to or less than 10 µM in the transactivation assay. Preferably, an IC₅₀ of 100 nM in the binding assay, and an EC₅₀ equal to or less than 100 nM in the transactivation assay. More preferably, the instant compounds have an IC₅₀ equal to or less than 50 nM in the binding assay, and an EC₅₀ equal to or less than 50 nM in the transactivation assay. Most preferably, the instant compounds have an IC₅₀ equal to or less than 10 nM in the binding assay, and an EC₅₀ equal to or less than 10 nM in the transactivation assay..

### PPAR Receptor Binding Assay

### A. Preparation of Human PPARγ2 and δ

Human PPARγ2 and PPARδ, independently, were prepared as gst-fusion proteins in *E. coli.* The full length human cDNA for PPARγ2 and PPARδ were subcloned into the PGEX-2T and PGEX-KT. respectively, expression vector (Pharmacia). *E. coli* containing the plasmid were grown, induced, and then harvested by centrifugation. The resuspended pellet was broken in a French press and debris was removed by centrifugation at 12,000Xg. Receptors were purified from the supernatant by affinity chromatography on glutathione sepharose. After application to the column, and 1 wash, receptor was eluted with glutathione. Glycerol was added to stabilize the receptor and aliquots were frozen at -80 °C for later use.

### B. [³H]AD-5075 and Example 11 Displacement Assay for PPARγ2 and PPARδ, respectively

For each assay, an aliquot of receptor (1:1000-1:3000 dilution) was incubated in TEGM (10 mM Tris, pH 7.2, 1 mM EDTA, 10% glycerol, 7 µl/100 ml β-mercaptoethanol, 10 mM Na molybdate, 1 mM dithiothreitol, 5 µg/ml aprotinin, 2 µg/ml leupeptin, 2 µg/ml benzamide and 0.5 mM PMSF) containing 5-10% COS-1 cell cytoplasmic lysate and 10 nM labeled thiazolidinedione ([³H₂]AD-5075, 21 Ci/mmole), ± test compound compound, [³H₂]Example 11, 17 Ci/mmole), ± test compound, respectively. Assays were incubated for ∼16 h at 4 °C in a final volume of 300 µl. Unbound ligand was removed by addition of 200 µl dextran/gelatin-coated charcoal, on ice, for ∼10 minutes. After centrifugation at 3000 rpm for 10 min at 4 °C, 200 µl of the supernatant fraction was counted in a liquid scintillation counter. In this assay the KD for AD-5075 and Example 11 is 1 nM, respectively.

### PPAR Receptor Transactivation Assay

### A. Activation of hPPARγ and hPPARδMethods

### 1. Plasmids

The chimeric receptor expression constructs, pSG5-hPPARγ2/GR and pSG5-hPPARδ/GR, were prepared by inserting the DNA binding domain of the murine glucocorticoid receptor adjacent to the ligand binding domain of hPPARγ2 or hPPARδ. These vectors were kindly provided by Dr. Azriel Schmidt (MRL). The glucocorticoid receptor -responsive reporter vector, pMMTV/luc/neo, contains the murine mammary tumour virus (MMTV) promoter adjacent to the luciferase gene (luc) and the neomycin resistance gene (neo). It was constructed from pMMTV/luc which was provided by Dr. Azriel Schmidt (Merck Research Laboratories). Prior to transfection into CHO-K1 cells, pSG5-hPPARγ2/GR and pSG5-hPPARδ/GR were linearized with Xba I. pMMTV/luc/neo DNA was cut with Pvu I. Wild type receptor constructs, pSG5-hPPAPγ2, pSG5-hPPARδ and pSG5-hPPARα were prepared by inserting the full-length hPPARγ2, hPPARδ and PPARα cDNAs adjacent to the SV40 promoter in pSG5. The PPAR-responsive reporter construct, pPPRE-luc, contained 3 copies of a generic PPRE placed adjacent to the thymidine kinase minimal promoter and the luciferase reporter gene. The transfection control vector, pCMV-lacZ, contains the galactosidase Z gene under the regulation of the cytomegalovirus promoter.

### 2. Production of stable cell lines

CHO-K1 cells were seeded overnight at 6x10⁵ cells /60 mm dish in alpha Minimum Essential Medium (MEM) containing 10% fetal calf serum (FCS), 10 mM HEPES, 100 units/ml PenicillinG and 100 µg/ml Streptomycin sulfate at 37°C in an atmosphere of 10% CO₂ in air. The cells were washed once with OptiMEM 1 Reduced-Serum Medium and then cotransfected with 4.5 µg of pSG5-hPPARγ2 /GR or pSG5-hPPARδ/GR expression vector and 0.5 µg of pMMTV/luc/neo in the presence of 100 µg Lipofectamine (GIBCO BRL) according to the instructions of the manufacturer. Transfection medium was removed 2 h later and replaced with growth medium. After being incubated for 3 days, cells were subcultured by diluting the cell suspension 1/1250 and 1/6250 and placing the cells in a 100 mm culture dish. Selection of the stable cell lines was initiated the next day by adding 500 µg/ml G418 to the medium. Cells were routinely fed with the selection media for 1 month at which time 120 colonies were picked and transferred to 24 well culture plates. Ten days later, confluent colonies were transferred to 6 well plates to maintain stocks and to 96 well plates to assay for luciferase activity. Positive clones were characterized and validated by titrating 4 known agonists on each clone. Two clones, g2B2P2D9 and d2A5P2G3, were selected for screening purposes.

### B. hPPAR/GR transactivation screens in stably transfected CHO-Kl cells

The hPPARγ2/GR and hPPARδ/GR stable CHO-K1 cell lines were seeded at 1x10⁴ cells/well in 96 well cell culture plates in alpha MEM containing 10% FCS, 10 mM HEPES, and 500 mg/ml G418 at 37°C in an atmosphere of 10% CO₂ in air. After a 20 hour incubation, cells were washed once with alpha MEM and then incubated in an atmosphere of 10% CO₂ in air in alpha MEM containing 5% charcoal stripped FCS, 10 mM HEPES, and 500 mg/ml G418. The cells were incubated for 24 hours in the absence of test compound or in the presence of a range of concentrations of test compound. Cell lysates were prepared from washed cells using Reporter Lysis Buffer (Promega) according to the manufacturer's directions. Luciferase activity in cell extracts was determined using Luciferase Assay Reagent buffer (Promega) in a ML3000 luminometer (Dynatech Laboratories).

### Transactivation Wild-Type Assay

### A. Characterization of ligand activity on wild-type hPPARγ, hPPARδ and hPPARα.

COS-1 cells were seeded at 0.5 X 10⁵ cells/dish into 24 well plates in Dulbecco's modified Eagle medium (high glucose) containing 10% charcoal stripped fetal calf serum, nonessential amino acids, 100 units/ml Penicillin G and 100 µg/ml Streptomycin sulfate at 37°C in a humidified atmosphere of 10% CO₂. After 24 hours, transfections were performed with Lipofectamine (Gibco-BRL, Gaithersburg, MD) according to the instructions of the manufacturer. In general, for transactivation experiments, transfection mixes contained 0.15 mg of hPPARγ2 hPPARα or hPPARδ expression vector, 0.15 mg of reporter vector pPPRE-luc and 0.001 mg of pCMV-lacZ as an internal control of transfection efficiency. Compounds demonstrating significant agonist activity in the above primary screen were further characterized by incubation with transfected cells for 48h across a range of concentrations. Luciferase activity was determined as described above.

In a similar manner, hPPARγ1 cDNA can be used in place of hPPARγ2 cDNA in the methods described in Example 5 to make the wild type receptor construct, pSG5-hPPARγ1.

### III.In Vivo Studies

### Methods

db/db Mice are obese, highly insulin resistant animals. The db locus has been shown to code for the leptin receptor. These animals are substantially hypertriglyceridemic and hyperglycemic.

Male db/db mice (10-11 week old C57B1/KFJ, Jackson Labs, Bar Harbor, ME) were housed 5/cage and allowed *ad lib.* access to ground Purina rodent chow and water. The animals, and their food, were weighed every 2 days and were dosed daily by gavage with vehicle (0.5% carboxymethylcellulose) ± test compound at the indicated dose. Drug suspensions were prepared daily. Plasma glucose, Cholesterol and triglyceride concentrations were determined from blood obtained by tail bleeds at 3-5 day intervals during the study period. Glucose, cholesterol and triglyceride, determinations were performed on a Boehringer Mannheim Hitachi 911 automatic analyzer (Boehringer Mannheim, Indianapolis, IN) using heparinized plasma diluted 1:5, or 1:6 (v/v) with normal saline. Lean animals were age-matched heterozygous mice maintained in the same manner. The instant compounds were found to lower triglyceride and glucose levels at a dose of about 100mg/kg, preferably a dose of about 10-50 mg/kg, when administered by oral gavage daily for a period of at least 5 days.

Lipoprotein analysis was performed on either serum, or EDTA treated plasma obtained by heart puncture from anesthetized animals at the end of the study. Apolipoprotein concentrations were determined by ELISA, and cholesterol particles were analyzed by FPLC, precipitation, or ultracentrifugation. Total liver RNA was prepared from tissue that had been frozen on liquid nitrogen at the time of euthanasia. Apolipoprotein mRNA was analyzed on Northern Blots using specific probes for mouse or rat proteins.

## Claims

1. A compound having the formula I: 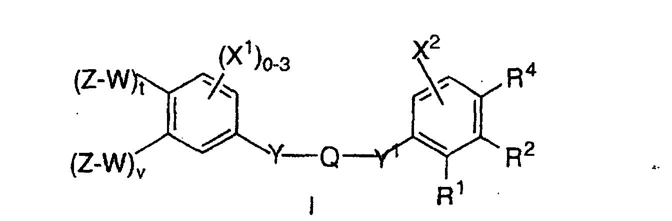 or a pharmaceutically acceptable salt thereof, wherein:
R is selected from the group consisting of H, C₁₋₆ alkyl, C₅₋₁₀ aryl, and C₅₋₁₀ heteroaryl, said alkyl, aryl and heteroaryl optionally substituted with 1 to 3 groups of R^{a};
R¹ is selected from a group consisting of: H, C₁₋₁₅ alkyl, C₂₋₁₅ alkenyl, C₂₋₁₅ alkynyl and C₃₋₁₀ cycloalkyl, said alkyl, alkenyl, alkynyl, and cycloalkyl optionally substituted with 1 to 3 groups of R^{a};
R² is selected from a group consisting of: H, C₁₋₁₅ alkyl, acyl, C₂₋₁₅ alkenyl, OR³, CO₂alkyl, C(O)R³, OH, -OC(O)R³, C₂₋₁₅ alkynyl, C₅₋₁₀ aryl, C₅₋₁₀ heteroaryl, said alkyl, alkenyl, alkynyl, aryl and heteroaryl optionally substituted with 1 to 3 groups of R^{a};
R³ is selected from a group consisting of: H, NHR¹, NHacyl, C₁₋₁₅ alkyl, C₂₋₁₅ alkenyl, C₁₋₁₅ alkoxy, CO₂alkyl, OH, C₂₋₁₅ alkynyl, C₅₋₁₀ aryl, C₅₋₁₀ heteroaryl said alkyl, alkenyl, alkynyl, aryl and heteroaryl optionally substituted with 1 to 3 groups of R^{a;}
R⁴ is selected from the group consisting of: R², -B-R⁵ or 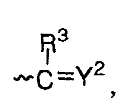 with the proviso that when (Z-W-) is Z-CR⁶R⁷, Y is O and R⁴ is R², then R² is not acetyl, H, alkyl, alkoxy or aryl; or when (Z-W-) is Z-CR⁶R⁷, Y is O and R4 is 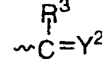 then Y² is not O, or when (Z-W-) is Z-CR⁶R⁷-, or 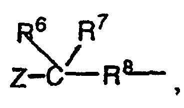 where R⁸ is O, Y is S or O and R⁴ is -B-R⁵, then B is not O or S;
R⁵ is selected from the group consisting of: C₅₋₁₀ aryl and C₅₋₁₀ heteroaryl, said aryl and heteroaryl optionally substituted with 1 to 3 groups of R^{a};
B is selected from the group consisting of: O, S(O)p and NR¹;
(Z-W-) is Z-CR⁶R⁷-, Z-CH=CH-, or 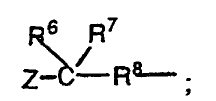
R⁸ is selected from the group consisting of CR⁶R⁷, O, NR⁶, and S(O)ₚ;
R⁶ and R⁷ are independently selected from the group consisting of H, C₁₋₆ alkyl;
X¹ and X² are independently selected from a group consisting of: H, OH, C₁₋₁₅ alkyl, C₂₋₁₅ alkenyl, C₂₋₁₅ alkynyl, halo, OR³, C₅₋₁₀ aryl, C₅₋₁₀ aralkyl, C₅₋₁₀ heteroaryl and C₁₋₁₀ acyl, said alkyl, alkenyl, alkynyl, aryl and heteroaryl optionally substituted with 1 to 3 groups of R^{a};
R^{a} represents a member selected from the group consisting of: halo, aryl, acyl, heteroaryl, CF₃, OCF₃, -O-, CN, NO₂, R³, OR³; SR³, S(O)R³, SO₂R³, NR³R³, NR³COR³, NR³CO₂R³, NR³CON(R³)₂, NR³SO₂R³, COR³, CO₂R³, CON(R³)₂, SO₂N(R³)₂, OCON(R³)₂ said aryl and heteroaryl optionally substituted with 1 to 3 groups of halo or C1-6 alkyl;
Y is selected from the group consisting of: S(O)ₚ, -CH₂-, -C(O)-, -C(O)NH-, -NR-, -O-, -SO₂NH, -NHSO₂;
Y¹ is selected from the group consisting of: O, NR and CH₂;
Y² is selected from the group consisting of: O, N(C₁₋₁₅) alkyl, N(CO₂)alkyl, N-Oalkyl, N-Oacyl and N-OH, with the proviso that if Y² is O and R³ is CH₃ then n is 2;
Z is selected from the group consisting of: CO₂R³, CONHSO₂R, CONH₂ and 5-(1H-tetrazole);
t and v are independently 0 or 1 such that t + v = 1
Q is a saturated or unsaturated straight chain hydrocarbon containing 2-4carbon atoms and
p is 0-2.

2. A compound of Claim 1 where X¹ & X² are independently H or halo.

3. A compound of Claim 1 where Y is O.

4. A compound of Claim 1 where Y is S(O)ₚ, wherein p is 0-2.

5. A compound of Claim 1 where Y is -CH₂-.

6. A compound of Claim 1 where Y is -CO-.

7. A compound of Claim 1 where Y is -NH-.

8. A compound of Claim 1 where Y is NHSO₂ or SO₂NH.

9. A compound of Claim 1 where Y is C(O)NH.

10. A compound of Claim 1 where R⁴ is R² with the proviso that when (Z-W-) is Z-CR⁶R⁷, and Y is O then R² is not acetyl, H, alkyl, alkoxy or aryl.

11. A compound of Claim 1 where R⁴ is -B-R⁵ with the proviso that when (Z-W-) is Z-CR⁶R⁷-, or 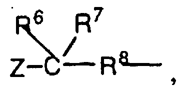 where R⁸ is O, and Y is S or O, then B is not O or S.

12. A compound of Claim I where R⁴ is 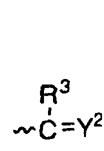 with the proviso that when (Z-W-) is Z-CR⁶R⁷, and Y is O then Y² is not O.

13. A compound of Claim 1 where W is -CR⁶R⁷- or

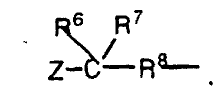

14. A compound of Claim 1 wherein:
R is C₁₋₆ alkyl or C₅₋₁₀ aryl, said alkylor aryl optionally substituted with 1 to 3 groups of R^{a};
R¹ is C₁₋₁₅ alkyl;
X¹ & X² are independently H, C₁₋₆ alkyl, or halo;
Y is O, NH or S;
Y¹ is O;
(Z-W-) is Z-CR⁶R⁷- or 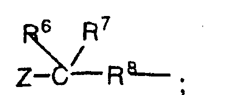
R⁴ is -B-R⁵ or 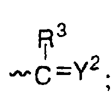 with the proviso that when (Z-W-) is Z-CR⁶R⁷,
Y is O and R4 is 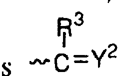 then Y² is not O, or when (Z-W-) is Z-CR⁶R⁷-, or 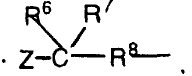 where R⁸ is O, Y is S or O and R⁴ is -B-R⁵, then B is not O or S;
R^{a} is a member selected from the group consisting of: halo, aryl, acyl heteroaryl, CF₃, OCF₃, -O-, CN, NO₂, R³, OR³; SR³, S(O)R³, SO₂R³, NR³COR³, COR³, CON(R³)₂, SO₂N(R³)₂, said aryl and heteroaryl optionally substituted with 1 to 3 groups of halo or C1-6 alkyl; and
Z is CO₂R³, CONHSO₂R, CONH₂ or 5-(1H-tetrazole).

15. A compound of Claim 1 selected from the group consisting of:
Methyl 3-chloro-4-(3-(2-propyl-3-hydroxy-4-propionylphenoxy)-propylthio)-phenylacetate;
3-Chloro-4-(3-(2-propyl-3-hydroxy-4-propionylphenoxy)propylthio)phenyl-acetic acid;
Methyl 3-chloro-4-(3-(2-propyl-3-hydroxy-4-(1-oxobutyl)phenoxy)-propylthio)phenyl acetate:
3-Chloro-4-(3-(2-propyl-3-hydroxy-4-(1-oxobutyl)phenoxy)-propylthio)-phenylacetic acid:
Methyl 3-chloro-4-(3-(2-propyl-3-hydroxy-4-(1-oxo-2-methylpropyl)-phenoxy)propylthio)phenylacetate;
Methyl 3-chloro-4-(3-(2-propyl-3-hydroxy-4-(1-oxopropyl)phenoxy)-propyloxy)phenylacetate;
3-Chloro-4-(3-(2-propyl-3-methoxy-4-propionylphenoxy)propylthio)-phenylacetic acid;
methyl-3-chloro-4-(3-(2-propyl-3-methoxy-4-propionylphenoxy) propylthio)phenylacetate;
Methyl 3-chloro-4-(3-(2-propyl-3-acetoxy-4-propionylphenoxy)propylthio)phenylacetate;
Methyl 3- chloro-4-(3-(2-propyl-3-hydroxy-4-(1-hydroxyliminopropyl)-phenoxy)propylthio)phenylacetate;
3-Chloro-4-(3-(2-propyl-3-hydroxy-4-(1-hydroxyliminopropyl)phenoxy)-propylthio)phenylacetic acid;
Methyl 3- chloro-4-(3-(2-propyl-3-methoxy-4-(1-hydroxyliminopropyl)-phenoxy)propylthio)phenyl acetate;
3-Chloro-4-(3-(2-propyl-3-methoxy-4-(1-hydroxyliminopropyl)phenoxy)-propylthio)phenyl acetic acid;
Methyl 3-chloro-4-(3-(2-propyl-3-hydroxy-4-(1-hydroxyliminopropyl)-phenoxy)propyloxy)phenyl acetate;
Methyl 3-chloro-4-(3-(2-propyl-3-hydroxy-4-(1-methoxyliminopropyl)-phenoxy)propylthio)phenylacetate;
Methyl 3-chloro-4-(3-(2-propyl-3-hydroxy-4-(1-hydroxyliminobutyl)-phenoxy)propylthio)phenylacetate;
3-Chloro-4-(3-(2-propyl-3-hydroxy-4-(1-hydroxyliminobutyl)phenoxy)-propylthio)phenylacetic acid;
Methyl 3-chloro-4-(3-(2-propyl-3-hydroxy-4-(1-acetoxyiminobutyl)-phenoxy)propylthio)phenylacetate;
Methyl 3-chloro-4-(3-(2-propyl-3-hydroxy-4-(1-hydroxylimino-2-methyl-propyl)phenoxy)propylthio)phenylacetate;
3-Chloro-4-(3-(2-propyl-3-hydroxy-4-(1-hydroxylimino-2-methylpropyl)-phenoxy)propylthio)phenylacetic acid;
Methyl 3-chloro-4-(3-(2-propyl-3-acetoxy-4-(1-hydroxyiminopropyl) phenoxy)propylthio)phenyl acetate;
3-chloro-4-(3-(2-propyl-3-hydroxy-4-(1-oxopropyl)phenoxy)propylthio)-phenylacetamide
Methyl 3-chloro-4-(3-(2-cyclopropylmethyl-3-hydroxy-4-propionylphenoxy)propylthio)phenylacetate;
3-Chloro-4-(3-(2-cyclopropylmethyl-3-hydroxy-4-propionylphenoxy)-propylthio)phenylacetic acid;
Methyl 3-chloro-4-(3-(2-cyclopropylmethyl-3-hydroxy-4-(1-hydroxyimino-propyl)phenoxy)propylthio)phenylacetate;
3-Chloro-4-(3-(2-cyclopropylmethyl-3-hydroxy-4-(1-hydroxyiminopropyl)-phenoxy)propylthio)phenylacetic acid;
Methyl 3-chloro-4-(4-(2-propyl-3-hydroxy-4-propionylphenyl)butylthio)-phenylacetate;
3-Chloro-4-(4-(2-propyl-3-hydroxy-4-propionylphenyl)-butylthio)phenyl-acetic acid;
Methyl 3-chloro-4-(4-(2-propyl-3-hydroxy-4-(1-hydroxyiminopropyl)-phenyl)butylthio)phenylacetate;
3-chloro-4-(4-(2-propyl-3-hydroxy-4-(1-hydroxyiminopropyl)-phenyl)butylthio)phenylacetic acid;
Methyl 3-chloro-4-(3-(2-(2-propenyl)-3-hydroxy-4-propionylphenoxy)-propylthio)phenylacetate;
Methyl 3-chloro-4-(3-(2-(2-propenyl)-3-hydroxy-4-(1-hydroxyiminopropyl)-phenoxy)propylthio)phenylacetate;
3-Chloro-4-(3-(2-(2-propenyl)-3-hydroxy-4-(1-hydroxyiminopropyl)-phenoxy)propylthio)phenylacetic acid;
Methyl 3-chloro-4-(3-(2-propyl-4-propionylphenoxy)propylthio)phenyl-acetate;
3-Chloro-4-(3-(2-propyl-4-propionylphenoxy)propylthio)phenylacetic acid;
Methyl 3-chloro-4-(3-(2-propyl-4-(1-hydroxyiminopropyl)phenoxy)propyl-thio)phenylacetate;
3-Chloro-4-(3-(2-propyl-4-( 1-hydroxyiminopropyl)phenoxy)propylthio)-phenylacetic acid;
Methyl 3-chloro-4-(4-(2-propyl-3-hydroxy-4-acetylphenoxy)butylthio)-phenylacetate;
3-Chloro-4-(4-(2-propyl-3-hydroxy-4-acetylphenoxy)butylthio)phenylacetic acid;
3-Chloro-4-(3-(2-propyl-3-hydroxy-4-acetyl)phenoxy)propylthiophenyl- 5-methyl-(1H)-tetrazole;
3-Chloro-4-(4-(2-propyl-3-hydroxy-4-acetylphenoxy)butylthio)phenyl-5-methyl-(1H)-tetrazole;
3-Chloro-4-(3-(2-propyl-3-hydroxy-4-propionylphenoxy)propylthio)-phenyl-5-methyl-(1H)-tetrazole;
3-Chloro-4-(3-(2-propyl-3-hydroxy-4-( 1-hydroxyiminopropyl)-phenoxy)-propylthio)-phenyl-5-methyl-(1H)-tetrazole;
3-Chloro-4-(4-(2-propyl-3-hydroxy-4-propionylphenoxy)butylthio)-phenyl-5-methyl-(1H)-tetrazole;
3-Chloro-4-(4-(2-propyl-3-hydroxy-4-(1-hydroxyiminopropyl)-phenoxy)-butylthio)phenyl-5-methyl-(1H)-tetrazole;
4-(3-(2-Propyl-3-hydroxy-4-acetylphenoxy)propyloxy)-phenyl-5-methyl-(1H)-tetrazole;
4-(4-(2-Propyl-3-hydroxy-4-propionylphenoxy)butyloxy)-phenyl-5-methyl-( 1H)-tetrazole;
4-(3-(2-Propyl-3-hydroxy-4-acetylphenoxy)propylthio)phenyl-5-methyl-( 1H)-tetrazole;
3-chloro-4-(3-(2-propyl-3-hydroxy-4-(1-oxopentyl))phenoxy)propylthio phenyl acetic acid methyl ester;
3-chloro-4-(3-(2-propyl-3-hydroxy-4-(1-hydroxyliminopentyl))phenoxy) propylthio phenyl acetic acid methyl ester;
3-chloro-4-(3-(2-propyl-3-hydroxy-4-(1-hydroxylimino propyl)phenoxy)-propylthio)phenylacetamide;
3-chloro-4-(3-(2-propyl-3-hydroxy-4-(1-(hydroxylimino-3-(phenyl) propyl)phenoxy)-propylthio)phenylacetic acid;
Methyl 3-chloro-4-(4-phenoxy-2-propylphenoxy)-propylamino)phenylacetate;
3-Chloro-4-(4-phenoxy-2-propylphenoxy)-propylamino)phenylacetic acid;
3-chloro-4-(3-(2-propyl-3-hydroxy-4-(1-hydroxyliminopropyl)) phenoxy)propylthio phenyl acetic acid;
4-(2-(4-benzyl-2-propylphenoxy)propylthio)-3-chlorophenylacetic acid;
Methyl 3-chloro-4-(3-(2-propyl-3-hydroxy-4-(1-oxopentyl)phenoxy)-propyl)-phenylacetate;
4-(3-(4-(N-hydroxyiminobenzoyl)-2-propylphenoxy)propylthio)-3-chlorophenylacetic acid;
4-(3-(4-benzoyl-3-hydroxy-2-propylphenoxy)propylthio)-3-chlorophenylacetic acid;
4-(3-(4-Phenoxy-2-propylphenoxy)propylamino)-phenyl(2,2-dimethyl)acetic acid;
4-(3-(4-Phenoxy-2-propylphenoxy)propylamino)-phenylpropan-3-oic acid;
N-[4-(3-(4-Phenoxy-2-propylphenoxy)propylamino)-phenyl]glycine;
4-(3-(4-Phenoxy-2-propylphenoxy)propylamino)-phenylacetic acid;
4-(3-(4-Phenoxy-2-propylphenoxy)propylamino)-3-chlorophenylacetic acid;
4-(3-(4-Phenoxy-2-propylphenoxy)propylamino)-phenoxyacetic acid;
4-(3-(4-Phenoxy-2-propylphenoxy)propylthio)-phenylpropan-3-oic acid;
4-(3-(4-Phenoxy-2-propylphenoxy)propyloxy)-2-phenyl-2,2-dimethylacetic acid;
4-(4-(4-Phenoxy-2-propylphenoxy)butylamino)-phenoxy-2,2-dimethylacetic acid;
4-(3-(4-Phenoxy-2-propylphenoxy)propylthio)-3-methylphenylacetic acid;
4-(3-(4-Phenoxy-2-propylphenoxy)propyloxy)-3-butylphenylacetic acid;
4-(3-(4-Phenoxy-2-propylphenoxy)propylamino)-2-propylphenylacetic acid;
4-(4-(4-Phenoxy-2-propylphenoxy)butylthio)-2-fluorophenylacetic acid;
4-(3-(4-Phenoxy-2-propylphenoxy)propyloxy)-phenoxy-2-spiro-cyclopropylacetic acid;
3-(3-(4-Phenoxy-2-propylphenoxy)propyloxy)-phenoxyacetic acid;
3-(3-(4-Phenoxy-2-propylphenoxy)propylamino)-phenylpropan-3-oic acid;
3-(3-(4-Phenoxy-2-propylphenoxy)propylthio)-2-phenyl-2,2-dimethylacetic acid;
3-(4-(4-Phenoxy-2-propylphenoxy)butyloxy)-phenoxy-2,2-dimethylacetic acid;
3-(3-(4-Phenoxy-2-propylphenoxy)propylthio)-3-methylphenylacetic acid;
3-(3-(4-Phenoxy-2-propylphenoxy)propylthio)-3-chlorophenylacetic acid;
4-(3-(4-Phenoxy-2-propylphenoxy)propylthio)-3-chlorophenylacetic acid;
3-(4-(4-Phenoxy-2-propylphenoxy)butylamino)-3-butylphenylacetic acid;
3-(3-(4-Phenoxy-2-propylphenoxy)propyloxy)-2-propylphenylacetic acid;
3-(3-(4-Phenoxy-2-propylphenoxy)propylthio)-2-fluorophenylacetic acid;
3-(4-(4-Phenoxy-2-propylphenoxy)butylamino)-phenoxy*-*2-*spiro*-cyclopropylacetic acid;
4-(3-(4-Phenoxy-2-(cyclopropylmethyl)phenoxy)propylamino)-phenylacetic acid;
4-(3-(4-Phenoxy-2-(prop-2-enyl)phenoxy)propylamino)-phenylacetic acid;
4-(3-(4-Phenoxy-2-butylphenoxy)propylamino)-phenylacetic acid;
4-(3-(4-Phenoxy-2-propoxyphenoxy)propylamino)-phenylacetic acid;
3-(4-(4-Phenoxy-2-(cyclopropylmethyl)phenoxy)butyloxy)-phenylacetic acid;
3-(4-(4-Phenoxy-2-(prop-2-enyl)phenoxy)butylamino)-phenylacetic acid;
3-(4-(4-Phenoxy-2-butylphenoxy)butyloxy)-phenylacetic acid;
3-(4-(4-Phenoxy-2-propoxyphenoxy)butyloxy)-phenylacetic acid;
4-(3-(4-Phenoxy-3-hydroxy-2-propylphenoxy)propylthio)-phenylacetic acid;
3-(3-(4-Phenoxy-3-methoxy-2-propylphenoxy)propylthio)-phenylacetic acid;
4-(4-(4-Phenoxy-3-butyloxy-2-propylphenoxy)butyloxy)-phenylacetic acid;
4-(4-(4-Phenoxy-3-chloro-2-propylphenoxy)butyloxy)-phenylacetic acid;
4-(3-(4-Benzoyl-2-propylphenoxy)propylthio)-phenylacetic acid;
4-(3-(4-(4-Chloro)benzoyl-2-propylphenoxy)propylthio)-phenylacetic acid;
4-(4-(4-(4-Tolyl)benzoyl-2-propylphenoxy)butyloxy)-phenylacetic acid;
3-(4-(4-(4-Butylcarbonyl)-2-propylphenoxy)butyloxy)-phenylacetic acid;
4-(3-(4-(N-Hydroxyiminoacetyl)-2-propylphenoxy)propylthio)-phenylacetic acid;
3-(3-(4-(2-Phenylethylcarbonyl)-2-propylphenoxy)propylthio)-phenylacetic acid;
4-(4-(4-(2-Phenylethoxycarbonyl-2-propylphenoxy)butyloxy)-phenylacetic acid;
4-(4-(4-(4-Phenylthiocarbonyl)-2-propylphenoxy)butyloxy)-phenylacetic acid;
3-chloro-4-(3-((1,2-benzisoxazol-3-yl)-2-propylphenoxy) propylthio)phenylacetic acid;
3-chloro-4-(3-((1,2-benzisoxazol-3-yl)-2-propylphenoxy) butyloxy)phenylacetic acid;
3-(4-(4-benzoyl-2-propylphenoxy)butyloxy)phenyl acetic acid;
3-chloro-4-(3-(4-fluorobenzoyl-2-propylphenoxy)propylthio)phenyl acetic acid;
3-chloro-4-(3-(4-phenyl-2-propylphenoxy)propylthio)phenyl acetic acid;
3-chloro-4-(3-(N-ethyl-N-(4-acetyl-3-hydroxy-2-propylphenyl)-amino)propylthio)phenyl acetic acid;
3-chloro-4-(3-(N-(4-hydroximinoacetyl-3-hydroxy-2-propylphenyl)-amino)propylthio)phenyl acetic acid;
3-chloro-4-(4--phenoxy (4-trifluoromethyl)-propylamino) phenyl acetic acid;
3-chloro-4-(4- phenoxy-2-propyl-(4-(4-fluoro)phenylsulfonyl)-propylthio)-phenyl acetic acid;
3-fluoro-4-(4-(4-phenoxy-2-propylphenoxy)-butyloxy)phenylacetic acid;
3-(4-(1-(2-propyl-4-phenethyl)hydroquinolyl)butyloxy)phenylacetic acid;
3-Chloro-4-(3-(2-propyl-3-hydroxy-4-(1-acetoxyiminopropyl))-phenoxy)propylthio)phenylacetic acid;
3-Chloro-4-(3-(2-propyl-3-methoxy-4-methylaminocarbonylphenoxy)propylthio)phenylacetic acid;
3-chloro-4-(3-(4-phenoxy-2-propylphenoxy)propyl-thio)phenylacetic acid;
3-chloro-4-((2-propyl-5-phenoxy) phenoxy-propylthio)phenylacetic acid;
3-Chloro-4-(3-(2-propyl-3-benzoylphenoxy)propylthio)phenylacetic acid; and
4-(3-(2-propyl-3-hydroxy-4-acetyl-phenoxy)propyloxy)-phenoxy acetic acid.

16. A compound according to Claim 7 which is:
Methyl 3-chloro-4-(3-(2-propyl-3-hydroxy-4-propionylphenoxy)-propylthio)-phenylacetate;
3-Chloro-4-(3-(2-propyl-3-hydroxy-4-propionylphenoxy)propylthio)phenyl-acetic acid;
Methyl 3-chloro-4-(3-(2-propyl-3-hydroxy-4-(1-oxobutyl)phenoxy)-propylthio)phenyl acetate;
3-Chloro-4-(3-(2-propyl-3-hydroxy-4-(1-oxobutyl)phenoxy)-propylthio)-phenylacetic acid;
Methyl 3-chloro-4-(3-(2-propyl-3-hydroxy-4-(1-oxopropyl)phenoxy)-propyloxy)phenylacetate;
3-Chloro-4-(3-(2-propyl-3-hydroxy-4-(1-hydroxyliminopropyl)phenoxy)-propylthio)phenylacetic acid;
Methyl 3-chloro-4-(3-(2-propyl-3-hydroxy-4-(1-hydroxyliminopropyl)-phenoxy)propyloxy)phenyl acetate;
Methyl 3-chloro-4-(3-(2-propyl-3-hydroxy-4-(1-hydroxyliminobutyl)-phenoxy)propylthio)phenylacetate;
3-Chloro-4-(3-(2-propyl-3-hydroxy-4-(1-hydroxyliminobutyl)phenoxy)-propylthio)phenylacetic acid;
Methyl 3- chloro-4-(3-(2-propyl-3-hydroxy-4-(1-hydroxyliminopropyl)-phenoxy)propylthio)phenylacetate
3-Chloro-4-(3-(2-propyl-3-hydroxy-4-(1-hydroxylimino-2-methylpropyl)-phenoxy)propylthio)phenylacetic acid;
Methyl 3-chloro-4-(3-(2-cyclopropylmethyl-3-hydroxy-4-propionylphenoxy)propylthio)phenylacetate;
3-Chloro-4-(3-(2-cyclopropylmethyl-3-hydroxy-4-propionylphenoxy)-propylthio)phenylacetic acid;
Methyl 3-chloro-4-(4-(2-propyl-3-hydroxy-4-(1-hydroxyiminopropyl)-phenyl)butylthio)phenylacetate;
3-chloro-4-(4-(2-propyl-3-hydroxy-4-( 1-hydroxyiminopropyl)-phenyl)butylthio)phenylacetic acid;
3-Chloro-4-(3-(2-propyl-3-hydroxy-4-propionylphenoxy)propylthio)-phenyl-5-methyl-( I H)-tetrazole;
3-chloro-4-(3-(2-propyl-3-hydroxy-4-(1-oxopentyl))phenoxy)propylthio phenyl acetic acid methyl ester;
3-ch loro-4-(3-(2-propyl-3-hydroxy-4-(1-hydroxyliminopentyl))phenoxy) propylthio phenyl acetic acid methyl ester;
3-chloro-4-(3-(2-propyl-3-hydroxy-4-(1-hydroxylimino propyl)phenoxy)-propylthio)phenylacetamide;
3-chloro-4-(3-(2-propyl-3-hydroxy-4-(1-(hydroxylimino-3-(phenyl) propyl)phenoxy)-propylthio)phenylacetic acid;
Methyl 3-chloro-4-(4-phenoxy-2-propylphenoxy)-propylamino)phenylacetate;
3-Chloro-4-(4-phenoxy-2-propylphenoxy)-propylamino)phenylacetic acid;
3-chloro-4-(3-(2-propyl-3-hydroxy-4-(1-hydroxyliminopropyl)) phenoxy)propylthio phenyl acetic acid;
4-(2-(4-benzyl-2-propylphenoxy)propylthio)-3-chlorophenylacetic acid;
Methyl 3-chloro-4-(3-(2-propyl-3-hydroxy-4-(1-oxopentyl)phenoxy)-propyl)-phenylacetate;
4-(3-(4-(N-hydroxyiminobenzoyl)-2-propylphenoxy)propylthio)-3-chlorophenylacetic acid;
4-(3-(4-benzoyl-3-hydroxy-2-propylphenoxy)propylthio)-3-chlorophenylacetic acid;
4-(3-(4-Phenoxy-2-propylphenoxy)propyloxy)-phenylacetic acid;
4-(3-(4-Benzoyl-2-propylphenoxy)propyloxy)-phenylacetic acid;
3-(4-(4-Phenoxy-2-propylphenoxy)butyloxy)-phenylacetic acid;
3-(4-(4-Benzoyl-2-propylphenoxy)butyloxy)-phenylacetic acid;
4-(3-(4-Phenoxy-2-propylphenoxy)propyloxy)-phenoxyacetic acid;
4-(3-(4-Benzoyl-2-propylphenoxy)propyloxy)-phenoxyacetic acid;
4-(3-(4-Phenoxy-2-propylphenoxy)propylthio)-3-propylphenylacetic acid;
4-(4-(4-Benzoyl-2-propylphenoxy)butylthio)-3-chlorophenylacetic acid;
4-(3-(4-Phenoxy-2-propylphenoxy)propylsulfono)-3-propylphenylacetic acid;
4-(3-(4-Benzoyl-2-propylphenoxy)propylsulfono)-3-chlorophenylacetic acid;
4-(4-(4-Phenoxy-2-propylphenoxy)butylthio)-3-propylbenzyl-tetrazole;
4-(4-(4-Benzoyl-2-propylphenoxy)butylthio)-3-chlorobenzyl-tetrazole;
4-(3-(4-Phenoxy-2-propylphenoxy)propylamino)-phenylacetic acid;
4-(3-(4-Benzoyl-2-propylphenoxy)propylamino)-phenylacetic acid;
3-(4-(4-(4-Fluorophenoxy)-2-propylphenoxy)butyloxy)-phenylacetic acid;
3-(4-(4-(4-Fluorobenzoyl)-2-propylphenoxy)butyloxy)-phenylacetic acid;
3-chloro-4-(3-((1,2-benzisoxazol-3-yl)-2-propylphenoxy) propylthio)phenylacetic acid;
3-chloro-4-(3-(4-fluorobenzoyl-2-propylphenoxy)propylthio)phenyl acetic acid; and
3-chloro-4-(3-(4-phenoxy-2-propylphenoxy)propyl-thio)phenylacetic acid.

17. A compound according to anyone of claims 1 to 16 which has an IC₅₀ equal to or less than 10 µM in the hPPARδ binding assay and an EC₅₀ equal to or less than 10 µM in the hPPARδ transactivation assay.

18. A compound according to Claim 17 which has an IC₅₀ equal to or less than 100 nM in the hPPARδ binding assay and an EC₅₀ equal to or less than 100 nM in the hPPARδ transactivation assay.

19. A compound according to Claim 18 which has an IC₅₀ equal to or less than 50 nM in the hPPARδ binding assay and an EC₅₀ equal to or less than 50 nM in the hPPARδ transactivation assay.

20. A compound according to Claim 19 which has an IC₅₀ equal to or less than 10 nM in the hPPARδ binding assay and an EC₅₀ equal to or less than 10 nM in the hPPARδ transactivation assay.

21. A pharmaceutical composition comprising a compound according to any one of claims 1 to 20, or a pharmaceutically acceptable salt thereof, and an inert carrier.

22. A compound according to any one of claims 1 to 20, or a pharmaceutically acceptable salt thereof, for use in a method of treatment of the human or animal body.

23. Use of a compound according to any one of claims 1 to 20, or a pharmaceutically acceptable salt thereof, for the manufacture of a medicament for the treatment or prevention of diabetes, for lowering triglyceride levels, for treating obesity, for halting, preventing or reducing the risk of developing atherosclerosis and related disease events or for raising high density lipoprotein plasma levels.

24. A combination of a compound according to any one of claims 1 to 20, or a pharmaceutically acceptable salt thereof, and a sulfonylurea, fibrate, HMG-CoA reductase inhibitor, beta-sitosterol inhibitor, cholesterol acyltransferase inhibitor, biguanide, cholestyramine, angiotensin II antagonist, melinamide, nicotinic acid, fibrinogen receptor antagonist, aspirin, α-glucosidase inhibitor, insulin secretogogue or insulin for separate, simultaneous or sequential administration.

25. A combination of a compound according to any one of claims 1 to 20, or a pharmaceutically acceptable salt thereof, and a fenfluramine, dexfenfluramine, phentiramine or β₃ adrenergic receptor agonist for separate, simultaneous or sequential administration.

## Patentansprüche

1. Eine Verbindung mit der Formel I: 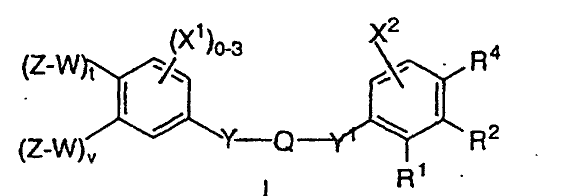 oder ein pharmazeutisch annehmbares Salz davon, wobei:
R ausgewählt ist aus der Gruppe, bestehend aus H, C₁₋₆-Alkyl, C₅₋₁₀-Aryl und C₅₋₁₀-Heteroaryl, wobei das Alkyl, Aryl und Heteroaryl gegebenenfalls mit 1 bis 3 R^{a}-Gruppen substituiert sind,
R¹ ausgewählt ist aus einer Gruppe, bestehend aus: H, C₁₋₁₅-Alkyl, C₂₋₁₅-Alkenyl, C₂₋₁₅-Alkinyl und C₃₋₁₀-Cycloalkyl, wobei das Alkyl, Alkenyl, Alkinyl und Cycloalkyl gegebenenfalls mit 1 bis 3 R^{a}-Gruppen substituiert sind,
R² ausgewählt ist aus einer Gruppe, bestehend aus: H, C₁₋₁₅-Alkyl, Acyl, C₂₋₁₅-Alkenyl, OR³, CO₂-Alkyl, C(O)R³, OH, -OC(O)R³, C₂₋₁₅-Alkinyl, C₅₋₁₀-Aryl, C₅₋₁₀-Heteroaryl, wobei das Alkyl, Alkenyl, Alkinyl, Aryl und Heteroaryl gegebenenfalls mit 1 bis 3 R^{a}-Gruppen substituiert sind,
R³ ausgewählt ist aus einer Gruppe, bestehend aus: H, NHR¹, NH-Acyl, C₁₋₁₅-Alkyl, C₂₋₁₅-Alkenyl, C₁₋₁₅-Alkoxy, CO₂-Alkyl, OH, C₂₋₁₅-Alkinyl, C₅₋₁₀-Aryl, C₅₋₁₀-Heteroaryl, wobei das Alkyl, Alkenyl, Alkinyl, Aryl und Heteroaryl gegebenenfalls mit 1 bis 3 R^{a}-Gruppen substituiert sind,
R⁴ ausgewählt ist aus der Gruppe, bestehend aus: R², -B-R⁵ oder 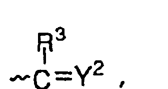 mit der Maßgabe, daß, wenn (Z-W-) Z-CR⁶R⁷ ist, Y O ist und R⁴ R² ist, R² dann nicht Acetyl, H, Alkyl, Alkoxy oder Aryl ist, oder wenn (Z-W-)
Z-CR⁶R⁷ ist, Y O ist und R⁴ 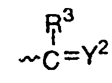 ist, Y² dann nicht O ist, oder wenn (Z-W-) Z-CR⁶R⁷- oder 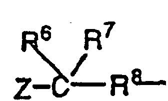 ist, wobei R⁸ O ist, Y S oder O ist und R⁴ -B-R⁵ ist, B dann nicht O oder S ist,
R⁵ ausgewählt ist aus der Gruppe, bestehend aus: C₅₋₁₀-Aryl und C₅₋₁₀-Heteroaryl, wobei das Aryl und Heteroaryl gegebenenfalls mit 1 bis 3 R^{a}-Gruppen substituiert sind,
B ausgewählt ist aus der Gruppe, bestehend aus: O, S(O)p und NR¹, (Z-W-) Z-CR⁶R⁷-, Z-CH=CH- oder 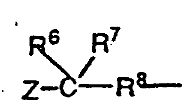 ist,
R⁸ ausgewählt ist aus der Gruppe, bestehend aus CR⁶R⁷, O, NR⁶ und S(O)ₚ,
R⁶ und R⁷ unabhängig ausgewählt sind aus der Gruppe, bestehend aus H, C₁₋₆-Alkyl,
X¹ und X² unabhängig ausgewählt sind aus einer Gruppe, bestehend aus: H, OH, C₁₋₁₅-Alkyl, C₂₋₁₅-Alkenyl, C₂₋₁₅-Alkinyl, Halogen, OR³, C₅₋₁₀-Aryl, C₅₋₁₀-Aralkyl, C₅₋₁₀-Heteroaryl und C₁₋₁₀-Acyl, wobei das Alkyl, Alkenyl, Alkinyl, Aryl und Heteroaryl gegebenenfalls mit 1 bis 3 R^{a}-Gruppen substituiert sind,
R^{a} ein Element bedeutet, ausgewählt aus der Gruppe, bestehend aus: Halogen, Aryl, Acyl, Heteroaryl, CF₃, OCF₃, -O-, CN, NO₂, R³, OR³, SR³, S(O)R³, SO₂R³, NR³R³, NR³COR³, NR³CO₂R³, NR³CON(R³)₂, NR³SO₂R³, COR³, CO₂R³, CON(R³)₂, SO₂N(R³)₂, OCON(R³)₂, wobei das Aryl und Heteroaryl gegebenenfalls mit 1 bis 3 Halogen- oder C₁₋₆-Alkylgruppen substituiert sind,
Y ausgewählt ist aus der Gruppe, bestehend aus: S(O)ₚ, -CH₂-, -C(O)-, -C(O)NH-, -NR-, -O-, -SO₂NH, -NHSO₂,
Y¹ ausgewählt ist aus der Gruppe, bestehend aus: O, NR und CH₂,
Y² ausgewählt ist aus der Gruppe, bestehend aus: O, N(C₁₋₁₅)-Alkyl, N(CO₂)-Alkyl, N-O-Alkyl, N-O-Acyl und N-OH, mit der Maßgabe, daß, wenn Y² O ist und R³ CH₃ ist, n dann 2 ist,
Z ausgewählt ist aus der Gruppe, bestehend aus: CO₂R³, CONHSO₂R, CONH₂ und 5-(1H-Tetrazol),
t und v unabhängig 0 oder 1 sind, so daß t + v = 1,
Q ein gesättigter oder ungesättigter geradkettiger Kohlenwasserstoff mit 2-4 Kohlenstoffatomen ist und
p 0-2 ist.

2. Eine Verbindung nach Anspruch 1, wobei X¹ & X² unabhängig H oder Halogen sind.

3. Eine Verbindung nach Anspruch 1, wobei Y O ist.

4. Eine Verbindung nach Anspruch 1, wobei Y S(O)ₚ ist, wobei p 0-2 ist.

5. Eine Verbindung nach Anspruch 1, wobei Y -CH₂- ist.

6. Eine Verbindung nach Anspruch 1, wobei Y -CO- ist.

7. Eine Verbindung nach Anspruch 1, wobei Y -NH- ist.

8. Eine Verbindung nach Anspruch 1, wobei Y NHSO₂ oder SO₂NH ist.

9. Eine Verbindung nach Anspruch 1, wobei Y C(O)NH ist.

10. Eine Verbindung nach Anspruch 1, wobei R⁴ R² ist, mit der Maßgabe, daß, wenn (Z-W-) Z-CR⁶R⁷ ist und Y O ist, R² dann nicht Acetyl, H, Alkyl, Alkoxy oder Aryl ist.

11. Eine Verbindung nach Anspruch 1, wobei R⁴ -B-R⁵ ist, mit der Maßgabe, daß, wenn (Z-W-) Z-CR⁶R⁷- odez 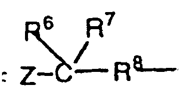 ist, wobei R⁸ O ist, und Y S oder O ist, B dann nicht O oder S ist.

12. Eine Verbindung nach Anspruch 1, wobei R⁴ 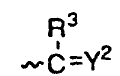 ist, mit der Maßgabe, daß, wenn (Z-W-) Z-CR⁶R⁷ ist und Y O ist, Y² dann nicht O ist.

13. Eine Verbindung nach Anspruch 1, wobei W -CR⁶R⁷- oder 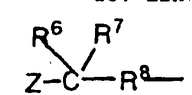 ist.

14. Eine Verbindung nach Anspruch 1, wobei:
R C₁₋₆-Alkyl oder C₅₋₁₀-Aryl ist, wobei das Alkyl oder Aryl gegebenenfalls mit 1 bis 3 R^{a}-Gruppen substituiert ist,
R¹ C₁₋₁₅-Alkyl ist,
X¹ & X² unabhängig H, C₁₋₆-Alkyl oder Halogen sind,
Y O, NH oder S ist,
Y¹ O ist,
(Z-W-) Z-CR⁶R⁷- oder 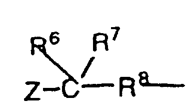 ist,
R⁴ -B-R⁵ oder 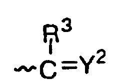 ist, mit der Maßgabe, daß, wenn (Z-W-) Z-CR⁶R⁷ ist, Y O ist und R⁴ 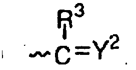 ist, Y² dann nicht O ist, oder wenn (Z-W-) Z-CR⁶R⁷- oder 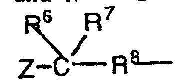 ist, wobei R⁸ O ist, Y S oder O ist und R⁴ -B-R⁵ ist, B dann nicht O oder S ist,
R^{a} ein Element ist, ausgewählt aus der Gruppe, bestehend aus: Halogen, Aryl, Acyl, Heteroaryl, CF₃, OCF₃, -O-, CN, NO₂, R³, OR³, SR³, S(O)R³, SO₂R³, NR³COR³, COR³, CON(R³)₂, SO₂N(R³)₂, wobei das Aryl und Heteroaryl gegebenenfalls mit 1 bis 3 Halogen- oder C₁₋₆-Alkylgruppen substituiert sind, und
Z CO₂R³, CONHSO₂R, CONH₂ oder 5-(1H-Tetrazol) ist.

15. Eine Verbindung nach Anspruch 1, ausgewählt aus der Gruppe, bestehend aus:
Methyl-3-chlor-4-(3-(2-propyl-3-hydroxy-4-propionylphenoxy)propylthio)-phenylacetat,
3-Chlor-4-(3- (2-propyl-3-hydroxy-4-propionylphenoxy)propylthio)phenylessigsäure,
Methyl-3-chlor-4-(3-(2-propyl-3-hydroxy-4-(1-oxobutyl)phenoxy)propylthio)-phenylacetat,
3-Chlor-4-(3-(2-propyl-3-hydroxy-4-(1-oxobutyl)phenoxy)propylthio)phenylessigsäure,
Methyl-3-chlor-4-(3-(2-propyl-3-hydroxy-4-(1-oxo-2-methylpropyl)phenoxy)-propylthio)phenylacetat,
Methyl-3-chlor-4-(3-(2-propyl-3-hydroxy-4-(1-oxopropyl)phenoxy)propyloxy)phenylacetat,
3-Chlor-4-(3-(2-propyl-3-methoxy-4-propionylphenoxy)propylthio)phenylessigsäure,
Methyl-3-chlor-4-(3-(2-propyl-3-methoxy-4-propionylphenoxy)propylthio)-phenylacetat,
Methyl-3-chlor-4-(3-(2-propyl-3-acetoxy-4-propionylphenoxy)propylthio)-phenylacetat,
Methyl-3-chlor-4-(3-(2-propyl-3-hydroxy-4-(1-hydroxyliminopropyl)phenoxy)-propylthio)phenylacetat,
3-Chlor-4- (3-(2-propyl-3-hydroxy-4-(1-hydroxyliminopropyl)phenoxy)propylthio)phenylessigsäure,
Methyl-3-chlor-4-(3-(2-propyl-3-methoxy-4-(1-hydroxyliminopropyl)phenoxy)-propylthio)phenylacetat,
3-Chlor-4- (3-(2-propyl-3-methoxy-4-(1-hydroxyliminopropyl)phenoxy)propylthio)phenylessigsäure,
Methyl-3-chlor-4-(3-(2-propyl-3-hydroxy-4-(1-hydroxyliminopropyl)phenoxy)-propyloxy)phenylacetat,
Methyl-3-chlor-4-(3-(2-propyl-3-hydroxy-4-(1-methoxyliminopropyl)phenoxy)-propylthio)phenylacetat,
Methyl-3-chlor-4-(3-(2-propyl-3-hydroxy-4-(1-hydroxyliminobutyl)phenoxy)-propylthio)phenylacetat,
3-Chlor-4-(3-(2-propyl-3-hydroxy-4-(1-hydroxyliminobutyl)phenoxy)propylthio)phenylessigsäure,
Methyl-3-chlor-4-(3-(2-propyl-3-hydroxy-4-(1-acetoxyiminobutyl)phenoxy)-propylthio)phenylacetat,
Methyl-3-chlor-4-(3-(2-propyl-3-hydroxy-4-(1-hydroxylimino-2-methylpropyl)phenoxy)propylthio)phenylacetat,
3-Chlor-4-(3-(2-propyl-3-hydroxy-4-(1-hydroxylimino-2-methylpropyl)-phenoxy)propylthio)phenylessigsäure,
Methyl-3-chlor-4-(3-(2-propyl-3-acetoxy-4-(1-hydroxyiminopropyl)phenoxy)-propylthio)phenylacetat,
3-Chlor-4-(3-(2-propyl-3-hydroxy-4-(1-oxopropyl)phenoxy)propylthio)-phenylacetamid,
Methyl-3-chlor-4-(3-(2-cyclopropylmethyl-3-hydroxy-4-propionylphenoxy)-propylthio)phenylacetat,
3-Chlor-4-(3-(2-cyclopropylmethyl-3-hydroxy-4-propionylphenoxy)propyl-. thio)phenylessigsäure,
Methyl-3-chlor-4-(3-(2-cyclopropylmethyl-3-hydroxy-4-(1-hydroxyiminopropyl)phenoxy)propylthio)phenylacetat,
3-Chlor-4-(3-(2-cyclopropylmethyl-3-hydroxy-4-(1-hydroxyiminopropyl)-phenoxy)propylthio)phenylessigsäure,
Methyl-3-chlor-4-(4-(2-propyl-3-hydroxy-4-propionylphenyl)butylthio)-phenylacetat,
3-Chlor-4- (4- (2-propyl-3-hydroxy-4-propionylphenyl)butylthio)phenylessigsäure,
Methyl-3-chlor-4-(4-(2-propyl-3-hydroxy-4-(1-hydroxyiminopropyl)phenyl)-butylthio)phenylacetat,
3-Chlor-4-(4-(2-propyl-3-hydroxy-4-(1-hydroxyiminopropyl)phenyl) -butylthio)phenylessigsäure,
Methyl-3-chlor-4-(3-(2-(2-propenyl)-3-hydroxy-4-propionylphenoxy)propylthio)phenylacetat,
Methyl-3-chlor-4-(3-(2-(2-propenyl)-3-hydroxy-4-(1-hydroxyiminopropyl)-phenoxy)propylthio)phenylacetat,
3-Chlor-4-(3-(2-(2-propenyl)-3-hydroxy-4-(1-hydroxyiminopropyl)phenoxy)propylthio)phenylessigsäure,
Methyl-3-chlor-4-(3-(2-propyl-4-propionylphenoxy)propylthio)phenylacetat, , 3-Chlor-4-(3-(2-propyl-4-propionylphenoxy)propylthio)phenylessigsäure,
Methyl-3-chlor-4-(3- (2-propyl-4-(1-hydroxyiminopropyl)phenoxy)propylthio)-phenylacetat,
3-Chlor-4-(3-(2-propyl-4-(1-hydroxyiminopropyl)phenoxy)propylthio)phenylessigsäure,
Methyl-3-chlor-4-(4-(2-propyl-3-hydroxy-4-acetylphenoxy)butylthio)phenylacetat,
3-Chlor-4-(4-(2-propyl-3-hydroxy-4-acetylphenoxy)butylthio)phenylessigsäure,
3-Chlor-4-(3-(2-propyl-3-hydroxy-4-acetyl)phenoxy)propylthiophenyl-5-methyl-(1H)-tetrazol,
3-Chlor-4-(4-(2-propyl-3-hydroxy-4-acetylphenoxy)butylthio)phenyl-5-methyl-(1H)-tetrazol,
3-Chlor-4-(3-(2-propyl-3-hydroxy-4-propionylphenoxy)propylthio)phenyl-5-methyl-(1H)-tetrazol,
3-Chlor-4-(3-(2-propyl-3-hydroxy-4-(1-hydroxyiminopropyl)phenoxy)propylthio) phenyl-5-methyl- (1H)-tetrazol,
3-Chlor-4-(4-(2-propyl-3-hydroxy-4-propionylphenoxy)butylthio)phenyl-5-methyl-(1H)-tetrazol,
3-Chlor-4-(4-(2-propyl-3-hydroxy-4-(1-hydroxyiminopropyl)phenoxy)butylthio)phenyl-5-methyl-(1H)-tetrazol,
4- (3- (2-Propyl-3-hydroxy-4-acetylphenoxy)propyloxy)phenyl-5-methyl-(1H)-tetrazol,
4- (4-(2-Propyl-3-hydroxy-4-propionylphenoxy)butyloxy)phenyl-5-methyl-(1H)-tetrazol,
4-(3-(2-Propyl-3-hydroxy-4-acetylphenoxy)propylthio)phenyl-5-methyl-(1H)-tetrazol,
3-Chlor-4-(3-(2-propyl-3-hydroxy-4-(1-oxopentyl))phenoxy)propylthiophenylessigsäuremethylester,
3-Chlor-4-(3-(2-propyl-3-hydroxy-4-(1-hydroxyliminopentyl))phenoxy)propylthiophenylessigsäuremethylester,
3-Chlor-4- (3- (2-propyl-3-hydroxy-4- (1-hydroxyliminopropyl)phenoxy)propylthio)phenylacetamid,
3-Chlor-4-(3-(2-propyl-3-hydroxy-4-(1-(hydroxylimino-3-(phenyl)propyl)-phenoxy)propylthio)phenylessigsäure,
Methyl-3-chlor-4- (4-phenoxy-2-propylphenoxy)propylamino)phenylacetat,
3-Chlor-4- (4-phenoxy-2-propylphenoxy)propylamino)phenylessigsäure,
3-Chlor-4-(3-(2-propyl-3-hydroxy-4-(1-hydroxyliminopropyl))phenoxy)propylthiophenylessigsäure,
4-(2-(4-Benzyl-2-propylphenoxy)propylthio)-3-chlorphenylessigsäure, Methyl-3-chlor-4-(3-(2-propyl-3-hydroxy-4- (1-oxopentyl)phenoxy)propyl)-phenylacetat,
4-(3-(4-(N-Hydroxyiminobenzoyl)-2-propylphenoxy)propylthio)-3-chlorphenylessigsäure,
4-(3-(4-Benzoyl-3-hydroxy-2-propylphenoxy)propylthio)-3-chlorphenylessigsäure,
4- (3- (4-Phenoxy-2-propylphenoxy)propylamino)phenyl (2, 2-dimethyl)essigsäure,
4-(3-(4-Phenoxy-2-propylphenoxy)propylamino)phenylpropan-3-säure,
N-[4-(3-(4-Phenoxy-2-propylphenoxy)propylamino)phenyl]glycin,
4-(3-(4-Phenoxy-2-propylphenoxy)propylamino)phenylessigsäure,
4-(3-(4-Phenoxy-2-propylphenoxy)propylamino)-3-chlorphenylessigsäure,
4-(3-(4-Phenoxy-2-propylphenoxy)propylamino)phenoxyessigsäure,
4-(3-(4-Phenoxy-2-propylphenoxy)propylthio)phenylpropan-3-säure,
4-(3-(4-Phenoxy-2-propylphenoxy)propyloxy)-2-phenyl-2,2-dimethylessigsäure,
4-(4-(4-Phenoxy-2-propylphenoxy)butylamino)phenoxy-2,2-dimethylessigsäure,
4-(3-(4-Phenoxy-2-propylphenoxy)propylthio)-3-methylphenylessigsäure,
4-(3-(4-Phenoxy-2-propylphenoxy)propyloxy)-3-butylphenylessigsäure,
4-(3-(4-Phenoxy-2-propylphenoxy)propylamino)-2-propylphenylessigsäure,
4-(4-(4-Phenoxy-2-propylphenoxy)butylthio)-2-fluorphenylessigsäure,
4-(3-(4-Phenoxy-2-propylphenoxy)propyloxy)phenoxy-2-spiro-cyclopropylessigsäure,
3-(3-(4-Phenoxy-2-propylphenoxy)propyloxy)phenoxyessigsäure,
3-(3- (4-Phenoxy-2-propylphenoxy)propylamino)phenylpropan-3-säure,
3-(3-(4-Phenoxy-2-propylphenoxy)propylthio)-2-phenyl-2,2-dimethylessigsäure,
3-(4-(4-Phenoxy-2-propylphenoxy)butyloxy)phenoxy-2,2-dimethylessigsäure,
3-(3-(4-Phenoxy-2-propylphenoxy)propylthio)-3-methylphenylessigsäure,
3-(3-(4-Phenoxy-2-propylphenoxy)propylthio)-3-chlorphenylessigsäure,
4-(3-(4-Phenoxy-2-propylphenoxy)propylthio)-3-chlorphenylessigsäure,
3- (4-(4-Phenoxy-2-propylphenoxy)butylamino)-3-butylphenylessigsäure,
3-(3-(4-Phenoxy-2-propylphenoxy)propyloxy)-2-propylphenylessigsäure,
3-(3-(4-Phenoxy-2-propylphenoxy)propylthio)-2-fluorphenylessigsäure,
3-(4-(4-Phenoxy-2-propylphenoxy)butylamino)phenoxy-2-spiro-cyclopropylessigsäure,
4-(3-(4-Phenoxy-2-(cyclopropylmethyl)phenoxy)propylamino)phenylessigsäure,
4-(3-(4-Phenoxy-2-(prop-2-enyl)phenoxy)propylamino)phenylessigsäure,
4-(3-(4-Phenoxy-2-butylphenoxy)propylamino)phenylessigsäure,
4-(3-(4-Phenoxy-2-propoxyphenoxy)propylamino)phenylessigsäure,
3-(4-(4-Phenoxy-2-(cyclopropylmethyl)phenoxy)butyloxy)phenylessigsäure,
3-(4- (4-Phenoxy-2-(prop-2-enyl)phenoxy)butylamino)phenylessigsäure,
3-(4-(4-Phenoxy-2-butylphenoxy)butyloxy)phenylessigsäure,
3-(4-(4-Phenoxy-2-propoxyphenoxy)butyloxy)phenylessigsäure,
4-(3- (4-Phenoxy-3-hydroxy-2-propylphenoxy)propylthio)phenylessigsäure,
3-(3-(4-Phenoxy-3-methoxy-2-propylphenoxy)propylthio)phenylessigsäure,
4-(4-(4-Phenoxy-3-butyloxy-2-propylphenoxy)butyloxy)phenylessigsäure,
4-(4-(4-Phenoxy-3-chlor-2-propylphenoxy)butyloxy)phenylessigsäure,
4-(3-(4-Benzoyl-2-propylphenoxy)propylthio)phenylessigsäure,
4-(3-(4-(4-Chlor)benzoyl-2-propylphenoxy)propylthio)phenylessigsäure,
4-(4-(4-(4-Tolyl)benzoyl-2-propylphenoxy)butyloxy)phenylessigsäure,
3-(4-(4-(4-Butylcarbonyl)-2-propylphenoxy)butyloxy) phenylessigsäure,
4-(3-(4-(N-Hydroxyiminoacetyl)-2-propylphenoxy)propylthio)phenylessigsäure,
3-(3-(4-(2-Phenylethylcarbonyl)-2-propylphenoxy)propylthio)phenylessigsäure,
4-(4-(4-(2-Phenylethoxycarbonyl-2-propylphenoxy)butyloxy)phenylessigsäure,
4-(4-(4-(4-Phenylthiocarbonyl)-2-propylphenoxy)butyloxy)phenylessigsäure,
3-Chlor-4-(3-((1,2-benzisoxazol-3-yl)-2-propylphenoxy)propylthio)phenylessigsäure,
3-Chlor-4-(3-((1,2-benzisoxazol-3-yl)-2-propylphenoxy)butyloxy)phenylessigsäure,
3-(4-(4-Benzoyl-2-propylphenoxy)butyloxy)phenylessigsäure,
3-Chlor-4-(3-(4-fluorbenzoyl-2-propylphenoxy)propylthio)phenylessigsäure,
3-Chlor-4-(3-(4-phenyl-2-propylphenoxy)propylthio)phenylessigsäure,
3-Chlor-4-(3-(N-ethyl-N-(4-acetyl-3-hydroxy-2-propylphenyl)amino)propylthio)phenylessigsäure,
3-Chlor-4-(3-(N-(4-hydroxyiminoacetyl-3-hydroxy-2-propylphenyl)amino)-propylthio)phenylessigsäure,
3-Chlor-4- (4-phenoxy- (4-trifluormethyl)propylamino)phenylessigsäure,
3-Chlor-4- (4-phenoxy-2-propyl- (4- (4-fluor)phenylsulfonyl)propylthio)phenylessigsäure,
3-Fluor-4- (4- (4-phenoxy-2-propylphenoxy)butyloxy)phenylessigsäure,
3-(4-(1-(2-Propyl-4-phenethyl)hydrochinolyl)butyloxy)phenylessigsäure,
3-Chlor-4-(3-(2-propyl-3-hydroxy-4-(1-acetoxyiminopropyl))phenoxy)propylthio)phenylessigsäure,
3-Chlor-4- (3-(2-propyl-3-methoxy-4-methylaminocarbonylphenoxy)propylthio)-phenylessigsäure,
3-Chlor-4- (3- (4-phenoxy-2-propylphenoxy)propylthio)phenylessigsäure,
3-Chlor-4-((2-propyl-5-phenoxy)phenoxypropylthio)phenylessigsäure,
3-Chlor-4-(3- (2-propyl-3-benzoylphenoxy)propylthio)phenylessigsäure und
4-(3-(2-Propyl-3-hydroxy-4-acetylphenoxy)propyloxy)phenoxyessigsäure.

16. Eine Verbindung gemäß Anspruch 7, die ist:
Methyl-3-chlor-4-(3-(2-propyl-3-hydroxy-4-propionylphenoxy)propylthio)-phenylacetat,
3-Chlor-4-(3-(2-propyl-3-hydroxy-4-propionylphenoxy)propylthio)phenylessigsäure,
Methyl-3-chlor-4-(3-(2-propyl-3-hydroxy-4-(1-oxobutyl)phenoxy)propylthio)-phenylacetat,
3-Chlor-4-(3-(2-propyl-3-hydroxy-4-(1-oxobutyl)phenoxy)propylthio)phenylessigsäure,
Methyl-3-chlor-4-(3-(2-propyl-3-hydroxy-4-(1-oxopropyl)phenoxy)propyloxy)-phenylacetat,
3-Chlor-4-(3-(2-propyl-3-hydroxy-4-(1-hydroxyliminopropyl)phenoxy)propylthio)phenylessigsäure,
Methyl-3-chlor-4-(3-(2-propyl-3-hydroxy-4-(1-hydroxyliminopropyl)phenoxy)-propyloxy)phenylacetat,
Methyl-3-chlor-4-(3-(2-propyl-3-hydroxy-4-(1-hydroxyliminobutyl)phenoxy)-propylthio)phenylacetat,
3-Chlor-4-(3-(2-propyl-3-hydroxy-4-(1-hydroxyliminobutyl)phenoxy)propylthio)phenylessigsäure,
Methyl-3-chlor-4-(3-(2-propyl-3-hydroxy-4-(1-hydroxyliminopropyl)phenoxy)-propylthio)phenylacetat,
3-Chlor-4-(3-(2-propyl-3-hydroxy-4-(1-hydroxylimino-2-methylpropyl)-phenoxy)propylthio)phenylessigsäure,
Methyl-3-chlor-4-(3-(2-cyclopropylmethyl-3-hydroxy-4-propionylphenoxy)-propylthio)phenylacetat,
3-Chlor-4-(3-(2-cyclopropylmethyl-3-hydroxy-4-propionylphenoxy)propylthio)phenylessigsäure,
Methyl-3-chlor-4-(4-(2-propyl-3-hydroxy-4-(1-hydroxyiminopropyl)phenyl)-butylthio)phenylacetat,
3-Chlor-4-(4-(2-propyl-3-hydroxy-4-(1-hydroxyiminopropyl)phenyl)-butylthio)phenylessigsäure,
3-Chlor-4-(3-(2-propyl-3-hydroxy-4-propionylphenoxy)propylthio)phenyl-5-methyl-(1H)-tetrazol,
3-Chlor-4-(3-(2-propyl-3-hydroxy-4-(1-oxopentyl))phenoxy)propylthiophenylessigsäuremethylester,
3-Chlor-4-(3-(2-propyl-3-hydroxy-4-(1-hydroxyliminopentyl))phenoxy)propylthiophenylessigsäuremethylester,
3-Chlor-4-(3-(2-propyl-3-hydroxy-4-(1-hydroxyliminopropyl)phenoxy)propylthio)phenylacetamid,
3-Chlor-4-(3-(2-propyl-3-hydroxy-4-(1-(hydroxylimino-3-(phenyl)propyl)-phenoxy)propylthio)phenylessigsäure,
Methyl-3-chlor-4-(4-phenoxy-2-propylphenoxy)propylamino)phenylacetat,
3-Chlor-4-(4-phenoxy-2-propylphenoxy)propylamino)phenylessigsäure,
3-Chlor-4-(3-(2-propyl-3-hydroxy-4-(1-hydroxyliminopropyl))phenoxy)-propylthiophenylessigsäure,
4-(2-(4-Benzyl-2-propylphenoxy)propylthio)-3-chlorphenylessigsäure,
Methyl-3-chlor-4-(3-(2-propyl-3-hydroxy-4-(1-oxopentyl)phenoxy)propyl)-phenylacetat,
4-(3-(4- (N-Hydroxyiminobenzoyl)-2-propylphenoxy)propylthio)-3-chlorphenylessigsäure,
4-(3- (4-Benzoyl-3-hydroxy-2-propylphenoxy)propylthio)-3-chlorphenylessigsäure,
4-(3-(4-Phenoxy-2-propylphenoxy)propyloxy)phenylessigsäure,
4-(3-(4-Benzoyl-2-propylphenoxy)propyloxy)phenylessigsäure,
3-(4-(4-Phenoxy-2-propylphenoxy)butyloxy)phenylessigsäure,
3-(4-(4-Benzoyl-2-propylphenoxy)butyloxy)phenylessigsäure,
4-(3-(4-Phenoxy-2-propylphenoxy)propyloxy)phenoxyessigsäure,
4-(3-(4-Benzoyl-2-propylphenoxy)propyloxy)phenoxyessigsäure,
4-(3-(4-Phenoxy-2-propylphenoxy)propylthio)-3-propylphenylessigsäure,
4-(4-(4-Benzoyl-2-propylphenoxy)butylthio)-3-chlorphenylessigsäure,
4-(3-(4-Phenoxy-2-propylphenoxy)propylsulfono)-3-propylphenylessigsäure,
4-(3-(4-Benzoyl-2-propylphenoxy)propylsulfono)-3-chlorphenylessigsäure,
4-(4-(4-Phenoxy-2-propylphenoxy)butylthio)-3-propylbenzyltetrazol,
4-(4-(4-Benzoyl-2-propylphenoxy)butylthio)-3-chlorbenzyltetrazol,
4-(3-(4-Phenoxy-2-propylphenoxy)propylamino)phenylessigsäure,
4-(3-(4-Benzoyl-2-propylphenoxy)propylamino)phenylessigsäure,
3-(4-(4-(4-Fluorphenoxy)-2-propylphenoxy)butyloxy)phenylessigsäure,
3-(4-(4-(4-Fluorbenzoyl)-2-propylphenoxy)butyloxy)phenylessigsäure,
3-Chlor-4-(3-((1,2-benzisoxazol-3-yl)-2-propylphenoxy)propylthio)phenylessigsäure,
3-Chlor-4-(3-(4-fluorbenzoyl-2-propylphenoxy)propylthio)phenylessigsäure und
3-Chlor-4-(3-(4-phenoxy-2-propylphenoxy)propylthio)phenylessigsäure.

17. Eine Verbindung gemäß irgendeinem der Ansprüche 1 bis 16, die im hPPARδ-Bindungsassay einen IC₅₀-Wert gleich oder geringer als 10 µM und im hPPARδ-Transaktivierungsassay einen EC₅₀-Wert gleich oder geringer als 10 µM besitzt.

18. Eine Verbindung gemäß Anspruch 17, die im hPPARδ-Bindungsassay einen IC₅₀-Wert gleich oder geringer als 100 nM und im hPPARδ-Transaktivierungsassay einen EC₅₀-Wert gleich oder geringer als 100 nM besitzt.

19. Eine Verbindung gemäß Anspruch 18, die im hPPARδ-Bindungsassay einen IC₅₀-Wert gleich oder geringer als 50 nM und im hPPARδ-Transaktivierungsassay einen EC₅₀-Wert gleich oder geringer als 50 nM besitzt.

20. Eine Verbindung gemäß Anspruch 19, die im hPPARδ-Bindungsassay einen IC₅₀-Wert gleich oder geringer als 10 nM und im hPPARδ-Transaktivierungsassay einen EC₅₀-Wert gleich oder geringer als 10 nM besitzt.

21. Eine pharmazeutische Zusammensetzung, die eine Verbindung gemäß irgendeinem der Ansprüche 1 bis 20 oder ein pharmazeutisch annehmbares Salz davon und einen inerten Träger enthält.

22. Eine Verbindung gemäß irgendeinem der Ansprüche 1 bis 20 oder ein pharmazeutisch annehmbares Salz davon zur Verwendung bei einem Verfahren zur Behandlung des Menschen- oder Tierkörpers.

23. Die Verwendung einer Verbindung gemäß irgendeinem der Ansprüche 1 bis 20 oder eines pharmazeutisch annehmbaren Salzes davon zur Herstellung eines Medikaments zur Behandlung oder Prävention von Diabetes, zur Senkung von Triglyceridspiegeln, zur Behandlung von Fettsucht, zum Stoppen, Verhindern oder Verringern des Risikos der Ausbildung von Atherosklerose und verwandten Erkrankungsfällen oder zur Erhöhung von High-Density-Lipoprotein-Plasmaspiegeln.

24. Eine Kombination aus einer Verbindung gemäß irgendeinem der Ansprüche 1 bis 20 oder einem pharmazeutisch annehmbaren Salz davon und einem Sulfonylharnstoff, Fibrat, HMG-CoA-Reduktaseinhibitor, beta-Sitosterol-Inhibitor, Cholesterinacyltransferaseinhibitor, Biguanidin, Cholestyramin, Angiotensin-II-Antagonisten, Melinamid, Nicotinsäure, Fibrinogenrezeptorantagonisten, Aspirin, α-Glucosidaseinhibitor, Insulinsekretagogum oder Insulin zur getrennten, gleichzeitigen oder aufeinanderfolgenden Verabreichung.

25. Eine Kombination aus einer Verbindung gemäß irgendeinem der Ansprüche 1 bis 20 oder einem pharmazeutisch annehmbaren Salz davon und einem Fenfluramin, Dexfenfluramin, Phentiramin oder β3-adrenergen Rezeptorantagonisten zur getrennten, gleichzeitigen oder aufeinanderfolgenden Verabreichung.

## Revendications

1. Composé de formule I : 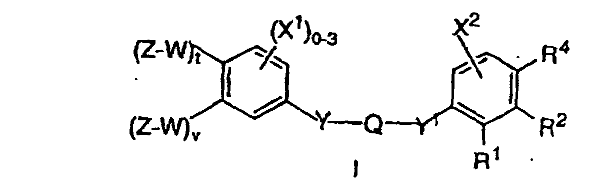 ou un sel pharmaceutiquement acceptable de celui-ci, où
R cst choisi dans le groupe constitué par : H, un reste alkyle en C₁₋₆, aryle en C₅₋₁₀ et hétéroaryle en C₅₋₁₀, lesdits restes alkyle, aryle et hétéroaryle étant substitués de façon optionnelle par 1 à 3 groupes R^{a};
R¹ est choisi dans le groupe constitué par : H, un reste alkyle en C₁₋₁₅, alcényle en C₂₋₁₅, alcynyle en C₂₋₁₅ et cycloalkyle en C₃₋₁₀, lesdits restes alkyle, alcényle, alcynyle et cycloalkyle étant substitués de façon optionnelle par 1 à 3 groupes R^{a};
R² est choisi dans le groupe constitué par : H, un reste alkyle en C₁₋₁₅, acyle, alcényle en C₂₋₁₅, OR³, CO₂alkyle, C(O)R³, OH, -OC(O)R³, alcynyle en C₂₋₁₅, aryle en C₅₋₁₀ et hétéroaryle en C₅₋₁₀, lesdits restes alkyle, alcényle, alcynyle, aryle et hétéroaryle étant substitués de façon optionnelle par 1 à 3 groupes R^{a};
R³ est choisi dans le groupe constitué par : H, NHR¹, NHacylc, un reste alkyle en C₁₋₁₅, alcényle en C₂₋₁₅, alcoxy en C₁₋₁₅, CO₂alkyle, OH, alcynyle en C₂₋₁₅, aryle en C₅₋₁₀, hétéroaryle en C₅₋₁₀, lesdits restes alkyle, alcénylc, alcynyle, aryle et hétéroaryle étant substitués de façon optionnelle par 1 à 3 groupcs R^{a};
R⁴ est choisi dans le groupe constitué par : R², -B-R⁵ ou 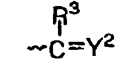 sous réserve que si (Z-W-) est Z-CR⁶R⁷-, Y est O et R⁴ est R², alors R² n'est pas acétyle, H, alkyle, alcoxy ni aryle ; ou si (Z-W-) est Z-CR⁶R⁷-, Y est O et R⁴ est 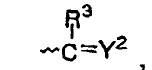 alors Y² n'est pas O; ou si (Z-W-) est Z-CR⁶R⁷- ou 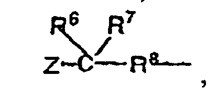 où R⁸ est O, Y est O ou S et R⁴ est -B-R⁵, alors B n'est ni O ni S ;
R⁵ est choisi dans le groupe constitué par ; un reste aryle en C₅₋₁₀ et hétéroaryle en C₅₋₁₀, lesdits restes aryle et hétéroaryle étant de façon optionnelle substitués par 1 à 3 groupes R^{a} ;
B est choisi dans le groupe constitué par : O, S(O)ₚ et NR¹ ;
(Z-W-) est Z-CR⁶R⁷-, Z-CH=CH- ou 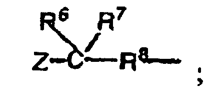 R⁸ est choisi dans le groupc constitué par CR⁶R⁷, O, NR⁶ et S(O)ₚ ;
R⁶ et R⁷ sont, chacun de façon indépendante, choisis dans le groupe constitué par un H et un reste alkyle en C₁₋₆;
X¹ et X² sont, chacun de façon indépendante, choisis dans le groupe constitué par : H, OH, un reste alkyle en C₁₋₁₅, alcényle en C₂₋₁₅, alcynyle en C₂₋₁₅, halogéno, OR³, aryle en C₅₋₁₀, (aryl en C₅₋₁₀)alkyle, hétéroaryle en C₅₋₁₀ et acyle en C₁₋₁₀, lesdits restes alkyle, alcényle, alcynyle, aryle et hétéroaryle étant de façon optionnelle substitués par 1 à 3 groupes R^{a};
R^{a} représente un élément choisi dans le groupe constitué par les restes : halogéno, aryle, acyle, hétéroaryle, CF₃, OCF₃, -O-, CN, NO₂, R³, OR³, SR³, S(O)R³, SO₂R³, NR³R³, NR³COR³, NR³CO₂R³, NR³CON(R³)₂, NR³SO₂R³, COR³, CO₂R³, CON(R³)₂, SO₂N(R³)₂, OCON(R³)₂, lesdits restes aryle et hétéroaryle étant de façon optionnelle substitués par 1 à 3 restes halogéno ou alkyle en C₁₋₆ ;
Y est choisi dans le groupe constitué par : S(O)ₚ, -CH₂-, -C(O)-, -C(O)NH-, -NR-, -O-, -SO₂NH, -NHSO₂ ;
Y¹ est choisi dans le groupe constitué par : O, NR et CH₂ ;
Y² est choisi dans le groupe constitué par : O, N(alkyle en C₁₋₁₅), N(CO₂)alkyle, N-O-alkyle, N-O-acyle et N-OH, sous réserve que si Y² est O et
R³ est CH₃, alors n est égal à 2;
Z est choisi dans le groupe constitué par : CO₂R³, CONHSO₂R, CONH₂ et 5-(1H-tétrazole) ;
t et v sont, chacun de façon indépendante, égaux à 0 ou 1, et t + v = 1
Q est un groupe hydrocarboné à chaîne linéaire saturée ou insaturée contenant 2 à 4 atomes de carbone et
p est un nombre de 0 à 2.

2. Composé selon la revendication 1, dans lequel X¹ et X² sont, chacun de façon indépendante, un atome d'hydrogène ou d'halogène.

3. Composé selon la revendication 1, dans lequel Y est O.

4. Composé selon la revendication 1, dans lequel Y est S(O)ₚ, où p est un nombre de 0 à 2.

5. Composé selon la revendication 1, dans lequel Y est -CH₂-.

6. Composé selon la revendication 1, dans lequel Y est -CO-.

7. Composé selon la revendication 1, dans lequel Y est -NH-.

8. Composé selon la revendication 1, dans lequel Y est NHSO₂ ou SO₂NH.

9. Composé selon la revendication 1, dans lequel Y est C(O)NH.

10. Composé selon la revendication 1, dans lequel R⁴ est R², sous réserve que si (Z-W-) est Z-CR⁶R⁷- et Y est O, alors R² n'est pas acétyle, H, alkyle, alcoxy ni aryle.

11. Composé selon la revendication 1, dans lequel R⁴ est -B-R⁵, sous réserve que si (Z-W-) est Z-CR⁶R⁷- ou 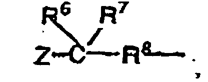 où R⁸ est O et Y est O ou S, alors B n'est ni O ni S.

12. Composé selon la revendication 1, dans lequel R⁴ est 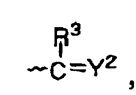 sous réserve que, si (Z-W-) est Z-CR⁶R⁷- et Y est O alors Y² n'est pas O.

13. Composé selon la revendication 1, dans lequel W est -CR⁶R⁷ ou

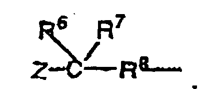

14. Composé selon la revendication 1, dans lequel :
R est un reste alkyle en C₁₋₆ ou aryle en C₅₋₁₀, lesdits restes alkyle ou aryle étant substitués de façon optionnelle par 1 à 3 groupes R^{a} ;
R¹ est un reste alkyle en C₁₋₁₅ ;
X¹ et X² sont, chacun de façon indépendante, H, un reste alkyle en C₁₋₆ ou halogéno ;
Y est O, NH ou S;
Y¹ est O;
(Z-W-) est Z-CR⁶R⁷- ou 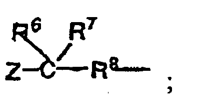
R⁴ est -B-R⁵ ou 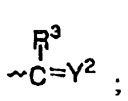 sous réserve que si (Z-W-) est Z-CR⁶R⁷-, Y est O et R⁴ est 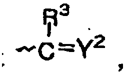 alors Y² n'est pas O ; ou si (Z-W-) est Z-CR⁶R⁷- ou 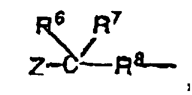 où R⁸ est O, Y est O ou S et R⁴ est -B-R⁵, alors B n'est ni O ni S ;
R^{a} est un élément choisi dans le groupe constitué par les restes : halogéno, aryle, acyle, hétéroaryle, CF₃, OCF₃, -O-, CN, NO₂, R³, OR³, SR³, S(O)R³, SO₂R³, NR³COR³, COR³, CON(R³)₂, SO₂N(R³)₂, lesdits restes aryle et hétéroaryle étant de façon optionnelle substitués par 1 à 3 restes halogéno ou alkyle en C₁₋₆ et
Z est CO₂R³, CONHSO₂R, CONH₂ ou 5-(1H-tétrazole).

15. Composé selon la revendication 1, choisi dans le groupe constitué par :
le 3-chloro-4-(3-(2-propyl-3-hydroxy-4-propionylphénoxy)propylthio)phénylacétate de méthyle ;
l'acide 3-chloro-4-(3-(2-propyl-3-hydroxy-4-propionylphénoxy)propylthio)-phénylacétique ;
le 3-chloro-4-(3-(2-propyl-3-hydroxy-4-(1-oxobutyl)phénoxy)propylthio)-phénylacétate de méthyle ;
l'acide 3-chloro-4-(3-(2-propyl-3-hydroxy-4-(1-oxobutyl)phénoxy)propylthio)-phénylacétique ;
le 3-chloro-4-(3-(2-propyl-3-hydroxy-4-(1-oxo-2-méthylpropyl)phénoxy)-propylthio)phénylacétate de méthyle ;
le 3-chloro-4-(3-(2-propyl-3-hydroxy-4-(1-oxopropyl)phénoxy)propyloxy)-phénylacctate de méthyle ;
l'acide 3-chloro-4-(3-(2-propyl-3-méthoxy-4-propionylphénoxy)propylthio)-phénylacétique ;
le 3-chloro-4-(3-(2-propyl-3-méthoxy-4-propionylphénoxy)propylthio)-phénylacétate de méthyle ;
le 3-chloro-4-(3-(2-propyl-3-acétoxy-4-propionylphénoxy)propylthio)-phénylacétate de méthyle ;
le 3-chloro-4-(3-(2-propyl-3-hydroxy-4-(1-hydroxyiminopropyl)phénoxy)-propylthio)phénylacétate de méthyle ;
l'acide 3-chloro-4-(3-(2-propyl-3-hydroxy-4-(1-hydroxyiminopropyl)-phénoxy)propylthio)phénylacétique ;
le 3-chloro-4-(3-(2-propyl-3-méthoxy-4-(1-hydroxyiminopropyl)phénoxy)-propylthio)phénylacétate de méthyle ;
l'acide 3-chloro-4-(3-(2-propyl-3-méthoxy-4-(1-hydroxyiminopropyl)-phénoxy)propylthio)phénylacétique ;
le 3-chloro-4-(3-(2-propyl-3-hydroxy-4-(1-hydroxyiminopropyl)phénoxy)-propyloxy)phénylacétate de méthyle ;
le 3-chloro-4-(3-(2-propyl-3-hydroxy-4-(1-méthoxyiminopropyl)phénoxy)-propylthio)phénylacétate de méthyle ;
le 3-chloro-4-(3-(2-propyl-3-hydroxy-4-(1-hydroxyiminobutyl)phénoxy)-propylthio)phénylacétate de méthyle ;
l'acide 3-chloro-4-(3-(2-propyl-3-hydroxy-4-(1-hydroxyiminobutyl)phénoxy)-propylthio)phénylacétique ;
le 3-chloro-4-(3-(2-propyl-3-hydroxy-4-(1-acétoxyiminobutyl)phénoxy)-propylthio)phénylacétate de méthyle ;
le 3-chloro-4-(3-(2-propyl-3-hydroxy-4-(1-hydroxyimino-2-méthylpropyl)-phénoxy)propylthio)phénylacétate de méthyle ;
l'acide 3-chloro-4-(3-(2-propyl-3-hydroxy-4-(1-hydroxyimino-2-méthylpropyl)phénoxy)propylthio)phénylacétique ;
le 3-chloro-4-(3-(2-propyl-3-acétoxy-4-(1-hydroxyiminopropyl)phénoxy)-propylthio)phénylacétate de méthyle ;
le 3-chloro-4-(3-(2-propyl-3-hydroxy-4-(1-oxopropyl)phénoxy)propylthio)-phénylacétamide ;
le 3-chloro-4-(3-(2-cyclopropylméthyl-3-hydroxy-4-propionyl)phénoxy)-propylthio)phénylacétate de méthyle ;
l'acide 3-chloro-4-(3-(2-cyclopropylméthyl-3-hydroxy-4-propionyl)phénoxy)-propylthio)phénylacétique ;
le 3-chloro-4-(3-(2-cyclopropylméthyl-3-hydroxy-4-(1-hydroxyiminopropyl)-phénoxy)propylthio)phénylacétate de méthyle ;
l'acide 3-chloro-4-(3-(2-cyclopropylméthyl-3-hydroxy-4-(1-hydroxyiminopropyl)phénoxy)propylthio)phénylacétique ;
le 3-chloro-4-(4-(2-propyl-3-hydroxy-4-propionylphényl)butylthio)phénylacétate de méthyle ;
l'acide 3-chloro-4-(4-(2-propyl-3-hydroxy-4-propionylphényl)butylthio)-phénylacétique ;
le 3-chloro-4-(4-(2-propyl-3-hydroxy-4-(1-hydroxyiminopropyl)phényl)-butylthio)phénylacétate de méthyle ;
l'acide 3-chloro-4-(4-(2-propyl-3-hydroxy-4-(1-hydroxyiminopropyl)phényl)-butylthio)phénylacétique ;
le 3-chloro-4-(3-(2-(2-propényl)-3-hydroxy-4-propionylphénoxy)propylthio)-phénylacétate de méthyle ;
le 3-chloro-4-(3-(2-(2-propényl)-3-hydroxy-4-(1-hydroxyiminopropyl)-phénoxy)propylthio)phénylacétate de méthyle ;
l'acide 3-chloro-4-(3-(2-(2-propényl)-3-hydroxy-4-(1-hydroxyiminopropyl)-phénoxy)propylthio)phénylacétique ;
le 3-chloro-4-(3-(2-propyl-4-propionylphénoxy)propylthio)phénylacétate de méthyle ;
l'acide 3-chloro-4-(3-(2-propyl-4-propionylphénoxy)propylthio)phénylacétique ;
le 3-chloro-4-(3-(2-propyl-4-(1-hydroxyiminopropyl)phénoxy)propylthio)-phénylacétate de méthyle ;
l'acide 3-chloro-4-(3-(2-propyl-4-(1-hydroxyiminopropyl)phénoxy)-propylthio)phénylacétique ;
le 3-chloro-4-(4-(2-propyl-3-hydroxy-4-acétylphénoxy)butylthio)phénylacétate de méthyle ;
l'acide 3-chloro-4-(4-(2-propyl-3-hydroxy-4-acétylphénoxy)butylthio)phényl-acétique ;
le 3-chloro-4-(3-(2-propyl-3-hydroxy-4-acétylphénoxy)propylthio)phényl-5-méthyl-(1H)-tétrazole ;
le 3-chloro-4-(4-(2-propyl-3-hydroxy-4-acétylphénoxy)butylthio)phényl-5-méthyl-(1H)-tétrazole ;
le 3-chloro-4-(3-(2-propyl-3-hydroxy-4-propionylphénoxy)propylthio)phényl-5-méthyl-(1H)-tétrazole ;
le 3-chloro-4-(3-(2-propyl-3-hydroxy-4-(1-hydroxyiminopropyl)phénoxy)-propylthio)phényl-5-méthyl-(1H)-tétrazole ;
le 3-chloro-4-(4-(2-propyl-3-hydroxy-4-propionylphénoxy)butylthio)phényl-5-méthyl-(1H)-tétrazole ;
le 3-chloro-4-(4-(2-propyl-3-hydroxy-4-(1-hydroxyiminopropyl)phénoxy)-butylthio)phényl-5-méthyl-(1H)-tétrazole ;
le 4-(3-(2-propyl-3-hydroxy-4-acétylphénoxy)propyloxy)phényl-5-méthyl(1H)-tétrazole ;
le 4-(4-(2-propyl-3-hydroxy-4-propionylphénoxy)butyloxy)phényl-5-méthyl-(1H)-tétrazole ;
le 4-(3-(2-propyl-3-hydroxy-4-acétylphénoxy)propy)thio)phényl-5-méthyl-(1H)-tétrazole ;
le 3-chloro-4-(3-(2-propyl-3-hydroxy-4-(1-oxopentyl))phénoxy)propylthio)-phénylacétate de méthyle ;
le 3-chloro-4-(3-(2-propyl-3-hydroxy-4-(1-hydroxyiminopentyl))phénoxy)-propylthio)phénylacétate de méthyle ;
le 3-chloro-4-(3-(2-propyl-3-hydroxy-4-(1-hydroxyiminopropyl))phénoxy)-propylthio)phénylacétaimde ;
l'acide 3-chloro-4-(3-(2-propyl-3-hydroxy-4-(1-(hydroxyimino-3-phényl)-propyl)phénoxy)propylthio)phénylacétique ;
le 3-chloro-4-(4-phénoxy-2-propylphénoxy)propylamino)phénylacétate de méthyle ;
l'acide 3-chloro-4-(4-phénoxy-2-propylphénoxy)propylamino)phénylacétique ;
l'acide 3-chloro-4-(3-(2-propyl-3-hydroxy-4-(1-hydroxyiminopropyl))-phénoxy)propylthiophénylacétique ;
l'acide 4-(2-(4-benzyl-2-propylphénoxy)propylthio)-3-chlorophénylacétique ;
le 3-chloro-4-(3-(2-propyl-3-hydroxy-4-(1-oxopentyl))phénoxy)propyl)-phénylacétate de méthyle ;
l'acide 4-(3-(4-(N-hydroxyiminobenzoyl-2-propylphénoxy)propylthio)-3-chloro-phénylacétique ;
l'acide 4-(3-(4-benzoyl-3-hydroxy-2-propylphénoxy)propylthio)-3-chlorophénylacétique ;
l'acide 4-(3-(4-phénoxy-2-propylphénoxy)propylamino)phényl-(2,2-diméthyl)-acétique ;
l'acide 4-(3-(4-phénoxy-2-propylphénoxy)propylamino)phénylpropan-3-oïque ;
la N-[4-(3-(4-phénoxy-2-propylphénoxy)propylamino)phényl]glycine ;
l'acide 4-(3-(4-phénoxy-2-propylphénoxy)propylamino)phénylacétique ;
l'acide 4-(3-(4-phénoxy-2-propylphénoxy)propylamino)-3-chlorophénylacétique ;
l'acide 4-(3-(4-phénoxy-2-propylphénoxy)propylamino)phénoxyacétique ;
l'acide 4-(3-(4-phénoxy-2-propylphénoxy)propylthio)phénylpropan-3-oïque ;
l'acide 4-(3-(4-phénoxy-2-propylphénoxy)propyloxy)-2-phényl-(2,2-diméthyl)acétique ;
l'acide 4-(4-(4-phénoxy-2-propylphénoxy)butylamino)phénoxy-(2,2-diméthyl)acétique ;
l'acide 4-(3-(4-phénoxy-2-propylphénoxy)propylthio)-3-méthylphénylacétique ;
l'acide 4-(3-(4-phénoxy-2-propylphénoxy)propyloxy)-3-butylphénylacétique ;
l'acide 4-(3-(4-phénoxy-2-propylphénoxy)propylamino)-2-propylphénylacétique ;
l'acide 4-(4-(4-phénoxy-2-propylphénoxy)butylthio)-2-fluorophénylacétique;
l'acide 4-(3-(4-phénoxy-2-propylphénoxy)propyloxy)phénoxy-2-*spiro*-cyclopropylacétique ;
l'acide 3-(3-(4-phénoxy-2-propylphénoxy)propyloxy)phénoxyacétique ;
l'acide 3-(3-(4-phénoxy-2-propylphénoxy)propylamino)phénylpropan-3-oïque ;
l'acide 3-(3-(4-phénoxy-2-propylphénoxy)propylthio)-2-phényl-(2,2-diméthyl)acétique ;
l'acide 3-(4-(4-phénoxy-2-propylphénoxy)butyloxy)phénoxy-(2,2-diméthyl)acétique ;
l'acide 3-(3-(4-phénoxy-2-propylphénoxy)propylthio)-3-méthylphénylacétique ;
l'acide 3-(3-(4-phénoxy-2-propylphénoxy)propylthio)-3-chlorophénylacétique ;
l'acide 4-(3-(4-phénoxy-2-propylphénoxy)propylthio)-3-chlorophénylacétique ;
l'acide 3-(4-(4-phénoxy-2-propylphénoxy)butylamino)-3-butylphénylacétique ;
l'acide 3-(3-(4-phénoxy-2-propylphénoxy)propyloxy)-2-propylphénylacétique ;
l'acide 3-(3-(4-phénoxy-2-propylphénoxy)propylthio)-2-fluorophénylacétique ;
l'acide 3-(4-(4-phénoxy-2-propylphénoxy)butylamino)phénoxy-2-*spiro*-cyclopropylacétique ;
l'acide 4-(3-(4-phénoxy-2-(cyclopropylméthyl)phénoxy)propylamino)-phénylacétique ;
l'acide 4-(3-(4-phénoxy-2-(prop-2-ényl)phénoxy)propylamino)phénylacétique ;
l'acide 4-(3-(4-phénoxy-2-butylphénoxy)propylamino)phénylacétique ;
l'acide 4-(3-(4-phénoxy-2-propoxyphénoxy)propylamino)phénylacétique ;
l'acide 3-(4-(4-phénoxy-2-(cyclopropylméthyl)phénoxy)butyloxy)phénylacétique ;
l'acide 3-(4-(4-phénoxy-2-(prop-2-ényl)phénoxy)butylamino)phénylacétique ;
l'acide 3-(4-(4-phénoxy-2-butylphénoxy)butyloxy)phenylacétique ;
l'acide 3-(4-(4-phénoxy-2-propoxyphénoxy)butyloxy)phénylacétique ;
l'acide 4-(3-(4-phénoxy-3-hydroxy-2-propylphénoxy)propylthio)phénylacétique ;
l'acide 3-(3-(4-phénoxy-3-méthoxy-2-propylphénoxy)propylthio)phénylacétique ;
l'acide 4-(4-(4-phénoxy-3-butyloxy-2-propylphénoxy)butyloxy)phénylacétique ;
l'acide 4-(4-(4-phénoxy-3-chloro-2-propylphénoxy)butyloxy)phénylacétique ;
l'acide 4-(3-(4-benzoyl-2-propylphénoxy)propylthio)phénylacétique ;
l'acide 4-(3-(4-(4-chlorobenzoyl)-2-propylphénoxy)propylthio)phénylacétique ;
l'acide 4-(4-(4-(4-toluyl)benzoyl-2-propylphénoxy)butyloxy)phénylacétique ;
l'acide 3-(4-(4-(4-butylcarbonyl)-2-propylphénoxy)butyloxy)phénylacétique ;
l'acide 4-(3-(4-(N-hydroxyiminoacétyl)-2-propylphénoxy)propylthio)phénylacétique ;
l'acide 3-(3-(4-(2-phényléthylcarbonyl)-2-propylphénoxy)propylthio)phénylacétique ;
l'acide 4-(4-(4-(2-phényléthoxycarbonyl)-2-propylphénoxy)butyloxy)phénylacétique ;
l'acide 4-(4-(4-(4-phénylthiocarbonyl)-2-propylphénoxy)butyloxy)phénylacétique ;
l'acide 3-chloro-4-(3-((1,2-benzisoxazol-3-yl)-2-propylphénoxy)propylthio)-phénylacétique ;
l'acide 3-chloro-4-(3-((1,2-benzisoxazol-3-yl)-2-propylphénoxy)butyloxy)-phénylacétique ;
l'acide 3-(4-(4-benzoyl-2-propylphénoxy)butyloxy)phénylacétique ;
l'acide 3-chloro-4-(3-(4-fluorobenzoyl-2-propylphénoxy)propylthio)phénylacétique ;
l'acide 3-chloro-4-(3-(4-phényl-2-propylphénoxy)propylthio)phénylacétique ;
l'acide 3-chloro-4-(3-(N-éthyl-N-(4-acétyl-3-hydroxy-2-propylphényl)amino)-propylthio)phénylacétique ;
l'acide 3-chloro-4-(3-(N-(4-hydroximinoacétyl-3-hydroxy-2-propylphényl)-amino)propylthio)phénylacétique ;
l'acide 3-chloro-4-(4-phénoxy-(4-trifluorométhyl)propylamino)phénylacétique ;
l'acide 3-chloro-4-(4-phénoxy-2-propyl-(4-(4-fluoro)phénylsulfonyl)-propylthio)phénylacétique ;
l'acide 3-fluoro-4-(4-(4-phénoxy-2-propylphénoxy)butyloxy)phénylacétique ;
l'acide 3-(4-(1-(2-propyl-4-phénéthyl)hydroquinoléyl)butyloxy)phénylacétique ;
l'acide 3-chloro-4-(3-(2-propyl-3-hydroxy-4-(1-acétoxyiminopropyl))-phénoxy)propylthio)phénylacétique ;
l'acide 3-chloro-4-(3-(2-propyl-3-méthoxy-4-méthylaminocarbonylphénoxy)-propylthio)phénylacétique ;
l'acide 3-chloro-4-(3-(4-phénoxy-2-propylphénoxy)propylthio)phénylacétique ;
l'acide 3-chloro-4-((2-propyl-5-phénoxy)phénoxy)propylthio)phénylacétique ;
l'acide 3-chloro-4-(3-(2-propyl-3-benzoylphénoxy)propylthio)phénylacétique ; et
l'acide 4-(3-(2-propyl-3-hydroxy-4-acétylphénoxy)propyloxy)phénoxyacétique.

16. Composé selon la revendication 7, qui est :
le 3-chloro-4-(3-(2-propyl-3-hydroxy-4-propionylphénoxy)propylthio)phénylacétate de méthyle ;
l'acide 3-chloro-4-(3-(2-propyl-3-hydroxy-4-propionylphénoxy)propylthio)-phénylacétique ;
le 3-chloro-4-(3-(2-propyl-3-hydroxy-4-(1-oxobutyl)phénoxy)propylthio)-phénylacétate de méthyle ;
l'acide 3-chloro-4-(3-(2-propyl-3-hydroxy-4-(1-oxobutyl)phénoxy)propylthio)-phénylacétique ;
le 3-chloro-4-(3-(2-propyl-3-hydroxy-4-(1-oxopropyl)phénoxy)propyloxy)-phénylacétate de méthyle ;
l'acide 3-chloro-4-(3-(2-propyl-3-hydroxy-4-(1-hydroxyiminopropyl)-phénoxy)propylthio)phénylacétique ;
le 3-chloro-4-(3-(2-propyl-3-hydroxy-4-(1-hydroxyiminopropyl)phénoxy)-propyloxy)phénylacétate de méthyle ;
le 3-chloro-4-(3-(2-propyl-3-hydroxy-4-(1-hydroxyiminobutyl)phénoxy)-propylthio)phénylacétate de méthyle ;
l'acide 3-chloro-4-(3-(2-propyl-3-hydroxy-4-(1-hydroxyiminobutyl)phénoxy)-propylthio)phénylacétique ;
le 3-chloro-4-(3-(2-propyl-3-hydroxy-4-(1-hydroxyiminopropyl)phénoxy)-propylthio)phénylacétate de méthyle ;
l'acide 3-chloro-4-(3-(2-propyl-3-hydroxy-4-(1-hydroxyimino-2-méthylpropyl)phénoxy)propylthio)phénylacétique ;
le 3-chloro-4-(3-(2-cyclopropylméthyl-3-hydroxy-4-propionylphénoxy)-propylthio)phénylacétate de méthyle ;
l'acide 3-chloro-4-(3-(2-cyclopropylméthyl-3-hydroxy-4-propionylphénoxy)-propylthio)phénylacétique ;
le 3-chloro-4-(4-(2-propyl-3-hydroxy-4-(1-hydroxyiminopropyl)phényl)-butylthio)phénylacétate de méthyle ;
l'acide 3-chloro-4-(4-(2-propyl-3-hydroxy-4-(1-hydroxyiminopropyl)phényl)-butylthio)phénylacétique ;
le 3-chloro-4-(3-(2-propyl-3-hydroxy-4-propionylphénoxy)propylthio)phényl-5-méthyl-(1H)-tétrazole ;
le 3-chloro-4-(3-(2-propyl-3-hydroxy-4-(1-oxopentyl))phénoxy)propylthiophénylacétate de méthyle ;
le 3-chloro-4-(3-(2-propyl-3-hydroxy-4-(1-hydroxyiminopentyl))phénoxy)-propylthiophénylacétate de méthyle ;
le 3-chloro-4-(3-(2-propyl-3-hydroxy-4-(1-hydroxyiminopropyl))phénoxy)-propylthio)phénylacétamide ;
l'acide 3-chloro-4-(3-(2-propyl-3-hydroxy-4-(1-(hydroxyimino-3-phényl)-propyl)phénoxy)propylthio)phénylacétique ;
le 3-chloro-4-(4-phénoxy-2-propylphénoxy)propylamino)phénylacétate de méthyle;
l'acide 3-chloro-4-(4-phénoxy-2-propylphénoxy)propylamino)phénylacétique ;
l'acide 3-chloro-4-(3-(2-propyl-3-hydroxy-4-(1-hydroxyiminopropyl))-phénoxy)propylthio)phénylacétique ;
l'acide 4-(2-(4-benzyl-2-propylphénoxy)propylthio)-3-chlorophénylacétique ;
le 3-chloro-4-(3-(2-propyl-3-hydroxy-4-(1-oxopentyl)phénoxy)propyl)-phénylacétate de méthyle ;
l'acide 4-(3-(4-(N-hydroxyiminobenzoyl)-2-propylphénoxy)propylthio)-3-chlorophénylacétique ;
l'acide 4-(3-(4-benzoyl-3-hydroxy-2-propylphénoxy)propylthio)-3-chlorophénylacétique ;
l'acide 4-(3-(4-phénoxy-2-propylphénoxy)propyloxy)phénylacétique ;
l'acide 4-(3-(4-benzoyl-2-propylphénoxy)propyloxy)phénylacétique ;
l'acide 3-(4-(4-phénoxy-2-propylphénoxy)butyloxy)phénylacétique ;
l'acide 3-(4-(4-benzoyl-2-propylphénoxy)butyloxy)phénylacétique ;
l'acide 4-(3-(4-phénoxy-2-propylphénoxy)propyloxy)phénoxyacétique ;
l'acide 4-(3-(4-benzoyl-2-propylphénoxy)propyloxy)phénoxyacétique ;
l'acide 4-(3-(4-phénoxy-2-propylphénoxy)propylthio)-3-propylphénylacétique ;
l'acide 4-(4-(4-benzoyl-2-propylphénoxy)butylthio)-3-chlorophénylacétique ;
l'acide 4-(3-(4-phénoxy-2-propylphénoxy)propylsulfono)-3-propylphénylacétique ;
l'acide 4-(3-(4-benzoyl-2-propylphénoxy)propylsulfono)-3-chlorophénylacétique ;
l'acide 4-(4-(4-phénoxy-2-propylphénoxy)butylthio)-3-propylbenzyltétrazole ;
l'acide 4-(4-(4-benzoyl-2-propylphénoxy)butylthio)-3-chlorobenzyltétrazole ;
l'acide 4-(3-(4-phénoxy-2-propylphénoxy)propylamino)phényl-acétique ;
l'acide 4-(3-(4-benzoyl-2-propylphénoxy)propylamino)phényl-acétique ;
l'acide 3-(4-(4-(4-fluorophénoxy)-2-propylphénoxy)butyloxy)phénylacétique ;
l'acide 3-(4-(4-(4-fluorobenzoyl)-2-propylphénoxy)butyloxy)phénylacétique ;
l'acide 3-chloro-4-(3-((1,2-benzisoxazol-3-yl)-2-propylphénoxy)propylthio)-phénylacétique ;
l'acide 3-chloro-4-(3-(4-fluorobenzoyl-2-propylphénoxy)propylthio)phénylacétique ; et
l'acide 3-chloro-4-(3-(4-phénoxy-2-propylphénoxy)propylthio)phénylacétique.

17. Composé selon l'une quelconque des revendications 1 à 16, qui a une CI₅₀ inférieure ou égale à 10 µM dans le test de fixation de hPPARδ et une CE₅₀ inférieure ou égale à 10 µM dans le test de transactivation de hPPARδ.

18. Composé selon la revendication 17, qui a une CI₅₀ inférieure ou égale à 100 nM dans le test de fixation de hPPARδ et une CE₅₀ inférieure ou égale à 100 nM dans le test de transactivation de hPPARδ.

19. Composé selon la revendication 18, qui a une CI₅₀ inférieure ou égale à 50 nM dans le test de fixation de hPPARδ et une CE₅₀ inférieure ou égale à 50 nM dans le test de transactivation de hPPARδ.

20. Composé selon la revendication 19, qui a une CI₅₀ inférieure ou égale à 10 nM dans le test de fixation de hPPARδ et une CE₅₀ inférieure ou égale à 10 nM dans le test de transactivation de hPPARδ.

21. Composition pharmaceutique comprenant un composé selon l'une quelconque des revendications 1 à 20 ou un sel pharmaceutiquement acceptable de celui-ci et un support inerte.

22. Composé selon l'une quelconque des revendications 1 à 20 ou un sel pharmaceutiquement acceptable de celui-ci, pour son utilisation dans une méthode de traitement de l'organisme humain ou animal.

23. Utilisation d'un composé selon l'une quelconque des revendications 1 à 20, ou d'un sel pharmaceutiquement acceptable de celui-ci, pour la fabrication d'un médicament pour le traitement ou la prévention du diabète, pour la diminution des taux de triglycérides, pour le traitement de l'obésité, pour l'arrêt, la prévention ou la réduction du risque de développement d'une athérosclérose et d'événements pathologiques reliés, ou pour augmenter les taux plasmatiques des lipoprotéines de haute densité.

24. Combinaison d'un composé selon l'une quelconque des revendications 1 à 20, ou d'un sel pharmaceutiquement acceptable de celui-ci, et d'une sulfonylurée, d'un fibrate, d'un inhibiteur de l'HMG-CoA réductase, d'un inhibiteur de béta-sitostérol, d'un inhibiteur de la cholestérolacyltransférase, d'un biguanide, de la cholestyramine, d'un antagoniste de l'angiotensine II, du mélinamide, de l'acide nicotinique, d'un antagoniste du récepteur du fibrinogène, de l'aspirine, d'un inhibiteur de l'α-glucosidase, d'un sécrétogogue de l'insuline ou de l'insuline pour une administration séparée, simultanée ou séquentielle.

25. Combinaison d'un composé selon l'une quelconque des revendications 1 à 20, ou d'un sel pharmaceutiquement acceptable de celui-ci, et de fenfluramine, dexfenfluramine, phentiramine ou d'un agoniste du récepteur β₃-adrénergique pour une administration séparée, simultanée ou séquentielle.
